# EUROPEAN PATENT APPLICATION

(11) **EP 2 546 232 A1**
(43) Date of publication of application: **16.01.2013**
(21) Application number: 12188238.5
(22) Date of filing: 16.06.2008
(51) Int. Cl.: C07D 209/30, C07D 213/65, C07D 237/20, C07D 401/10, C07D 401/12, C07D 403/04, C07D 403/10, C07D 413/10, C07D 413/12, C07D 417/12, C07D 471/04, C07D 487/04, C07D 513/04, A61K 31/506, A61P 9/00, A61P 11/02, A61P 11/06

(54) **Diphenyl Substituted Alkanes**

(30) Priority: 20.06.2007 US 936630 P
(62) Divisional of application: 08768495.7
(71) Applicant: Merck Sharp & Dohme Corp., Rahway, NJ 07065 (US)
(72) Inventor: Ogawa, Anthony, Rahway, NJ New Jersey 07065-0907 (US); Ujjainwalla, Feroze, Rahway, NJ New Jersey 07065-0907 (US); Vande Bunte, Ellen, K., Rahway, NJ New Jersey 07065-0907 (US); Chu, Lin, Rahway, NJ New Jersey 07065-0907 (US); Ondeyka, Debra, Rahway, NJ New Jersey 07065-0907 (US); Kopka, Ihor, E., Rahway, NJ New Jersey 07065-0907 (US); Li, Bing, Rahway, NJ New Jersey 07065-0907 (US); Ok, Hyun, O., Rahway, NJ New Jersey 07065-0907 (US); Patel, Minal, Rahway, NJ New Jersey 07065-0907 (US); Sisco, Rosemary, Rahway, NJ New Jersey 07065-0907 (US)
(74) Representative: Horgan, James Michael Frederic

(57) **Abstract**

The instant invention provides compounds of Formula I which are 5-lipoxygenase activating protein inhibitors: 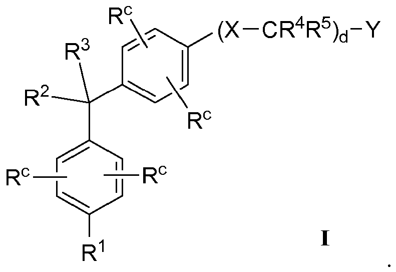 Compounds of Formula I are useful as anti-atherosclerotic, anti-asthmatic, anti-allergic, anti-inflammatory and cytoprotective agents.

## Description

### FIELD OF THE INVENTION

The instant invention involves compounds that inhibit 5-lipoxygenase activating protein (FLAP), compositions containing such compounds and methods of treatment using such compounds for the treatment and prevention of atherosclerosis and related diseases and conditions.

### BACKGROUND OF THE INVENTION

Inhibition of leukotriene biosynthesis has been an active area of pharmaceutical research for many years. Leukotrienes are potent contractile and inflammatory mediators derived through the oxygenation of arachidonic acid by 5-lipoxygenase.

One class of leukotriene biosynthesis inhibitors are those known to act through inhibition of 5-lipoxygenase (5-LO). In general, 5-LO inhibitors have been sought for the treatment of allergic rhinitis, asthma and inflammatory conditions including arthritis. One example of a 5-LO inhibitor is the marketed drug zileuton, which is indicated for the treatment of asthma. More recently, it has been reported that 5-LO may be an important contributor to the atherogenic process; see Mehrabian, M. et al., Circulation Research, 2002 Jul 26, 91(2):120-126.

A new class of leukotriene biosynthesis inhibitors (now known as FLAP inhibitors) distinct from 5-LO inhibitors is described in Miller, D.K. et al., "Identification and isolation of a membrane protein necessary for leukotriene production," Nature, vol. 343, No. 6255, pp. 278-281 (18 Jan 1990). See also Dixon, R.A. et al, "Requirement of a 5-lipoxygenase-activating protein for leukotriene synthesis," Nature, vol 343, no. 6255, pp. 282-4 (18 Jan 1990). 5-LO inhibitor compounds were used to identify and isolate the inner nuclear membrane 18,000 dalton protein 5-lipoxygenase-activating protein (FLAP). These compounds inhibit the formation of cellular leukotrienes but have no direct effect on soluble 5-LO activity. In cells, arachidonic acid is released from membrane phospholipids by the action of cytosolic phospholipase 2. This arachidonic acid is transferred to nuclear membrane bound 5-lipoxygenase by FLAP. The presence of FLAP in cells is essential for the synthesis of leukotrienes. Additionally, based on studies described in Helgadottir, A., et al., Nature Genetics, vol 36, no. 3 (March 2004) pp. 233-239, it is believed that the gene encoding 5-lipoxygenase activating protein confers risk for myocardial infarction and stroke in humans.

Despite significant therapeutic advances in the treatment and prevention of atherosclerosis and ensuing atherosclerotic disease events, such as the improvements that have been achieved with HMG-CoA reductase inhibitors, further treatment options are clearly needed. The instant invention addresses that need by providing compounds, compositions and methods for the treatment or prevention of atherosclerosis as well as related conditions.

### SUMMARY OF THE INVENTION

The instant invention relates to compounds of Formula I which are FLAP inhibitors, methods for their preparation, and methods and pharmaceutical formulations for using these compounds in mammals, especially humans. This invention provides compounds of structural Formula I: 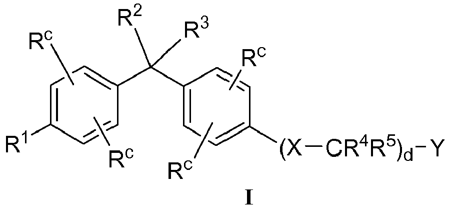 and the pharmaceutically acceptable salts thereof. This invention also involves the use of compounds described herein to slow or halt atherogenesis. Therefore, one object of the instant invention is to provide a method for treating atherosclerosis, which includes halting or slowing the progression of atherosclerotic disease once it has become clinically evident, comprising administering a therapeutically effective amount of a compound of Formula I to a patient in need of such treatment. Another object is to provide methods for preventing or reducing the risk of developing atherosclerosis and atherosclerotic disease events, comprising administering a prophylactically effective amount of a compound of Formula I to a patient who is at risk of developing atherosclerosis or having an atherosclerotic disease event.

The compounds of Formula I are also useful as anti-asthmatic, anti-allergic, anti-inflammatory and cytoprotective agents. They are also useful in treating angina, cerebral spasm, glomerular nephritis, hepatitis, endotoxemia, uveitis, and allograft rejection. The instant invention provides methods of treatment comprising administering a therapeutically effective amount of a compound of Formula I to a patient in need of the above-described treatments.

A further object is to provide the use of FLAP inhibitors of Formula I in combination with other therapeutically effective agents, including other anti-atherosclerotic drugs. These and other objects will be evident from the description contained herein.

### DETAILED DESCRIPTION OF THE INVENTION

The instant invention provides FLAP inhibitor compounds having structural Formula I: 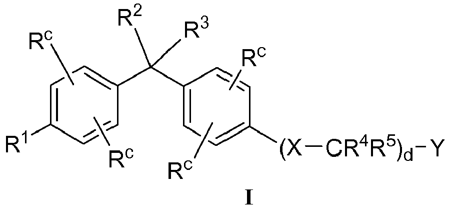 and the pharmaceutically acceptable salts thereof wherein:
**R¹** is selected from the group consisting of:
   (a) a 5-membered aromatic or partially unsaturated heterocyclic ring containing 2 to 4 heteroatoms selected from N, S and O, wherein the heterocyclic ring is optionally substituted with (i) R⁶, or (ii) oxo and R⁶ provided R⁶ is not oxo;
   (b) a 6-membered aromatic or partially unsaturated heterocyclic ring containing 1 to 2 heteroatoms selected from N and O, wherein the heterocyclic ring is optionally substituted with (i) R⁶, (ii) oxo and R⁶ provided R⁶ is not oxo, or (iii) methyl and R⁶;
   (c) an 8-membered aromatic or partially unsaturated ortho-fused bicyclic ring system containing 3-5 heteroatoms selected from one sulfur and 2-4 of nitrogen wherein one carbon in the ring is optionally substituted with a group selected from =O, =S, -SMe, -NH₂,
      - CF₃, -Cl, -C₁₋₄alkyl optionally substituted with 1-3 of fluoro or a group selected from -NH₂, - OH, -OC₁₋₄alkyl, and -CN;
   (d) a 9-membered aromatic or partially unsaturated ortho-fused bicyclic ring system containing 3-4 nitrogen atoms, wherein one carbon in the ring is optionally substituted with a group selected from =O, =S, -SMe, -NH₂, -CF₃, -Cl, pyrrolidinyl, tetrahydrofuranyl, -C₁₋₄alkyl optionally substituted with 1-3 of fluoro or a group selected from -NH₂, - OH, -OC₁₋₄alkyl, -CN, and R¹⁰;
   (e) -C₁₋₆alkyl, -C₂₋₆alkenyl, and -C₂₋₆alkynyl, said alkyl, alkenyl and alkynyl groups being optionally substituted with R¹² and optionally substituted with R¹³;
   (f) -C₃₋₆cycloalkyl optionally substituted with 1-3 substituents selected from the group consisting of fluoro, -NH₂, -OH and -C₁₋₃alkyl optionally substituted with 1-3 of fluoro;
   (g) -O-R6a; and
   (h) -H, -OH, -CN, -CO₂R⁹, -C(O)NR⁷R⁸, -NR⁷R⁸, -NRbSOₚR^{a}, -NR^{b}C(O)R^{a}, -NR^{b}C(O)NR^{a}R^{b}, -S(O)ₚR^{a}, and -S(O)ₚNR^{a}R^{b};
**p** is an integer selected from 0, 1 and 2;
**R²** is selected from the group consisting of (a) -C₁₋₆alkyl optionally substituted with 1-5 of fluoro or hydroxy, (b) -C₃₋₆cycloalkyl, and (c) 1-methyl-C₃₋₆cycloalkyl;
**R³** is selected from the group consisting of -H, -F, -OH, and -C₁₋₃alkyl optionally substituted with 1-5 fluoro (including for example -CF₃);
**X** is selected from the group consisting of -O-, -S- and -CR^{2a} R^{3a}-;
**R^{2a}** is selected from the group consisting of -H, -OH, -OC₁₋₆alkyl optionally substituted with 1-3 of fluoro, -O-benzyl and -fluoro;
**R^{3a}** is selected from the group consisting of -H, fluoro and -C₁₆alkyl;
   or **CR^{2a}R^{3a}** represents carbonyl;
**R⁴** is selected from the group consisting of -H, -C₁₋₆alkyl optionally substituted with 1-3 of fluoro, and -C₃₋₆cycloalkyl optionally substituted with 1-3 of fluoro;
**R⁵** is selected from the group consisting of -H, fluoro and methyl;
or **R⁴** and **R⁵** taken together with the carbon to which they are attached represents a -C₃₋₆cycloalkyl ring, for example cyclopropyl-1,1-diyl;
**d** is an integer selected from 0 (zero) and 1 (one);
**R⁶** is selected from the group consisting of (a) -C₁₋₆alkyl optionally substituted with one or more substituents selected from the group consisting of -OH, -NH₂, -N(CH₃)₂, -NH(C=O)O^{t}Bu, -CN, -O-C₁₋₄alkyl and fluoro (for example, 1-3 of fluoro), (b) -C₁₋₆alkyl-R¹⁰, (c) -OC₁₋₆alkyl optionally substituted with one or more substituents selected from the group consisting of -OH, -NH₂ and fluoro, (d) -C₃₋₆ cycloalkyl optionally substituted with one or more substituents selected from the group consisting of methyl, -OH, -NH₂, -NH(C=O)O^{t}Bu, -CF₃ and fluoro, (e) -NR⁷R⁸, (f) -SO₂C₁₋₃alkyl, (g) -(CH₂)₀₋₃CO₂-R⁸, (h) -OH, (i) =O (oxo), (j) -SH, (k) =S, (1) -SMe, (m) -Cl, (n) 1-5 of fluoro, (o) -CF₃, (p) -CN and (q) R¹⁰;
**R^{6a}** is selected from the group consisting of (1) -C₁₋₆alkyl optionally substituted with R¹² and optionally substituted with R¹³, (2) -C₃₋₆cycloalkyl optionally substituted with R¹² and optionally substituted with R¹³ and (3) -C₂₋₆alkyl-R¹⁰;
**R⁷** is selected from the group consisting of (a) -H, (b) -C₁₋₆alkyl optionally substituted with one or more substituents selected from the group consisting of -F, -NH₂ and -OH, (c) -C₃₋₆cycloalkyl optionally substituted with one or more substituents selected from the group consisting of methyl, -CF₃, -F, -NH₂ and -OH, (d) -COC₁₋₆alkyl optionally substituted with one or more substituents selected from the group consisting of -F and -OH, (e) -C(O)OC₁₋₆alkyl optionally substituted with one or more substituents selected from the group consisting of methyl, phenyl, -CF₃, -F and -OH, (f) a 4-6 membered saturated heterocyclic ring containing one N and/or one O, wherein the ring is bonded to the nitrogen in -NR⁷R⁸ through a carbon atom in the ring, and wherein the ring is optionally substituted with one or more substituents selected from the group consisting of methyl, -CF₃, -F, -CH₂CH₂F, -CH2CHF2, -CH₂CF₃, -NH₂ and -OH, and wherein the ring is optionally bridged by a -CH₂CH₂- group, (g) a 5-membered unsaturated heterocyclic ring comprising one, two or three heteroatoms selected from N, O and S, and (h) benzyl;
**R⁸** is selected from the group consisting of (a) -H, (b) -C₁₋₆alkyl optionally substituted with one or more substituents selected from the group consisting of -F, -NH₂ and -OH, and (c) -C₃₋₆cycloalkyl optionally substituted with one or more substituents selected from the group consisting of methyl, -CF₃, -F, -NH₂ and -OH;
or **R⁷** and **R⁸** together represent - (CH₂)₃₋₅- which is bonded with the nitrogen to which **R⁷** and **R⁸** are attached to form a 4-6 membered ring, wherein the ring is optionally substituted with a substituent selected from the group consisting of -CH₃, -CF₃, -F and -OH;
**R⁹** is selected from the group consisting of -H, -C₁₋₆alkyl and -C₃₋₆cycloalkyl;
**R¹⁰** is a heterocyclic ring selected from the group consisting of (a) azetidinyl optionally substituted with one or more of methyl, -F and -OH, (b) pyrrolidinyl optionally substituted with one or more of methyl, -F and -OH, (c) piperidinyl optionally substituted with one or more of methyl, -F and -OH, (d) piperazinyl optionally substituted with =O, and (e) morpholinyl optionally substituted with one or more of methyl and -F;
**Y** is selected from the group consisting of (a) a 5-membered aromatic or partially unsaturated heterocyclic ring containing 1 to 4 heteroatoms selected from 1 to 4 ofN and zero to 1 of S, wherein the heterocyclic ring is optionally substituted with R¹¹, (b) a 6-membered aromatic or partially unsaturated heterocyclic ring containing 1 to 2 N heteroatoms, wherein the heterocyclic ring is optionally substituted with R¹¹, (c) a 9-membered bicyclic aromatic or partially unsaturated heterocyclic ring containing 1 to 4 N heteroatoms, wherein the heterocyclic ring is optionally substituted with R¹ and (d) a 10-membered bicyclic aromatic or partially unsaturated heterocyclic ring containing 1 to 4 N heteroatoms, wherein the heterocyclic ring is optionally substituted with R¹¹;
**R¹¹** is selected from the group consisting of -F, -NH₂, -OH, -OC₃₋₄cycloalkyl, -C₁₋₃alkyl optionally substituted with 1-3 fluoro, and -OC₁₋₃alkyl optionally substituted with phenyl or 1-3 fluoro;
**R¹²** is selected from the group consisting of -CO₂R⁹, - C(O)NR⁷R⁸, -N(R^{a})₂, -NRbSOₚR^{a}, -NR^{b}C(O)R^{a}, -NR^{b}C(O)NR^{a}R^{b}, -S(O)ₚNR^{a}R^{b}, -S(O)ₚR^{a}, -F, -CF₃, phenyl, Het and Z¹;
**R¹³** is selected from the group consisting of -OH, -NH₂ and 1-5 of -F;
each **R^{a}** is independently selected from the group consisting of (a) -H, (b) -C₁₋₆alkyl, -C₂₋₆alkenyl and -C₂₋₆alkynyl, wherein each is optionally substituted with 1-3 of fluoro, and optionally substituted with 1-2 substituents selected from the group consisting of: -OH, - OC₁₋₄alkyl, cyano, -NH₂, -NHC₁₋₄alkyl, and -N(C₁₋₄alkyl)₂, (c) -C₃₋₆cycloalkyl, optionally substituted with 1-3 of fluoro, and optionally substituted with 1-2 substituents selected from the group consisting of: -C₁₋₄alkyl, -OH, -OC₁₋₄alkyl, -CN, -NH₂, -NHC₁₋₄alkyl, -N(C₁₋₄alkyl)₂, and -CF₃, (d) Het and Het-C₁₋₄alkylene-, the Het moieties being optionally substituted on carbon with 1-2 substituents selected from the group consisting of -F, -OH, -CO₂H, -C₁₋₄alkyl, -CO₂C₁₋₄alkyl, -OC₁₋₄alkyl, -NH₂, -NHC₁₋₄alkyl, -N(C₁₋₄alkyl)₂, -NHC(O)C₁₋₄alkyl, oxo, -C(O)NHC₁₋₄alkyl and -C(O)N(C₄alkyl)₂; and optionally substituted on nitrogen when present with a group selected from -C₁₋₄alkyl and -C₁₋₄acyl; and the alkylene portion of Het-C₁₋₄alkylene- being optionally substituted with 1-3 of fluoro and optionally substituted with a member selected from the group consisting of -OH, -CN, -OC₁₋₄alkyl, -NH₂, -NHC₁₋₄alkyl, and -N(C₁₋₄alkyl)₂, (e) Z² and Z²-C₁₋₄alkylene-, the alkylene portion of Z²-C₁₋₄alkylene- being optionally substituted with 1-3 of fluoro and optionally substituted with a member selected from the group consisting of -OH, -CN, -OC₁₋₄alkyl, -NH₂, -NHC₁₋₄alkyl, and -N(C₁₋₄alkyl)₂;
each **R^{b}** is independently selected from the group consisting of -H and -C₁₋₃alkyl optionally substituted with 1-2 members selected from the group consisting of -NH₂, -OH, -F, -CN and -CF₃;
each **R^{c}** is independently selected from the group consisting of -H, -F, -Cl, -OH, -CN, -C₁₋₄alkyl optionally substituted with 1-3 of fluoro, and -OC₁₋₄alkyl optionally substituted with 1-3 of fluoro;
**Het** is selected from the group consisting of azetidinyl, pyrrolidinyl, piperidinyl, piperazinyl, morpholinyl, tetraydrofuranyl and β-lactamyl, δ- lactamyl, γ-lactamyl and tetrahydropyranyl;
**Z¹** is selected from the group consisting of: (a) Z², (b) an 8-membered aromatic or partially unsaturated ortho-fused bicyclic ring system containing 3-5 heteroatoms selected from one sulfur and 2-4 of nitrogen wherein one carbon in the ring is optionally substituted with a group selected from =O, =S, -SMe, -NH₂, -CF₃, -Cl, -C₁₋₄alkyl optionally substituted with 1-3 of fluoro and optionally substituted with a group selected from -NH₂, -OH, -OC₁₋₄alkyl, and - CN, and (c) a 9-membered aromatic or partially unsaturated ortho-fused bicyclic ring system containing 3-4 nitrogen atoms, wherein one carbon in the ring is optionally substituted with a group selected from =O, =S, -SMe, -NH₂, -CF₃, -Cl, -C₁₋₄alkyl optionally substituted with 1-3 of fluor and optionally substituted with a group selected from -NH₂, -OH, -OC₁₋₄alkyl, and -CN;
and **Z²** is selected from the group consisting of: (a) a 5-membered aromatic or partially unsaturated heterocyclic ring containing 2-4 nitrogen atoms, wherein one nitrogen in the ring is optionally substituted with a group selected from -C₁₋₄alkyl optionally substituted with 1-3 of fluoro and optionally substituted with a group selected from -NH₂, -OH, and -CN, and one carbon in the ring is optionally substituted with a group selected from =O, =S, -SMe, -NH₂, - CF₃, -Cl, -C₁₋₄alkyl optionally substituted with 1-3 of fluoro and optionally substituted with a group selected from -NH₂, -OH, -OC₁₋₄alkyl, and -CN, and -OC₁₋₄alkyl optionally substituted with -OH or 1-3 of fluoro; (b) a 5-membered aromatic or partially unsaturated heterocyclic ring containing 2-3 heteroatoms selected from one oxygen or one sulfur and 1-2 of nitrogen, wherein one nitrogen in the ring is optionally substituted with a group selected from C₁₋₄alkyl optionally substituted with 1-3 of fluoro and optionally substituted with a group selected from -NH₂, -OH, and -CN, and one carbon in the ring is optionally substituted with a group selected from =O, =S, - SMe, -NH₂, -CF₃, -Cl, C₁₋₄alkyl optionally substituted with 1-3 of fluoro and optionally substituted with a group selected from -NH₂, -OH, -OC₁₋₄alkyl, and -CN, and -OC₁₋₄alkyl optionally substituted with -OH or 1-3 of fluoro; and (c) a 6-membered aromatic or partially unsaturated heterocyclic ring containing 1-2 nitrogen atoms, wherein one nitrogen in the ring is optionally substituted with a group selected from -C₁₋₄alkyl optionally substituted with 1-3 of fluoro and optionally substituted with a group selected from -NH₂, -OH, and -CN, and one carbon in the ring is optionally substituted with a group selected from =O, =S, -SMe, -NH₂, - CF₃, -Cl, -C₁₋₄alkyl optionally substituted with 1-3 of fluoro and optionally substituted with a group selected from -NH₂, -OH, -OC₁₋₄alkyl, and -CN, and -OC₁₋₄alkyl optionally substituted with -OH or 1-3 of fluoro;
   provided that when -(X-CR⁴R⁵)_{d}-Y is 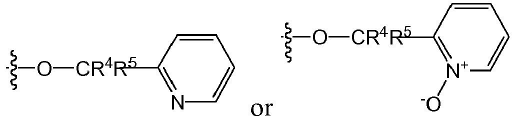 and R⁴ is -H or -C₁₋₄alkyl and R⁵ is -H or methyl, then R¹ is not -CO₂R⁹, OH, CN, 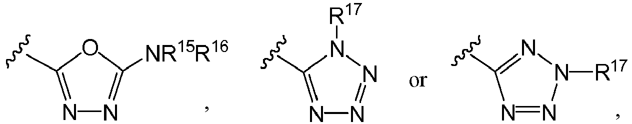 wherein
**R¹⁵** is selected from the group consisting of -H, -C₁₋₆alkyl optionally substituted with 1 to 3 fluoro, -C₃₋₆cycloalkyl optionally substituted with 1-3 fluoro, -COC₁₋₆alkyl and -COC₃₋₆cycloalkyl;
**R¹⁶** is selected from the group consisting of -H, -C₁₋₆alkyl optionally substituted with 1 to 3 fluoro, and -C₃₋₆cycloalkyl optionally substituted with 1 to 3 fluoro;
**R¹⁷** is selected from the group consisting of -H, -C₁₋₆alkyl optionally substituted with 1 to 3 fluoro, -C₃₋₆cycloalkyl optionally substituted with 1 to 3 fluoro and -CH₂-R¹⁸;
**R¹⁸** is selected from the group consisting of pyrrolidinyl optionally substituted on nitrogen with methyl, piperidinyl optionally substituted on nitrogen with methyl, and morpholinyl optionally substituted on nitrogen with methyl.

In an embodiment of this inventions are compounds of Formulas I and Ia wherein X is O, and compounds of Formula Ia-1, wherein Y is unsubstituted or substituted pyridinyl, and R¹ is not -CO₂R⁹, OH, CN, oxadiazole, 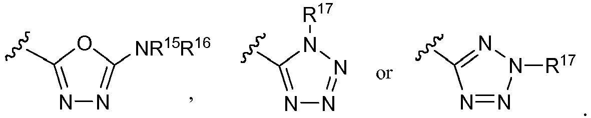 In one subset of this embodiment R¹ is selected from 1- or 2-carboxymethyl-5-tetrazolyl, optionally substituted 2,3-dihydro-3-methyl-2-oxo-1,3,4-oxadiazol-5-yl, optionally substituted 3-pyrazolyl, -C(O)NHR⁸, optionally substituted 1,3,4-thiadiazolyl, optionally substituted 1,2,4-oxadiazol-3-yl, optionally substituted 4,5-dihydro-5-oxo-1,2,4-oxadiazol-3-yl, optionally substituted 1,2,4-triazol-3-yl, optionallyl substituted 1,2,4-triazol-4-yl (including 2,3-dihydro-3-oxo-1,2,4-triazol-4-yl), optionally substituted 1,2,3-triazol-4-yl, optionally substituted 1-tetrazolyl, optionally substituted 1,2-oxazol-5-yl, optionally substituted 1,3-oxazol-2-yl, optionally substituted 2-, 3- and 4-pyridyl (including dihydropyridone), optionally substituted 2-, 4- and 5-pyrimidinyl, optionally substituted 3- and 4-pyridazinyl, optionally substituted pyrazinyl, optionally substituted 4-imidazolyl, optionally substituted 8-membered aromatic or partially unsaturated ortho-fused bicyclic ring system containing 3-5 heteroatoms selected from one sulfur and 2-4 of nitrogen, and optionally substituted 9-membered aromatic or partially unsaturated ortho-fused bicyclic ring system containing 3-4 nitrogen atoms.

In another embodiment of the present invention are compounds of Formula I having structural Formula Ia: 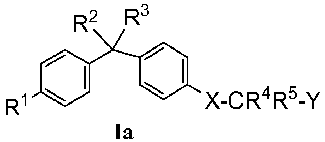 and pharmaceutically acceptable salts thereof, wherein the variables, e. g. R¹, R², R³, R⁴, R⁵, X, Y, etc., are as defined in relation to Formula I.

In another embodiment of the present invention are compounds of Formula I and Ia having structural Formula Ia-1: 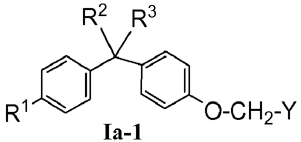 and pharmaceutically acceptable salts thereof, wherein the variables, e. g. R¹, R², R³, Y, etc., are as defined in relation to Formula I. In one subset of this embodiment Y is selected from optionally substituted 2-pyrimidinyl, optionally substituted 2-thiazolyl, 3-F- and 5-F-2-pyridyl. Within this subset there is a group wherein R¹ is (a) a 5-membered aromatic or partially unsaturated heterocyclic ring containing 2 to 4 heteroatoms selected from N, S and O, wherein the heterocyclic ring is optionally substituted with (i) R⁶, or (ii) oxo and R⁶ provided R⁶ is not oxo; (b) a 6-membered aromatic or partially unsaturated heterocyclic ring containing 1 to 2 heteroatoms selected from N and O, wherein the heterocyclic ring is optionally substituted with (i) R6, (ii) oxo and R⁶ provided R⁶ is not oxo, or (iii) methyl and R⁶; (c) an 8-membered aromatic or partially unsaturated ortho-fused bicyclic ring system containing 3-5 heteroatoms selected from one sulfur and 2-4 of nitrogen wherein one carbon in the ring is optionally substituted with a group selected from =O, =S, -SMe, -NH₂, -CF₃, -Cl, -C₁₋₄alkyl optionally substituted with 1-3 of fluoro or a group selected from -NH₂, -OH, -OC₁₋₄alkyl, and -CN; and (d) a 9-membered aromatic or partially unsaturated ortho-fused bicyclic ring system containing 3-4 nitrogen atoms, wherein one carbon in the ring is optionally substituted with a group selected from =O, =S, -SMe, -NH₂, -CF₃, -Cl, pyrrolidinyl, tetrahydrofuranyl, -C₁₋₄alkyl optionally substituted with 1-3 of fluoro or a group selected from -NH₂, -OH, -OC₁₋₄alkyl, -CN, and R¹⁰.

In another embodiment of the present invention are compounds of Formula I and Ia having structural Formula Ib: 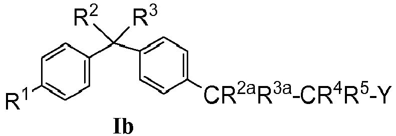 and pharmaceutically acceptable salts thereof wherein the variables, e. g. R¹, R², R³, R^{2a}, R^{3a}, Y, etc., are as defined in relation to Formula I.

In another embodiment of the present invention are compounds of Formula I having structural Formula Ic: 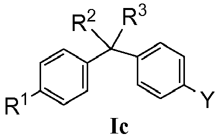 and pharmaceutically acceptable salts thereof, wherein the variables, e. g. R¹, R², R³, Y, etc., are as defined in relation to Formula I. In one subset Y is selected from optionally substituted pyridyl, optionally substituted pyrimidinyl and optionally substituted pyridazinyl. Within this subset is a group in which Y is 3-pyridyl, 5-methoxy-3-pyridyl, 6-amino-3-pyridazinyl and 2-amino-5-pyrimidinyl.

In another embodiment of the present invention are compounds of Formula I wherein R^{c} is selected from hydrogen, -F, -Cl, -OH or -C₁₋₆alkyl. Preferably, R^{c} is hydrogen or -C₁₋₆alkyl. Most preferably, R^{c} is hydrogen.

In another embodiment of the present invention are compounds of Formulas I, Ia, Ia-1 or I-b wherein Y is not pyridinyl. In another embodiment are compounds of Formulas I, Ia, Ia-1 or I-b wherein R¹ is not oxadiazolyl or tetrazolyl.

In another embodiment of the present invention are compound of Formulas I, Ia, Ia-1, Ib and Ic wherein Y is selected from the group consisting of (a) a 5-membered aromatic heterocyclic ring containing 1 to 2 heteroatoms selected from 1 to 2 of N and zero to 1 of S, wherein the heterocyclic ring is optionally substituted with R¹ as hereinbefore defined, and (b) a 6-membered heterocyclic ring, containing 1 to 2 N heteroatoms, wherein the heterocyclic ring is optionally substituted with R¹¹.

Preferably, for compounds of Formula I wherein d is 1, and Formulas Ia, Ia-1 and Ib, Y is selected from thiazolyl, pyridinyl, pyridazinyl and pyrimidinyl, optionally substituted with - F, -OCH₃ and -NH₂. Examples of suitable Y groups include 1,3-thiazol-2-yl, 1,3-thiazol-4-yl, 2-pyridyl, 2-pyrimdinyl, 3-fluoro-2-pyridyl, 5-fluoro-2-pyridyl, and pyridyl-N-oxide .

In another embodiment of the invention are compounds of (1) Formula I wherein d is 0 and (2) Formula Ic, wherein Y is a 6-membered heterocyclic ring, containing 1 to 2 N heteroatoms, wherein the heterocyclic ring is optionally substituted with R¹¹. Examples of such susbtituted Y groups include: 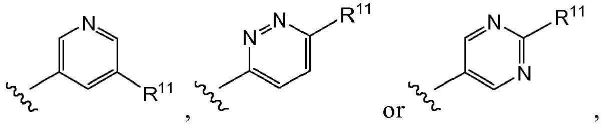 and more particularly 3-methoxy-5-pyridyl, 3-amino-6-pyridazinyl, or 2-amino-5-pyrimidinyl.

In another embodiment of the present invention are compounds of Formulas I and Ia wherein X is -O-.

In another embodiment of the present invention are compounds of Formulas I and Ia wherein X is -CR^{2a} R^{3a}, and more particularly it is -CH₂-, -CHF-, -CH(OH)-, -C(O)-, -CH(OC₁₋₃alkyl)-, -CH(O-benzyl)- or -CH(OCF₃)-.

In another embodiment of the present invention are compounds of Formulas I, Ia, and Ib wherein -CR⁴R⁵- is -CH₂-, -CH(CH₃)- or -CH(CH₂CH₃)-. Examples of suitable - X-CR⁴R⁵- moieties include but are not limited to: ethylene, -OCH₂-, -OCH(CH₃)-, - OCH(CH₂CH₃)-, -C(O)CH(CH₂CH₃)-, -CH(F)CH₂-, -CH(OH)CH₂-, -CH(OCH3)CH₂-, - CH(OCH₂CH₃)CH₂-, -CH(OCF₃)CH₂-, and -CH(OCH₂Ph)CH₂-.

In another embodiment of the present invention are compounds of Formula I wherein d is zero.

In another embodiment of the present invention are compound of Formulas I, Ia, Ia-1, Ib and Ic wherein R² is -C₁₋₄alkyl optionally substituted with one to three fluorine atoms, -C₃₋₆cycloalkyl or 1-methyl-C₃₋₄cycloalkyl. Preferably, R² is -C₃₋₄alkyl, -C₃₋₄cycloalkyl or 1-methyl-C₃₋₄cycloalkyl. More preferably, R² is propyl or butyl. Most preferably, R² is ⁱpropyl or ^{t}butyl. Examples of suitable R² groups include methyl, ethyl, n-propyl, isopropyl, tert-butyl, cyclopropyl, cyclobutyl, cyclopentyl, cyclohexyl, -C(CH₃)₂CHF₂, -C(CH₃)₂CF₃, 1-methylcyclopropyl, and 1-methylcyclobutyl.

In another embodiment of the present invention are compound of Formulas I, Ia, Ia-1, Ib and Ic wherein R³ is -H, -C₁₋₃alkyl or -OH. Preferably, R³ is -H, -OH or methyl. More preferably, R³ is -H or methyl, and most preferably, R³ is methyl when R² is ⁱpropyl and R³ is -H when R² is ^{t}butyl.

In another embodiment of the present invention are compounds of Formulas I, Ia, Ia-1, Ib, and Ic wherein R¹ is a 5-membered aromatic or partially unsaturated heterocyclic ring containing 2 to 4 heteroatoms selected from N, S and O, wherein the heterocyclic ring is optionally substituted with (i) R⁶, or (ii) oxo and R⁶ provided R⁶ is not oxo. Examples of suitable R¹ groups that can be optionally substituted as described include: 1H-pyrazol-3-yl and -5-yl, 1H-imidazol-4-yl and -5-yl, 1H-imidazol-2-yl, 2H-1,2,3-triazol-4-yl, 1H-1,2,3-triazol-4-yl and -5-yl, 4H-1,2,4-triazol-4-yl and -3-yl, 1H-1,2,4-triazol-3-yl and -5-yl, 1H- and 2H-tetrazol-5-yl, 1H-tetrazol-1-yl, 2,3-dihydro-2-oxo-1,3,4-oxadiazol-5-yl, 1,2-oxazol-2-yl, 1,2-oxadiazol-5-yl, 1,3-oxazol-5-yl, 1,3-oxazol-4-yl, 1,2-oxazol-3-yl, 1,3-thiazol-2-yl, 1,2-thiazol-5-yl, 1,3-thiazol-5-yl, 1,3-thiazol-4-yl, 1,2-thiazole-3-yl, 1,2,4-thiadiazol-3-yl, 1,2,4-thiadiazol-5-yl, 1,3,4-thiadiazole-5-yl, 1,2,4-oxadiazol-3-yl, 1,2,4-oxadiazol-5-yl and 1,3,4-oxadiazol-5-yl. Examples of suitable R⁶ groups include: methyl, ethyl, iPr, -CH₂F, -NH₂, -OH, -CH₂OH, -CH₂NH₂, -CO₂CH₃, -CH2CN, -CH₂CO₂H, - CH₂CO₂CH₃,-(CH₂)₂F, -CH₂CHF₂, -CH₂CF₃, -(CH₂)₂N(CH₃)₂, -CH₂C(CH₃)₂OH, - C(CH₃)₂OH, -C(CH₃)₂NH₂, -(CH₂)₂OH, 1-hydroxycyclopropyl, 1-aminocyclopropyl, - NHC(O)CH₃, 2-pyrrolidinyl, 3-pyrrolidinyl, 4-morpholinyl, 2-pyrrolidinylmethyl, 4-morpholinylmethyl, 1-pyrrolidinylmethyl, 3-fluoro-1-pyrrolidinylmethyl, 4-pyrrolidinylmethyl, 1-piperidinylmethyl, 4-fluoro-1-piperidinylmethyl, 4-piperidinylmethyl, N-methyl-4-piperidinylmethyl, 2-morpholinylmethyl, 1-azetidinylmethyl, 3-hydroxy-1-azetidinylmethyl, 3-fluoro- and 3,3-difluoro-1-azetidinylmethyl, -C(CH₃)₂NHCO₂C(CH₃)₃, 1-(t-butoxycarbonylamino)-1-cyclopropyl, and 2-and 3-oxo-4-piperazinylmethyl.

In another embodiment of the present invention are compounds of Formulas I, Ia, Ia-1, Ib, and Ic wherein R¹ is a 6-membered aromatic or partially unsaturated heterocyclic ring containing 1 to 2 heteroatoms selected from N and O, wherein the heterocyclic ring is optionally substituted with (i) R⁶, (ii) oxo and R⁶ provided R⁶ is not oxo, or (iii) methyl and R⁶. Examples of suitable R¹ groups that can be optionally substituted as described include pyridyl, pyridazinyl, pyrimidinyl and pyrazinyl. Examples of suitable R⁶ groups include: methyl, fluoro, chloro, -OH, - OCH₃, -OCH₂CH₃, -CF₃, -NH₂, -CO₂CH₂CH₃, -SO₂CH₃, -CO₂CH₃, -CH₂OH, -C(CH₃)₂OH, 1-azetidinyl, 3-hydroxy-1-azetidinyl, 3-fluoro- and 3,3-difluoro-1-azetidinyl, 3-hydroxy-1-pyrrolidinyl, 1-pyrrolidinyl, 4-morpholinyl, 1-azetidinylmethyl, 3-fluoro-1-azetidinylmethyl, 3-hydroxy-1-azetidinylmethyl, 1-pyrrolidinylmethyl, 3-fluoro-1-azetidinylmethyl, and 4-morpholinylmethyl.

In another embodiment of the present invention are compounds of Formulas I, Ia, Ia-1, Ib, and Ic wherein R¹ is an 8-membered aromatic or partially unsaturated ortho-fused bicyclic ring system containing 3 to 5 heteroatoms selected from one sulfur and 2 to 4 nitrogen atoms wherein one carbon in the ring is optionally substituted with a group selected from =O, =S, -SMe, -NH₂, - CF₃, -Cl, -C₁₋₄alkyl and -C₁₋₄alkyl substituted with a group selected from -NH₂, -OH, -OC₁₋₄alkyl, -CN and 1 to 3 fluoro. Examples of suitable R¹ groups which may optionally be substituted include:

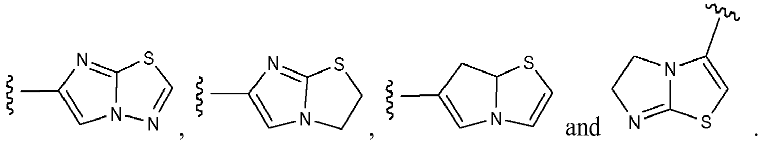

In another embodiment of the present invention are compounds of Formulas I, Ia, Ia-1, Ib, and Ic wherein R¹ is a 9 membered aromatic or partially unsaturated ortho-fused bicyclic ring system containing 3 to 4 nitrogen atoms, wherein one carbon in the ring is optionally substituted with a group selected from =O, =S, -SMe, -NH₂, CF₃, -Cl, pyrrolidinyl, tetrahydrofuranyl, C₁₋₄alkyl and -C₁₋₄alkyl substituted with a group selected from -NH₂, -OH, -OC₁₋₄alkyl, -CN and 1 to 3 fluoro and piperidinyl. Examples of suitable R¹ groups include: 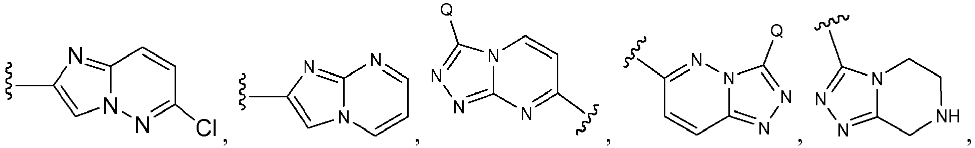 where Q is -H, -CH₃, -CF₃, ⁿPr, ⁱPr, ^{t}Bu, -NH₂, -C(CH₃)₂OH, -C(CH₃)₂NH₂, 3-tetrahydrofuranyl, 2-pyrrolidinyl, or 4-piperidinylmethyl.

In another embodiment of the present invention are compounds of Formulas I, Ia, Ia-1, Ib, and Ic wherein R¹ is -H, -CN, -C(O)NHR⁷ or -CO₂R⁹. Examples of suitable R¹ groups include: hydrogen, -CN, -CO₂CH₃, -C(O)NHCH₃, -C(O)NHCH(CH₃)₂, -C(O)NH-cyclopropyl, - C(O)NH-benzyl, -C(O)NH-(2-thiazolyl), -C(O)NH-(4-tetrahydropyranyl), -C(O)NH-(1-methyl-4-piperidinyl), -C(O)NHCH₂C(CH₃)₂OH,

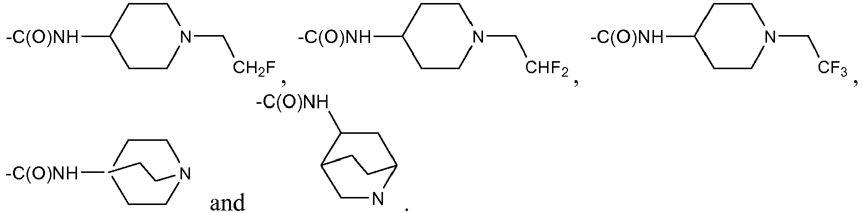

In another embodiment of the present invention are compounds of Formulas I, Ia, Ia-1, Ib, and Ic wherein R¹ is -C₁₋₆alkyl optionally substituted with R¹² and optionally substituted with R13, where R¹² and R¹³ are as defined in relation to formula (I). Preferably, R¹ is C₁₋₆alkyl optionally substituted with R¹³. More preferably, R¹ is -C₁₋₄alkyl optionally substituted with -OH, -NH₂ or 1-3 fluoro. An examples of a suitable R¹ group is -C(CH₃)₂OH.

In another embodiment of the present invention are compounds of Formulas I, Ia, Ia-1, Ib, and Ic wherein R¹ is -OR^{6a}, where R^{6a} is -C₁₋₄alkyl optionally substituted with R¹³, where R¹³ is defined in relation to formula (I). Preferably R¹³ is 1-5 of fluoro.

Each embodiment, class or sub-class described above for each variable (e.g., R¹, R², R³, X, Y, etc.) in Formulas I, Ia, Ia-1, Ib and Ic, may be combined with one or more of the embodiments, classes or sub-classes described above for one or more other variables, and all such sub-generic combinations are included within the scope of this invention.

The term "alkyl" means carbon chains which may be linear or branched, or combinations thereof, containing the indicated number of carbon atoms. Examples of alkyl groups include methyl, ethyl, propyl (n-propyl, ⁿpropyl or *ⁿ*Pr), iso-propyl (i-propyl or ⁱpropyl or *i*Pr), butyl, sec-butyl (s-butyl or ^{s}butyl or *^{s}*Bu), tert-butyl (t-butyl or ^{t}butyl or *^{t}*Bu), pentyl, hexyl, and the like. "Cycloalkyl" is intended to be a cyclized alkyl ring having the indicated number of carbon atoms. Examples of cycloalkyl include cyclopropyl (c-Pr), cyclobutyl (c-Bu), cyclopentyl (c-Pen), and cyclohexyl (c-Hex). The cycloalkyl ring may be substituted on any available carbon which results in the creation of a stable structure, including the ring carbon which serves as the point of attachment to the rest of the molecule. Preferably, cycloalkyl is cyclopropyl or cyclobutyl, and more particularly, when it is substituted with -CH₃ or -CF₃, the substituent is on the ring carbon which serves as the point of attachment to the rest of the molecule.

The term "alkylene" means an acyclic carbon or a saturated acyclic carbon chain represented by the formula -CₙH₂ₙ-. Examples of alkylene include branched and straight carbon chains, for example -CH₂-, -CH₂-CH₂-, -CH₂-CH₂-CH₂-, -CH₂-CH₂-CH₂-CH₂-, -CH₂-CH(CH₃)-, etc.

"Aryl" (Ar) means mono- and bicyclic aromatic rings containing 6-12 carbon atoms. Examples of aryl include phenyl, naphthyl, indenyl and the like. Phenyl is preferred.

The term"heterocycle" and derivatives thereof such as "heterocyclyl" and "heterocyclic ring" mean mono- and bicyclic saturated, partially saturated and aromatic rings and ring systems containing at least one heteroatom selected from N, S and O, each of said ring or ring system having from 3 to 10 atoms in which the point of attachment may be carbon or nitrogen, unless otherwise specified. The term includes, for example, partially unsaturated monocyclic rings that are not aromatic, such as 2- or 4-pyridones attached through the nitrogen or *N*-substituted-(1*H*,3*H*)-pyrimidine-2,4-diones (*N*-substituted uracils). Heterocyclyl moreover includes such moieties in charged form, e.g., piperidinium. Substituents, when present, may be on any available carbon in the ring; suitable substituents may also be on available nitrogens in the ring.

The phrase "optionally substituted with one or more substituents" is intended to mean that the total number of substituents on the optionally substituted moiety overall may be zero, one or more than one , and that each carbon and heteroatom (when present) available for substitution in the given moiety may independently be unsubstituted or mono- or poly-substituted, with one or more substituents that are the same or different at each occurrence and which result in the creation of a stable structure. The term "poly-substituted" is intended to mean two or more substituents, e.g. di-, tri-, tetra-, penta- substitution and higher as appropriate, valence and stability permitting. For example, C₁₋₃alkyl optionally substituted with one or more of fluoro includes, but is not limited to, -CH₃, -CH₂F, -CHF₂, -CF₃, -CH₂CH₃, -CH₂-CH₂F, -CHF-CH₂F, -CF₂-CF₃, -CH(CF₃)-CH₃, -CF₂-CF₂-CF₃, and the like. In some instances, the number of substituents which may optionally be present on a moiety is specified, for example but not limited to, 1-3 of -F (fluoro). For example, methyl optionally substituted with 1-3 of -F includes -CH₃, -CH₂F, -CHF₂ and -CF₃.

Some of the compounds encompassed herein may exist as tautomers, e.g., keto-enol tautomers. For the purpose of illustration, when R¹ is a 5-membered heterocyclic ring and R6 is oxo, the resulting compound may be capable of tautomerism, as exemplified below: 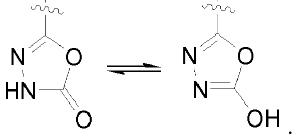 Where compounds of this invention are capable of tautomerization, all individual tautomers as well as mixtures thereof are included in the scope of this invention.

Reference to the compounds of this invention as those of "Formula I" "Formula Ia," "Formula Ib," or any other generic structural formulas used herein is intended to encompass compounds falling within the scope of the structural Formula including pharmaceutically acceptable salts, esters and solvates thereof where such forms are possible, unless specified otherwise. The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc, and the like. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, *N,N*-dibenzylethylenediamine, diethylamine, 2-diethylaminoethanol, 2-dimethylaminoethanol, ethanolamine, ethylenediamine, *N*-ethyl-morpholine, *N*-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, pamoic, pantothenic, phosphoric, propionic, succinic, sulfuric, tartaric, p-toluenesulfonic acid, trifluoroacetic acid, and the like.

Pharmaceutically acceptable esters can optionally be made by esterification of an available carboxylic acid group or by formation of an ester on an available hydroxy group in a compound. Such esterified compounds may serve as pro-drugs which can be hydrolyzed back to their acid or hydroxy form. Examples of pharmaceutically acceptable esters include, but are not limited to, -C₁₋₄alkyl and -C₁₋₄alkyl substituted with phenyl.

The compounds of Formula I may contain one or more asymmetric centers, and can thus occur as racemates, enantiomeric mixtures, single enantiomers, diastereoisomeric mixtures and individual diastereoisomers. The present invention includes all such isomers, as well as salts, esters and solvates of such racemates, mixtures, enantiomers and diastereoisomers. Furthermore, some of the crystalline forms of compounds of the present invention may exist as polymorphs and as such are intended to be included in the scope of the present invention. In addition, some of the compounds of the instant invention may form solvates with water or common organic solvents. Such solvates and hydrates are likewise encompassed within the scope of this invention.

Compounds of structural Formula I may be separated into their individual diastereoisomers by, e.g., fractional crystallization from suitable solvents, e.g., DCM/hexanes or EtOAc/hexanes, or via chiral chromatography using an optically active stationary phase. Absolute stereochemistry may be determined by X-ray crystallography of crystalline products or crystalline intermediates which are derivatized, if necessary, with a reagent containing a stereogenic center of known configuration.

The ability of the compounds of this invention to inhibit biosynthesis of the leukotrienes makes them useful for preventing or reversing the symptoms induced by the leukotrienes in a human subject. Accordingly, this invention provides a method for preventing the synthesis, the action, or the release of leukotrienes in a mammal which comprises administering to said mammal a FLAP inhibitory effective amount of a compound of this invention. Such FLAP inhibitory activity can be measured using the FLAP Assay described herein. Since leukotrienes are potent inflammatory mediators, also provided is a method of treating an inflammatory condition in a mammal which comprises administering a therapeutically effective amount of a compound of this invention to a mammal in need of such treatment.

The inhibition of the mammalian biosynthesis of leukotrienes also indicates that the compounds and pharmaceutical compositions thereof are useful to treat, prevent or ameliorate atherosclerosis in mammals, and especially in humans. Therefore, the compounds of Formula I can be used for the treatment of atherosclerosis comprising administering a therapeutically effective amount of a compound of Formula I to a patient in need of such treatment. A further aspect of this invention involves a method for preventing or reducing the risk of developing atherosclerosis, comprising administering a prophylactically effective amount of a compound of Formula I to a patient in need of such treatment, for example, a patient who is at risk of developing atherosclerosis.

Atherosclerosis is characterized by the deposition of atheromatous plaques containing cholesterol and lipids on the innermost layer of the walls of large and medium-sized arteries. Atherosclerosis encompasses vascular diseases and conditions that are recognized and understood by physicians practicing in the relevant fields of medicine. Atherosclerotic cardiovascular disease including restenosis following revascularization procedures, coronary heart disease (also known as coronary artery disease or ischemic heart disease), cerebrovascular disease including multi-infarct dementia, and peripheral vessel disease including erectile dysfunction, are all clinical manifestations of atherosclerosis and are therefore encompassed by the terms "atherosclerosis" and "atherosclerotic disease."

A FLAP inhibitor may be administered to prevent or reduce the risk of occurrence, or recurrence where the potential exists, of a coronary heart disease (CHD) event, a cerebrovascular event, and/or intermittent claudication. Coronary heart disease events are intended to include CHD death, myocardial infarction (i.e., a heart attack), and coronary revascularization procedures. Cerebrovascular events are intended to include ischemic or hemorrhagic stroke (also known as cerebrovascular accidents) and transient ischemic attacks. Intermittent claudication is a clinical manifestation of peripheral vessel disease. The term "atherosclerotic disease event" as used herein is intended to encompass coronary heart disease events, cerebrovascular events, and intermittent claudication. It is intended that persons who have previously experienced one or more non-fatal atherosclerotic disease events are those for whom the potential for recurrence of such an event exists.

Accordingly, the instant invention also provides a method for preventing or reducing the risk of a first or subsequent occurrence of an atherosclerotic disease event comprising the administration of a prophylactically effective amount of a FLAP inhibitor to a patient at risk for such an event. The patient may already have atherosclerotic disease at the time of administration, or may be at risk for developing it.

The method of this invention particularly serves to prevent or slow new atherosclerotic lesion or plaque formation, and to prevent or slow progression of existing lesions or plaques, as well as to cause regression of existing lesions or plaques. Accordingly, one aspect of this invention encompassed within the scope of treatment of atherosclerosis involves a method for halting or slowing the progression of atherosclerosis, including halting or slowing atherosclerotic plaque progression, comprising administering a therapeutically effective amount of a FLAP inhibitor to a patient in need of such treatment. This method also includes halting or slowing progression of atherosclerotic plaques existing at the time the instant treatment is begun (i.e., "existing atherosclerotic plaques"), as well as halting or slowing formation of new atherosclerotic plaques in patients with atherosclerosis.

Another aspect of this invention encompassed within the scope of treatment of atherosclerosis involves a method for regression of atherosclerosis, including regression of atherosclerotic plaques existing at the time the instant treatment is begun, comprising administering a therapeutically effective amount of a FLAP inhibitor to a patient in need of such treatment. Another aspect of this invention involves a method for preventing or reducing the risk of atherosclerotic plaque rupture comprising administering a prophylactically effective amount of a FLAP inhibitor to a patient in need of such treatment.

The ability of the compounds of Formula I to inhibit biosynthesis of the leukotrienes makes them useful for preventing or reversing the symptoms induced by the leukotrienes in a human subject. This inhibition of the mammalian biosynthesis of leukotrienes indicates that the compounds and pharmaceutical compositions thereof are useful to prevent or reduce the risk for, treat or ameliorate in mammals and especially in humans: 1) pulmonary disorders including diseases such as asthma, chronic bronchitis, and related obstructive airway diseases, 2) allergies and allergic reactions such as allergic rhinitis, contact dermatitis, allergic conjunctivitis, and the like, 3) inflammation such as arthritis or inflammatory bowel disease, 4) pain, 5) skin disorders such as atopic eczema, and the like, 6) cardiovascular disorders such as angina, formation of atherosclerotic plaques, myocardial ischemia, hypertension, platelet aggregation and the like, 7) renal insufficiency arising from ischaemia induced by immunological or chemical (cyclosporin) etiology and 8) migraine or cluster headache, 9) ocular conditions such as uveitis, 10) hepatitis resulting from chemical, immunological or infectious stimuli, 11) trauma or shock states such as bum injuries, endotoxemia and the like, 12) allograft rejection, 13) prevention of side effects associated with therapeutic administration of cytokines such as Interleukin II and tumor necrosis factor, 14) chronic lung diseases such as cystic fibrosis, bronchitis and other small- and large-airway diseases, 15) cholecystitis, 16) multiple sclerosis, 17) proliferation of myoblastic leukemia cells, 18) pulmonary fibrosis, 19) respiratory syncytial virus, 20) acne and 21) sleep apnea. Moreover, the compounds of this invention can be administered to patients, including adult and pediatric patients, for the relief of symptoms of allergic rhinitis, including seasonal allergic rhinitis.

Particularly, the compounds of this invention can be administered to patients, including adult and pediatric patients, for the prophylaxis of asthma and for chronic treatment of asthma. The compounds of this invention can be administered to patients, including adult and pediatric patients, for the treatment of asthma: (1) as an alternative to low-dose inhaled corticosteroids (ICS) for patients with mild persistent asthma, (2) as concomitant therapy with low-dose inhaled corticosteroids (ICS) for patients with mild persistent asthma, or (3) as concomitant therapy in patients with persistent asthma who are inadequately controlled on inhaled corticosteroids (ICS) or on combined ICS/long-acting beta-agonist (LABA) therapy. The compounds can be used for treatment of asthmatic patients including, but not limited to, steroid resistant/non-responder asthmatics, asthmatics for whom leukotriene modifiers have previously failed, smoking asthmatics, and aspirin sensitive asthmatics.

The compounds can be administered to patients to: (1) improve FEV1 (Forced Expitory Volume in one minute), (2) improve morning and evening PEF (Peak Expitory flow), (3) reduce beta-agonist use (measured by puffs/day), (4) reduce inhaled / systemic steroid use. (5) improve daytime asthma symptoms, (6) reduce number of nocturnal awakenings, 7) improve asthma control days, (8) reduce number of asthma exacerbations, wherein an exacerbation is defined as: requiring systemic steroid, an emergency room visit, hospitalization, an unscheduled asthma related doctor visit, decrease in A.M. PEF by >20% or A.M. PEF <1801/min, increased SABA (short-acting beta-agonist) use >70% from baseline (minimum increase 2 puffs), or increased symptom score of >50%, (9) reduce the number of asthma attacks (measured as % of days with at least one attack over a specified period of total days), wherein the attack is one that requires systemic steroid use, an emergency room visit, hospitalization, or an unscheduled asthma related doctor visit, (10) reduce the number of acute asthma attacks, (11) reduce blood and sputum eosinophils, and/or (12) prevent and treat EIB (exercised induced bronchoconstriction).

The FLAP inhibitors of this invention can also be used in a therapeutically effective amount for promoting osteogenesis in a patient in need of such treatment. For example, the compounds could be used to promote osteogenesis to accelerate or enhance bone fracture healing, treat bone defects, and enhance bone formation. The compounds can be administered alone or in combination with one or more additional active agents that inhibit bone resorption, regulate calcium resorption from bone, enhance bone accumulation, enhance bone formation, induce bone formation, impair growth of microorganisms, reduce inflammation, and/or reduce pain.

Thus, the compounds of the present invention may also be used to treat or prevent mammalian (especially, human) disease states such as erosive gastritis; erosive esophagitis; diarrhea; cerebral spasm; premature labor; spontaneous abortion; dysmenorrhea; ischemia; noxious agent-induced damage or necrosis of hepatic, pancreatic, renal, or myocardial tissue; liver parenchymal damage caused by hepatoxic agents such as CCl₄ and D-galactosamine; ischemic renal failure; disease-induced hepatic damage; bile salt induced pancreatic or gastric damage; trauma- or stress-induced cell damage; and glycerol-induced renal failure. Leukotriene biosynthesis inhibitors also act as inhibitors of tumor metastasis and exhibit cytoprotective action and therefore the compounds of this invention may also be useful in this regard.

The FLAP inhibitors of this invention can also be administered for prevention, amelioration and treatment of glomerulonephritis (see Guasch A., Zayas C.F., Badr KF. (1999), "MK-591 acutely restores glomerular size selectivity and reduces proteinuria in human glomerulonephritis," Kidney Int., 56:261-267); and also for and prevention, amelioration and treatment of kidney damage resulting from diabetes complications (see Valdivielso JM, Montero A., Badr KF., Munger KA. (2003), "Inhibition of FLAP decreases proteinuria in diabetic rats," J. Nephrol., 16(1):85-940.)

In addition, the compounds of this invention can also be used for the treatment of chronic obstructive pulmonary disease (COPD). As described in S. Kilfeather, Chest, 2002, vol 121, 197, airway neutrophilia in COPD patients is believed to be a contributing source of inflammation and is associated with airway remodeling. The presence of neutrophils is mediated in part by LTB₄, and treatment with the instant compounds could be used to reduce neutrophilic inflammation in patients with COPD and reduce the rate of COPD exacerbations. In particular, the compounds of this invention could be used for daily, preferably once-daily, maintenance treatment of airflow obstruction associated with COPD, including chronic bronchitis and emphysema.

The cytoprotective activity of a compound may be observed in both animals and man by noting the increased resistance of the gastrointestinal mucosa to the noxious effects of strong irritants, for example, the ulcerogenic effects of aspirin or indomethacin. In addition to lessening the effect of non-steroidal anti-inflammatory drugs on the gastrointestinal tract, animal studies show that cytoprotective compounds will prevent gastric lesions induced by oral administration of strong acids, strong bases, ethanol, hypertonic saline solutions, and the like. Two assays can be used to measure cytoprotective ability. These assays are: (A) an ethanol-induced lesion assay and (B) an indomethacin-induced ulcer assay and are described in EP 140,684.

In particular, the compounds of the invention would be useful to reduce the gastric erosion caused by co-administration of a cyclooxygenase-2 selective inhibitor and low-dose aspirin. Cyclooxygenase-2 selective inhibitors are widely used as effective anti-inflammatory drugs with less potential for gastrointestinal complications as compared to traditional, non-selective non-steroidal anti-inflammatory drugs. However, the combined use of a cyclooxygenase-2 selective inhibitor with low-dose aspirin for cardio protection may compromise the gastrointestinal safety of this class of compounds. By virtue of its activity as a 5-lipoxygenase inhibitor, the compounds of the invention would be expected to be gastric protective in this regard. See Fiorucci, et al. FASEB J. 17:1171-1173, 2003. Cyclooxygenase-2 selective inhibitors for use with the invention include but are not limited to etoricoxib (ARCOXIA™) and celecoxib (CELEBREX®). A compound of this invention in combination with a cyclooxygenase-2 selective inhibitor could be administered in unit dosage form or separately to a patient on low-dose aspirin therapy. Alternatively, the cyclooxygenase-2 inhibitor could be administered in unit dosage form with low-dose aspirin, in which case a compound of this invention would be administered separately. All three active ingredients in unit dosage form is also encompassed. Conventional dosage amounts of the cyclooxygenase-2 selective inhibitor and aspirin (for cardio protection) may be utilized. Aspirin could be administered at 81 mg once daily.

The term "patient" includes mammals, especially humans, who use the instant active agents for the prevention or treatment of a medical condition. Administering of the drug to the patient includes both self-administration and administration to the patient by another person. The patient may be in need of treatment for an existing disease or medical condition, or may desire prophylactic treatment to prevent or reduce the risk of onset of atherosclerosis.

The term "therapeutically effective amount" is intended to mean that amount of a drug or pharmaceutical agent that will elicit the biological or medical response of a tissue, a system, animal or human that is being sought by a researcher, veterinarian, medical doctor or other clinician. The term "prophylactically effective amount" is intended to mean that amount of a pharmaceutical drug that will prevent or reduce the risk of occurrence of the biological or medical event that is sought to be prevented in a tissue, a system, animal or human by a researcher, veterinarian, medical doctor or other clinician. It is understood that a specific daily dosage amount can simultaneously be both a therapeutically effective amount, e.g., for treatment to slow progression of existing atherosclerosis, and a prophylactically effective amount, e.g., for prevention of an atherosclerotic disease event or formation of new lesions.

In general, FLAP inhibitors can be identified as those compounds which have an IC₅₀ in the "FLAP Binding Assay" that is less than or equal to 1 µM, and preferably 500 nM or less, more preferably 100 nM or less, and most preferably 25 nM or less.

An effective amount of a FLAP inhibitor in the method of this invention is in the range of about 0.01 mg/kg to about 30 mg/kg of body weight per day, preferably 0.1 mg to about 15 mg per kg, and most preferably 0.5 to 7.5 mg per kg, in single or divided doses. A single daily dose is preferred but not necessary. For an average body weight of 70 kg, the dosage level is therefore from about 1 mg to about 2000 mg of drug per day, e.g. 10 mg, 25 mg, 50 mg, 75 mg, 100 mg, 125 mg, 150 mg, 175 mg, 200 mg, 250 mg or 500 mg per day, preferably given as a single daily dose or in divided doses two to four times a day, or in sustained release form. It will be understood, however, that the specific dose level for any particular patient will depend upon a variety of factors including the age, body weight, general health, sex, diet, time of administration, route of administration, rate of excretion, drug combination and the severity of the patient's condition. A consideration of these factors is well within the purview of the ordinarily skilled clinician for the purpose of determining the therapeutically effective or prophylactically effective dosage amount needed to prevent, counter, or arrest the progress of the condition. It is expected that the FLAP inhibitor will administered chronically on a daily basis for a length of time appropriate to treat or prevent the medical condition relevant to the patient, including a course of therapy lasting months, years or the life of the patient.

One or more additional active agents may be administered with a compound of Formula I. The term "additional active agent (or agents)" is intended to mean a pharmaceutically active agent (or agents) different from the compound of Formula I. In a broad embodiment, any suitable additional active agent or agents, including but not limited to anti-atherosclerotic agents such as a lipid modifying compound, anti-diabetic agents and/or anti-obesity agents, may be used in combination with the compound of Formula I in a single dosage formulation, or may be administered to the patient in a separate dosage formulation, which allows for concurrent or sequential administration of the active agents. The additional active agent or agents may have more than one pharmaceutical activity, for example it may have both lipid-modifying effects and anti-diabetic activity. Examples of additional active agents which may be employed include but are not limited to HMG-CoA reductase inhibitors, which include statins in their lactonized or dihydroxy open acid forms and pharmaceutically acceptable salts and esters thereof, including but not limited to lovastatin (see US Patent No. 4,342,767), simvastatin (see US Patent No. 4,444,784), pravastatin, particularly the sodium salt thereof (see US Patent No. 4,346,227), fluvastatin particularly the sodium salt thereof (see US Patent No. 5,354,772), atorvastatin, particularly the calcium salt thereof (see US Patent No. 5,273,995), pitavastatin also referred to as NK-104 (see PCT international publication number WO 97/23200) and rosuvastatin (CRESTOR^{®}; see US Patent No. 5,260,440); 5-lipoxygenase inhibitors; cholesterol ester transfer protein (CETP) inhibitors, for example JTT-705 and torcetrapib, also known as CP529,414; HMG-CoA synthase inhibitors; squalene epoxidase inhibitors; squalene synthetase inhibitors (also known as squalene synthase inhibitors), acyl-coenzyme A: cholesterol acyltransferase (ACAT) inhibitors including selective inhibitors of ACAT-1 or ACAT-2 as well as dual inhibitors of ACAT-1 and -2; microsomal triglyceride transfer protein (MTP) inhibitors; niacin; niacin receptor agonists such as acipimox and acifran, as well as niacin receptor partial agonists; bile acid sequestrants; LDL (low density lipoprotein) receptor inducers; platelet aggregation inhibitors, for example glycoprotein IIb/IIIa fibrinogen receptor antagonists and aspirin; human peroxisome proliferator activated receptor gamma (PPARγ) agonists including the compounds commonly referred to as glitazones for example pioglitazone and rosiglitazone and, including those compounds included within the structural class known as thiazolidinediones as well as those PPARγ agonists outside the thiazolidinedione structural class; PPARα agonists such as clofibrate, fenofibrate including micronized fenofibrate, and gemfibrozil; PPAR dual α/γ agonists; vitamin B₆ (also known as pyridoxine) and the pharmaceutically acceptable salts thereof such as the HCl salt; vitamin B₁₂ (also known as cyanocobalamin); folic acid or a pharmaceutically acceptable salt or ester thereof such as the sodium salt and the methylglucamine salt; anti-oxidant vitamins such as vitamin C and E and beta carotene; beta-blockers; angiotensin II antagonists such as losartan; angiotensin converting enzyme inhibitors such as enalapril and captopril; calcium channel blockers such as nifedipine and diltiazam; endothelian antagonists; agents that enhance ABCA1 gene expression; FXR and LXR ligands including both inhibitors and agonists; bisphosphonate compounds such as alendronate sodium; and cyclooxygenase-2 inhibitors such as etoricoxib, celecoxib and valdecoxib. Anti-obesity agents can be employed in combination with a compound of this invention including, but not limited to, sibutramine, orlistat, topiramate, naltrexone, bupriopion, phentermine, and phentermine/topiramate combination (QNEXA®); NPY5 antagonists; Acetyl-CoA Carboxylase-1 and -2 (ACC) inhibitors; MCH1R antagonists; and CB1 antagonists/inverse agonists such as those described in WO03/077847 and WO05/000809. Additional anti-diabetes agents which may be employed in combination with a compound of this invention include but are not limited to DPP-4 (dipeptidylpeptidase-4) inhibitors such as sitagliptin (JANUVIA®) and vildagliptin (GALVUS®); sulfonylureas e.g., chlorpropamide, tolazamide, glyburide, glipizide, and glimepiride; biguanides, e.g., metformin; alpha-glucosidase inhibitors e.g., acarbose and miglitol; meglitinides e.g., repaglinide; glucagon-receptor antagonists; and glucokinase activators.

Other advantageous pharmaceutical combinations comprise the compounds of this invention in combination with anti-cholinergics such as ipratropium bromide and tiotropium, bronchodilators such as the beta agonist salbutamol, metaproterenol, terbutaline, fenoterol, salmeterol, formoterol and the like, and the anti-asthmatic drugs theophylline, choline theophyllinate and enprofylline, the calcium antagonists nifedipine, diltiazem, nitrendipine, verapamil, nimodipine, felodipine, etc., and the corticosteroids, hydrocortisone, methylprednisolone, betamethasone, dexamethasone, beclomethasone, and the like. Particularly, for the prophylaxis and treatment of asthma, compounds of this invention can be used in combination with orally inhaled corticosteroids, such as beclomethasone (e.g. QVAR® Inhalation Aerosol), budesonide (e.g. Pulmicort Respules), flunisolide (e.g., AEROBID® and AEROBID®-M Inhaler System), fluticasone (e.g., FLOVENT® DISKUS® inhalation powder, FLOVENT® HFA Inhalation Aerosol), mometasone (e.g., ASMANEX® TWISTHALER®), and triamcinolone (e.g., AZMACORT® Inhalation Aerosol), and also with inhaled corticosteroid/LABA products such as fluticasone propionate/salmeterol (e.g., ADVAIR DISKUS®). The instant compounds could also be used in combination with leukotriene receptor antagonists such as montelukast (e.g., SINGULAIR®); phosphodiesterase 4 (PDE4) inhibitors such as roflumilast, N-Cyclopropyl-1-[3-(1-oxido-3-pyridinylethynyl)phenyl]-1,4-dihydro[1,8]naphthyridin-4-one-3-carboxamide and the compounds disclosed in PCT Publication WO2003/018579; and Very Late Antigen 4 (VLA4) inhibitors such as the compounds disclosed in U.S. Pat. No. 6,229,011, particularly R411 (N-(2-Chloro-6-methylbenzoyl)-4-[(2,6- dichlorobenzoyl) amino]-L-phenylalanine-2-(diethylamino)ethyl ester which is an ester pro-drug of the active moiety, N-(2-chloro-6-methylbenzoyl)-4- [(2,6-dichlorobenzoyl)amino]-L-phenylalanine), and the compounds disclosed in PCT publication WO2006/023396.

Still another type of agent that can be used in combination with the compounds of this invention are cholesterol absorption inhibitors, for example ZETIA® (ezetimibe) or the combination of ezetimibe with a statin such as VYTORIN® (ezetimibe/simvastatin). Cholesterol absorption inhibitors block the movement of cholesterol from the intestinal lumen into enterocytes of the small intestinal wall.

In the method of treatment of this invention, the FLAP inhibitors may be administered via any suitable route of administration such as orally, parenterally, or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. Oral formulations are preferred.

For oral use, the pharmaceutical compositions of this invention containing the active ingredient may be in forms such as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients, which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example, magnesium stearate, stearic acid or talc.

Oral immediate-release and time-controlled release dosage forms may be employed, as well as enterically coated oral dosage forms. Tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. One example of a time-controlled release device is described in U.S. Patent No. 5,366,738. They may also be coated by the technique described in U.S. Patent Nos. 4,256,108; 4,166,452; and 4,265,874 to form osmotic therapeutic tablets for controlled release.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredients is mixed with water or miscible solvents such as propylene glycol, PEGs and ethanol, or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxy-propylmethycellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more colouring agents, one or more flavouring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of an oil-in-water emulsions. The oily phase may be a vegetable oil, for example olive oil or arachis oil, or a mineral oil, for example liquid paraffin or mixtures of these. Suitable emulsifying agents may be naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. Cosolvents such as ethanol, propylene glycol or polyethylene glycols may also be used. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The instant invention also encompasses a process for preparing a pharmaceutical composition comprising combining a compound of Formula I with a pharmaceutically acceptable carrier. Also encompassed is the pharmaceutical composition which is made by combining a compound of Formula I with a pharmaceutically acceptable carrier.

A therapeutically effective amount of a compound of Formula I can be used for the preparation of a medicament useful for treating or preventing any of the medical conditions described herein, in dosage amounts described herein. For example, a compound of Formula I can be used for the preparation of a medicament useful for preventing or reducing the risk of developing atherosclerotic disease, halting or slowing the progression of atherosclerotic disease once it has become clinically manifest, and preventing or reducing the risk of a first or subsequent occurrence of an atherosclerotic disease event. Additionally, the medicament may be useful for the treatment of asthma, allergies and allergic conditions, inflammation, COPD or erosive gastritis. The medicament comprised of a compound of Formula I may also be prepared with one or more additional active agents, such as those described herein.

The compounds of structural Formula I of the present invention can be prepared according to the procedures of the following Schemes and Examples, using appropriate materials and are further exemplified by the specific examples which follow. Moreover, by utilizing the procedures described herein, one of ordinary skill in the art can readily prepare additional compounds of the present invention claimed herein. The compounds illustrated in the examples are not, however, to be construed as forming the only genus that is considered as the invention. All temperatures are degrees Celsius unless otherwise noted. Mass spectra (MS) were measured by electron-spray ion-mass spectroscopy (ES-MS).

The instant compounds are generally isolated in a pharmaceutically acceptable form which can either be the free base/free acid or an appropriate salt derivative, such as those described above. The free amine bases corresponding to the isolated salts can be generated by neutralization with a suitable base, such as aqueous sodium hydrogen carbonate, sodium carbonate, sodium hydroxide, or potassium hydroxide, extracted into an organic solvent followed by evaporation. The amine free base isolated in this manner can be further converted into another pharmaceutically acceptable salt by dissolution in an organic solvent followed by addition of the appropriate acid and subsequent evaporation, precipitation, or crystallization. Similarly, the free acids can be generated from a base salt derivative by neutralization with a suitable acid such as hydrochloric acid, tartaric acid, and the like. The free acid may be isolated and converted into another pharmaceutically acceptable salt by treatment with an appropriate base.

Some abbreviations used herein are as follows: ABCAl is adenosyltriphosphate-binding cassette-family Al; Ac is acetyl; AcOH is acetic acid; AIBN is 2,2'-azobis(2-methylpropionitrile); aq. is aqueous; Ar is Aryl; Bn is benzyl; Boc is *tert*butyloxycarbonyl; br is broad; Bu is butyl; *^{t}*Bu is tert-butyl; celite is Celite^{®} diatomaceous earth; cpm is counts per minute; δ is chemical shift; DAST is diethylaminosulfur trifluoride; DBU is 1,8-diazabicyclo[5.4.0]undec-7-ene; DCM is dichloromethane; d is doublet; DEAD is diethylazodicarboxylate; DIAD is diisopropylazodicarboxylate; DIBAL-H is diisobutylaluminum hydride; DIPEA is diisopropylethylamine; DMAP is 4-dimethylaminopyridine; DME is 1,2-dimethoxyethane; DMF is *N,N* dimethylformamide; dppf is 1,1'-bis(diphenylphosphino)ferrocene; DMSO is dimethyl sulfoxide; EDC is *N*-(3-dimethylaminopropyl)-*N*'-ethylcarbodiimide hydrochloride; EDTA is ethylenediamine tetraacetic acid; equiv. is equivalent(s); ES-MS is electrospray ion-mass spectroscopy; Et is ethyl; Et₂O is diethyl ether; EtOH is ethanol, EtOAc is ethyl acetate; FXR is farnesoid X receptor; g is gram; h is hours; HATU is *O*-(7-azabenzotriazol-1-yl)-*N,N,N',N*'-tetramethyl-uronium hexafluorophosphate; HetAr or HAR is Heteroaryl; HMG-CoA is 3-hydroxy-3-methylglutaryl coenzyme A; ¹HNMR is proton nuclear magnetic resonance; HOAt is 1-hydroxy-7-azabenzotriazole; HOBt is 1-hydroxybenzotriazole; HPLC is high performance liquid chromatography; Hz is hertz; *i* is Iso; IC₅₀ is concentration at which 50 % inhibition exists; *J* is internuclear coupling constant; kg is kilogram; LDA is lithium diisopropylamide; LG is leaving group; LHMDS is lithium bis(trimethylsilyl)amide; LTB₄ is leukotriene B₄; LXR is liver X receptor; m is multiplet; M is molar; Me is methyl; m.p. is melting point; mg is milligram; µg is microgram; MeCN is acetonitrile; MeOH is methanol; MHz is megahertz; min is minute; mL is milliliter; mm is millimeter; µL is microliter; mM is milimolar; µM is micromolar; mmol is milimoles; Ms is methanesulfonyl; MS is mass spectrum, and a mass spectrum obtained by ES-MS may be denoted herein by "ES"; *m*/*z* is mass to charge ratio; *n* is normal; N is normal; nm is nanometer; nM is nanomolar; NMM is *N*-methylmorpholine; NMO is *N*-methylmorpholine-*N-*oxide; NMP is *N*-methylpyrolidin-2-one; *ⁿ*Pr is *n*-propyl; p is pentet; *p* is para; PEG is polyethylene glycol; Ph is phenyl; Phth is phthalimidoyl; PPARα is peroxisome proliferator activated receptor alpha; Pr is propyl; *ⁱ*Pr is isopropyl; *p*-TSA is para-toluenesulfonic acid; PyBOP is benzotriaxole-1-yl-oxy-tris-pyrrolidino-phosphonium hexafluorophosphate; q is quartet; rt is room temperature; s is singlet; *sec* is secondary; t is triplet; *^{t}*BuOH is *tert*-butanol; *tert* is tertiary; Tf is trifluoromethane-sulfonyl; TFA is trifluoroacetic acid; and THF is tetrahydrofuran; Ts is tosyl; UV is ultraviolet; x g is times gravity; °C is degrees Celsius. In the Schemes, all substituents are as defined above unless indicated otherwise. When present, "-(R^{c})₂" represents two -R^{c} groups separately bonded to the phenyl ring, i.e.

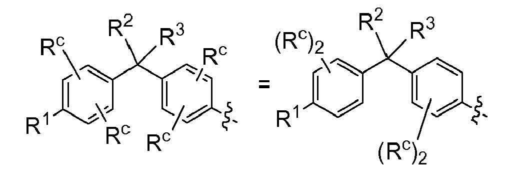

Reaction schemes A-U illustrate the methods employed in the synthesis of the compounds of the present invention of structural Formula **I.** All abbreviations are as defined above unless indicated otherwise.

Reaction scheme A illustrates a method for the synthesis of compounds **3** and **4.** Benzophenone **1** is treated with an organometallic reagent **2,** capable of transferring an alkyl group, to provide compound **3.** Preferred organometallic reagents for this transformation include organomagnesium (Grignard) and organolithium compounds. When a Grignard reagent is employed, it is customary to conduct the reaction in a suitable ethereal solvent such as diethyl ether, or THF, or mixtures thereof, at temperatures between -78 °C and the boiling temperature of the solvent. When an organolithium reagent is used, the reaction can be conducted in a variety of solvents such as diethyl ether or hexanes, at temperatures between -78 °C and room temperature. The Grignard and the organolithium reagents are often purchased commercially, but can be prepared synthetically according to known methods in organic synthesis. The method for removal of the tertiary hydroxyl group in **3** will depend upon the identity of the V¹ and V² substituents. If these substituents are unaffected by hydrogenation conditions, then the hydroxyl group may be removed by hydrogenolysis using a palladium-on-carbon catalyst in a solvent such as methanol or ethanol, and in the presence of hydrogen gas or a hydrogen donor such as formic acid. If one or both of the V¹ and V² substituents are sensitive to hydrogenation conditions, it is preferable to use an organosilane such as triethylsilane in the presence of a protic acid like TFA or a Lewis acid like boron trifluoride. It is customary to conduct the reaction in an inert organic solvent like DCM or 1,2-dichloroethane at temperatures between 0 °C and boiling point of the solvent. Depending on the nature of the V¹ and V² substituents, compound **4** can then be transformed to other compounds of the present invention (**I**).

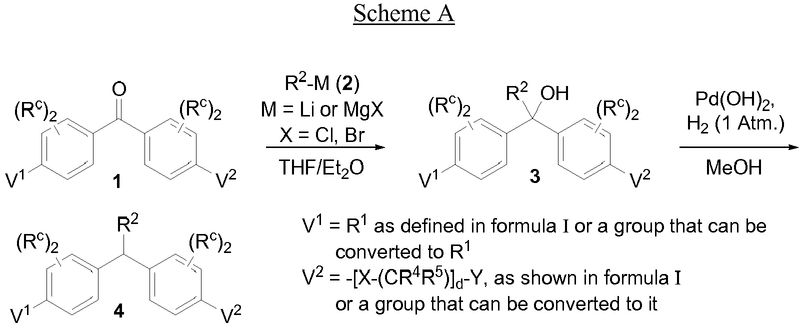

Reaction scheme B illustrates an alternative method for the synthesis of diarylalcohols **3.** In this method, an alkyl-aryl ketone **5** is treated with an organometallic reagent **6,** capable of transferring an aryl group. Preferred organometallic reagents for effecting this transformation include organomagnesium (Grignard) and organolithium compounds, and are used in a similar manner to that described above. In yet another variation of this method, **3** can also be prepared from the reaction of an alkyl-aryl ketone **7** and an organometallic reagent **8.**

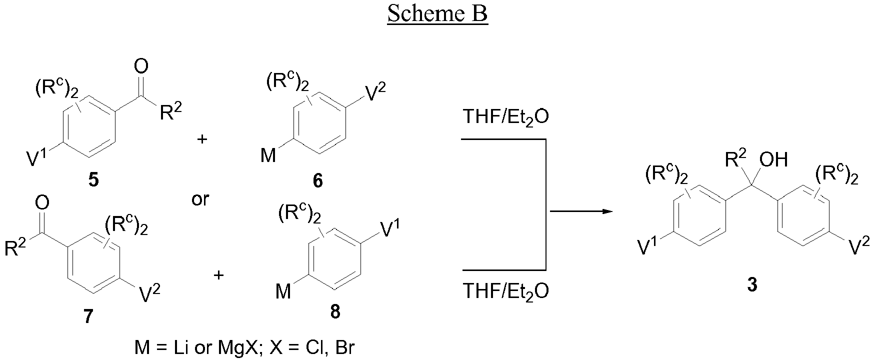

Reaction scheme C illustrates a general method for the synthesis of compounds of type **13.** Aldehyde or ketone **9** can be arylated twice in an electrophilic aromatic substitution process called the Friedel-Crafts reaction. Typical conditions for effecting such an arylation include initial addition of one aromatic-coupling partner **10** to the aldehyde or ketone **9** to afford an intermediary alcohol **11,** subsequent generation of an intermediate carbocation **12,** derived from **11,** followed by *in situ* trapping with a second aromatic-coupling partner **10** which may or may not be the same as the first aromatic coupling partner. Formation of **12** may occur spontaneously in solution or it may be promoted with a reagent capable of ionizing **11,** for example a protic acid such as *p*-TSA, or concentrated hydrochloric acid or a suitable Lewis acid. In certain cases, it may be preferable to conduct the reaction in the presence of a free radical scavenger such as 3-mercaptopropionic acid or the like. The reaction is conducted typically in an inert organic solvent, at temperatures between -20 °C and the boiling temperature of the solvent. The product **13** can be elaborated to compounds of the present invention **(I)** as described in the subsequent schemes.

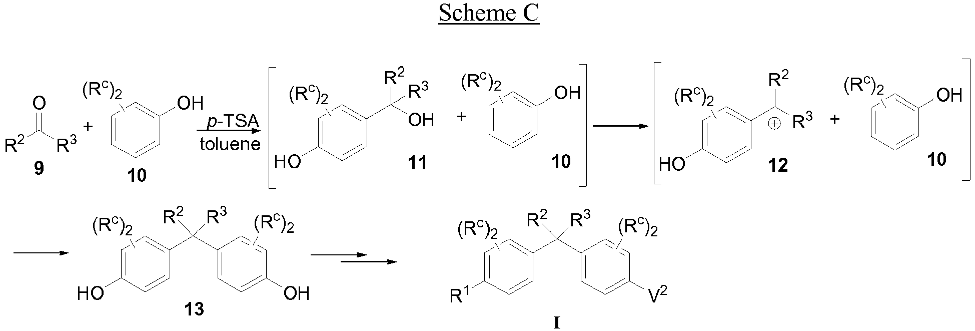

Reaction scheme D illustrates a method for the generation of compounds of type **15** (V¹ ≠ OH). In this method, each of the aromatic coupling partners is introduced sequentially, but in separate chemical manipulations. For example, the aromatic coupling partners are introduced using a combination of the aforementioned Grignard and Friedel-Crafts arylation methodologies. Conditions for effecting the latter transformations are as described above.

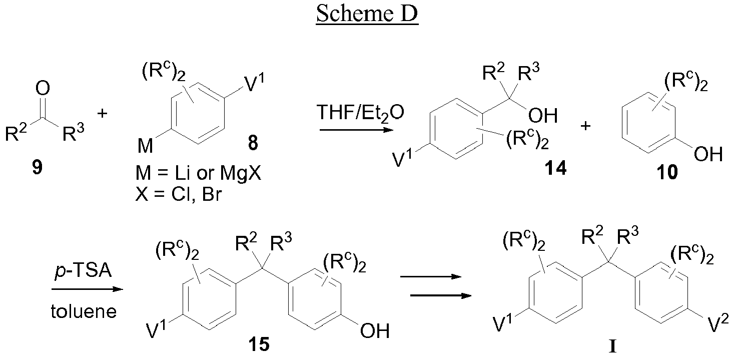

Reaction scheme E illustrates an alternate method of synthesis of compound **4.** Benzophenone **1** is treated with a phosphonium ylide **17** to afford olefin **18.** Ylide **17** can be generated from phosphonium salt **16** that is reacted with a suitable base, such as lithium bis(trimethylsilyl)amide, LDA, or similar bases possessing alternate counterions, such as sodium or potassium, typically in an ethereal solvent such as diethyl ether or THF, at temperatures between -78 °C and the solvent boiling temperature. Alternatively, ketone **1** can be treated with other suitable carbon nucleophiles, such as those described for Scheme B, that are capable of reacting selectively with the ketone moiety. The subsequent tertiary hydroxyl intermediate **3** can be dehydrated to afford olefin **18.** The dehydration step is commonly promoted with a protic acid source, such as *p*-TSA or concentrated hydrochloric acid, or a Lewis Acid, such as boron trifluoride, typically at elevated reaction temperatures up to and including the boiling temperature of the reaction solvent. The reduction of olefin **18** can be effected in the presence of a suitable reducing catalyst, such as palladium(0) on activated carbon, or the like, under a hydrogen atmosphere (≥1 atm), in a suitable inert organic solvent, such as methanol, ethanol, EtOAc, or mixtures thereof, to afford the reduced product **4,** which can be elaborated to compounds of the present invention **(I)** as described in the subsequent schemes.

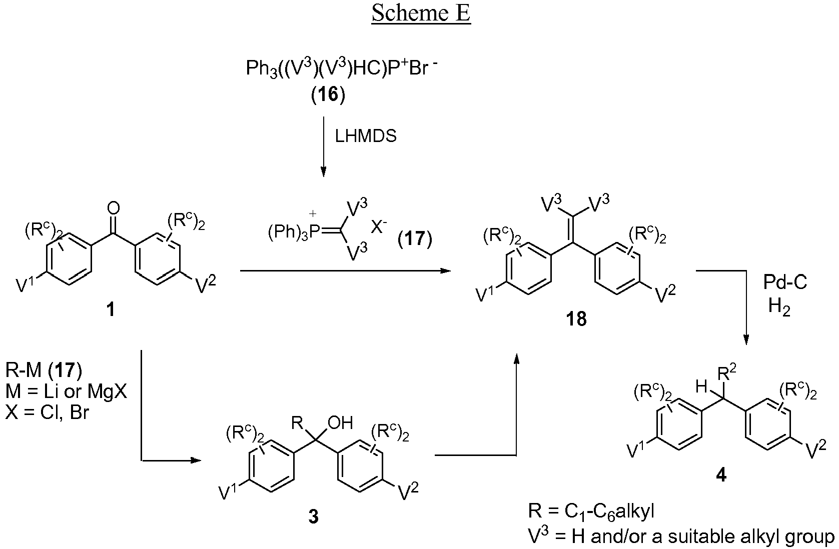

Reaction scheme F illustrates a method of synthesis of a compound of type **20.** Olefin **18** is cyclopropanated in the presence of a metallocarbene to afford cyclopropane **19.** An example of this reaction is known as the Simmons-Smith reaction. Typical conditions for effecting such a cyclopropanation include the generation of a reactive organozinc intermediate by reacting diiodomethane with zinc-copper couple, and the like, typically in an ethereal solvent, such as diethyl ether, at temperatures between 0 °C and room temperature. Since the reaction mixture is heterogeneous, conditions that favor reagent mixing, such as the use of a sonicating water bath, may be employed. Alternatively, compound **19** is obtained by reacting olefin **18** with diiodomethane in the presence of a suitable organometallic reagent, such as diethyl zinc, typically in halogenated solvents, such as dichloroethane, at temperatures between 0 °C and room temperature. Cyclopropane **19** can be treated with a substoichiometric amount of a suitable reducing catalyst, such as platinum(0) on activated carbon, or the like, under a hydrogen atmosphere (≥1 atm), in a suitable inert organic solvent, such as methanol, ethanol, or the like to provide compound **20**, which can be elaborated to compounds of the present invention (I) as described in the subsequent schemes.

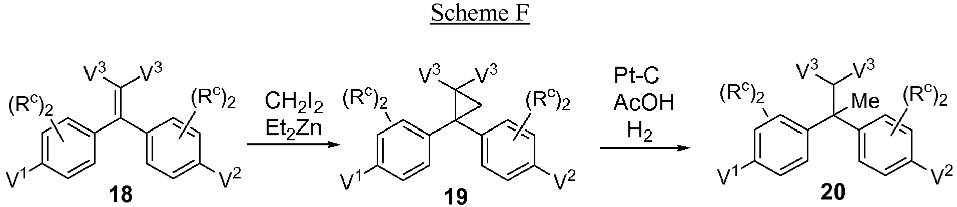

Reaction scheme G illustrates a method for the elaboration of compound **21** to afford compound **22.** In this method, **21** is treated with methanol in the presence of a suitable palladium catalyst, such as[1,1'-bis(diphenylphosphino)-ferrocene]dichloropalladium(II), or the like, and a tertiary amine base, such as triethylamine, or diisopropylethylamine, or the like, in an inert organic solvent like dimethylformamide. The reaction is usually conducted at elevated temperature, typically between 50 °C and 100 °C, for periods of 3-24 h, under an atmosphere of carbon monoxide (J. Org. Chem. 1974, 39, 3318-3326). In certain cases, it may be preferable t use elevated pressures of carbon monoxide, or an additive, such as lithium chloride, to promote or accelerate the reaction. The product ester **22** can be elaborated to compounds of the present invention **(I)** as described in the subsequent schemes.

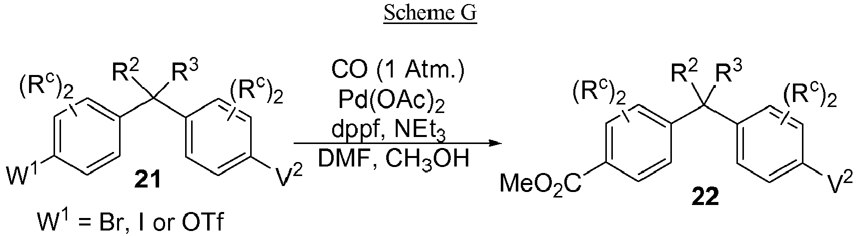

Reaction scheme H illustrates a method for the elaboration of compound **21** to afford compound **23.** In this method, **21** is reacted with potassium cyanide, or a similar cyanide source, such as trimethylsilylcyanide, or the like, in the presence of a suitable palladium catalyst/ligand system. It may be preferable to use an inorganic additive, such as copper(I) iodide, and/or a mild base, such as triethylamine, to accelerate or promote the reaction. The reaction is usually performed in a suitable degassed inert organic solvent, preferably a polar aprotic solvent, such as acetonitrile, DMF or NMP, at elevated temperatures, generally between 50-140 °C, for a period of 3-24 h. The product nitrile **23** can be elaborated to compounds of the present invention **(I)** as described in the subsequent schemes.

### Scheme H

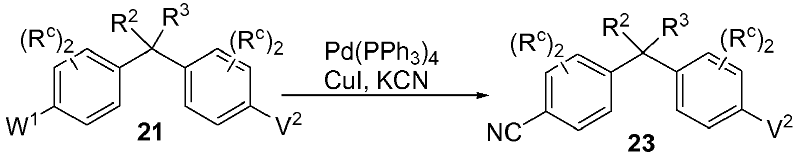

Reaction scheme I illustrates a method of synthesis of compound **24.** In this method, compound **22** can be hydrolyzed to carboxylic acid **24** using a variety of methods known to those skilled in organic synthesis. The product carboxylic acid **24** can be used as a coupling partner in reaction Schemes L and M, or synthetically modified using a variety of methods known in organic synthesis to afford compounds of the present invention **(I).**

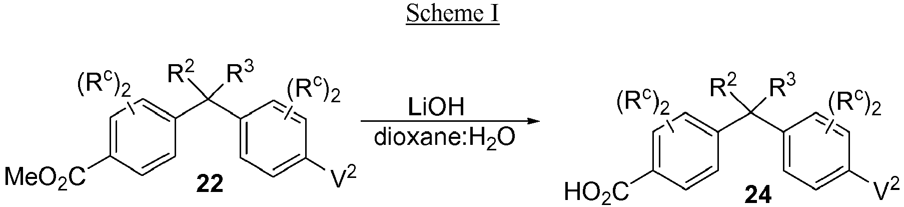

Reaction scheme J illustrates a method of synthesis of compounds of structural formulas **25, 26** and **27.** In this method, **21** is treated with either allyltributylstannane or vinyltributylstannane in the presence of a suitable a palladium catalyst such as [1,1'-bis(diphenyl-phosphino)ferrocene]dichloropalladium(II), or the like, in an inert organic solvent like DMF or NMP. The reaction is usually conducted at elevated temperatures, typically between 50-120 °C, for periods of 2-24 hours. In certain cases, it may be necessary to use an additive such as lithium chloride to promote the reaction. Often, the reaction times can be significantly reduced if the reaction is conducted under microwave irradiation. The product of the reaction is alkene **25** which can be synthetically elaborated, using a variety of methods known in organic synthesis. For example, **25** can be oxidatively cleaved to afford aldehyde **26,** which can be further oxidized to carboxylic acid **27.** A preferred method for the oxidative cleavage reaction is the two-step process shown in reaction scheme J. Alkene **25** is first oxidized to a vicinal diol using catalytic osmium tetraoxide in the presence of a stoichiometric reoxidant such as NMO, in a solvent system such as acetone-water. The intermediate vicinal diol which forms is generally not isolated, but is in turn subjected to cleavage with sodium periodate in a suitable mixed solvent system like THF-water to afford **26.** Both steps in the oxidative cleavage sequence are generally completed during periods of several minutes to a few hours, at temperatures between 0 °C and room temperature. Alternatively, the oxidative cleavage of **25** may also be accomplished using ozone, or by other methods known to those skilled in the art. Aldehyde **26** can then be further oxidized to **27** using a buffered chlorite oxidation system. In this method, **26** is treated with sodium chlorite and monobasic sodium phosphate in the presence of a chlorine scavenger, such as 2-methyl-2-butene. The reaction is conducted typically in a solvent system like *n*-butanol-water, for periods of 1-6 hours, at temperatures between 0 °C and room temperature. In certain cases, **25** can be directly converted to **27** using the sodium periodate/ruthenium trichloride reagent system. Both **26** and **27** can be elaborated in numerous ways known in organic synthesis to furnish other compounds of the present invention **(I).**

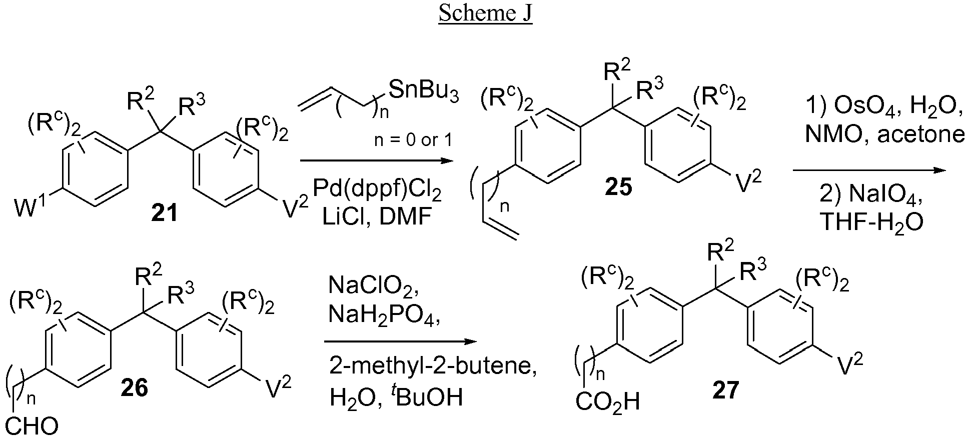

Reaction scheme K illustrates a method of synthesis of a compound of type **28.** In this method, the W¹ group in **21** can be removed by treatment with an appropriate reducing agent, such as a trialkylammonium formate, ammonium formate, triethylsilane, or the like, in the presence of a suitable homogeneous palladium catalyst, such as [1,1'-bis(diphenylphosphino)-ferrocene]dichloropalladium(II) in an inert organic solvent, preferably a polar aprotic solvent, such as DMF, or NMP. The reaction is usually run at elevated temperatures, typically between 50-90 °C, to afford aryl compound **28.**

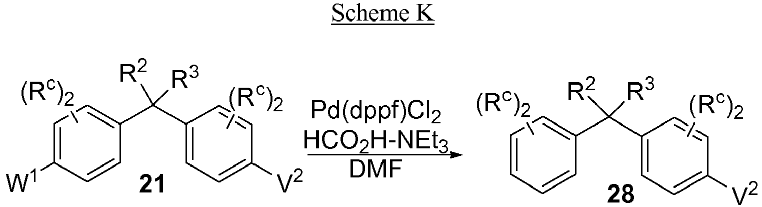

Reaction scheme L illustrates a method of synthesis of compounds of structural formula **30.** In the most general case, **27** is treated with an amine **29** to afford an amide **30.** The amide bond coupling reaction illustrated in reaction scheme L is conducted in an appropriate inert solvent such as DMF, DCM or the like and may be performed with a variety of reagents suitable for amide coupling reactions such as HATU, EDC or PyBOP. Preferred conditions for the amide bond coupling reaction shown in reaction Scheme L are known to those skilled in organic synthesis. Modifications may include, but are not limited to, the use of basic reagents such as triethylamine, DIPEA, or NMM, or the addition of an additive such as HOAt or HOBt. Alternatively, **29** may be treated with an activated ester or acid chloride derivative of **27**to afford **30.** The amide bond coupling shown in reaction Scheme L is usually conducted at temperatures between 0 °C and room temperature, occasionally at elevated temperatures, and the coupling reaction is typically conducted for periods of 1 to 24 hours. 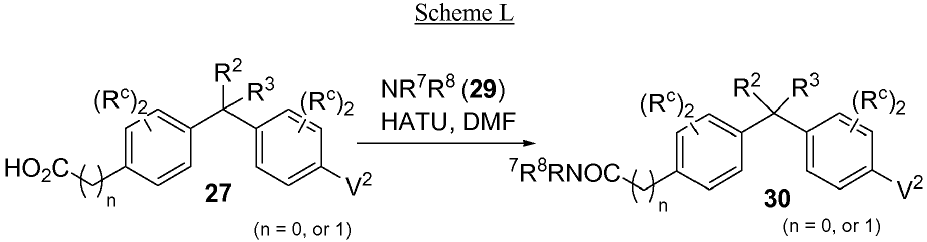 Reaction scheme M illustrates a method for the synthesis of a compound of type **32.** In this method, **27** is subjected to the Curtius reaction to afford an *N*-Boc protected amine **31.** The reaction is performed by reacting **27** with diphenylphosphoryl azide in the presence of a tertiary amine such as triethylamine or DIPEA in a solvent such as toluene. The initial product is generally accepted to be the acyl azide, which is rearranged to the isocyanate in a thermal process analogous to the Wolff rearrangement of acyl carbenes. The rearrangment is conducted typically at the reflux temperature of the solvent, for instance 110 °C, and the rearrangement is usually completed in a period of 1-5 hours. The intermediate isocyanate which forms is generally not isolated, but is in turn subjected to *in situ* reaction with a suitable alcohol such as tert-butyl alcohol to afford carbamate **31.** The N-Boc group can be removed by a suitable deprotection method such as treatment with hydrogen chloride in EtOAc or TFA in DCM. The deprotection is conducted typically at temperatures between 0 °C and room temperature, and the reaction is usually complete in 0.5-3 hours. The product amine **32** can be used as a coupling partner in Schemes N and O, or elaborated using a variety of methods known in organic synthesis to afford compounds of the present invention **(I).**

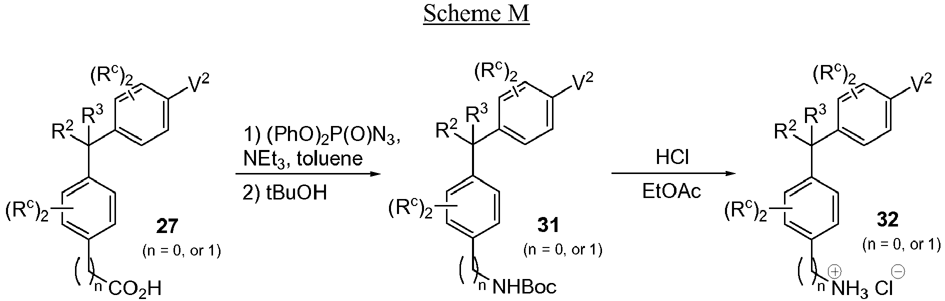

Reaction scheme N illustrates methods for the syntheses of compounds of type **35.** For example, **32** can participate in amide bond coupling reactions with a carboxylic acid **33** to afford amide **35,** using the reagents and conditions described for the generalized amide coupling protocol shown in reaction Scheme L in the presence of a suitable tertiary amine base, such as triethylamine, or diisopropylethylamine, or the like. Alternatively, **32** may also be treated with an activated ester or acid chloride derivative of type **34,** which also affords **35.** Typical conditions for effecting such a transformation include treatment of **32** with acid chloride **34** in the presence of excess tertiary amine base such as triethylamine. It is customary to perform the reaction in an inert organic solvent such as DMF or DCM, at temperatures between 0 °C and the reflux temperature of the solvent, frequently at room temperature and for periods of 1-24 hours.

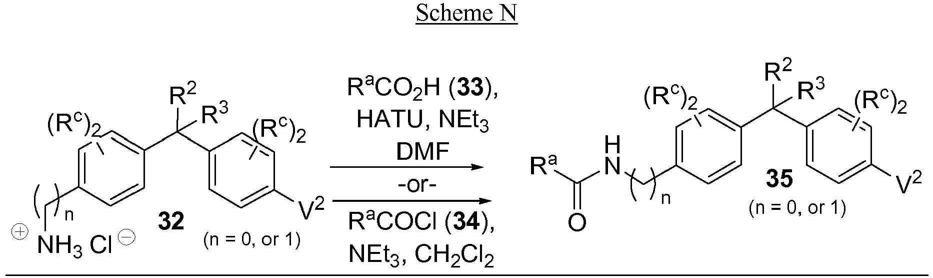

As shown in reaction scheme O, **32** can also be elaborated using the Fukuyama modification of the Mitsunobu reaction (Fukuyama, T.; Jow, C.-K.; Cheung, M. Tetrahedron Lett. 1995, 36, 6373-74). For example, **32** may be reacted with an arylsulfonyl chloride such as 2-nitrobenzenesulfonyl chloride, 4-nitrobenzenesulfonyl chloride or 2,4-dinitrobenzenesulfonyl chloride and a tertiary amine base such as 2,4,6-collidine or 2,6-lutidine in an inert organic solvent such as DCM. Alternatively, the reaction can also be performed under the classical Schotten-Baumann conditions as shown in scheme O, in which **32** and the arylsulfonyl chloride are allowed to react in aqueous alkaline solution. The product of this reaction is sulfonamide **36,** which can be further modified by reaction with an alcohol **37** in the presence of triphenylphosphine and an activating agent such as DEAD, DIAD, or the like. The reaction is performed in a suitable inert organic solvent such as benzene, toluene, THF, or mixtures thereof, typically at room temperature, and the reaction is generally complete in 0.5-3 hours. The product of this reaction is a dialkylsulfonamide **38,** which can be desulfonylated by treatment with either a nucleophilic amine like n-propylamine, in a solvent such as DCM, or with mercaptoacetic acid and triethylamine in DCM. In either case, the reaction is conducted typically at room temperature, for a period of 5 minutes to 1 hour. When a 2- or 4-nitrobenzenesulfonyl derivative is employed, the cleavage of the sulfonamide is accomplished with either the combination of thiophenol and potassium carbonate in a solvent like DMF, or with mercaptoacetic acid and lithium hydroxide in DMF. In either case, the reaction is conducted at room temperature, for a period of 1-3 hours. The secondary amine product **39** can be modified further using a variety of methods known in organic synthesis to provide other compounds of the present invention. For example, **39** may be subjected to a reductive amination reaction with aldehyde or ketone **40** using the conditions described in the bottom of reaction Scheme O to afford compounds of type **41.** 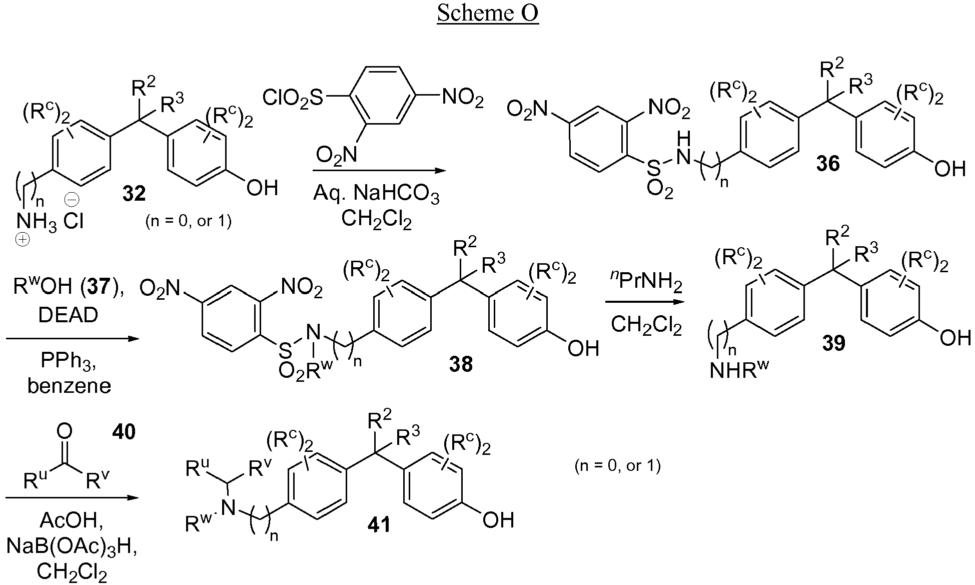 In compound **41** when n = 1, -CHR^{u}R^{v} = a group within the scope of R^{a} or a group that can be converted to R^{a} as defined in formula **I** and R^{w} = a group within the scope of R^{a} or a group that can be converted to R^{a} as defined in formula **I**, or when n = 0, -CHR^{u}R^{v} = a group within the scope of R⁷ or a group that can be converted to R⁷ as defined in formula **I** and R^{w} = a group within the scope of R⁸ or a group that can be converted to R⁸ as defined in formula **I**

Reaction scheme P illustrates using the Suzuki reaction for the synthesis of compounds of type **44.** Compound **21a** can be treated with an aryl- or heteroaryl-boronic acid of type **42,** or alternatively, an aryl- or heteroaryl-boronate of type **43,** in the presence of a suitable palladium catalyst, such as [1,1'-bis(diphenylphosphino)ferrocene]dichloropalladium(II), or tetrakis(triphenylphosphine) palladium (0), or the like, and a mild base, such as sodium carbonate, sodium phosphate tribasic, or the like (Pure Appl. Chem. 1991, 63, 419-422). The reaction is usually performed in a suitable degassed aqueous mixture of inert organic solvents, such as toluene, ethanol or dioxane, at elevated temperatures, generally between 70 °C and the boiling temperature of the solvent mixture, for a period of 3-24 h. Conditions suitable for performing Suzuki reactions at room temperature have been reported in the literature (see: J. Am. Chem. Soc. 2000, 122, 4020-4028, and references therein).

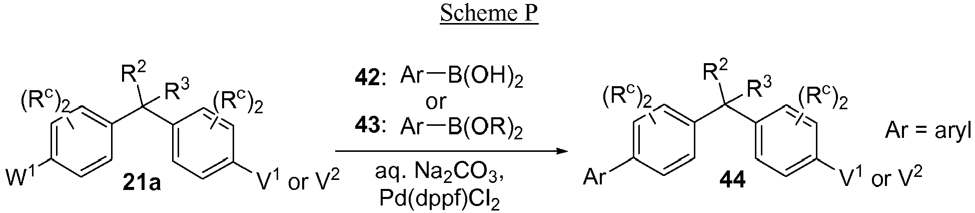

Reaction scheme Q illustrates an alternate method of synthesis of compounds of type **44.** In this method, compound **21a** is treated with bis(pinacolato)diboron in the presence of a suitable palladium catalyst, such as [1,1'-bis(diphenylphosphino)ferrocene]-dichloropalladium(II), and an activating reagent, such as potassium acetate, or the like. The reaction is usually performed in a suitable degassed inert organic solvent, such as dimethyl sulfoxide or dioxane, or the like, at elevated temperatures, generally between 70 °C and 100 °C, for a period of 1-24 h (J. Org. Chem. 1995, 60, 7508-7510), to provide boronate **45**, which can participate in organotransition metal catalyzed cross-coupling reactions, such as the Suzuki reaction (Scheme P), to afford compounds of the present invention (**I**).

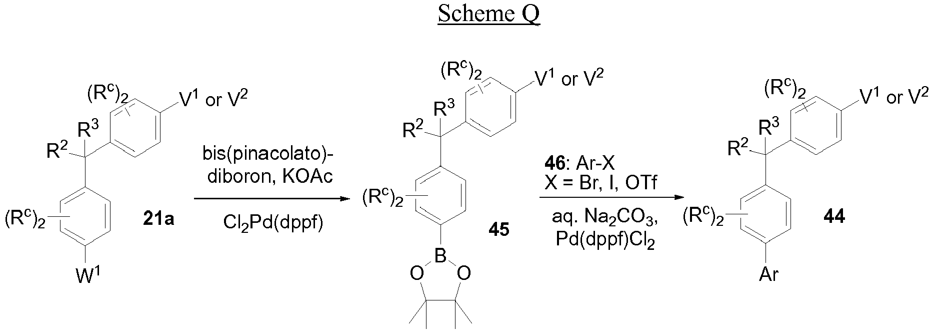

Reaction scheme R illustrates a method of synthesis of compounds of structural formulas **51** and **52.** In this method, **47** is treated with a triflating agent, such as trifluoromethanesulfonic anhydride or 2-(*N*,*N*,-bis(trifluoromethansulfonyl)amino pyridine, or the like, in the presence of a tertiary amine base, such as triethylamine or diisopropylethylamine, to afford intermediate **48.** The triflating reaction is typically performed in aprotic organic solvents, such as DCM or THF, at temperatures that range from -78 °C to room temperature. Compound **48** can be treated with a terminal alkyne **49** in an organotransition metal catalyzed cross-coupling process commonly referred to as the Sonogashira reaction. The reaction is performed in the presence of a suitable palladium catalyst and a copper(I) co-catalyst, such as copper(I) iodide, and typically employs an excess of an amine base, such as triethylamine or diethylamine. The reaction is conducted in an inert organic solvent such as DMF, at temperatures ranging from ambient temperature to about 100 °C, for a period of 3-24 hours. The product of the reaction is alkyne **50** which can then be converted into alkene **51** or alkane **52.** If **51** is desired, preferred conditions for performing the partial reduction of **50** involve the use of a Lindlar catalyst reagent system under an atmospheric or elevated pressure of hydrogen. The reaction is usually conducted in an inert organic solvent, such as EtOH and EtOAc, or combinations thereof, and at room temperature for a period of 3-15 hours. If **52** is desired, then the reduction of **50** is performed with any one of a variety of palladium-on-carbon catalysts, at either atmospheric or elevated pressure of hydrogen.

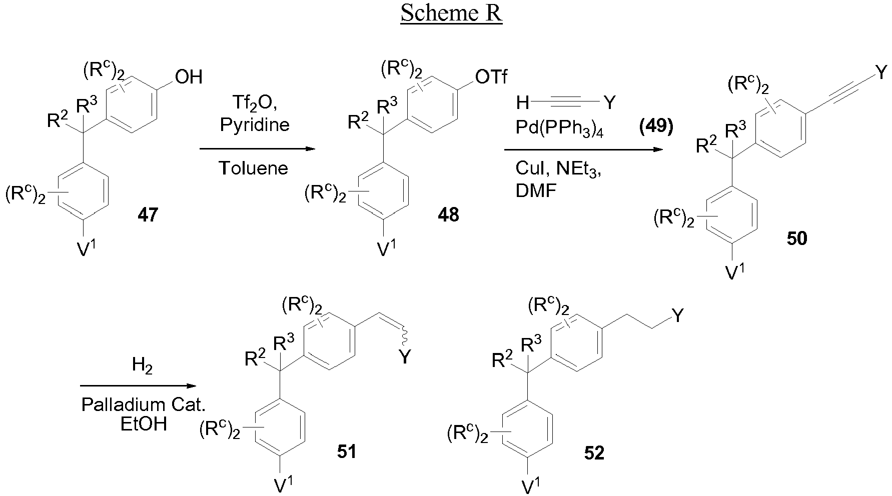

Scheme S illustrates a method of synthesis for compounds of type **56.** In this method, which is a modification of the method commonly referred to as the Kucherov reaction, alkyne **50** is treated with a concentrated acid, such as sulfuric acid, or the like, in water or an alternate protic solvent, at temperatures ranging between 0 °C and room temperature. This method can also be performed using mercuric sulfate, as a substitute for concentrated acid, to promote the alkyne hydration. The product of this reaction is ketone **53,** which can be treated with a reducing agent, such as sodium borohydride or lithium borohydride, under a variety of conditions known to those skilled in the art. In addition, several methods exist for effecting stereoselective reduction of **53** to either antipode of alcohol **54.** A preferred method includes the application of sub-stoichiometric amounts of chiral oxazaborolidine reagents in conjunction with a stoichiometric reducing agent, such as borane-dimethylsulfide, to effect the aforementioned stereoselective reduction of **53** (Angew. Chem. Int. Ed. 1998, 37, p. 1986-2012, and references therein). Alcohol **54** can be treated with an electrophile **55** in the presence of a suitable base, such as sodium hydride. It is customary to conduct the alkylation reaction in a polar aprotic solvent, such as THF, DMF or *N*-methyl-2-pyrrolidinone, or the like, at temperatures generally between - 20 °C and room temperature.

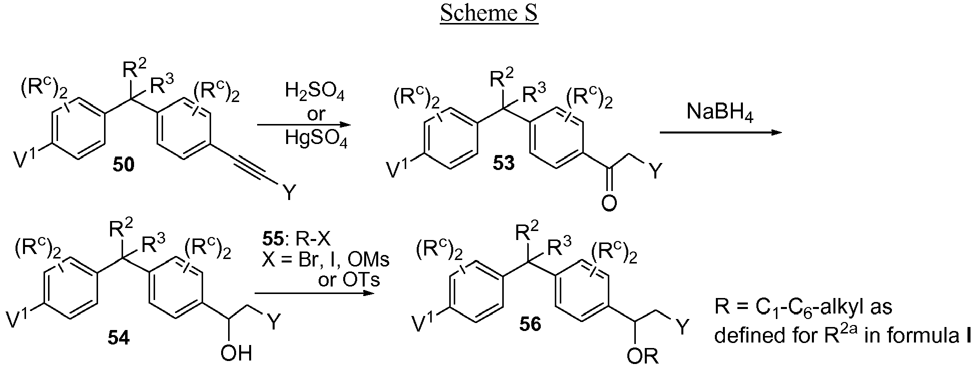

Scheme T illustrates that compounds of structural formula **57** can be elaborated to a variety of heterocyclic (HAR) derivatives of structural formula **58** using known methods in organic synthesis. Specific examples of such transformations are shown in the Examples section. Leading references for effecting such transformations include:
1) Joule, J.A; Mills, K and Smith, G.F. Heterocyclic Chemistry, Chapman & Hall, 1995, 3rd Edn., and references cited therein;
2) Katrittzky, A.R.; Rees, C.W. (Eds), Comprehensive Heterocyclic Chemistry: The Structure, Reactions, Synthesis, and Uses of Heterocyclic Compounds, Pergamon Press, Oxford, 1984, 8v, and references cited therein; and
3) Comprehensive Heterocyclic Chemistry II: Review of the Literature 1982-1995: The Structure, Reactions, Synthesis and Uses of Heterocyclic Compounds, Pergamon Press, New York, 2nd Edn., 1996, 1 1v, and references cited therein.

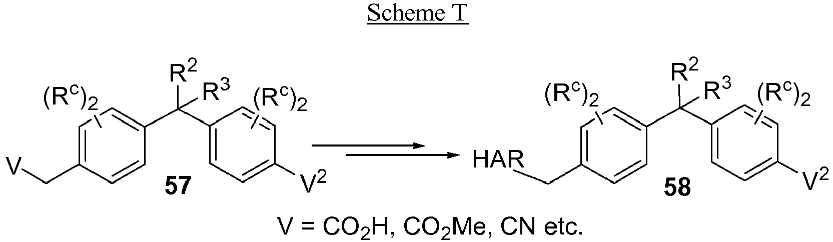

Scheme U illustrates a method for the resolution of a compound of structural formula **59** in which the asterisked carbon is a center of chirality. Generally, the latter, or intermediates en route to their preparation, may be resolved to afford enantiomerically pure compounds such as **60** and **61** by chiral stationary phase liquid chromatography techniques or other suitable methods known in organic synthesis.

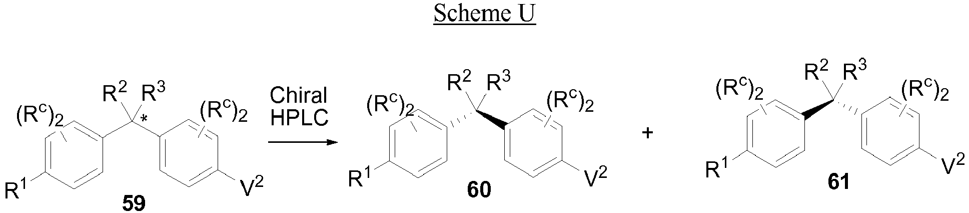

Intermediates used in the synthesis of compounds of this invention can be prepared using the following procedures. In the Tables associated with the following Schemes, compounds having mass spectral data were synthetically prepared. As used hereinafter, PYR is pyridyl, PYM is pyrimidinyl, and TZE is 1,3-thiazolyl.

### (1) Preparation of i-1h and i-1i (Scheme i-1)

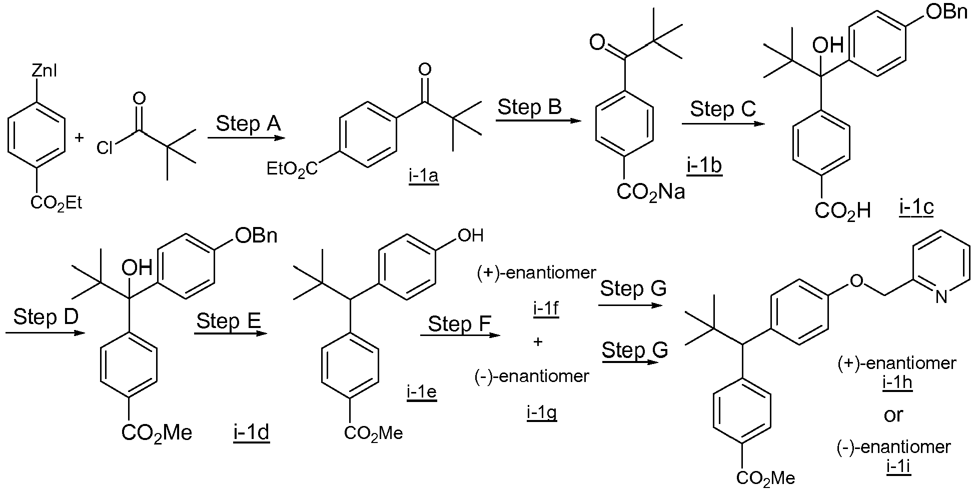

### Step A: Preparation of ethyl 4-(2,2-dimethylpropanoyl)benzoate (i-1a) a)

4-(Ethoxycarbonyl)phenyl-zinc iodide (50.0 mL of a 0.5 M solution in THF, 25.0 mmol) was added slowly via cannula to a stirred solution of dichlorobis(triphenylphosphine)-palladium(II) (484 mg, 0.690 mmol) in THF (50 mL) at 0 °C. After 15 min, trimethylacetyl chloride (2.80 mL, 22.7 mmol) was added and the resulting mixture was stirred at 0 °C. After 1.5 h, the reaction mixture was poured into 1 N HCl and extracted three times with EtOAc. The combined organic extracts were washed with water and brine, dried (MgSO₄) and concentrated *in vacuo.* Purification of the crude residue by flash chromatography on silica gel (gradient elution; 0-10% EtOAc/hexanes as eluent) afforded the title compound i-1a. ¹HNMR (500 MHz, CDCl₃): δ 8.08 (d, 2H, *J* = 8.5 Hz), 7.67 (d, 2H, *J* = 8.5 Hz), 4.42 (q, 2H, *J* = 7.2 Hz), 1.42 (t, 3H, *J* = 7.2 Hz), 1.36 (s, 9H).

### Step B: Preparation of sodium 4-(2,2-dimethylpropanoyl)benzoate (i-1b)

Lithium hydroxide monohydrate (1.50 g, 35.7 mmol) was added to a stirred solution of i-1a (3.20 g, 13.7 mmol) in dioxane/water (20 mL:8.0 mL, respectively) and the resulting mixture was heated to 50 °C. After 1 h, the reaction mixture was cooled to rt, poured into 0.5 N HCl and extracted three times with EtOAc. The combined organic extracts were washed with water and brine, dried (MgSO₄) and concentrated *in vacuo.* The crude residue was suspended in methanol, and sodium methoxide (4.0 mL of 25% wt solution in methanol) was added to the resulting mixture. After 30 min, the volatiles were evaporated *in vacuo* to afford the title compound i-1b, which was used without further purification in the subsequent step.

### Step C: Preparation of 4-{1-[4-(benzyloxy)phenyl]-1-hydroxy-2,2-dimethylpropyl}benzoic acid i-1c)

Lithium chloride (2.00 g, 47.2 mmol) was added to an appropriately sized round bottom flask and then fused under vacuum using a gentle flame source. Magnesium turnings (730 mg, 30.4 mmol), iodine (a few crystals), 1-(benzyloxy)-4-bromobenzene (7.90 g, 30.0 mmol) and THF (30 mL) were added and the resulting mixture was heated at 50 °C until the magnesium metal was consumed. After cooling to rt the resulting solution was added slowly via syringe pump to a stirred solution of i-1b (3.30 g, 14.5 mmol) in THF (100 mL) at 0 °C. After approximately 3 h, the reaction mixture was poured into 1 N HCl and extracted three times with EtOAc. The combined organic extracts were washed with water and brine, dried (MgSO₄) and concentrated *in vacuo* to give the title compound i-1c, which was used without further purification in the subsequent step. ¹HNMR (500 MHz, CDCl₃): δ 8.04 (d, 2H, *J* = 8.6 Hz), 7.63 (d, 2H, *J* = 8.6 Hz), 7.50 (d, 2H, *J* = 9.0 Hz), 7.52-7.36 (m, 5H), 6.93 (d, 2H, *J* = 9.0 Hz) 5.08 (s, 2H), 1.22(s, 9H).

### Step D: Preparation of methyl 4-{1-[4-(benzyloxy)phenyl]-1-hydroxy-2,2-dimethylpropyl}-benzoate (i-1d)

Cesium carbonate (5.70 g, 17.5 mmol) and iodomethane (2.70 mL, 43.4 mmol) were added to a solution of i-1c (5.70 g, 14.6 mmol) in DMF (70 mL). After approximately 2 h, the reaction mixture was quenched by the addition of satd. aq. ammonium chloride. The resulting mixture was poured into water and extracted three times with EtOAc. The combined organic extracts were washed with water and brine, dried (MgSO₄) and concentrated *in vacuo.* Purification of the crude residue by flash chromatography on silica gel (gradient elution; 0-10% EtOAc/hexanes as eluent) afforded the title compound i-1d. ¹HNMR (500 MHz, CDCl₃): δ 7.94 (d, 2H, *J* = 8.6 Hz), 7.58 (d, 2H, *J* = 8.6 Hz), 7.47 (d, 2H, *J* = 9.0 Hz), 7.47-7.34 (m, 5H), 6.90 (d, 2H, *J* = 9.0 Hz), 5.05 (s, 2H), 3.92 (s, 3H), 1.18 (s, 9H).

### Step E: Preparation of methyl 4-[1-(4-hydroxyphenyl)-2,2-dimethylpropyl]benzoate (i-1e)

A mixture of i-1d (2.60 g, 6.70 mmol) and palladium hydroxide (800 mg of 20 wt. % on activated carbon) in ethanol (60 mL) was hydrogenated at atmospheric pressure. After 72 h, the reaction mixture was filtered through a short column of Celite^{®}, eluting copiously with DCM. The filtrate was concentrated *in vacuo* and the crude residue was purified by flash chromatography on silica gel (gradient elution; 5-20% EtOAc/hexanes as eluent) to afford the title compound i-1e. ¹HNMR (500 MHz, CDCl₃): δ 7.96 (d, 2H, *J* = 8.4 Hz), 7.49 (d, 2H, *J* = 8.4 Hz), 7.30 (d, 2H, *J* = 8.5 Hz), 6.78 (d, 2H, *J* = 8.5 Hz), 3.92 (s, 3H), 3.75 (s, 1H), 1.03 (s, 9H).

### Step F: Preparation of (i-1f) and (i-1g)

Enantiomers i-1f and i-1g were separated using preparative supercritical fluid chromatography. A solution of i-1e (1.8 g) in methanol (9 mL) was injected (9 × 1 mL) onto a Chiralpak^{®} AD (available from Chiral Technologies, Inc., Exton, Pa.) semi-preparative (250 × 20 mm) HPLC column (eluting with 40% methanol/CO₂ at 50 mL/min, 100 bar outlet pressure with UV detection at 220 nm). The enantiomers were separated with the faster eluting enantiomer i-1f having a retention time of∼3.25 min and the slower eluting enantiomer i-1g having a retention time of∼4.90 min. The respective fractions were concentrated to provide the enantiomers i-1f (α_{D} +9.21° (c = 0.01, chloroform)) and i-1g (α_{D} -10.2° (c = 0.01, chloroform)).

### Step G: Preparation of methyl 4-{2,2-dimethyl-l-[4-(pyridin-2-ylmethoxy)phenyl]propyl}-benzoate (i-1i)

Cesium carbonate (2.10 g, 6.45 mmol), potassium iodide (490 mg, 2.95 mmol), and 2-picolyl chloride hydrochloride (460 mg, 2.80 mmol) were added to a stirred solution of i-1g (800 mg, 2.68 mmol) in DMF (25.0 mL). After approximately 18 h, the reaction mixture was quenched by the addition of satd. aq. ammonium chloride. The resulting mixture was poured into water and extracted three times with EtOAc. The combined organic extracts were washed with sat. aq. sodium bicarbonate, water and brine, dried (MgSO₄) and concentrated *in vacuo* to afford the title compound i-1i (α_{D} -4.80°, c = 0.01, chloroform). ¹HNMR (500 MHz, CDCl₃): δ 8.59 (d, 1H, *J* = 4.3 Hz), 7.94 (d, 2H, *J* = 8.3 Hz), 7.72 (dt, 1H, *J* = 1.8, 7.8 Hz), 7.52 (d, 1H, *J* = 8.0 Hz), 7.49 (d, 2H, *J* = 8.3 Hz), 7.34 (d, 2H, *J* = 8.8 Hz), 7.23 (dd, 1H, *J* = 5.1, 7.4 Hz), 6.92 (d, 2H, *J* = 8.8 Hz), 5.19 (s, 2H) 3.90 (s, 3H), 3.75 (s, 1H), 1.02 (s, 9H). In a similar manner, intermediate i-1f was converted to i-1h (α_{D} +7.70°, c = 0.01, chloroform).

### (2) Preparation of i-2e and i-2f (Scheme i-2)

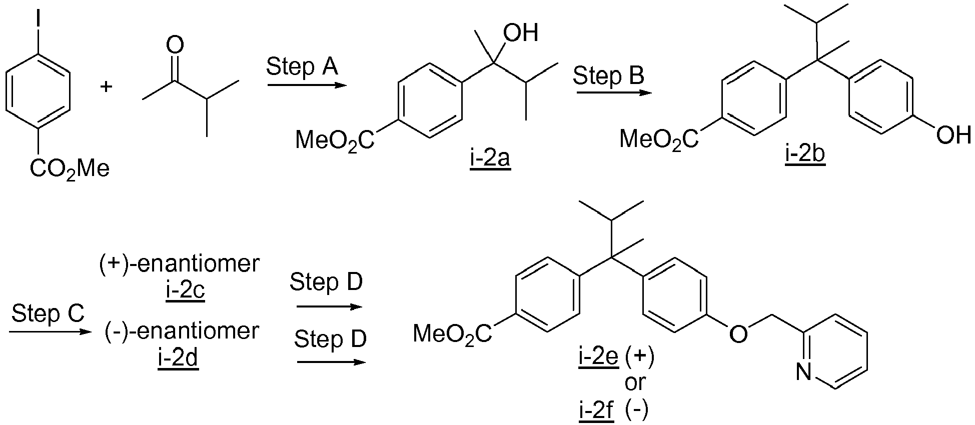

### Step A: Preparation of methyl 4-(1-hydroxy-1,2-dimethylpropyl)benzoate (i-2a)

Isopropyl magnesium chloride (12.0 mL of a 2 M solution in THF, 24.0 mmol) was added to a solution of methyl 4-iodobenzoate (5.24 g, 20.0 mmol) in THF (50 mL) at -40 °C. After 1 h, a second portion of isopropyl magnesium chloride (5.00 mL of a 2 M solution in THF, 10.0 mmol) was added and the resulting mixture allowed to stir at -40 °C for 4 h. 3-Methyl-2-butanone (2.10 mL, 19.6 mmol) was then added and the resulting mixture allowed to warm to rt overnight. The reaction mixture was poured into 1 N HCl and extracted three times with EtOAc. The combined organic extracts were washed water and brine, dried (MgSO₄) and concentrated *in vacuo.* Purification of the crude residue by flash chromatography on silica gel (gradient elution; 0%-20% EtOAc/hexanes as eluent) afforded the title compound i-2a. ¹HNMR (500 MHz, CDCl₃): δ 8.02 (d, 2H, *J* = 8.5 Hz), 7.52 (d, 2H, *J* = 8.5 Hz), 3.95 (s, 3H), 2.05 (p, 1H, *J* = 6.6 Hz), 1.57 (s, 3H), 0.95 (d, 3H, *J* = 6.6 Hz), 0.80 (d, 3H, *J* = 6.9 Hz).

### Step B: Preparation of methyl 4-[1-(4-hydroxyphenyl)-1,2-dimethylpropyl]benzoate (i-2b)

*p*-TSA (600 mg, 3.15 mmol), phenol (900 mg, 9.54 mmol) and i-2a (1.41 g, 6.35 mmol) were added to a preheated round-bottom flask at 95 °C, and the resulting mixture was then heated to 120 °C for 2.0 h. After cooling to rt, the crude residue was purified by flash chromatography on silica gel (gradient elution; 0%-10% EtOAc/hexanes as eluent) to afford the title compound i-2b. ¹HNMR (500 MHz, CDCl₃): δ 7.93 (d, 2H, *J* = 8.5 Hz), 7.33 (d, 2H, *J* = 8.5 Hz), 7.10 (d, 2H, *J* = 8.7 Hz), 6.74 (d, 2H, *J* = 8.7 Hz), 3.91 (s, 3H), 2.70 (p, 1H, *J* = 6.7 Hz), 1.61 (s, 3H), 0.87 (d, 3H, *J* = 6.7 Hz), 0.83 (d, 3H, *J* = 6.7 Hz).

### Step C: Preparation of (i-2c) and (i-2d)

Enantiomers i-2c and i-2d were separated using preparative normal phase chiral HPLC. A solution of i-2b (360 mg) in isopropanol:heptane (4.5 mL of a 1:4 mixture) was injected (9 × 0.5 mL) onto a Chiralpak® AD (available from Chiral Technologies, Inc., Exton, Pa.) semi-preparative (250 × 20 mm) HPLC column (eluting with 30% isopropanol/heptanes at 9 mL/min with UV detection at 254 nm). The enantiomers were separated with the faster eluting enantiomer i-2c having a retention time of 18.9 min and the slower eluting enantiomer i-2d having a retention time of 21.6 min. The respective fractions were concentrated to provide the enantiomers i-2c and i-2d.

### Step D: Preparation of methyl 4-{1,2-dimethyl-1-[4-(pyridin-2-ylmethoxy)phenyl]propyl}-benzoate (i-2e)

Intermediate i-2e was prepared following procedures similar to those described for the preparation of i-1i, substituting i-2c for i-1g. *m*/z (ES) 390 (MH)⁺. ¹HNMR (500 MHz, CDCl₃): δ 8.61 (d, 1H, *J* = 4.6 Hz), 7.93 (d, 2H, *J* = 8.7 Hz), 7.74 (t, 1H, *J* = 7.7 Hz), 7.55 (d, 1H, *J* = 7.8 Hz), 7.33 (d, 2H, *J* = 8.7 Hz), 7.23 (m, 1H), 7.16 (d, 2H, *J* = 8.7 Hz), 6.90 (d, 2H, *J* = 8.7 Hz), 5.20 (s, 2H), 3.90 (s, 3H), 2.71 (p, 1H, *J* = 6.6 Hz), 1.62 (s, 3H), 0.87 (d, 3H, *J* = 6.6 Hz), 0.83 (d, 3H, *J* = 6.6 Hz).
In a similar manner, intermediate i-2d was converted to i-2f.

### (3) Preparation of i-3d (Scheme i-3)

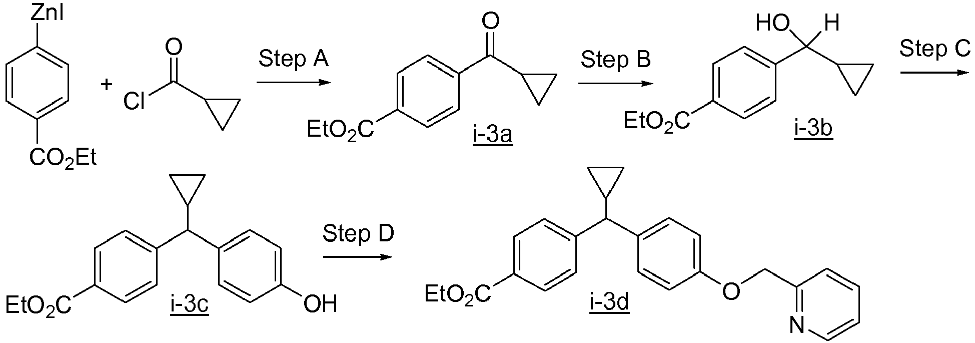

### Step A: Preparation of ethyl 4-(cyclopropylcarbonyl)benzoate (i-3a)

Intermediate i-3a was prepared from 4-ethoxycarbonylphenyl zinc iodide and cyclopropanecarbonyl chloride following the above procedure as described previously in the preparation of intermediate i-1a. *m*/*z* (ES) 219 (MH)⁺. ¹HNMR (500 MHz, CDCl₃): δ 8.17 (d, 2H, *J* = 8.5 Hz), 8.08 (d, 2H, *J* = 8.4 Hz), 4.44 (q, 2H, *J* = 7.1 Hz), 2.71 (m, 1H), 1.45 (t, 3H, *J* = 7.1 Hz),1.31 (m, 2H,), 1.13 (m, 2H).

### Step B: Preparation of ethyl 4-[cyclopropyl(hydroxyl)methyl]benzoate (i-3b)

Sodium borohydride (107 mg, 2.82 mmol) was added in several portions to a stirred solution of i-3a (1.23 g, 5.64 mmol) in ethanol (30 mL) at rt. After 2 h, an additional portion of sodium borohydride (75.0 mg, 1.98 mmol) was added. After 1 h, the volatiles were removed *in vacuo,* and the crude residue partitioned between EtOAc and 0.5N HCl. The organic phase was separated, washed with brine, dried (Na₂SO₄) and concentrated *in vacuo.* Purification of the crude residue by flash chromatography on silica gel (gradient elution; 10%-25% EtOAc/hexanes as eluent) afforded the title compound i-3b. ¹HNMR (500 MHz, CDCl₃): δ 8.06 (d, 2H, *J* = 8.2 Hz), 7.53 (d, 2H, *J* = 8.3 Hz), 4.41 (q, 2H, *J* = 7.1 Hz), 4.11 (d, 1H, *J* = 8.2 Hz), 1.42 (t, 3H, *J* = 7.1 Hz), 1.23 (m, 1H), 0.68 (m, 1H), 0.62 (m, 1H), 0.51 (m, 1H), 0.46 (m, 1H).

### Step C: Preparation of ethyl 4-[cyclopropyl(4-hydroxyphenyl)methyl]benzoate (i-3c)

Intermediate i-3c was prepared following the procedure as described for the preparation of intermediate i-2b, substituting i-3b for i-2a. *m*/*z* (ES) 297 (MH)⁺.

### Step D: Preparation of ethyl 4-{cyclopropyl[4-(pyridin-2-ylmethoxy)phenyl]methyl} - benzoate (i-3d)

Intermediate i-3d was prepared following the procedure as described for the preparation of i-1i, substituting i-3c for intermediate i-1g. *m*/*z* (ES) 388 (MH)⁺. ¹HNMR (500 MHz, CDCl₃): δ 8.62 (d, 1H, *J* = 4.8 Hz), 7.99 (d, 2H, *J* = 8.3 Hz), 7.74 (dt, 1H, *J* = 1.8, 7.8 Hz), 7.56 (d, 1H, *J* = 7.7 Hz), 7.34 (d, 2H, *J* = 8.2 Hz), 7.25 (dd, 1H, *J* = 5.4, 7.0 Hz), 7.19 (d, 2H, *J* = 8.7 Hz), 6.95 (d, 2H, *J* = 8.7 Hz), 5.21 (s, 2H), 4.39 (q, 2H, *J* = 7.1 Hz), 3.24 (d, 1H, *J* = 9.4 Hz), 1.40 (t, 3H, *J* = 7.1 Hz), 1.39 (m, 1H), 0.69 (m, 2H), 0.31 (m, 2H).

### (4) Preparation of ethyl 4-{2-methyl-1-[4-(pyridin-2-ylmethoxy)phenyl]propyl}benzoate (i-4a)

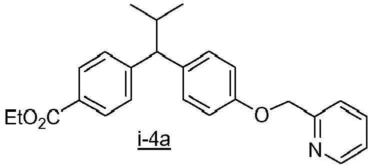

Intermediate i-4a was prepared using isobutyryl chloride in place of cyclopropanecarbonyl chloride following the procedures as described in scheme i-3, Step A through D. *m*/*z* (ES) 390 (MH)⁺. ¹HNMR (500 MHz, CDCl₃): δ 8.60 (d, 1H, *J* = 5.2 Hz), 7.96 (d, 2H, *J* = 8.5 Hz), 7.72 (dt, 1H, *J* = 1.6, 7.6 Hz), 7.53 (d, 1H, *J* = 7.7 Hz), 7.35 (d, 2H, *J* = 8.3 Hz), 7.24 (m, 1H), 7.20 (d, 2H, *J* = 8.7 Hz), 6.92 (d, 2H, *J* = 8.7 Hz), 5.18 (s, 2H), 4.37 (q, 2H, *J* = 7.1 Hz), 3.45 (d, 1H, *J* = 10.7 Hz), 2.48 (m, 1H), 1.29 (t, 3H, *J* = 7.1 Hz), 0.91 (d, 3H, *J* = 6.7 Hz), 0.88 (d, 3H, *J*= 6.4 Hz).

### (5) Preparation of i-5d (Scheme i-5)

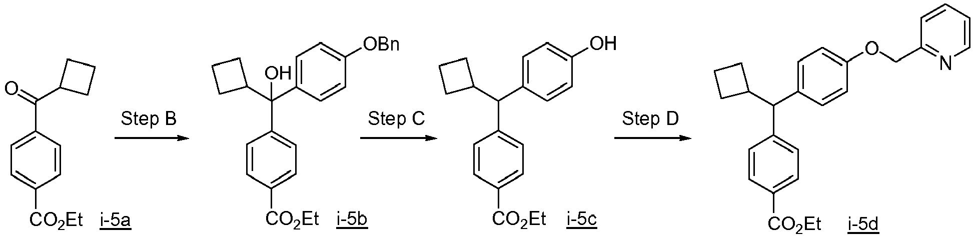

### Step A: Preparation of ethyl 4-(cyclobutylcarbonyl)benzoate (i-5a)

Intermediate i-5a was prepared following procedures as described for the preparation of i-1a, substituting cyclobutanecarbonyl chloride for trimethylacetyl chloride. ¹HNMR (500 MHz, CDCl₃): δ 8.13 (d, 2H, *J* = 8.2 Hz), 7.96 (d, 2H, *J* = 8.3 Hz), 4.43 (q, 2H, *J* = 7.1 Hz), 4.04 (dt, 1H, *J* = 6.9, 7.4 Hz), 2.44 (m, 2H), 2.34 (m, 2H), 2.14 (m, 1H), 1.96 (m, 1H), 1.43 (t, 3H, *J* = 7.0 Hz).

### Step B: Preparation of ethyl 4-{[4-(benzyloxy)phenyl] (cyclobutyl)hydroxymethyl} benzoate (i-5b)

A stirred mixture of magnesium turnings (80.0 mg, 3.33 mmol), iodine (a few crystals) and 1-(benzyloxy)-4-bromobenzene (873 mg, 3.32 mmol) in THF (10 mL) was heated at reflux until the magnesium metal was consumed. The resulting mixture was cooled to rt and added dropwise to a stirred solution of i-5a (774 mg, 3.33 mmol) in THF (5.0 mL) at 0 °C. After approximately 5 h, the reaction mixture was poured into 0.5 N HCl and extracted twice with EtOAc. The combined organic extracts were washed with brine, dried (Na₂SO₄) and concentrated *in vacuo.* Purification of the crude residue by flash chromatography on silica gel (isocratic elution; 10% EtOAc/hexanes as eluent) afforded the title compound i-5b. *m*/*z* (ES) 399 (M-OH)⁺. ¹HNMR (500 MHz, CDCl₃): δ 7.96 (d, 2H, *J* = 8.4 Hz), 7.45 (m, 7H), 7.28 (d, 2H, *J* = 8.7 Hz), 6.91 (d, 2H, *J* = 8.9 Hz), 5.05 (s, 2H), 4.37 (q, 2H, *J* = 7.1 Hz), 3.39 (dt, 1H, *J* = 7.2, 8.3 Hz), 2.10 (m, 3H), 1.87 (m, 1H), 1.72 (m, 2H), 1.39 (t, 3H, *J* = 7.0 Hz).

### Step C: Preparation of ethyl 4-[cyclobutyl(4-hydroxyphenyl)methyl]benzoate (i-5c)

A mixture of i-5b (386 mg, 0.927 mmol) and palladium hydroxide (50.0 mg of 20 wt. % on activated carbon) in methanol (10 mL) was hydrogenated at atmospheric pressure for 9 h. The resulting mixture was filtered through a short column of Celite^{®}, eluting copiously with EtOAc. The filtrate was concentrated *in vacuo* and the crude residue purified by flash chromatography on silica gel (gradient elution; 10%-15% EtOAc/hexanes as eluent) to afford the title compound i-5c. ¹HNMR (500 MHz, CDCl₃): δ 7.96 (d, 2H, *J* = 8.3 Hz), 7.26 (d, 2H, *J* = 8.2 Hz), 7.06 (d, 2H, *J* = 8.7 Hz), 6.76 (d, 2H, *J* = 8.4 Hz), 4.37 (q, 2H, *J* = 7.1 Hz), 3.88 (d, 1H, *J* = 11 Hz), 3.03 (dt, 1H, *J* = 8.1, 11 Hz), 2.05 (m, 2H), 1.90 (m, 2H), 1.75 (m, 2H), 1.39 (t, 3H, *J* = 7.1 Hz).

### Step D: Preparation of ethyl 4- {cyclobutyl[4-(pyridin-2-ylmethoxy)phenyl]methyl} - benzoate (i-5d)

Intermediate i-5d was prepared following procedures as described for the preparation of i-1i, substituting i-5c for i-1g. *m*/*z* (ES) 402 (MH)⁺. ¹HNMR (500 MHz, CDCl₃): δ 8.61 (d, 1H, J = 4.8 Hz), 7.95 (d, 2H, *J* = 8.2 Hz), 7.73 (dt, 1H, *J* = 1.7, 7.7 Hz), 7.54 (d, 1H, *J* = 8.0 Hz), 7.26 (d, 2H, *J* = 8.3 Hz), 7.24 (m, 1H), 7.11 (d, 2H, *J* = 8.6 Hz), 6.92 (d, 2H, *J* = 8.7 Hz), 5.19 (s, 2H), 4.37 (q, 2H, *J* = 7.1 Hz), 3.89 (d, 1H, *J* = 11 Hz), 3.03 (dt, 1H, *J* = 8.1, 11 Hz), 2.07 (m, 1H), 2.02 (m, 1H), 1.88 (m, 2H), 1.78 (m, 2H), 1.39 (t, 3H, *J* = 7.1 Hz).

### (6) Preparation of i-6d (Scheme i-6)

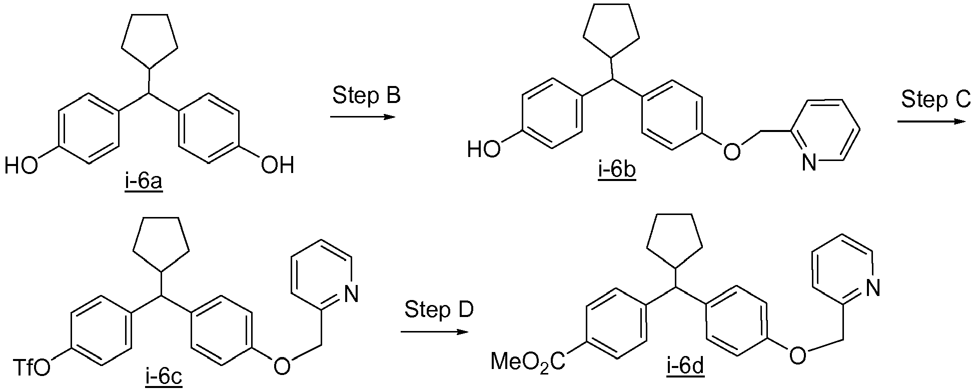

### Step A: Preparation of 4,4'-(cyclopentylmethylene)diphenol (i-6a)

Chlorotrimethylsilane (1.50 mL, 11.8 mmol) was added to a stirred solution of cyclopentanecarboxaldehyde (1.00 g, 10.2 mmol), phenol (2.90 g, 30.8 mmol) and 3-mercaptopropionic acid (87.0 µL, 1.00 mmol), and the resulting mixture was heated to 65 °C. After 2 h, the reaction mixture was cooled to rt, poured into 0.1N HCl and extracted three times with EtOAc. The combined organic extracts were washed with water and brine, dried (Na₂SO₄) and concentrated *in vacuo.* Purification of the crude residue by flash chromatography on silica gel (gradient elution; 10%-20% EtOAc/hexanes as eluent) afforded the title compound i-6a. ¹HNMR (500 MHz, CDCl₃): δ 7.14 (d, 4H, *J* = 8.5 Hz), 6.73 (d, 4H, *J* = 8.7 Hz), 3.47 (d, 1H, *J* = 11.2 Hz), 2.58 (m, 1H), 1.64 (m, 4H), 1.55 (m, 2H), 1.14 (m, 2H).

### Step B: Preparation of 4-{cyclopentyl[4-(pyridin-2-ylmethoxy)phenyl]methyl}phenol (i-6b)

Cesium carbonate (2.20 g, 6.75 mmol), potassium iodide (1.10 g, 6.63 mmol) and 2-picolyl chloride hydrochloride (1.10 g, 6.71 mmol) were added to a stirred solution of i-6a (1.80 g, 6.71 mmol) in DMF (25 mL). After approximately 20 h, the reaction was quenched by the addition of satd. aq. ammonium chloride. The resulting mixture was extracted three times with EtOAc. The combined organic extracts were washed with water and brine, dried (Na₂SO₄) and concentrated *in vacuo.* Purification of the crude residue by flash chromatography on silica gel (gradient elution; 15%-40% EtOAc/hexanes as eluent) afforded the title compound i-6b. *m*/*z* (ES) 360 (MH)⁺. ¹HNMR (500 MHz, CDCl₃): δ 8.60 (d, 1H, *J* = 4.9 Hz), 7.75 (m, 1H), 7.56 (d, 1H, *J* = 8.0 Hz), 7.26 (m, 1H), 7.18 (d, 2H, *J* = 8.7 Hz), 7.13 (d, 2H, *J* = 8.7 Hz), 6.89 (d, 2H, *J* = 8.4 Hz), 6.73 (d, 2H, *J* = 8.4 Hz), 5.19 (s, 2H), 3.47 (d, 1H, *J* = 11 Hz), 2.60 (m, 1H), 1.50-1.70 (m, 6H), 1.14 (m, 2H).

### Step C: Preparation of 4-{cyclopentyl[4-(pyridin-2-ylmethoxy)phenyl]methyl}phenyl trifluoromethanesulfonate (i-6c)

Lithium bis(trimethylsilyl)amide (2.20 mL of a 1.0 M solution in THF, 2.20 mmol) was added to a stirred solution of i-6b (650 mg, 1.81 mmol) in THF (18 mL) at 0 °C. After 5 min, *N*-phenyltrifluoromethanesulfonimide (790 mg, 2.21 mmol) was added, and the resulting mixture stirred at 0 °C for 20 min. The reaction mixture was poured into water and extracted three times with EtOAc. The combined organic extracts were washed with water and brine, dried (Na₂SO₄) and concentrated *in vacuo.* Purification of the crude residue by flash chromatography on silica gel (gradient elution; 10%-30% EtOAc/hexanes as eluent) afforded the title compound i-6c. *m*/*z* (ES) 492 (MH)⁺.

### Step D: Prepartation of methyl 4-{cyclopentyl[4-(pyridin-2-ylmethoxy)phenyl]methyl} - benzoate (i-6d)

Palladium (II) acetate (76.0 mg, 0.339 mmol) and 1,1'-bis(diphenylphosphino)-ferrocene (280 mg, 0.505 mmol) were added successively to a solution of i-6c (840 mg, 1.71 mmol) in triethylamine:DMF:methanol (30 mL of a 1:10:10 mixture, respectively). The reaction mixture was saturated with carbon monoxide and then heated to 80 °C under a carbon monoxide atmosphere (balloon) for 16 h. After cooling to rt, the reaction mixture was poured into 0.1 N HCl (aq) and extracted three times with EtOAc. The combined organic extracts were washed with water, brine, dried (Na₂SO₄) and concentrated *in vacuo.* Purification of the crude residue by flash chromatography on silica gel (gradient elution; 10%-30% EtOAc/hexanes as eluent) afforded the title compound i-6d. *m*/*z* (ES) 492 (MH)⁺. ¹HNMR (500 MHz, CDCl₃): δ 8.64 (d, 1H, *J* = 3.9 Hz),7.93 (d, 2H, *J* = 8.5 Hz), 7.91 (m, 1H), 7.69 (d, 1H, *J* = 7.8 Hz), 7.40 (m, 1H), 7.34 (d, 2H, *J* = 8.3 Hz), 7.21 (d, 2H, *J* = 8.4 Hz), 6.92 (d, 2H, *J* = 8.7 Hz), 5.32 (s, 2H), 3.89 (s, 3H), 3.60 (d, 1H, *J* = 11.2 Hz), 2.68 (m, 1H), 1.53-1.72 (m, 6H), 1.16 (m, 2H).

### (7) Preparation of i-7a and i-7b (Scheme i-7)

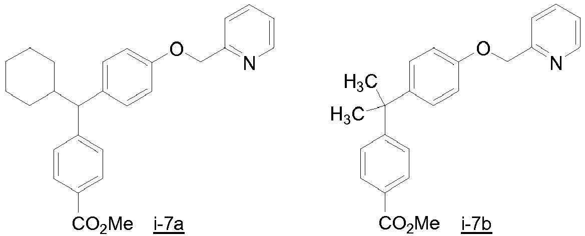

i-7a and i-7b were prepared following the procedures as described in scheme i-6, Steps A through D, substituting cyclohexanecarboxaldehyde and acetone, respectively, in place of cyclopentanecarboxaldehyde for the preparation of intermediate i-6a.

Intermediate i-7a: *m*/*z* (ES) 416 (MH)⁺. ¹HNMR (500 MHz, CDCl₃): δ 8.63 (d, 1H, *J* = 2.8 Hz), 7.94 (d, 2H, *J* = 8.3 Hz), 7.88 (dd, 1 H, J = 7.6, 7.7 Hz), 7.67 (d, 1H, *J* = 8.0 Hz), 7.38 (dd, 1H, *J* = 5.2, 6.7 Hz), 7.33 (d, 2H, *J* = 8.5 Hz), 7.20 (d, 2H, *J* = 8.5 Hz), 6.92 (d, 2H, *J* = 8.4 Hz), 5.30 (s, 2H), 3.88 (s, 3H), 3.51 (d, 1H, *J* = 10.7 Hz), 2.09 (m, 1H), 1.66 (m, 4H), 1.20 (m, 4H), 0.87 (m, 2H).

Intermediate i-7b: *m*/*z* (ES) 362 (MH)⁺. ¹HNMR (500 MHz, CDCl₃): δ 8.62 (d, 1H, *J* = 4.4 Hz), 7.95 (d, 2H, *J* = 8.5 Hz), 7.76 (dt, 1H, *J* = 1.4, 7. 8 Hz), 7.57 (d, 1H, *J* = 7.8 Hz), 7.32 (d, 2H, *J* = 8.5 Hz), 7.26 (dd, 1H, *J* = 5.9, 6.0 Hz), 7.15 (d, 2H, *J* = 8.9 Hz), 6.93 (d, 2H, *J* = 8.9 Hz), 5.22 (s, 2H), 3.92 (s, 3H), 1.70 (s, 6H).

### (8) Preparation of i-8d (Scheme i-8)

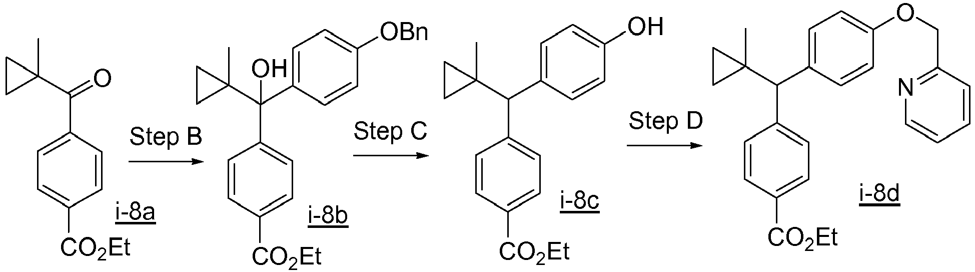

### Step A: Preparation of ethyl 4-[(1-methylcyclopropyl)carbonyl]benzoate (i-8a)

Lithium bis(trimethylsilyl)amide (8.60 mL of a 1.0 M solution in THF, 8.60 mmol) was added dropwise to a stirred solution of i-3a (1.70 g, 7.79 mmol) in THF (40 mL) at -78 °C. After 10 min, iodomethane (0.590 mL, 9.48 mmol) was added, and the resulting mixture was allowed to warm to rt over 4 h. After another 10 h, the reaction mixture was quenched by addition of satd. aq. ammonium chloride and extracted twice with EtOAc. The combined organic extracts were washed with 1 N HCl and brine, dried (Na₂SO₄) and concentrated *in vacuo.* Purification of the crude residue by flash chromatography on silica gel (gradient elution; 0%-10% EtOAc/hexanes as eluent) afforded the title compound i-8a. *m*/*z* (ES) 233 (MH)⁺. ¹HNMR (500 MHz, CDCl₃): δ 8.13 (d, 2H, *J* = 8.2 Hz), 7.80 (d, 2H, *J* = 8.2 Hz), 4.43 (q, 2H, *J* = 7.1 Hz), 1.45 (s, 3H), 1.44 (t, 3H, *J* = 7.1 Hz), 1.35 (m, 2H), 0.87 (m, 2H).

### Steps B - D: Preparation of ethyl 4-{(1-methylcyclopropyl)[4-(pyridin-2-ylmethoxy)phenyl]-methyl}benzoate (i-8d)

Intermediates i-8b, i-8c and i-8d were prepared following the above procedures as described in scheme i-5, substituting i-8a for intermediate i-5a. For i-8d: *m*/*z* (ES) 402 (MH)⁺. ¹HNMR (500 MHz, CDCl₃): δ 8.61 (d, 1H, *J* = 4.6 Hz), 7.97 (d, 2H, *J* = 8.4 Hz), 7.73 (dt, 1H, *J* = 1.6, 7.8 Hz), 7.57 (d, 1H, *J* = 7.8 Hz), 7.34 (d, 2H, *J* = 8.5 Hz), 7.26 (m, 1H), 7.14 (d, 2H, *J* = 8.7 Hz), 6.92 (d, 2H, *J* = 8.7 Hz), 5.22 (s, 2H), 4.38 (q, 2H, *J* = 7.1 Hz), 3.83 (s, 1H), 1.41 (t, 3H, *J* = 7.1 Hz), 1.09 (s, 3H), 0.46 (m, 4H).

### (9) Preparation of ethyl 4-{(1-methylcyclobutyl)[4-(pyridin-2-ylmethoxy)phenyl]methyl}benzoate (i-9a)

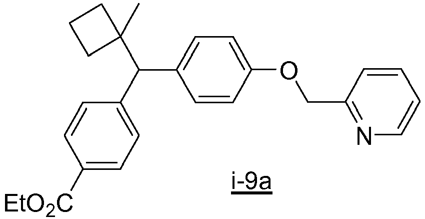

Intermediate i-9a was prepared from i-5a following the procedures as described in scheme i-8, substituting i-5a for i-3a. For i-9a: *m*/*z* (ES) 416 (MH)⁺. ¹HNMR (500 MHz, CDCl₃): δ 8.61 (d, 1H, *J*= 5.6 Hz), 7.96 (d, 2H, *J*= 8.2 Hz), 7.74 (dt, 1H, *J* = 1.8, 7.7 Hz), 7.56 (d, 1H, *J* = 8.1 Hz), 7.28 (d, 2H, *J* = 8.5 Hz), 7.25 (m, 1H), 7.12 (d, 2H, *J*= 8.7 Hz), 6.93 (d, 2H, *J* = 8.7 Hz), 5.21 (s, 2H), 4.38 (q, 2H, *J* = 7.1 Hz), 4.01 (s, 1H), 2.21 (m, 2H), 1.99 (m, 1H), 1.76 (m, 1H), 1.63 (m, 2H), 1.40 (t, 3H, *J* = 7.1 Hz), 1.29 (s, 3H).

### (10) Preparation of i-10d (Scheme i-10)

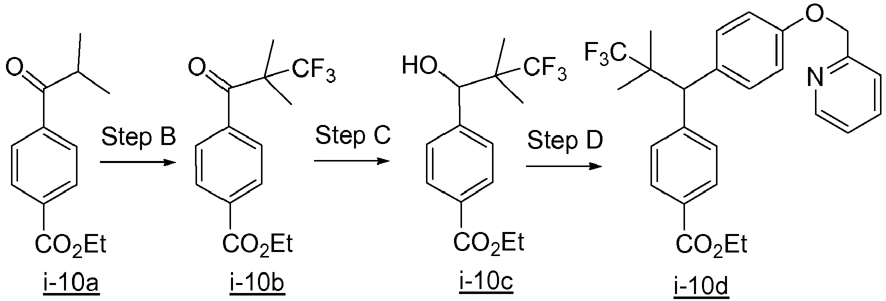

### Step A: Preparation of ethyl 4-isobutyrylbenzoate (i-10a)

Intermediate i-10a was prepared following the procedure as described for the preparation of i-1a, substituting isobutyryl chloride for trimethylacetyl chloride. For i-10a: *m*/*z* (ES) 221 (MH)⁺. ¹HNMR (500 MHz, CDCl₃): δ 8.14 (d, 2H, *J* = 8.5 Hz), 8.01 (d, 2H, *J* = 8.4 Hz), 4.43 (q, 2H, *J*= 7.0 Hz), 3.57 (hept., 1H, *J* = 6.8 Hz), 1.43 (t, 3H, *J* = 7.1 Hz), 1.24 (d, 6H, *J* = 6.9 Hz).

### Step B: Preparation of ethyl 4-(3,3,3-trifluoro-2,2-dimethylpropanoyl)benzoate (i-10b)

Sodium hexamethyldisilazide (3.0 mL of a 1.0 M solution in THF, 3.0 mmol) was added to a stirred solution of i-10a (0.51 g, 2.3 mmol) in THF (18 mL) at -78 °C. After 10 min, the reaction mixture was warmed to 0 °C. After 5 min, the reaction mixture was recooled to -78 °C, and iodotrifluoromethane (excess) was bubbled through the reaction mixture for 15 min. After 1 h, the reaction mixture was quenched with 1N HCl and extracted three times with EtOAc. The combined organic extracts were washed with water and brine, dried (Na₂SO₄) and concentrated *in vacuo.* Purification of the crude residue by flash chromatography on silica gel (gradient elution; 5%-10% EtOAc/hexanes as eluent) afforded the title compound i-10b.

### Step C: Preparation of ethyl 4-(3,3,3-trifluoro-1-hydroxy-2,2-dimethylpropyl)benzoate (i-10c)

Sodium borohydride (3.0 mg, 0.061 mmol) was added to a stirred solution of i-10b (35 mg, 0.12 mmol) in EtOH (1.0 mL) at rt. After 2 h, the reaction mixture was concentrated, and resuspended in EtOAc. The organics were washed with water and brine, dried (Na₂SO₄) and concentrated *in vacuo.* Purification of the crude residue by flash chromatography on silica gel (gradient elution; 5%-20% EtOAc/hexanes as eluent) afforded the title compound i-10c. *m*/*z* (ES) 291 (MH)⁺. ¹HNMR (500 MHz, CDCl₃): δ 8.06 (d, 2H, *J* = 8.3 Hz), 7.46 (d, 2H, *J* = 8.3 Hz), 5.00 (d, 1H, *J* = 2.7 Hz), 4.41 (q, 2H, *J* = 7.1 Hz), 1.43 (t, 3H, *J* = 7.1 Hz), 1.23 (s, 3H), 0.98 (s, 3H).

Step D: Intermediate i-10d was prepared following procedures as described for the preparation of i-2e, substituting i-10c for i-2a. For i-10d: *m*/*z* (ES) 458 (MH)⁺.

### (11) Preparation of i-11b (Scheme i-11)

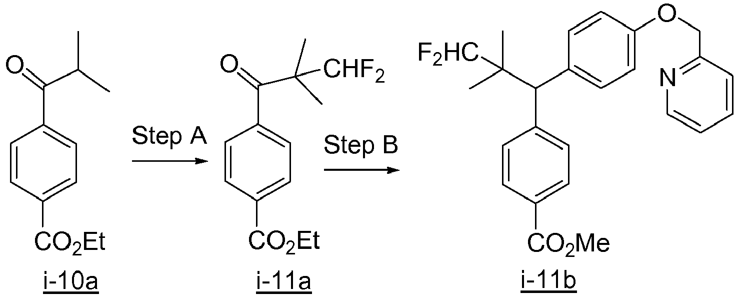

### Step A: Preparation of ethyl 4-(3,3-difluoro-2,2-dimethylpropanoyl)benzoate (i-11a)

Sodium hexamethyldisilazide (1.9 mL of a 1.0 M solution in THF, 1.9 mmol) was added to a stirred solution of i-10a (0.32 g, 1.5 mmol) in THF (10 mL) at -78 °C. After 15 min, chlorodifluoromethane (excess) was condensed into the reaction mixture. After 1 h, the reaction mixture was quenched with 0.5N HCl and extracted three times with EtOAc. The combined organic extracts were washed with water and brine, dried (Na₂SO₄) and concentrated *in vacuo.* Purification of the crude residue by flash chromatography on silica gel (isocratic elution; 5% EtOAc/hexanes as eluent) afforded the title compound i-11a. *m*/*z* (ES) 271 (MH)⁺. ¹HNMR (500 MHz, CDCl₃): δ 8.14 (d, 2H, *J* = 8.4 Hz), 7.68 (d, 2H, *J* = 8.5 Hz), 6.19 (t, 2H, *J* = 56 Hz), 4.44 (q, 2H, *J* = 7.1 Hz), 1.46 (s, 9H), 1.45 (t, 3H, *J* = 7.1 Hz).

Step B: Intermediate i-11b was prepared following the procedures as described in scheme i-1, steps B-E and G, substituting i-11a for i-1a, to afford the title compound i-11b. For i-11b: *m*/*z* (ES) 426 (MH)⁺. ¹HNMR (500 MHz, CDCl₃): δ 8.62 (d, 1H, J = 4.3 Hz), 7.98 (d, 2H, *J* = 8.2 Hz), 7.78 (m, 1H), 7.57 (d, 1H, *J* = 8.2 Hz), 7.52 (d, 2H, *J* = 8.2 Hz), 7.37 (d, 2H, *J* = 8.7 Hz), 7.29 (m, 1H), 6.96 (d, 2H, *J* = 8.7 Hz), 5.37 (t, 2H, *J* = 57 Hz), 5.24 (s, 2H), 4.09 (s, 1H), 3.91 (s, 3H), 1.14 (s, 3H), 1.11 (s, 3H).

Following procedures similar to those described in i-1 through i-11, the following compounds in Table i-11 can be prepared:

**Table i-11**

| | | | | | |
|---|---|---|---|---|---|
| 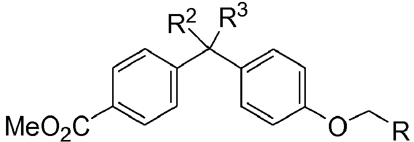 | | | | | |

| Ex. i-11A | Ex. i-11B | Ex. i-11C | Ex. i-11D | R² | R³ |
|---|---|---|---|---|---|
| R=2-PYM | R=2-TZE | R=5-F-2-PYR | R=3-F-2-PYR | | |
| a | a | a | a | ⁱPr | H |
| b | b | b | b | ^{t}Bu | H |
| c | c | c | c | c-Pr | H |
| d | d | d | d | c-Bu | H |
| e | e | e | e | 1-Me-c-Pr | H |
| f | f | f | f | 1-Me-c-Bu | H |
| g | g | g | g | *ⁱ*Pr | Me |
| h | h | h | h | *ⁱ*Pr | OH |
| i | i | i | i | *^{t}*Bu | OH |

Table i-11. Parent Ion *m*/*z* (MH)⁺ data for compounds.
For i-11Ag: methyl 4-{1,2-dimethyl-1-[4-(pyrimidin-2-ylmethoxy)phenyl]propyl}benzoate: *m*/*z* (ES) = 391 (MH)⁺
For i-HBg: methyl 4- {1,2-dimethyl-1-[4-(1,3-thiazol-2-ylmethoxy)phenyl]propyl}benzoate: *m*/*z* (ES) = 396 (MH)⁺

### (12) Preparation of i-12e and i-12f (Scheme i-12)

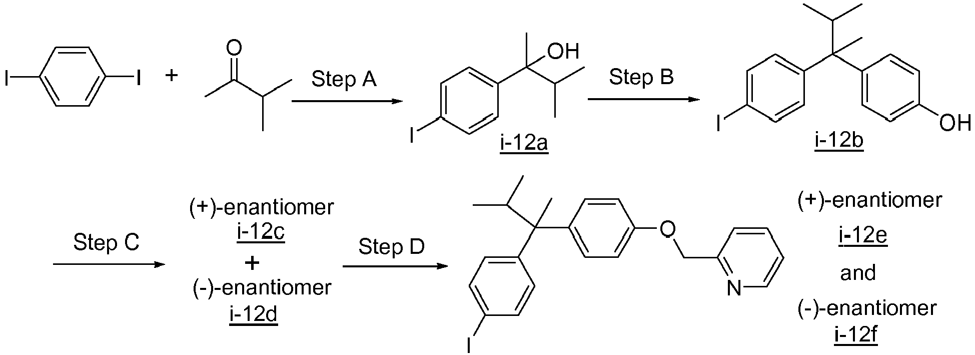

### Step A: Preparation of 2-(4-iodophenyl)-3-methylbutan-2-ol (i-12a)

*n*-Butyl lithium (139 mL of a 2M solution in hexanes, 277 mmol) was added via cannula to a stirred solution 1,4-diiodobenzene (89.0 g , 277 mmol) in THF (500 mL) at -78 °C, such that the internal temperature was maintained below -65 °C during the addition process. After 0.5 h, 3-methyl-2 butanone (24.0 g, 280 mmole) was added via syringe, again maintaining the internal temperature below -65 °C. After 0.5 h, the reaction mixture was warmed to 0 °C and quenched with satd. aq. ammonium chloride. The resulting mixture was dried (MgSO₄), concentrated *in vacuo,* and the crude residue purified by flash chromatography on silica gel (gradient elution; 10-25% EtOAc/hexanes as eluent) to afford the title compound i-12a. ¹H NMR (500 MHz, CDCl₃) δ 7.67 (d, 2H, *J* = 7.5 Hz), 7.20 (d, 2H, *J* = 7.5 Hz), 3.79 (s, 1H), 2.00 (m, 1H), 1.52 (s, 3H), 0.92 (d, 3H, *J* = 7 Hz), 0.81 (d, 3H, *J* = 7 Hz).

### Step B: Preparation of 4-[1-(4-iodophenyl)-1,2-dimethylpropyl]phenol (i-12b)

A mixture of i-12a (56.0 g, 193 mmol), *p*-TSA (36.0 g, 193 mmol) and phenol (25.0 g, 251 mmol) was heated to 95 °C for 1 h. After cooling to rt, the reaction mixture was partitioned between DCM and water. The separated organic phase was washed with water, three times with satd. aq. sodium bicarbonate, and once with brine. The organics were dried (MgSO₄) and concentrated *in vacuo* to afford the title compound i-12b, which was used without further purification in the subsequent step.

### Step C: Preparation of (i-12c) and (i-12d)

Enantiomers i-12c and i-12d were separated using preparative supercritical fluid chromatography. A solution of i-12b (1.8 g) in methanol (9 mL) was injected (9 × 1 mL) onto a Chiralpak^{®} AD-H (available from Chiral Technologies, Inc., Exton, Pa.) semi-preparative (250 × 20 mm) HPLC column (eluting with 30% methanol/CO₂ at 50 mL/min, 100 bar outlet pressure with UV detection at 220 nm). The enantiomers were separated with the faster eluting enantiomer i-12c having a retention time of∼3.25 min and the slower eluting enantiomer i-12d having a retention time of∼4.90 min. The respective fractions were concentrated to provide the enantiomers i-12c (α_{D} +9.21° (c = 0.01, chloroform)) and i-12d (α_{D} -10.2° (c = 0.01, chloroform)).

### Step D: Preparation of 2-({4-[1-(4-iodophenyl)-1,2-dimethylpropyl]phenoxy}methyl)-pyridine (i-12f)

Intermediate i-12f was prepared following procedures as described for the preparation of i-1i, substituting i-12d for i-1g. *m*/*z* (ES) 458 (MH)⁺. ¹HNMR (500 MHz, CDCl₃): δ 8.61 (d, 1H, *J* = 4.8 Hz), 7.74 (dd, 1H, *J* = 6.2, 7.6 Hz), 7.57 (d, 2H, *J* = 8.4 Hz), 7.56 (d, 1H, *J* = 4.4 Hz), 7.24 (m, 1H), 7.15 (d, 2H, *J* = 8.9 Hz), 7.00 (d, 1H, *J* = 8.4 Hz), 6.90 (d, 2H, *J* = 8.9 Hz), 5.20 (s, 2H), 2.63 (m, 1H), 0.84 (d, 3H, *J* = 6.7 Hz), 0.82 (d, 3H, *J* = 6.6 Hz).

Following procedures similar to those described in schemes i-1 through i-12, the following compounds in Table i-12 can be prepared:

**Table i-12**

| | | | | | | |
|---|---|---|---|---|---|---|
| 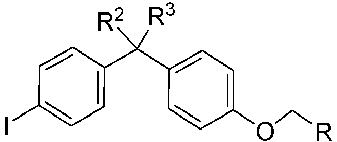 | | | | | | |

| Ex. i-12A | Ex. i-12B | Ex. i-12C | Ex. i-12D | Ex. i-12E | R² | R³ |
|---|---|---|---|---|---|---|
| R=2-PYR | R=2-PYM | R=2-TZE | R=5-F-2-PYR | R=3-F-2-PYR | | |
| a | a | a | a | a | *ⁱ*Pr | H |
| b | b | b | b | b | *^{t}*Bu | H |
| c | c | c | c | c | c-Pr | H |
| d | d | d | d | d | c-Bu | H |
| e | e | e | e | e | 1-Me-c-Pr | H |
| f | f | f | f | f | 1-Me-c-Bu | H |
| -- | g | g | g | g | *ⁱ*Pr | Me |
| h | h | h | h | h | *ⁱ*Pr | OH |
| i | i | i | i | i | Bu | OH |

Table i-12. Parent Ion *m*/*z* (MH)⁺ data for compounds.
For i-12Bg: 2-({4-[1-(4-iodophenyl)-1,2-dimethylpropyl]phenoxy}methyl)pyrimidine: *m*/*z* (ES) = 459 (MH)⁺
For i-12Cg: 2-({4-[1-(4-iodophenyl)-1,2-dimethylpropyl]phenoxy}methyl)thiazole: *m*/*z* (ES) = 464 (MH)⁺
For i-12Dg: 5-fluoro-2-({4-[1-(4-iodophenyl)-1,2-dimethylpropyl]phenoxy}methyl)pyridine: *m*/*z* (ES) = 476 (MH)⁺

### (13) Preparation of i-13a and i-13b (Scheme i-13)

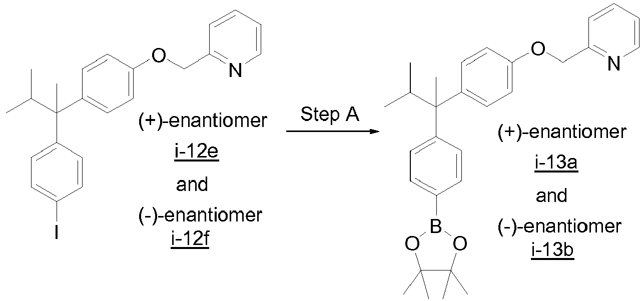

### Step A: Preparation of 2-[(4-{1,2-dimethyl-1-[4-(4,4,5,5-tetramethyl-1,3,2-dioxaborolan-2-yl)phenyl]propyl}phenoxy)methyl]pyridine (i-13b)

Bis(pinacolato)diboron (62 mg, 0.24 mmol), potassium acetate (65 mg, 0.66 mmol) and [1,1'-bis(diphenylphosphino)ferrocene]dichloropalladium(II) (5.0 mg, 0.066 mmol) were added to a degassed solution of i-12f (100 mg, 0.22 mmol) in DMSO (2.0 mL) at rt, and the resulting mixture was heated to 80 °C. After 1 h, the reaction mixture was quenched with water and extracted three times with EtOAc. The combined organic extracts were washed with three times with water and once with brine, dried (Na₂SO₄) and concentrated *in vacuo.* Purification of the crude residue by flash chromatography on silica gel (isocratic elution; 30% EtOAc/hexanes as eluent) afforded the title compound i-13b. *m*/*z* (ES) 458 (MH)⁺.

Following procedures similar to those described in schemes i-1 through i-13, the following compounds in Table i-13 can be prepared:

**Table i-13**

| | | | | | | |
|---|---|---|---|---|---|---|
| 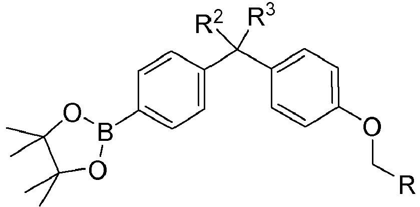 | | | | | | |

| Ex. i-13A | Ex. i-13B | Ex. i-13C | Ex. i-13D | Ex. i-13E | R² | R³ |
|---|---|---|---|---|---|---|
| R=2-PYR | R=2-PYM | R=2-TZE | R=5-F-2-PYR | R=3-F-2-PYR | | |
| a | a | a | a | a | *ⁱ*Pr | H |
| b | b | b | b | b | *^{t}*Bu | H |
| c | c | c | c | c | c-Pr | H |
| d | d | d | d | d | c-Bu | H |
| e | e | e | e | e | 1-Me-c-Pr | H |
| f | f | f | f | f | 1-Me-c-Bu | H |
| -- | g | g | g | g | *ⁱ*Pr | Me |
| h | h | h | h | h | *ⁱ*Pr | OH |
| i | i | i | i | i | *^{t}*Bu | OH |

Table i-13. Parent Ion *m*/*z* (MH)⁺ data for compounds.
For i-13Ab: 2-[(4-{2,2-dimethyl-1-[4-(4,4,5,5-tetramethyl-1,3,2-dioxaborolan-2-yl)phenyl]-propyl}phenoxy)methyl]pyridine: *m*/*z* (ES) = 458 (MH)⁺
For i-13Bg: 2-[(4- {1,2-dimethyl-1-[4-(4,4,5,5-tetramethyl-1,3,2-dioxaborolan-2-yl)phenyl]-propyl}phenoxy)methyl]pyrimidine: *m*/*z* (ES) = 459 (MH)⁺
For i-13Cg: 2-[(4-{2,2-dimethyl-1-[4-(4,4,5,5-tetramethyl-1,3,2-dioxaborolan-2-yl)phenyl]-propyl}phenoxy)methyl]-1,3-thiazole: *m*/*z* (ES) = 464 (MH)⁺

### (14) Preparation of i-14a and i-14b (Scheme i-14)

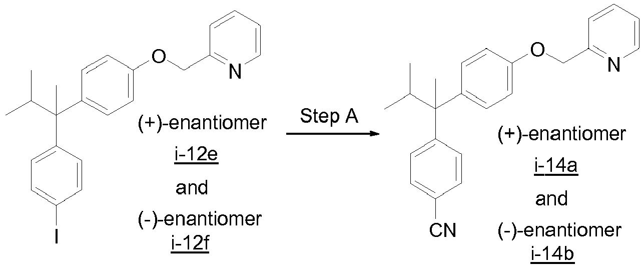

### Step A: Preparation of 4-{1,2-dimethyl-1-[4-(pyridin-2-ylmethoxy)phenyl]propyl}-benzonitrile (i-14b)

Tetrakis(triphenylphosphine) palladium(0) (126 mg, 0.109 mmol), copper (I) iodide (42 mg, 0.219 mmol) and potassium cyanide (285 mg, 4.37 mmol) were added successively to a solution of i-12f (840 mg, 1.71 mmol) in acetonitrile (20 mL). The reaction mixture was degassed and then heated to 85 °C for 2 h. After cooling to rt, the reaction mixture was filtered through celite, and filter plug was rinsed three times with EtOAc. The combined organic extracts were washed with brine, dried (Na₂SO₄) and concentrated *in vacuo.* Purification of the crude residue by flash chromatography on silica gel (gradient elution; 0%-50% EtOAc/hexanes as eluent) afforded the title compound i-14b. *m*/*z* (ES) 357 (MH)⁺. ¹HNMR (500 MHz, CDCl₃): δ 8.64

Following procedures similar to those described in schemes i-1 through i-12 and i-14, the following compounds in Table i-14 can be prepared:

**Table i-14**

| | | | | | | |
|---|---|---|---|---|---|---|
| 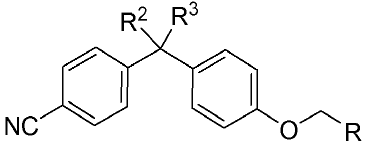 | | | | | | |

| Ex. i-14A | Ex. i-14B | Ex. i-14C | Ex. i-14D | Ex. i-14E | R² | R³ |
|---|---|---|---|---|---|---|
| R=2-PYR | R=2-PYM | R=2-TZE | R=5-F-2-PYR | R=3-F-2-PYR | | |
| a | a | a | a | a | *ⁱ*Pr | H |
| b | b | b | b | b | *^{t}*Bu | H |
| c | c | c | c | c | c-Pr | H |
| d | d | d | d | d | c-Bu | H |
| e | e | e | e | e | 1-Me-c-Pr | H |
| f | f | f | f | f | 1-Me-c-Bu | H |
| -- | g | g | g | g | *ⁱ*Pr | Me |
| h | h | h | h | h | *ⁱ*Pr | OH |
| i | i | i | i | i | *^{t}*Bu | OH |

Table i-14. Parent Ion *m*/*z* (MH)⁺ data for compounds.
For i-14Aa: 4-{2-methyl-1-[4-(pyridin-2-ylmethoxy)phenyl]propyl}benzonitrile: *m*/*z* (ES) = 343 (MH)⁺
For i-14Ab: 4-{2,2-dimethyl-1-[4-(pyrimidin-2-ylmethoxy)phenyl]propyl}benzonitrile: *m*/*z* (ES) = 357 (MH)⁺
For i-14Bg: 4-{1,2-dimethyl-1-[4-(pyrimidin-2-ylmethoxy)phenyl]propyl}benzonitrile: *m*/*z* (ES) = 358 (MH)+
For i-14Cg: 4-{1,2-dimethyl-1-[4-(1,3-thiazol-2-ylmethoxy)phenyl]propyl}benzonitrile: *m*/*z* (ES) = 363 (MH)⁺
For i-14Dg: 4-(1-{4-[(5-fluoropyridin-2-yl)methoxy]phenyl}-1,2-dimethylpropyl)benzonitrile: *m*/*z* (ES) = 375 (MH)⁺

### (15) Preparation of i-15c (Scheme i-15)

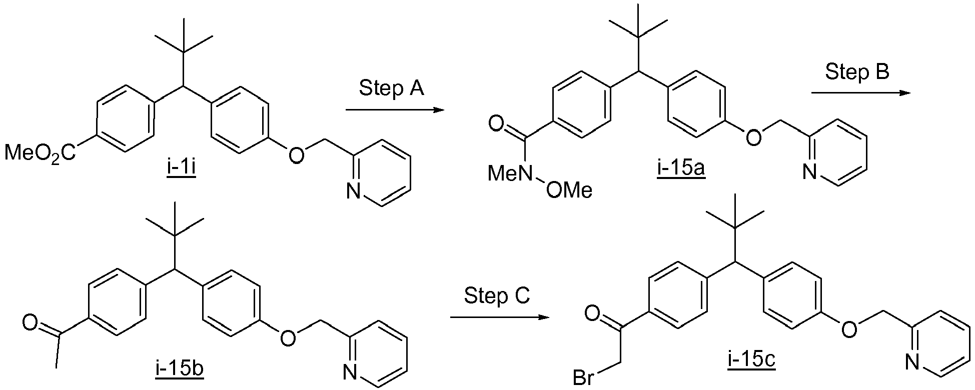

### Step A: Preparation of 4-{2,2-dimethyl-1-[4-(pyridin-2-ylmethoxy)phenyl]propyl}-N-methoxy-N-methylbenzamide (i-15a)

Trimethylaluminum (8.73 mL of a 2.0M solution in toluene, 17.5 mmol) was added to a prestirred solution of *N,O*-dimethylhydroxylamine hydrochloride (1.70 g, 17.5 mmol) in DCM (35 mL) at rt. After 15 min, a solution of i-1i (1.36 g, 3.50 mmol) in DCM (15 ml) was added dropwise. After 16 h, 1N Rochelle's salt solution was added, and after 1 h of vigorous stirring, the mixture was filtered, diluted with water, and extracted three times with EtOAc. The combined organic extracts were washed with brine, dried (MgSO₄), and concentrated *in vacuo.* Purification of the crude residue by flash chromatography on silica gel (gradient elution; 0-80% EtOAc/hexanes as eluent) furnished the title compound i-15a. *m*/*z* (ES) 419 (MH)⁺. ¹HNMR (500 MHz, CD₃OD): δ 8.52 (d, 1H, *J*= 4.6 Hz), 7.84 (dt, 1H, *J*= 1.8, 7.7 Hz), 7.55 (m, 5H), 7.40 (d, 2H, *J*= 8.6 Hz), 7.35 (dd, 1H, *J*= 5.2, 7.1 Hz), 6.93 (d, 2H, *J*= 8.7 Hz), 5.14 (s, 2H), 3.79 (s, 1H), 3.56 (s, 3H), 2.81 (s, 3H), 1.00 (s, 9H).

### Step B: Preparation of 1-(4-{2,2-dimethyl-1-[4-(pyridin-2-ylmethoxy)phenyl]propyl}-phenyl)ethanone (i-15b)

Methyl magnesium bromide (2.90 mL of a 3M solution in diethyl ether, 8.60 mmol) was added to a stirred solution of i-15a (1.20 g, 2.90 mmol) in THF (29 mL) at 0 °C, and the resulting reaction mixture allowed to warm to rt. After 1h, the reaction mixture was quenched with satd. aq. ammonium chloride and extracted three times with EtOAc. The combined organic extracts were washed with brine, dried (MgSO₄), and concentrated *in vacuo.* Purification of the crude residue by flash chromatography on silica gel (gradient elution; 0-40% EtOAc/hexanes as eluent) furnished the title compound i-15b. *m*/*z* (ES) 374 (MH)⁺. ¹HNMR (500 MHz, CDCl₃): δ 8.61 (d, 1H, *J*= 4.3 Hz), 7.89 (d, 2H, *J*= 8.2 Hz), 7.72 (dt, 1H, *J*= 1.8, 7.7 Hz), 7.53 (m, 3H), 7.35 (d, 2H, *J*= 8.7 Hz), 7.24 (dd, 1H, *J*= 5.0, 6.9 Hz), 6.93 (d, 2H, *J*= 8.7 Hz), 5.20 (s, 2H), 3.77 (s, 1H), 2.59 (s, 3H), 1.04 (s, 9H).

### Step C: Preparation of 2-bromo-1-(4-{2,2-dimethyl-1-[4-(pyridin-2-ylmethoxy)phenyl]-propyl}phenyl)ethanone (i-15c)

Pyrrolidone hydrotribromide (0.44 mmol) was added to a stirred solution of i-15b (151 mg, 0.400 mmol) in THF (4 mL), and the resulting mixture was heated to 60 °C. After 0.5 h, the reaction mixture was cooled to rt, diluted with water, and extracted three times with EtOAc. The combined organic extracts were washed with brine, dried (MgSO₄), and concentrated *in vacuo.* Purification of the crude residue by flash chromatography on silica gel (gradient elution; 0-30% EtOAc/hexanes as eluent) furnished the title compound i-15c. *m*/*z* (ES) 453 (MH)⁺.

Following procedures similar to those described in schemes i-1 through i-11 and i-15, the following compounds in Table i-15 can be prepared:

**Table i-15**

| | | | | | | | |
|---|---|---|---|---|---|---|---|
| 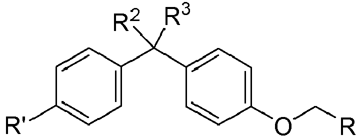 | | | | | | | |

| Ex. i-15A | Ex. i-15B | Ex. i-15C | Ex. i-15D | Ex. i-15E | Ex. i-15F | R² | R³ |
|---|---|---|---|---|---|---|---|
| R=2-PYR | R=2-PYM | R=2-TZE | R=2-PYR | R=2-PYM | R=2-TZE | | |
| R'=Ac | R'=Ac | R'=Ac | R'=Br-Ac | R'=Br-Ac | R'=Br-Ac | | |
| a | a | a | a | a | a | *i*Pr | H |
| -- | b | b | -- | b | b | *t*Bu | H |
| c | c | c | c | c | c | c-Pr | H |
| d | d | d | d | d | d | c-Bu | H |
| e | e | e | e | e | e | 1-Me-cPr | H |
| f | f | f | f | f | f | 1-Me-cBu | H |
| g | g | g | g | g | g | *i*Pr | Me |
| h | h | h | h | h | h | *i*Pr | OH |
| i | i | i | i | i | i | Bu | OH |

Table i-15. Parent Ion *m*/*z* (MH)⁺ data for compounds.
For i-15Ag: 1-(4-{1,2-dimethyl-1-[4-(pyridin-2-ylmethoxy)phenyl]propyl}phenyl)ethanone: *m*/*z* (ES) = 374 (MH)⁺

### (16) Preparation of i-16g (Scheme i-16)

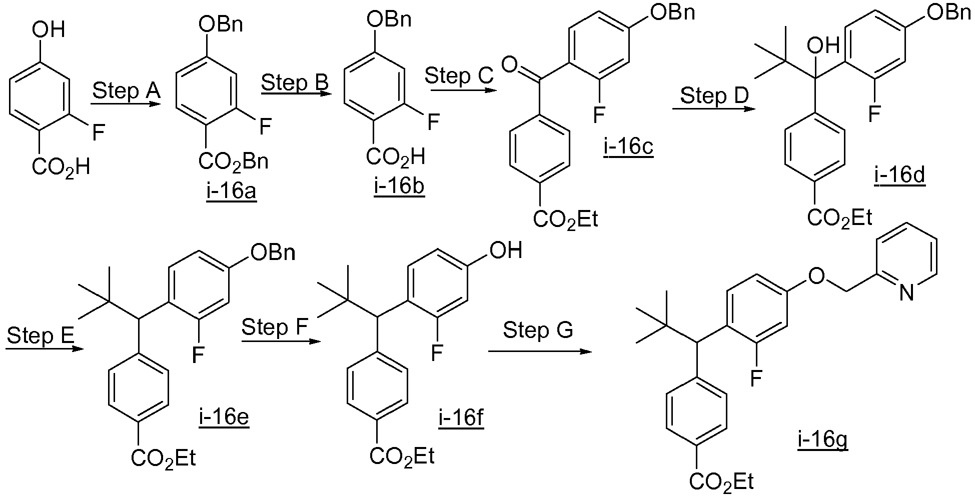

### Step A: Preparation of benzyl 4-(benzyloxy)-2-fluorobenzoate (i-16a)

Sodium hydride (3.04 g of a 60% dispersion in mineral oil, 75.7 mmol) was added in several portions to a solution of 2-fluoro-4-hydroxybenzoic acid (5.14 g, 32.9 mmol) in DMF (100 mL) at 0 °C. After 10 min, benzyl bromide (8.2 mL, 69.2 mmol) was added, and the reaction mixture was allowed to warm slowly to rt. After 17 h, the reaction was quenched with water and extracted with EtOAc. The organic layer was washed three times with satd. aq. sodium bicarbonate, dried (Na₂SO₄) and concentrated *in vacuo.* Purification of the crude residue by flash chromatography on silica gel (isocratic elution; 5% EtOAc/hexanes as eluent) afforded the title compound i-16a. *m*/*z* (ES) 337 (MH)⁺. ¹HNMR (500 MHz, CDCl₃): δ 7.97 (t, 1H, *J*= 8.6 Hz), 7.49 (d, 2H, *J*= 7.3 Hz), 7.35-7.45 (m, 8H), 6.82 (dd, 1H, *J*= 2.4, 8.8 Hz), 6.75 (dd, 1H, *J* = 2.6, 12.5 Hz), 5.39 (s, 2H), 5.13 (s, 2H).

### Step B: Preparation of 4-(benzyloxy)-2-fluorobenzoic acid (i-16b)

Potassium hydroxide (39 mL of a 2.0 M aq. solution, 78 mmol) was added to a solution of i-16a (5.76 g, 15.7 mmol) in dioxane (80 mL), and the reaction mixture was heated to 60 °C. After 5 h, the reaction mixture was diluted with 5% aq. sodium hydroxide and extracted four times with diethyl ether. The aqueous layer was acidifed to∼ pH 1 and extracted five times with EtOAc. The combined organic extracts were dried (Na₂SO₄) and concentrated to afford crude i-16b. ¹HNMR (500 MHz, CDCl₃): δ 11.0 (br, 1H), 8.02 (t, 1H, *J*= 8.7 Hz), 7.45 (m, 4H), 7.41 (m, 1H), 6.86 (dd, 1H, *J*= 2.5, 8.9 Hz), 6.78 (dd, 1H, *J*= 12.3 Hz), 5.16 (s, 2H).

### Step C: Preparation of ethyl 4-[4-(benzyloxy)-2-fluorobenzoyl]benzoate (i-16c)

Oxalyl chloride (5.1 mL, 59 mmol), followed by DMF:DCM (4:1; 1.5 mL), were added to a solution of i-16b (6.58 g, 26.8 mmol) in DCM (100 mL) and DME (100 mL) at 0 °C. After 0.5 h, the reaction mixture was concentrated *in vacuo,* and the resulting crude powder was converted to intermediate i-16c following the procedure as described for the preparation of intermediate i-1a. *m*/*z* (ES) 379 (MH)⁺. ¹HNMR (500 MHz, CDCl₃): δ 8.16 (d, 2H, *J*= 8.5 Hz), 7.86 (dd, 2H, *J* = 1.2, 8.5 Hz), 7.64 (t, 1H, *J*= 8.5 Hz), 7.45 (m, 5H), 6.91 (dd, 1H, *J* = 2.6, 8.7 Hz), 6.77 (dd, 1H, *J*= 2.4, 12.0 Hz), 5.16 (s, 2H), 4.44 (q, 2H, *J*= 7.1 Hz), 1.44 (t, 3H, *J*= 7.1 Hz).

### Step D: Preparation of ethyl 4-{1-[4-(benzyloxy)-2-fluorophenyl]-1-hydroxy-2,2-dimethylpropyl}benzoate (i-16d)

*tert*-Butyl magnesium chloride (65 mL of a 1.0 M THF solution, 65 mmol) was added to a solution of i-16c (5.91 g, 15.6 mmol) and lithium chloride (3.10 g, 93.8 mmol) in THF (20 mL) at 0 °C. After 16 h, the reaction mixture was poured into a mixture of 2N HCl and ice, and extracted with EtOAc. The organic layer was washed with 0.1N HCl and satd. aq. sodium bicarbonate, dried (Na₂SO₄) and concentrated. The resulting crude oil (i-16d) was used without purification in the subsequent reaction.

### Step E: Preparation of ethyl 4-[1-(2-fluoro-4-hydroxyphenyl)-1-hydroxy-2,2-dimethylpropyl]benzoate (i-16e)

Triethylsilane (75.0 mL, 469 mmol), followed by TFA (36.0 mL, 469 mmol), were added to a solution of crude i-16d (15.6 mmol) in DCM (250 mL) at 0 °C. After 2 h, the reaction mixture was quenched with satd. aq. sodium bicarbonate and extracted three times with EtOAc. The combined organic extracts were washed with sat. aq. sodium bicarbonate and brine, dried (Na₂SO₄) and concentrated. Purification of the crude residue by flash chromatography on silica gel (gradient elution; 0-100% EtOAc/hexanes as eluent) furnished the title compound i-16e. ¹HNMR (500 MHz, CDCl₃): δ 7.98 (d, 2H, *J*= 8.3 Hz), 7.54 (t, 1H, *J*= 8.9 Hz), 7.52 (d, 2H, *J* = 8.5 Hz), 7.35-7.45 (m, 5H), 6.77 (dd, 1H, *J*= 2.4, 8.8 Hz), 6.69 (dd, 1H, *J*= 2.5, 12.1 Hz), 5.04 (s, 2H), 4.39 (q, 2H, *J*= 7.1 Hz), 4.27 (s, 1H), 1.41 (t, 3H, *J*= 7.1 Hz), 1.07 (s, 9H).

### Step F: Preparation of ethyl 4-[1-(2-fluoro-4-hydroxyphenyl)-2,2-dimethylpropyl]benzoate (i-16f)

A mixture of i-16e (3.26 g, 7.76 mmol) and palladium (1.2 g of 10 wt. % on activated carbon) in ethanol (250 mL) was hydrogenated at atmospheric pressure for approximately 2 h. The resulting mixture was filtered through a short column of Celite@, eluting copiously with EtOAc. The filtrate was concentrated *in vacuo* and the crude residue was purified by flash chromatography on silica gel (gradient elution; 0-40% EtOAc/hexanes as eluent) to afford the title compound i-16f. ¹HNMR (500 MHz, CD₃OD): δ 7.92 (d, 2H, *J*= 8.5 Hz), 7.54 (d, 2H, *J*= 8.2 Hz), 7.51 (t, 1H, *J*= 8.9 Hz), 6.59 (dd, 1H, *J*= 2.3, 8.7 Hz), 6.47 (dd, 1H, *J*= 2.5, 12.4 Hz), 4.34 (q, 2H, *J*= 7.1 Hz), 4.22 (s, 1H), 1.37 (t, 3H, *J*= 7.1 Hz), 1.02 (s, 9H).

### Step G: Preparation of ethyl 4-{1-[2-fluoro-4-(pyridin-2-ylmethoxy)phenyl]-2,2-dimethylpropyl}benzoate (i-16g)

Intermediate i-16g was prepared from i-16f following the procedure as described in scheme i-1, Step G. *m*/*z* (ES) 422 (MH)⁺. ¹HNMR (500 MHz, CDCl₃): δ 8.55 (d, 1H, *J*= 4.6 Hz), 7.95 (d, 2H, *J*= 8.4 Hz), 7.61 (dt, 1H, *J*= 1.7, 7.7 Hz), 7.51 (t, 1H, *J*= 8.8 Hz), 7.47 (d, 2H, *J* = 8.2 Hz), 7.13 (dd, 1H, *J* = 5.1, 7.1 Hz), 6.75 (dd, 1H, *J* = 2.6, 8.8 Hz), 6.68 (dd, 1H, *J*= 2.6, 12.0 Hz), 5.14 (s, 2H), 4.33 (q, 2H, *J*= 7.1 Hz), 4.24 (s, 1H), 1.33 (t, 3H, *J*= 7.1 Hz), 1.02 (s, 9H).

### (17) Preparation of 4-{2,2-dimethyl-1-[4-(pyridin-2-ylmethoxy)phenyl]propyl-benzoic acid (i-17b)

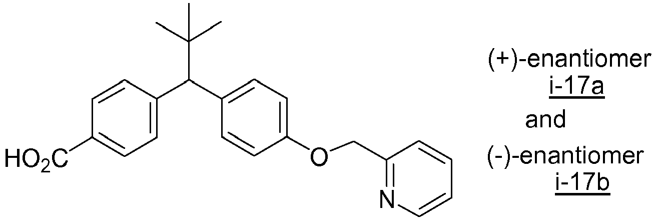

Lithium hydroxide monohydrate (162 mg, 3.86 mmol) was added to i-1i (600 mg, 1.54 mmol) in dioxane:water (15 mL of a 2 :1 mixture), and the resulting mixture was heated to 55 °C. After 1 h, the reaction mixture was cooled to rt, quenched with 0.5 N hydrochloric acid and extracted three times with EtOAc. The combined organic extracts were washed successively with water, brine, dried (Na₂SO₄) and concentrated *in vacuo* to afford the title compound i-17b.

Following procedures similar to those described above, the following compounds in Table i-17 can be prepared:

**Table i-17**

| | | | | | | |
|---|---|---|---|---|---|---|
| 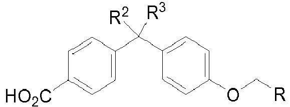 | | | | | | |

| Ex. i-17A | Ex. i-17B | Ex. i-17C | Ex. i-17D | Ex. i-17E | R² | R³ |
|---|---|---|---|---|---|---|
| R=2-PYR | R=2-PYM | R=2-TZE | R=5-F-2-PYR | R=3-F-2-PYR | | |
| a | a | a | a | a | *ⁱ*Pr | H |
| -- | b | b | b | b | *^{t}*Bu | H |
| c | c | c | c | c | c-Pr | H |
| d | d | d | d | d | c-Bu | H |
| e | e | e | e | e | 1-Me-c-Pr | H |
| f | f | f | f | f | 1-Me-c-Bu | H |
| g | g | g | g | g | *i*Pr | Me |
| h | h | h | h | h | *i*Pr | OH |
| i | i | i | i | i | *^{t}*Bu | OH |

Table i-17. Parent Ion *m*/*z* (MH)⁺ data for compounds.
For i-17Ac: 4-{cyclopropyl[4-(pyridin-2-ylmethoxy)phenyl]methyl}benzoic acid: *m*/*z* (ES) 360 (MH)⁺.
For i-17Ad: 4-{cyclobutyl[4-(pyridin-2-ylmethoxy)phenyl]methyl}benzoic acid: *m*/*z* (ES) 374 (MH)⁺.
For i-17Ag: 4-{1,2-dimethyl-1-[4-(pyridin-2-ylmethoxy)phenyl]propyl}benzoic acid: *m*/*z* (ES) 376 (MH)⁺.
For i-17Cg: 4-{1,2-dimethyl-1-[4-(1,3-thiazol-2-ylmethoxy)phenyl]propyl}benzoic acid: *m*/*z* (ES) 382 (MH)⁺.

### (18) Preparation of 2-[(4-{2,2-dimethyl-1-[4-(1H-tetrazol-5-yl)phenyl]propyl}phenoxy)methyl]-pyridine ammoniate (i-18a) (Scheme i-18)

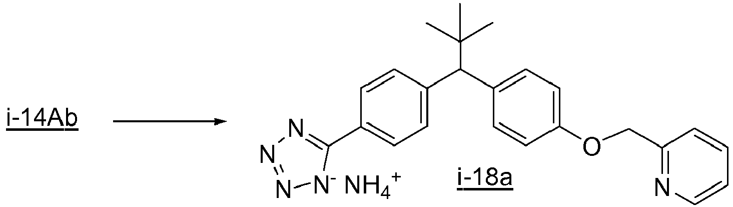

Azidotrimethyltin (1.45 g, 7.04 mmol) was added to a stirred solution of i-14Ab (626 mg, 1.76 mmol) in toluene (15 mL) and the resulting solution heated to reflux. After 18 h, the reaction mixture was cooled to rt, partially concentrated and diluted with ethanol. Hydrochloric acid (4 N in dioxane) was added, and after 1 h of vigorous agitation, the volatiles were removed *in vacuo* and the crude residue purified by flash chromatography on silica gel (gradient elution; 0%-100% ((85:15:1) DCM:methanol:ammonium hydroxide)/DCM as eluent) to afford the title compound 18a. ¹HNMR (500 MHz, CD₃OD): δ 8.53 (d, 1H, *J*= 4.8 Hz), 7.92 (d, 2H, *J* = 8.3 Hz), 7.86 (dt, 1H, *J* = 1.6, 7.6 Hz), 7.68 (d, 2H, *J* = 8.3 Hz), 7.60 (d, 1H, *J* = 7.8 Hz), 7.43 (d, 2H, *J*= 8.7 Hz), 7.37 (dd, 1H, *J*= 4.8, 7.0 Hz), 6.95 (d, 2H, *J*= 8.7 Hz), 5.16 (s, 2H), 3.85 (s, 1H), 1.04 (s, 9H).

Following procedures similar to those described above, the following compounds in Table i-18 can be prepared:

**Table i-18**

| | | | | | | |
|---|---|---|---|---|---|---|
| 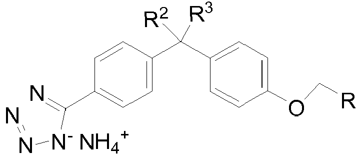 | | | | | | |

| Ex. i-18A | Ex. i-18B | Ex. i-18C | Ex. i-18D | Ex. i-18E | R² | R³ |
|---|---|---|---|---|---|---|
| R=2-PYR | R=2-PYM | R=2-TZE | R=F-2-PYR | R=3-F-2-PYR | | |
| a | a | a | a | a | *ⁱ*Pr | H |
| -- | b | b | b | b | *^{t}*Bu | H |
| c | c | c | c | c | c-Pr | H |
| d | d | d | d | d | c-Bu | H |
| e | e | e | e | e | 1-Me-c-Pr | H |
| f | f | f | f | f | 1-Me-c-Bu | H |
| g | g | g | g | g | *ⁱ*Pr | Me |
| h | h | h | h | h | *ⁱ*Pr | OH |
| i | i | i | i | i | *^{t}*Bu | OH |

Table i-18. Parent Ion *m*/*z* (MH)⁺ data for compounds.
For i-18Aa: ammonium 5-(4-{2-methyl-1-[4-(pyridin-2-ylmethoxy)phenyl]propyl}phenyl)tetrazol-1-ide: *m*/*z* (ES) 386 (MH)⁺.
For i-18Ag: ammonium 5-(4-{1,2-dimethyl-1-[4-(pyridin-2-ylmethoxy)phenyl]propyl}phenyl)-tetrazol-1-ide: *m*/*z* (ES) 400 (MH)⁺.
For i-18Bg: ammonium 5-(4-{1,2-dimethyl-1-[4-(pyrimidin-2-ylmethoxy)phenyl]propyl}phenyl)-tetrazol-1-ide: *m*/*z* (ES) 401 (MH)⁺.
For i-18Cg: ammonium 5-(4-{1,2-dimethyl-1-[4-(1,3-thiazol-2-ylmethoxy)phenyl]propyl}phenyl)-tetrazol-1-ide: *m*/*z* (ES) 406 (MH)⁺.

### (19) Preparation of i-19b (Scheme i-19)

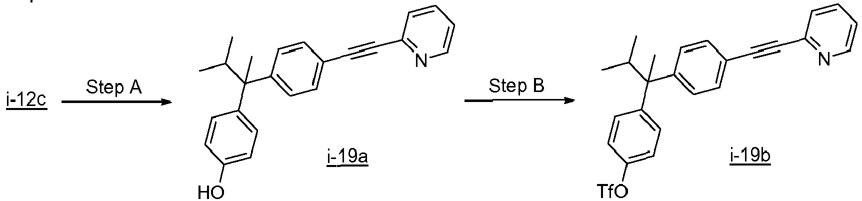

### Step A: Preparation of 4- {1,2-dimethyl-1-[4-(pyridin-2-ylethynyl)phenyl]propyl}phenol (i-19a)

Tetrakis(triphenylphosphine)palladium (0) (693 mg, 0.600 mmol) was added to a mixture of copper(I) iodide (1.16 g, 5.98 mmol), triethylamine (11.7 mL, 84.0 mmol), pyridine (2.56 mL, 24.2 mmol), tetrabutylammonium iodide (8.90 g, 24.1 mmol) and i-12c (4.40 g, 12.0 mmol) in DMF (80.0 mL). The resulting mixture was degassed and heated to 80 °C for 18 h. The reaction mixture was cooled to rt and diluted with EtOAc. The organics were washed with satd. aq. sodium bicarbonate and brine, dried and concentrated *in vacuo.* The crude residue was purified by flash chromatography on silica gel (gradient elution; 0-40% EtOAc/hexanes as eluent) to afford the title compound i-19a. *m*/*z* (ES) 342 (MH)⁺.

### Step B: Preparation of 4-{1,2-dimethyl-1-[4-(pyridin-2-ylethynyl)phenyl]propyl}phenyl

### trifluoromethanesulfonate (i-19b)

Compound i-19b can be prepared following the procedures described in scheme i-6, step C, substituting intermediate i-19a for i-6b. *m*/*z* (ES) 474 (MH)⁺.

Following procedures similar to those described above, the following compounds in Table i-19 can be prepared:

**Table i-19**

| | | |
|---|---|---|
| 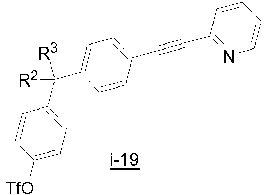 | | |

| Ex. i-19 | R² | R³ |
|---|---|---|
| c | *ⁱ*Pr | H |
| d | *^{t}*Bu | H |
| e | c-Pr | H |
| f | c-Bu | H |
| g | 1-Me-c-Pr | H |
| h | 1-Me-c-Bu | H |

### (20) Preparation of 3-chloro-6-(2,5-dimethyl-1H-pyrrol-1-yl)pyridazine (i-20a) and 5-bromo-2-(2,5-dimethyl-1H-pyrrol-1-yl)pyrimidine (i-20b)

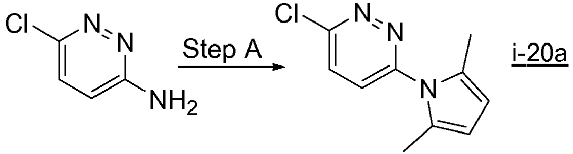

A mixture of *p*-TSA (117 mg, 0.618 mmol), 2,5-hexanedione (4.36 mL, 37.1 mmol) and 3-amino-6-chloropyridazine (4.00 g, 30.9 mmol) in toluene (150 mL) was heated at 140 °C for 5h in a round bottom flask equipped with a condenser and Dean-Stark apparatus. The reaction mixture was cooled to rt and charcoal was added. The mixture was filtered through Celite^{®} and concentrated *in vacuo* to afford the title compound i-20a. *m*/*z* (ES) 208 (MH)⁺.

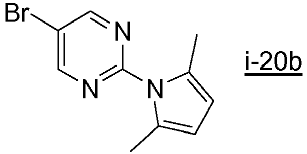

Compound i-20b was prepared following the procedures as described for the preparation of i-20a, substituting 2-amino-5-bromopyrimidine for 3-amino-6-chloropyridazine. *m*/*z* (ES) 252 (MH)⁺.

The Examples provided herein are intended only to illustrate the present invention and are not to be construed as limiting the scope of the claims in any way. In the Tables in the following Examples, compounds having mass spectral data were synthetically prepared; the abbreviations used are PYR = pyridyl, PYM = pyrimidinyl, TZE = thiazolyl.

### EXAMPLE 1

### Preparation of 5-(4-{2,2-dimethyl-1-[4-(pyrimidin-2-ylmethoxy)phenyl]propyl}phenyi)-1, 3,4-oxadiazol-2-amine (1a)

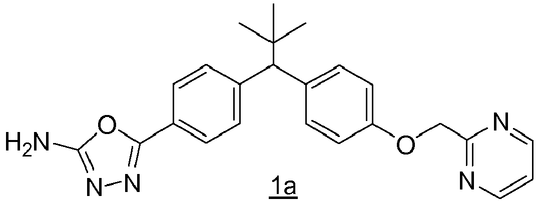

Hydrazine monohydrate (15 equiv.) is added to a stirred solution of i-11Ab (1.0 equiv.) in ethanol and the resulting solution is heated at reflux until the reaction is deemed complete. After cooling to rt, the volatiles are removed *in vacuo,* and the residue is partitioned between EtOAc and water. The organic phase is washed with water and brine, dried and concentrated *in vacuo.* The crude residue is dissolved in dioxane, to which a solution of sodium bicarbonate (1.1 equiv.) in water is added. A solution of cyanogen bromide (1.1 equiv.) in dioxane is then added slowly, and the resulting mixture is aged at rt until the reaction is deemed complete. The reaction mixture is poured into satd. aq. sodium bicarbonate and extracted with EtOAc. The combined organic extracts are washed with brine, dried and concentrated *in vacuo.* The crude residue can be purified by flash chromatography on silica gel can afford the title compound 1a.

Following procedures similar to those described above for making Compound 1a, the following compounds in Table 1 can be prepared:

**Table 1**

| | | | | | |
|---|---|---|---|---|---|
| 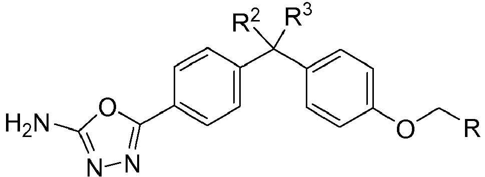 | | | | | |

| Ex. #1A | Ex. #1B | Ex. #1C | Ex. #1D | R² | R³ |
|---|---|---|---|---|---|
| R=2-PYM | R=2-TZE | R=5-F-2-PYR | R=3-F-2-PYR | | |
| -- | a | a | a | *^{t}*Bu | H |
| b | b | b | b | Et | H |
| c | c | c | c | Pr | H |
| d | d | d | d | *ⁱ*Pr | H |
| e | e | e | e | c-Pr | H |
| f | f | f | f | c-Bu | H |
| g | g | g | g | c-Pen | H |
| h | h | h | h | c-Hex | H |
| i | i | i | i | 1-Me-c-Pr | H |
| j | J | J | J | 1-Me-c-Bu | H |
| k | k | k | k | Me | Me |
| 1 | 1 | 1 | 1 | Et | Me |
| m | m | m | m | *ⁱ*Pr | Me |
| n | n | n | n | *^{t}*Bu | Me |
| o | o | o | o | c-Pr | Me |
| p | p | p | p | c-Bu | Me |
| q | q | q | q | 1-Me-c-Pr | Me |
| r | r | r | r | 1-Me-c-Bu | Me |
| s | s | s | s | -C(CH₃)₂CF₃ | H |
| t | t | t | t | -C(CH₃)₂CF₂H | H |
| u | u | u | u | *ⁱ*Pr | OH |
| v | v | v | v | *^{t}*Bu | OH |

### EXAMPLE 2

### Preparation of compound 2c

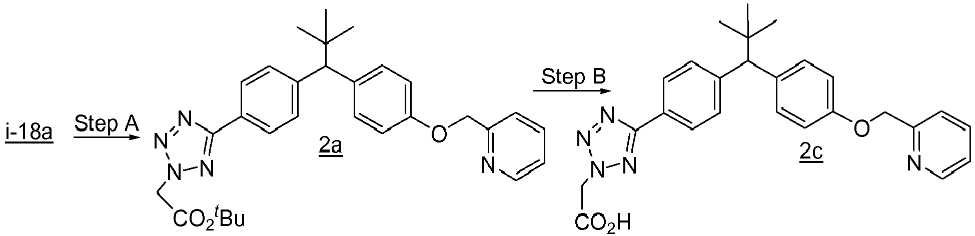

### Step A: Preparation of t-butyl [5-(4-{2,2-dimethyl-1-[4-(pyridin-2-ylmethoxy)phenyl] - propyl}phenyl)-2H-tetrazol-2-yl]acetate (2a)

*tert*-Butyl bromoacetate (14 µL, 0.095 mmol) was added to a solution of i-18a (15 mg, 0.036 mmol) and triethylamine (15 µL, 0.11 mmol) in DCM (500 µL), and the resulting mixture was allowed to stir at rt. After 20 h, additional portions of triethylamine (20 µL) and *tert*-butyl bromoacetate (15 µL) were added, and after a total reaction time of 25 h, the reaction was concentrated *in vacuo.* The crude residue was purified by flash chromatography on silica gel (gradient elution; 0%-70% (EtOAc/hexanes as eluent) to afford two regioisomers: 2a (major regioisomer) and 2b (minor regioisomer).
For 2a: ¹HNMR(500 MHz, CDCl₃): δ 8.61 (d, 1H, *J* = 4.6 Hz), 7.73 (dt, 1H, *J*= 1.6, 7.6 Hz), 7.60 (s, 4H), 7.54 (d, 1H, *J*= 7.8 Hz), 7.36 (d, 2H, *J*= 8.7 Hz), 7.24 (m, 1H), 6.95 (d, 2H, *J*= 8.7 Hz), 5.20 (s, 2H), 5.11 (s, 2H), 3.78 (s, 1H). 1.39 (s, 9H), 1.05 (s, 9H). 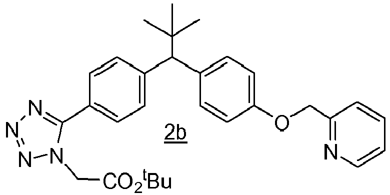 For 2b: ¹HNMR (500 MHz, CDCl₃): δ 8.61 (d, 1H, *J*= 4.6 Hz), 7.73 (dt, 1H, *J*= 1.6, 7.6 Hz), 7.60 (m, 4H), 7.54 (d, 1H, *J* = 7.8 Hz), 7.35 (d, 2H, *J* = 8.7 Hz), 7.25 (dd, 1H, *J* = 7.1, 7.2 Hz), 6.94 (d, 2H, *J*= 8.7 Hz), 5.20 (s, 2H), 5.11 (s, 2H), 3.78 (s, 1H), 1.39 (s, 9H), 1.05 (s, 9H).

### Step B: Preparation of [5-(4-{2,2-dimethyl-1-[4-(pyridin-2-ylmethoxy)phenyl]propyl}-phenyl)-2H-tetrazol-2-ylacetic acid (2c)

A solution of HCl in dioxane:water (1.00 mL of a (20:1) mixture of (4 M HCl in dioxane):water) was added to 2a (12 mg, 0.023 mmol) in dioxane (1.0 mL) at 5 °C. After 30 min, and additional portion of the above HCl-dioxane:water solution (1.0 mL) was added. After 18 h, the reactions mixture was concentrated, and the resulting crude residue was purified by preparative reversed phase HPLC on YMC Pack Pro C18 stationary phase (acetonitrile/water as eluent, 0.1% TFA as modifier). Lyophilization of the purified fractions affords the title compound 2c. *mlz* (ES) 458 (MH)⁺.

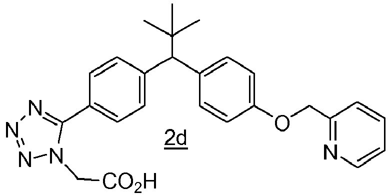

Compound 2d was prepared following the procedures described in example 2, step B, substituting compound 2b for compound 2a. *m*/*z* (ES) 458 (MH)⁺.

### Preparation of compound 2e

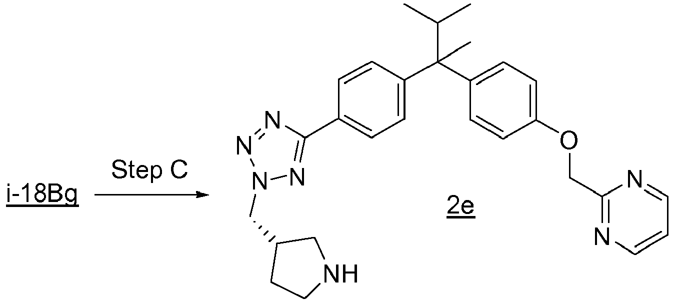

### Step C: Preparation of 2-({4-[1,2-dimethyl-1-(4-{2-[(3S)-pyrrolidin-3-ylmethyl]-2H-tetrazol-5-yl}phenyl)propyl]phenoxy}methyl)pyrimidine (2e)

Diethyl azodicarboxylate (80 µL, 0.51 mmol) was added dropwise to a solution of i-18Bg (50 mg, 0.11 mmol), triphenylphosphine (130 mg, 0.50 mmol) and (S)-Boc-prolinol (73 mg, 0.36 mmol) in DCM (1.0 mL). The resulting mixture was stirred at rt for 18 h, and then passed through a short column of silica gel (gradient elution; 0%-70% (EtOAc/hexanes as eluent), from which the major regioisomer was isolated to afford an intermediate that was converted to the title compound 2e using the procedures described in step B. *m*/*z* (ES) 484 (MH)⁺.

Following procedures similar to those described above, the following compounds in Table 2 can be prepared:

**Table 2**

| | | | | | | |
|---|---|---|---|---|---|---|
| 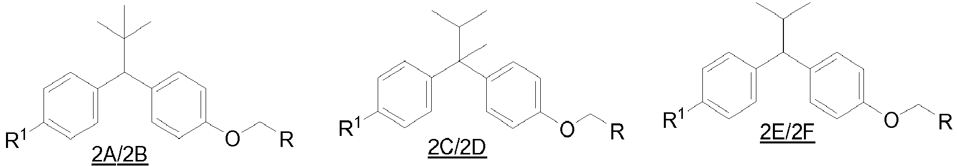 | | | | | | |
| 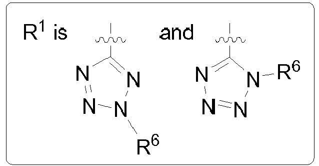 | | | | | | |

| Ex. 2A | Ex.2B | Ex. 2C | Ex. 2D | Ex. 2E | Ex. 2F | R⁶ |
|---|---|---|---|---|---|---|
| R=2-PYM | R=2-TZE | R=2-PYM | R=2-TZE | R=2-PYM | R=2-TZE | |
| a | a | a | a | a | a | Me |
| b | b | b | b | b | b | Et |
| c | c | c | c | c | c | ⁱPr |
| d | d | d | d | d | d | CHF₂ |
| e | e | e | e | e | e | 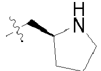 |
| f | f | f | f | f | f | 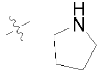 |
| g | g | g | g | g | g | 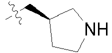 |
| h | h | -- | h | h | h | 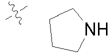 |
| i | i | i | i | i | i | 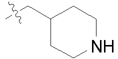 |
| j | j | j | j | j | j | 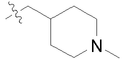 |
| k | k | k | k | k | k | 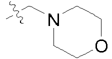 |
| -- | l | l | l | l | l | 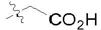 |

Table 2. Parent Ion *m*/*z* (MH)⁺ data for compounds
For 2Ca: 2-[(4-{1,2-dimethyl-1-[4-(2-methyl-2*H*-tetrazol-5-yl)phenyl]propyl}phenoxy)methyl]-pyrimidine: *m*/*z* (ES) = 415 (MH)⁺
For 2Cb: 2-[(4-{1,2-dimethyl-1-[4-(2-ethyl-2*H*-tetrazol-5-yl)phenyl]propyl}phenoxy)methyl]-pyrimidine: *m*/*z* (ES) = 429 (MH)⁺
For 2Cd: 2- {[4-(1-{4-[2-(difluoromethyl)-2*H*-tetrazol-5-yl]phenyl}-1,2-dimethylpropyl)-phenoxy]methyl}pyrimidine: *m*/*z* (ES) = 451 (MH)⁺
For 2Cg: 2-({4-[1,2-dimethyl-1-(4-{2-[(3*R*)-pyrrolidin-3-ylmethyl]-2H-tetrazol-5-yl}phenyl)-propyl]phenoxy}methyl)pyrimidine: *m*/*z* (ES) = 484 (MH)⁺
For 2Da: 5-(4-{1,2-dimethyl-1-[4-(1,3-thiazol-2-ylmethoxy)phenyl]propyl}phenyl)-2-methyl-2*H-*tetrazole: *m*/*z* (ES) = 420 (MH)⁺
For 2Db: 5-(4-{1,2-dimethyl-1-[4-(1,3-thiazol-2-ylmethoxy)phenyl]propyl}phenyl)-2-ethyl-2*H-*tetrazole: *m*/*z* (ES) = 434 (MH)⁺
For 2Dd: 2- {[4-(1-{4-[2-(difluoromethyl)-2*H*-tetrazol-5-yl]phenyl}-1,2-dimethylpropyl)-phenoxy]methyl}thiazole: *m*/*z* (ES) = 456 (MH)⁺

### EXAMPLE 3

### Preparation of 5-(4-{2,2-dimethyl-1-[4-(pyridin-2-ylmethoxy)phenyl]propyl}phenyl)-3-methyl-1,3,4-oxadiazol-2(3H)-one (3c)

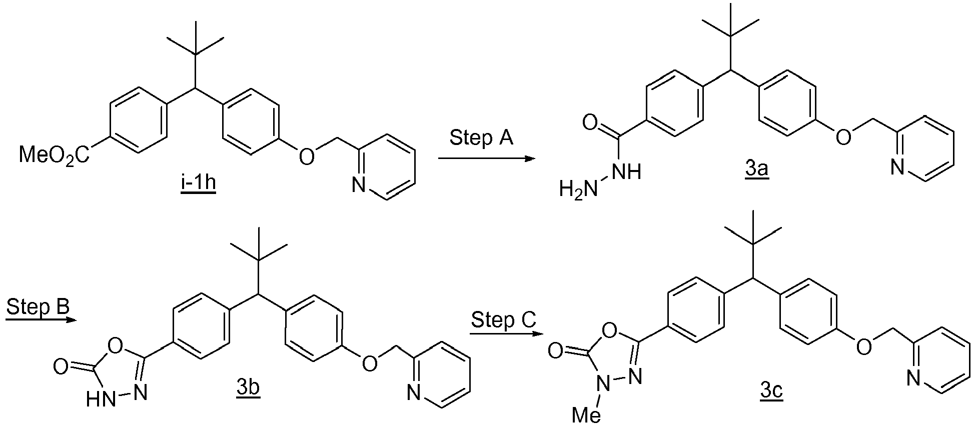

### Step A: Preparation of 4-{2,2-dimethyl-1-[4-(pyridin-2-ylmethoxy)phenyl]propyl} - benzohydrazide (3a)

Hydrazine monohydrate (1.5 mL) was added to a solution of i-1h (40 mg, 0.11 mmol) in ethanol (5.0 mL), and the resulting reaction mixture was heated to reflux. After 2 h, the reaction mixture was cooled to rt and diluted with EtOAc. The organics were washed successively with water and brine, dried and concentrated *in vacuo* to afford the title compound 3a, *m*/*z* (ES) 390 (MH)⁺.

### Step B: Preparation of 5-(4-{2,2-dimethyl-1-[4-(pyridin-2-ylmethoxy)phenyl]propyl} - phenyl)-1,3,4-oxadiazol-2(3H)-one (3b)

Phosgene (0.11 mL, 0.20 mmol) was added to a solution of 3a (44 mg, 0.11 mmol) in THF (1.0 mL) at -78 °C. After 30 min, the reaction mixture was quenched with satd. aq. sodium bicarbonate and extracted with DCM. The combined organic extracts were washed successively with water and brine, dried and concentrated *in vacuo.* The crude residue was dissolved in DMSO and purified by preparative reversed phase high performance liquid chromatography on YMC Pack Pro C18 phase (gradient elution; 25%-70% acetonitrile/water as eluent, 0.05% TFA modifier) to give the title compound 3b. *m*/*z* (ES) 416 (MH)⁺. ¹HNMR (500 MHz, *d₆*-DMSO): δ 12.50 (s, 1H), 8.59 (d, 1H, *J*= 5.0 Hz), 7.90 (m, 1H), 7.68 (d, 2H, *J*= 8.3 Hz), 7.60 (d, 2H, *J* = 8.3 Hz), 7.56 (d, 1H, *J* = 7.7 Hz), 7.40 (m, 1H), 7.38 (d, 2H, *J* = 8.7 Hz), 6.94 (d, 2H, *J*= 8.7 Hz), 5.16 (s, 2H), 3.84 (s, 1H), 0.93 (s, 9H).

### Step C: Preparation of 5-(4-{2,2-dimethyl-1-[4-(pyridin-2-ylmethoxy)phenyl]propyl}-phenyl)-3-methyl-1,3,4-oxadiazol-2(3H)-one (3c)

Sodium hydride (7.4 mg of a 60% dispersion in mineral oil, 0.17 mmol) was added to a solution of 3b (33 mg, 0.079 mmol) and iodomethane (7.4 µL, 0.12 mmol) in DMF (0.75 mL) at 0 °C. After 1 h, an additional portion of iodomethane (7.4 µL, 0.12 mmol) was added. After 2 h, the reaction mixture was poured into satd. aq. sodium bicarbonate and extracted with EtOAc. The combined organic extracts are washed with water and brine, dried and concentrated *in vacuo.* The crude residue was purified by preparative reversed phase high performance liquid chromatography on YMC Pack Pro C18 phase (gradient elution; 30%-80% acetonitrile/water as eluent, 0.05% TFA modifier) to give the title compound 3c. *m*/*z* (ES) 431 (MH)⁺.

Following procedures similar to those described above for making Compound 3c, the following compounds in Table 3 can be prepared:

**Table 3**

| | | | | | | |
|---|---|---|---|---|---|---|
| 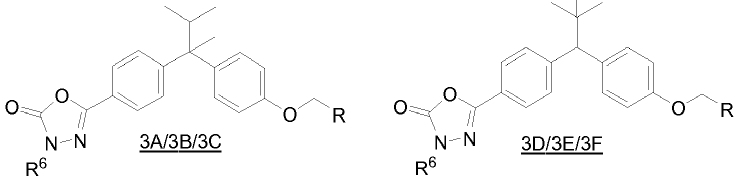 | | | | | | |

| Ex.3A R=2-PYR | Ex. 3B R=2-PYM | Ex.3C R=2=TZ E | Ex. 3D R=2-PYR | Ex.3E R=2-PYM | Ex.3F R=2=TZ E | R⁶ |
|---|---|---|---|---|---|---|
| a | a | a | -- | a | a | H |
| b | b | b | -- | b | b | Me |
| c | c | c | c | c | c | Et |
| d | d | d | d | d | d | *ⁱ*Pr |
| e | e | e | e | e | e | 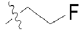 |
| f | f | f | f | f | f | 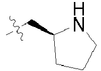 |
| g | g | g | g | g | g | 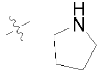 |
| h | h | h | h | h | h | 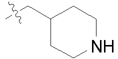 |
| i | i | i | i | i | i | 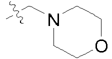 |

Table 3. Parent Ion *m*/*z* (MH)⁺ data for compounds:
For 3Dc: 5-(4-{2,2-dimethyl-1-[4-(pyridin-2-ylmethoxy)phenyl]propyl]phenyl)-3-ethyl-1,3,4-oxadiazol-2(3*H*)-one: *m*/*z* (ES) = 444 (MH)⁺
For 3De: 5-(4-{2,2-dimethyl-1-[4-(pyridin-2-ylmethoxy)phenyl]propyl}phenyl)-3-(2 - fluoroethyl)-1,3,4-oxadiazol-2(3*H*)-one: *m*/*z* (ES) = 462 (MH)⁺

### EXAMPLE 4

### Preparation of 2-{[4-(1-{4-[1-(2-fluoroethyl)-1H-pyrazol-3-yl]phenyl}-2,2-dimethylpropyl)-phenoxy]methyl}pyridine (4c)

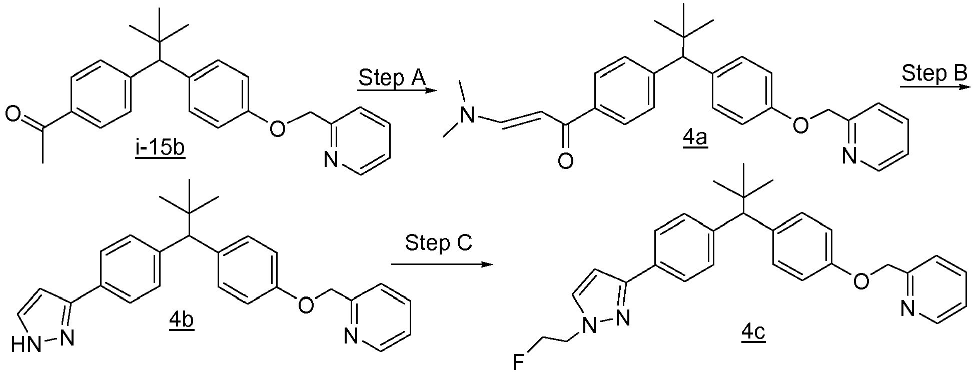

### Step A: Preparation of (2E)-3-(dimethylamino)-1-(4-{2,2-dimethyl-1-[4-(pyridin-2-yl-methoxy)phenyl]propyl)phenyl)prop-2-en-1-one (4a)

*N,N*-Dimethylformamide dimethylacetal (98 µL, 0.74 mmol) was added to a stirred solution of i-15b (55 mg, 0.15 mmol) in EtOH (0.40 mL) at rt, and the resulting mixture was irradiated at 150 °C in a microwave reactor. After 20 min, an additional portion of *N,N-*dimethylformamide dimethylacetal (0.20 mL, 1.5 mmol) was added, and heating at 150 °C was continued. After an additional 20 min, the reaction mixture was purified by flash chromatography on silica gel [gradient elution; 0%-10% ((9:1) methanol:ammonium hydroxide)/ DCM as eluent] to furnish the title compound 4a. *m*/*z* (ES) 453 (MH)⁺.

### Step B: Preparation of 2-[(4-{2,2-dimethyl-1-[4-(1H-pyrazol-3-yl)phenyl]propyl}-phenoxy)methyl]pyridine (4b)

Hydrazine monohydrate (43 µL, 0.88 mmol) was added to a stirred solution of 4a (63 mg, 0.15 mmol) in EtOH (1.2 mL), and the reaction mixture was heated to 80 °C. After 2 h, the reaction mixture was cooled to rt, concentrated *in vacuo,* and purified by flash chromatography on silica gel [gradient elution; 0%-10% ((9:1) methanol:ammonium hydroxide)/DCM as eluent] to furnish the title compound 4b. *m*/*z* (ES) 398 (MH)⁺.

### Step C: Preparation of 2-{[4-(1-{4-[1-(2-fluoroethyl-1H-pyrazol-3-yl]phenyl}-2,2-dimethylpropyl)phenoxy]methyl}pyridine (4c)

Sodium hydride (10 mg of a 60%wt dispersion in mineral spirits, 0.25 mmol) was added to a solution of 4b (51 mg, 0.13 mmol) in DMF (1.0 mL) at rt. After 15 min, 1-bromo-2-fluoroethane (24 µL, 0.32 mmol) was added, and the reaction was maintained at rt. After 45 min, the reaction was quenched with water and extracted with EtOAc. The combined organics were washed with brine, dried and concentrated *in vacuo.* The crude residue was dissolved in DMSO and purified by preparative reversed phase high performance liquid chromatography on YMC Pack Pro C18 phase (gradient elution; 10%-70% acetonitrile/water as eluent, 0.05% TFA modifier) to give the title compound 4c. *m*/*z* (ES) 444 (MH)⁺.

Following procedures similar to those described above for making Compound 4c, the following compounds in Table 4 can be prepared:

**Table 4**

| | | | | | | |
|---|---|---|---|---|---|---|
| 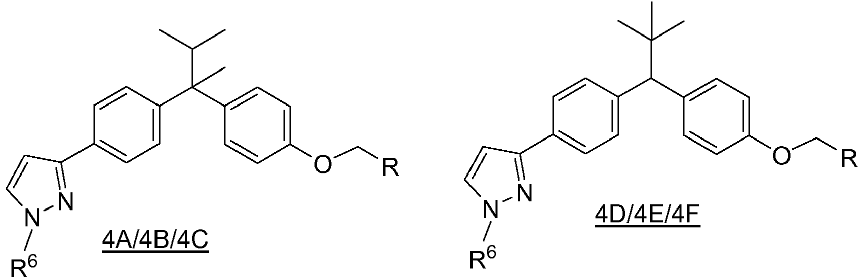 | | | | | | |

| Ex.4A | Ex. 4B | Ex. 4C | Ex.4D | Ex. 4E | Ex. 4F | R⁶ |
|---|---|---|---|---|---|---|
| R=2-PYR | R=2-PYM | R=2-TZE | R=2-PYR | R=2-PYM | R=2-TZE | |
| a | a | a | a | a | a | Me |
| b | b | b | b | b | b | Et |
| c | c | c | c | c | c | *ⁱ*Pr |
| d | d | d | -- | d | d | -CH₂CH₂F |
| e | e | e | e | e | e | -CH₂CHF₂ |
| f | f | f | f | f | f | -CH₂CF₃ |
| g | g | g | g | g | g | -CH₂C(CH₃)₂OH |
| h | h | h | h | h | h | -CH₂CH₂N(CH₃)₂ |
| i | i | i | i | i | i | 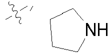 |
| j | J | J | J | J | J | 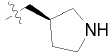 |

### EXAMPLE 5

### Preparation of N-cyclopropyl-4-{2,2-dimethyl-1-[4-(pyridin-2-ylmethoxy)phenyl]propyl}-benzamide (5 a)

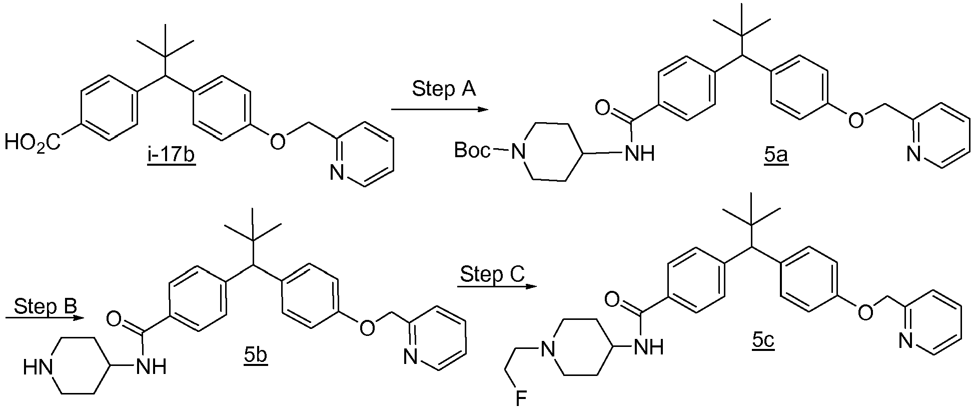

### Step A : Preparation of tert-butyl 4-[(4-{2,2-dimethyl-1-[4-(pyridin-2-ylmethoxy)phenyl]-propyl}benzoyl)amino]piperidine-1-carboxylate (5a)

*tert*-Butyl 4-aminopiperidine-1-carboxylate (318 mg, 1.59 mmol) was added to a stirred solution of HATU (658 mg, 1.73 mmol), *N,N*-diisopropylethylamine (927 µL, 5.32 mmol) and i-17b (500 mg, 1.33 mmol) in DMF (13.0 mL) at rt. After 18 h, the reaction mixture was poured into satd. aq. sodium bicarbonate and extracted with EtOAc. The combined organic extracts were dried, filtered and concentrated *in vacuo,* and the resulting crude product was purified by flash chromatography on silica gel (gradient elution; 0%-50% EtOAc/hexanes as eluent) to afford the title compound 5a. *m*/*z* (ES) 558 (MH)⁺.

### Step B: Preparation of 4-{2,2-dimethyl-1-[4-(pyridin-2-ylmethoxy)phenyl]propyl}-N-piperidin-4-ylbenzamide (5b)

Compound 5b was prepared following procedures as described in example 2, step B, substituting compound 5a for compound 2a. *m*/*z* (ES) 458 (MH)⁺.

### Step C: Preparation of 4-{2,2-dimethyl-1-[4-(pyridin-2-ylmethoxy)phenyl]propyl}-N-[1-(2-fluoroethyl)piperidin-4-yl]benzamide (5c)

2-Bromofluoroethane (83.0 mg, 0.655 mmol) was added to a solution of *N,N-*diisopropylethylamine (115 µL, 0.665 mmol), potassium iodide (14.0 mg, 0.220 mmol), DMAP (1.33 mg, 0.011 mmol) and 5b (50.0 mg, 0.109 mmol) in 1,2-dichloroethane (1.00 mL), and the reaction mixture was heated at 50 °C in a sealed tube. After 18 h, the reaction mixture was cooled to rt, poured into satd. aq. sodium bicarbonate and extracted with EtOAc. The combined organic extracts were washed with brine, dried and concentrated *in vacuo.* The crude product was purified by flash chromatography on silica gel (gradient elution; 0%-2.5% methanol/DCM as eluent) to afford the title compound 5c. *m*/*z* (ES) 504 (MH)⁺.

Following procedures similar to those described above in Example 5, the following compounds in Table 5, as well as corresponding compounds in which R is 2-thiazolylmethyl or 2-pyrimidinylmethyl, can be prepared:

**Table 5**

| | | | | | | | |
|---|---|---|---|---|---|---|---|
| 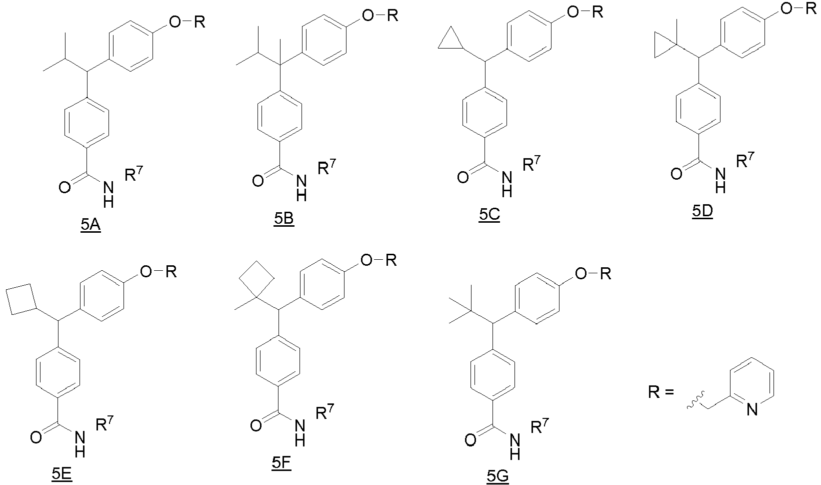 | | | | | | | |

| Ex. 5A | Ex. 5B | Ex. 5C | Ex. 5D | Ex. 5E | Ex. 5F | Ex. 4G | R⁷ |
|---|---|---|---|---|---|---|---|
| a | a | a | a | a | a | a | Me |
| b | b | b | b | b | b | b | 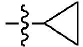 |
| c | c | c | c | c | c | c | 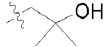 |
| d | d | d | d | d | d | d | 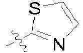 |
| e | e | e | e | e | e | e | 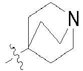 |
| f | f | f | f | f | f | f | 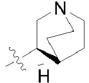 |
| g | g | g | g | g | g | g | 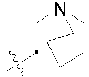 |
| h | h | h | h | h | h | -- | 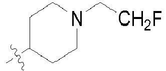 |
| i | i | i | i | i | i | i | 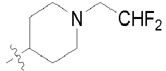 |
| J | J | J | J | J | J | J | 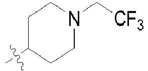 |

Table 5. Parent Ion *m*/*z* (MH)⁺ data for compounds.
For 5Bb: *N*-cyclopropyl-4-{1,2-dimethyl-1-[4-(pyridin-2-ylmethoxy)phenyl]propyl}benzamide: *m*/*z* (ES) 415 (MH)⁺.
For 5Cb: *N*-cyclopropyl-4-{cyclopropyl[4-(pyridin-2-ylmethoxy)phenyl]methyl}benzamide: *mlz* (ES) 399 (MH)⁺.
For 5Eb: *N*-cyclopropyl-4-{cyclobutyl[4-(pyridin-2-ylmethoxy)phenyl]methyl}benzamide: *mlz* (ES) 413 (MH)⁺.
For 5Gb: *N*-cyclopropyl-4-{2,2-dimethyl-1-[4-(pyridin-2-ylmethoxy)phenyl]propyl}benzamide: *m*/*z* (ES) 415 (MH)⁺.
For 5Gc: 4-{2,2-dimethyl-1-[4-(pyridin-2-ylmethoxy)phenyl]propyl}-N-(2-hydroxy-2-methylpropyl)benzamide: *m*/*z* (ES) 447 (MH)⁺.
For 5Gd: 4-{2,2-dimethyl-1-[4-(pyridin-2-ylmethoxy)phenyl]propyl}-*N*-1,3-thiazol-2-yl-benzamide: *m*/*z* (ES) 458 (MH)⁺.
For 5Ge: *N-*1-azabicyclo[2.2.2]oct-4-yl-4-{2,2-dimethyl-1-[4-(pyridin-2-ylmethoxy)phenyl]-propyl}benzamide: *m*/*z* (ES) 484 (MH)⁺.
For 5Gf: *N*-[(3*R*)-1-azabicyclo[2.2.2]oct-3-yl]-4-{2,2-dimethyl-1-[4-(pyridin-2-ylmethoxy)-phenyl]propyl}benzamide: *m*/*z* (ES) 484 (MH)⁺.
For 5Ggj: *N*-[(3*S*)-1-azabicyclo[2.2.2]oct-3-yl]-4-{2,2-dimethyl-1-[4-(pyridin-2-ylmethoxy)-phenyl]propyl}benzamide: *m*/*z* (ES) 484 (MH)⁺.
For 5Gi: 4-{2,2-dimethyl-1-[4-(pyridin-2-ylmethoxy)phenyl]propyl}-*N*-[1-(2,2-difluoroethyl)-piperidin-4-yl]benzamide: *m*/*z* (ES) 522 (MH)⁺.
For 5Gj: 4-{2,2-dimethyl-1-[4-(pyridin-2-ylmethoxy)phenyl]propyl}-*N*-[1-(2,2,2-trifluoroethyl)-piperidin-4-yl]benzamide: *m*/*z* (ES) 540 (MH)⁺.

### EXAMPLE 6

### Preparation of 5-(4-{2,2-dimethyl-1-[4-(pyridin-2-ylmethoxy)phenyl]propyl}phenyl)-1,3,4-thiadiazol-2-amine (6b)

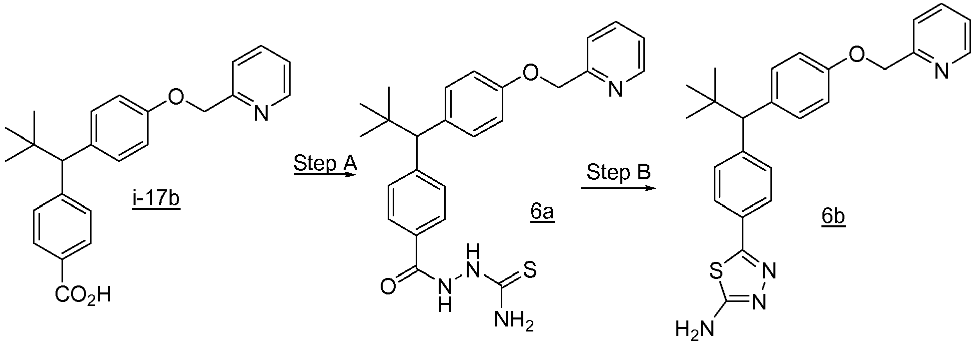

### Step A: Preparation of 2-(4-{2,2-dimethyl-1-[4-(pyridin-2-ylmethoxy)phenyl]propyl} benzoyl)hydrazinecarbothioamide (6a)

Thiosemicarbazide (5 equiv) is added to a prestirred solution of i-17b in DMF (0.1 M) at rt, followed by HATU (2 equiv). DIPEA (5 equiv) is then added, and the reaction mixture is stirred at ambient temperature until the reaction is deemed complete. The reaction mixture is poured into satd. aq. sodium bicarbonate and extracted with EtOAc. The combined organic extracts are washed with water, dried, and concentrated *in vacuo.* The crude residue can be purified by flash chromatography on silica gel to afford the title compound 6a.

### Step B: Preparation of 5-(4-{2,2-dimethyl-1-[4-(pyridin-2-ylmethoxy)phenyl]propyl}-phenyl)-1,3,4-thiadiazol-2-amine (6b)

Methanesulfonic acid (3 equiv) is added to a prestirred suspension of 6a (1equiv) in toluene (0.1M) at rt. The resulting mixture is heated at reflux until completion of reaction.

After cooling to rt, the reaction mixture is poured into satd. aq. sodium bicarbonate and extracted with EtOAc. The combined organic extracts are separated and washed once further with satd. aq. sodium bicarbonate, dried, and concentrated *in vacuo.* The residue can be purified by preparative reversed phase HPLC on YMC Pack Pro C18 stationary phase (acetonitrile/water as eluent, 0.1 % TFA as modifier) to afford the title compound 6b.

Following procedures similar to those described above for making Compound 6b, the following compounds in Table 6 can be prepared:

**Table 6**

| | | | | |
|---|---|---|---|---|
| 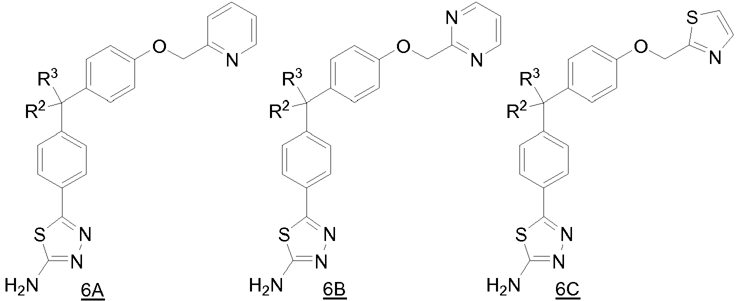 | | | | |

| Ex. 6A | Ex. 6B | Ex. 6C | R2 | R3 |
|---|---|---|---|---|
| a | a | a | *ⁱ*Pr | H |
| -- | b | b | *^{t}*Bu | H |
| c | c | c | c-Pr | H |
| d | d | d | c-Bu | H |
| e | e | e | 1-Me-c-Pr | H |
| f | f | f | 1-Me-c-Bu | H |
| g | g | g | *ⁱ*Pr | Me |

### EXAMPLE 7

### Preparation of 4-{[3-(4-{1,2-dimethyl-1-[4-(pyridin-2-ylmethoxy)phenyl]propyl}phenyl)-1,2,4-oxadiazol-5-yl]methyl}morpholine (7f)

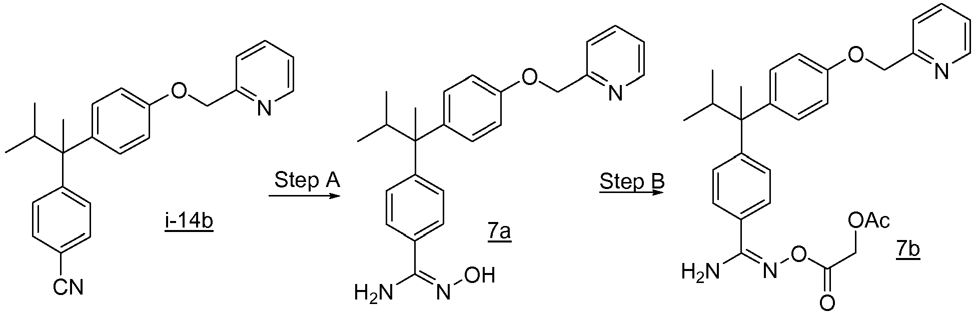

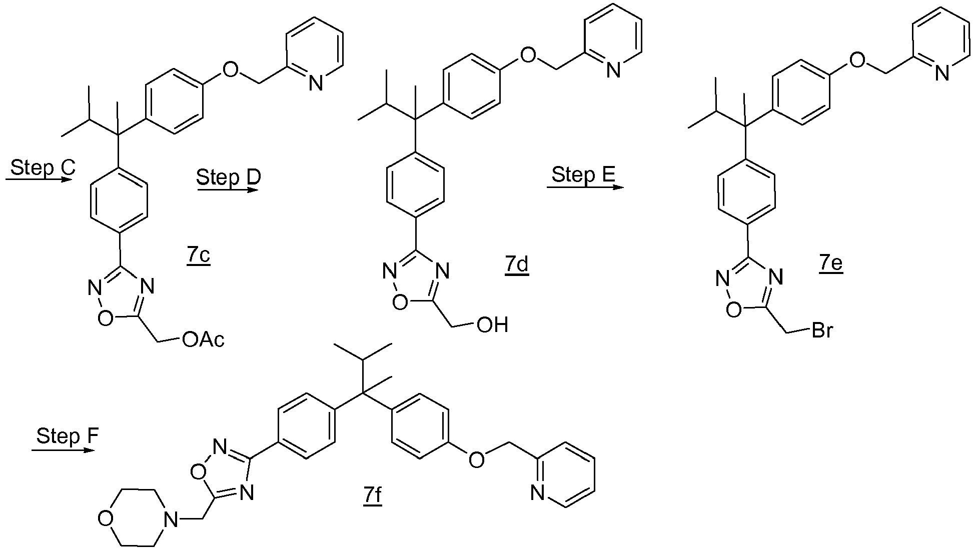

### Step A: Preparation of 4-{1,2-dimethyl-1-[4-(pyridin-2-ylmethoxy)phenyl]propyl}-N'-hydroxybenzenecarboximidamide (7a)

Hydroxylamine (0.39 mL of a 50% aqueous solution, 5.86 mmol) was added to a stirred solution of i-14b (0.67 g, 1.9 mmol) in EtOH (8 mL) at rt, and the resulting mixture was heated to 80 °C. After 16 h, the reaction mixture was concentrated *in vacuo,* and the crude residue was purified by flash chromatography on silica gel (gradient elution; 10%-30% EtOAc/hexanes as eluent) to furnish the title compound 7a. *m*/*z* (ES) 390 (MH)⁺.

### Step B: Preparation of 2-({[(1E)-amino(4-{1,2-dimethyl-1-[4-(pyridin-2-ylmethoxy)-phenyl]propyl}phenyl)methylene]amino}oxy)-2-oxoethyl acetate (7b)

Compound 7a (100 mg, 0.258 mmol) was added to a prestirred solution of acetoxyacetic acid (37.0 mg, 0.308 mmol), 1[3-(dimethylamino)propyl]-3-ethylcarbodiimide hydrochloride (59.0 mg, 0.308 mmol) and 1-hydroxybenzotriazole (45.0 mg, 0.333 mmol) in DCM (1.20 mL) at rt. After 3 h, the reaction mixture was poured into satd. aq. sodium bicarbonate and extracted with EtOAc. The combined organic extracts were washed with water and brine, dried and concentrated *in vacuo.* The crude product was purified by flash chromatography on silica gel (gradient elution; 0%-3% methanol/DCM as eluent) to afford the title compound 7b. *m*/*z* (ES) 490 (MH)⁺.

### Step C: Preparation of [3-(4-{1,2-dimethyl-1-[4-(pyridin-2-ylmethoxy)phenyl]propyl} - phenyl)-1,2,4-oxadiazol-5-yl]methyl acetate (7c)

A solution of 7b (118 mg, 0.242 mmol) in xylene (2.00 mL) was heated to 110 °C for 1.5 h. The reaction mixture was concentrated *in vacuo* to afford the title compound 7c, which was used in the subsequent step without further purification. *m*/*z* (ES) 472 (MH)⁺.

### Step D: Preparation of [3-(4-{1,2-dimethyl-1-[4-(pyridin-2-ylmethoxy)phenyl]propyl}-phenyl)-1,2,4-oxadiazol-5-yl]methanol (7d)

Potassium carbonate (200 mg, 1.45 mmol) in water (400 µL) was added to a solution of 7c (crude product from Step C, 0.242 mmol, theoretical) in methanol (1.60 mL) at rt. After 1.5 h, the reaction mixture was poured into satd. aq. sodium bicarbonate and extracted with EtOAc. The combined organic extracts were washed with water and brine, dried and concentrated *in vacuo.* The crude product was purified by preparative reversed phase HPLC on YMC Pack Pro C18 stationary phase (acetonitrile/water as eluent, 0.1% TFA as modifier) to afford the title compound 7d. *m*/*z* (ES) 430 (MH)⁺.

### Step E: Preparation of 2-{[4-(1-{4-[5-(bromomethyl)-1,2,4-oxadiazol-3-yl]phenyl}-1,2-dimethylpropyl)phenoxy]methyl}pyridine (7e)

Triphenylphospine (82.0 mg, 0.321 mmol) was added to a stirred solution of carbon tetrabromide (106 mg, 0.321 mmol) and 7d (92.0 mg, 0.214 mmol) in DCM (2.00 mL)at rt. After 30 min, the reaction mixture was purified directly by flash chromatography on silica gel (gradient elution; 0%-70% EtOAc/hexanes as eluent) to afford the title compound 7e. *m*/*z* (ES) 492 (MH)⁺.

### Step F: Preparation of 4-{[3-(4-[1,2-dimethyl-1-[4-(pyridin-2-ylmethoxy)phenyl]propyl} - phenyl)-1,2,4-oxadiazol-5-yl]methyl}morpholine (7f)

Morpholine (69.0 µL, 0.789 mmol) was added to a stirred solution of 7e (39.0 mg, 0.0792 mmol) in DCM (1.00 mL) at rt. After 1 h, the reaction mixture was poured into satd. aq. sodium bicarbonate and extracted with EtOAc. The combined organic extracts were washed with water and brine, dried (Na₂SO₄) and concentrated *in vacuo.* The crude residue was purified by preparative reversed phase HPLC on YMC Pack Pro C18 stationary phase (acetonitrile/water as eluent, 0.1% TFA as modifier) to afford the title compound 7f. *m*/*z* (ES) 499 (MH)⁺.

Following procedures similar to those described above for making Compound 7f, the following compounds in Table 7 can be prepared:

**Table 7**

| | | | | | | |
|---|---|---|---|---|---|---|
| 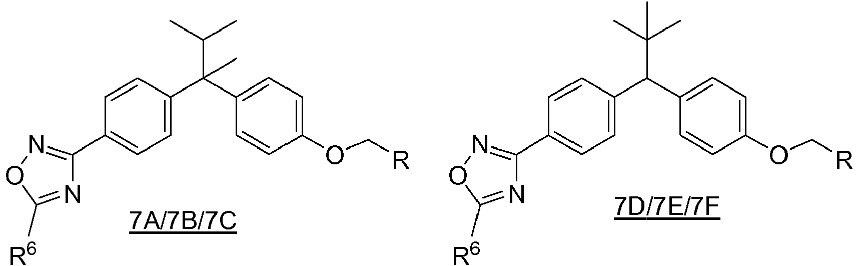 | | | | | | |

| Ex.7A R=2-PYR | Ex. 7B R=2-PYM | Ex. 7C R=2-TZE | Ex.7D R=2-PYR | Ex. 7E R=2-PYM | Ex. 7F R=2-TZE | R⁶ |
|---|---|---|---|---|---|---|
| a | a | a | a | a | a | Me |
| b | b | b | b | b | b | -CH₂F |
| c | c | c | c | c | c | Et |
| d | d | d | d | d | d | *ⁱ*Pr |
| e | e | e | e | e | e | -CH₂OH |
| f | f | f | f | f | f | 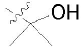 |
| g | g | g | g | g | g | 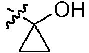 |
| h | h | h | h | h | h | 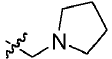 |
| i | i | i | i | i | i | 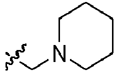 |
| -- | J | J | J | j | J | 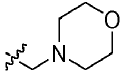 |
| k | k | k | k | k | k | 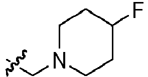 |
| l | l | l | l | l | l | 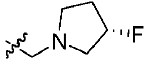 |
| m | m | m | m | m | m | 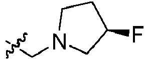 |
| n | n | n | n | n | n | 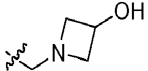 |
| o | o | o | o | o | o | 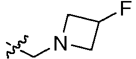 |
| p | p | p | p | p | p | 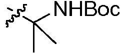 |
| q | q | q | q | q | q | 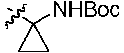 |

Table 7. Parent Ion *m*/*z* (MH)⁺ data for compounds.
For 7Aa: 2-[(4- {1,2-dimethyl-1-[4-(5-methyl-1,2,4-oxadiazol-3-yl)phenyl]propyl}phenoxy)-methyl]pyridine: *m*/*z* (ES) 414 (MH)⁺.
For 7Ae: [3-(4-{1,2-dimethyl-1-[4-(pyridin-2-ylmethoxy)phenyl]propyl}phenyl)-1,2,4-oxadiazol-5-yl]methanol: *m*/*z* (ES) 430 (MH)⁺.
For 7Af: 2-[3-(4-{1,2-dimethyl-1-[4-(pyridin-2-ylmethoxy)phenyl]propyl}phenyl)-1,2,4-oxadiazol-5-yl]propan-2-ol: *m*/*z* (ES) 458 (MH)⁺.
For 7Ag: 1-[3-(4-{1,2-dimethyl-1-[4-(pyridin-2-ylmethoxy)phenyl]propyl}phenyl)-1,2,4-oxadiazol-5-yl]cyclopropanol: *m*/*z* (ES) 456 (MH)⁺.
For 7Ap: *tert*-butyl {1-[3-(4-{1,2-dimethyl-1-[4-(pyridin-2-ylmethoxy)phenyl]propyl}phenyl)-1,2,4-oxadiazol-5-yl]-1-methylethyl}carbamate: *m*/*z* (ES) 557 (MH)⁺.
For 7Aq: *tert*-butyl {1-[3-(4-{1,2-dimethyl-1-[4-(pyridin-2-ylmethoxy)phenyl]propyl}phenyl)-1,2,4-oxadiazol-5-yl]cyclopropyl}carbamate: *m*/*z (ES)* 555 (MH)⁺.
For 7Bf: 2-[3-(4-{1,2-dimethyl-1-[4-(pyrimidin-2-ylmethoxy)phenyl]propyl}phenyl)-1,2,4-oxadiazol-5-yl]propan-2-ol: *m*/*z* (ES) 459 (MH)⁺.
For 7Cf: 2-[3-(4- {1,2-dimethyl-1-[4-(thiazol-2-ylmethoxy)phenyl]propyl}phenyl)-1,2,4-oxadiazol-5-yl]propan-2-ol: *m*/*z* (ES) 464 (MH)⁺.
For 7Cg: 1-[3-(4- {1,2-dimethyl-1-[4-(thiazol-2-ylmethoxy)phenyl]propyl}phenyl)-1,2,4-oxadiazol-5-yl]cyclopropanol: *m*/*z* (ES) 462 (MH)⁺.
For 7Da: 2-[(4- {2,2-dimethyl-1-[4-(5-methyl-1,2,4-oxadiazol-3-yl)phenyl]propyl}phenoxy)-methyl]pyridine: *m*/*z* (ES) 414 (MH)⁺.
For 7Db: 2- {[4-(1-{4-[5-(fluoromethyl)-1,2,4-oxadiazol-3-yl]phenyl}-2,2-dimethylpropyl)-phenoxy]methyl}pyridine: *m*/*z* (ES) 432 (MH)⁺.
For 7Df: 2-[3-(4- {2,2-dimethyl-1-[4-(pyridin-2-ylmethoxy)phenyl]propyl}phenyl)-1,2,4-oxadiazol-5-yl]propan-2-ol: *m*/*z* (ES) 458 (MH)⁺.
For 7Dp: *tert*-butyl {1-[3-(4-{2,2-dimethyl-1-[4-(pyridin-2-ylmethoxy)phenyl]propyl}phenyl)-1,2,4-oxadiazol-5-yl]-1-methylethyl}carbamate: *m*/*z* (ES) 557 (MH)⁺.

### EXAMPLE 8

### Preparation of 2-[3-(4-{2,2-dimethyl-1-[4-(pyridin-2-ylmethoxy)phenyl]propyl}phenyl)-1,2,4-oxadiazol-5-yl]propan-2-amine (8a)

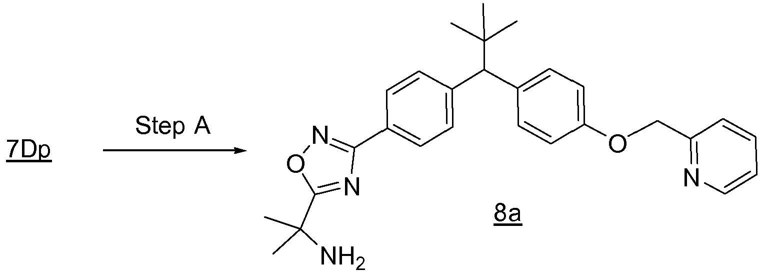

Compound 8a was prepared following the procedures described in example 2, step B, substituting compound 7Dp for compound 2a. *m*/*z* (ES) 457 (MH)⁺.

Following procedures similar to those described above for making Compound 8a, the following compounds in Table 8 can be prepared:

**Table 8**

| | | | | | | | |
|---|---|---|---|---|---|---|---|
| 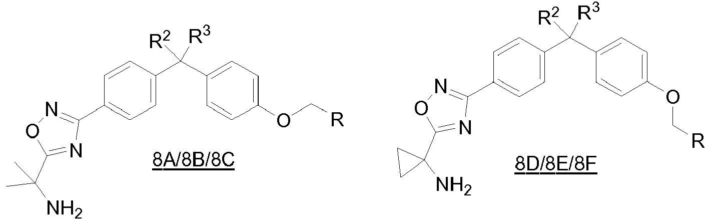 | | | | | | | |

| Ex.8A | Ex. 8B | Ex. 8C | Ex.8D | Ex. 8E | Ex. 8F | R² | R³ |
|---|---|---|---|---|---|---|---|
| R=2-PYR | R=2-PYM | R=2-TZE | R=2-PYR | R=2-PYM | R=2-TZE | | |
| a | a | a | a | a | a | *ⁱ*Pr | H |
| -- | b | b | b | b | b | *^{t}*Bu | H |
| c | c | c | c | c | c | c-Pr | H |
| d | d | d | d | d | d | c-Bu | H |
| e | e | e | e | e | e | 1-Me-c-Pr | H |
| f | f | f | f | f | f | 1-Me-c-Bu | H |
| g | g | g | g | g | g | *ⁱ*Pr | Me |

Table 8. Parent Ion *m*/*z* (MH)⁺ data for compounds.
For 8Ag: 2-[3-(4- {1,2-dimethyl-l-[4-(pyridin-2-ylmethoxy)phenyl]propyl}phenyl)-1,2,4-oxadiazol-5-yl]propan-2-amine: *m*/*z* (ES) 457 (MH)⁺.
For 8Cg: 1-[3-(4- {1,2-dimethyl-1-[4-(thiazol-2-ylmethoxy)phenyl]propyl}phenyl)-1,2,4-oxadiazol-5-yl]cyclopropanamine: *m*/*z* (ES) 463 (MH)⁺.
For 8Dg: 1-[3-(4- {1,2-dimethyl-1-[4-(pyridin-2-ylmethoxy)phenyl]propyl}phenyl)-1,2,4-oxadiazol-5-yl]cyclopropanamine: *m*/*z* (ES) 455 (MH)⁺.
For 8Fe: 1-[3-(4- {1,2-dimethyl-1-[4-(thiazol-2-ylmethoxy)phenyl]propyl}phenyl)-1,2,4-oxadiazol-5-yl]cyclopropanamine: *m*/*z* (ES) 461 (MH)⁺.

### EXAMPLE 9

### Preparation of 3-(4-{2,2-dimethyl-1-[4-(pyridin-2-ylmethoxy)phenyl]propyl}phenyl)-1,2,4-oxadiazol-5(4H)-one (9c)

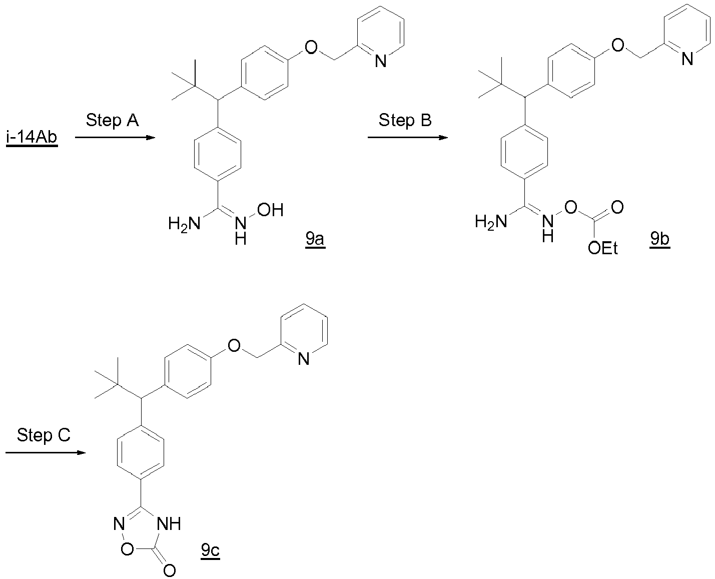

### Step A: Preparation of 4-{2,2-dimethyl-1-[4-(pyridin-2-ylmethoxy)phenyl]propyl}-N'-hydroxybenzenecarboximidamide (9a)

Compound 9a was prepared following the procedures described in example 7, step A, substituting intermediate i-14Ab for intermediate i-14b. *m*/*z* (ES) 390 (MH)⁺.

### Step B : Preparation of 4-{2,2-dimethyl-1-[4-(pyridin-2-ylmethoxy)phenyl]propyl}-N'-[(ethoxycarbonyl)oxy]benzenecarboximidamide (9b)

Ethyl chloroformate (39 µL, 0.40 mmol) was added to a solution of pyridine (40 µL, 0.50 mmol) and 9a (0.15 g, 0.39 mmol) in DCM (6.0 mL) at 0 °C. After 1 h, the reaction mixture was loaded directly onto a silica gel column and purified by flash chromatography (gradient elution; 0%-3% methanol/DCM as eluent) to furnish the title compound 9a. *m*/*z* (ES) 462 (MH)⁺.

### Step C : Preparation of 3-(4-{2,2-dimethyl-1-[4-(pyridin-2-ylmethoxy)phenyl]propyl}-phenyl)-1,2,4-oxadiazol-5(4H)-one (9c)

Compound 9c was prepared following the procedures described in example 7, step C, substituting compound 9b for compound 7b. *m*/*z* (ES) 416 (MH)⁺.

Following procedures similar to that described above for making Compound 9c, the following compounds in Table 9 can be prepared:

| | | | | |
|---|---|---|---|---|
| 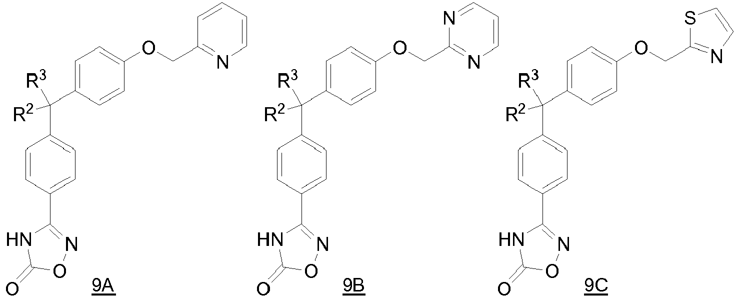 | | | | |

| Ex. 9A | Ex. 9B | Ex. 9C | R² | R³ |
|---|---|---|---|---|
| a | a | a | *i-Pr* | H |
| b | b | b | *t*Bu | H |
| c | c | c | c-Pr | H |
| d | d | d | c-Bu | H |
| e | e | e | 1-Me-c-Pr | H |
| f | f | f | 1-Me-c-Bu | H |
| g | g | g | *ⁱ*Pr | Me |

### EXAMPLE 10

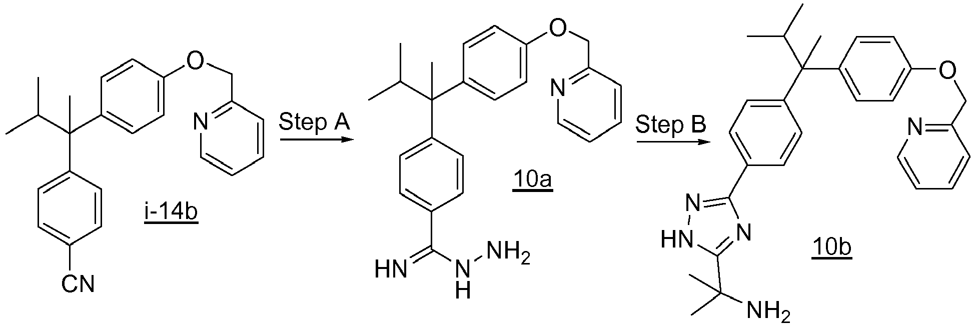

### Step A: Preparation of 4-{1,2-dimethyl-1-[4-(pyridin-2-ylmethoxy)phenyl]propyl}benzene-carboximidohydrazide (10a)

A solution of i-14b (455 mg, 1.27 mmol) in THF (2.00 mL) was added dropwise to a stirred suspension of sodium hydride (101 mg, 2.54 mmol) and anhydrous hydrazine (80.0 µL, 2.54 mmol) in THF (4.00 mL) at 0 °C. After 45 min, the reaction was quenched with satd. aq. ammonium chloride and extracted with EtOAc. The combined organic extracts were washed with satd. aq. sodium bicarbonate, water and brine, dried, and concentrated *in vacuo* to afford the title compound 10a. *m*/*z* (ES) 389 (MH)⁺.

### Step B: Preparation of 2-[5-(4-{1,2-dimethyl-1-[4-(pyridin-2-ylmethoxy)phenyl]propyl}-phenyl)-4H-1,2,4-triazol-3-yl]propan-2-amine (10b)

Compound 10b was prepared following the procedures described in example 7, steps B-C, substituting compound 10a for compound 7a, and the product of this reaction for compound 7b, that yielded an intermediate that was substituted for compound 7Dp in example 8 to afford the title compound 10b. *m*/*z* (ES) 456 (MH)⁺.

Following procedures similar to those described above for making Compound 10b, the following compounds in Table 10 can be prepared:

**Table 10**

| | | | | | | |
|---|---|---|---|---|---|---|
| 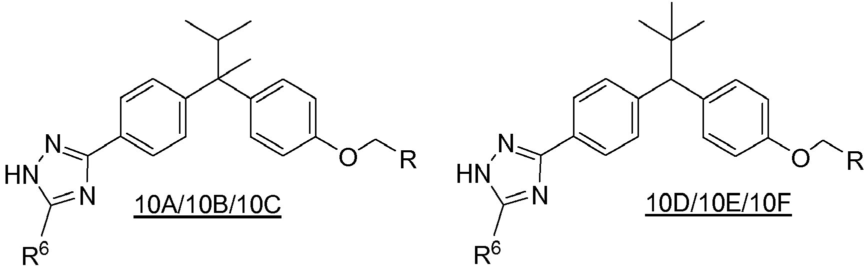 | | | | | | |

| Ex. 10A | Ex. 10B | Ex. 10C | Ex. 10D | Ex. 10E | Ex. 10F | R⁶ |
|---|---|---|---|---|---|---|
| R=2-PYR | R=2-PYM | R=2-TZE | R=2-PYR | R=2-PYM | R=2-TZE | |
| a | a | a | a | a | a | -CH₂NH₂ |
| -- | b | b | b | b | b | 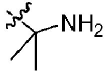 |
| c | c | c | c | c | c | 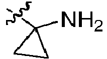 |
| d | d | d | d | d | d | 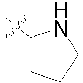 |
| e | e | e | e | e | e | 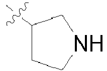 |
| f | f | f | f | f | f | 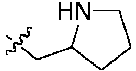 |
| g | g | g | g | g | g | 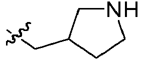 |
| h | h | h | h | h | h | 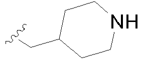 |

Table 10. Parent Ion *m*/*z* (MH)⁺ data for compounds.
For 10Aa: 1-[3-(4-{1,2-dimethyl-1-[4-(pyridin-2-ylmethoxy)phenyl]propyl}phenyl)-1*H*-1,2,4-triazol-5-yl]methanamine: *m*/*z* (ES) 428 (MH)⁺
For 10Ae: 2-[(4-{1,2-dimethyl-1-[4-(5-pyrrolidin-3-yl-1*H*-1,2,4-triazol-3-yl)phenyl]propyl}-phenoxy)methyl]pyridine: *m*/*z* (ES) 468 (MH)⁺
For 10Af: 2- {[4-(1,2-dimethyl-1-(4-[5-(pyrrolidin-2-ylmethyl)-1*H*-1,2,4-triazol-3-yl]phenyl}-propyl)phenoxy]methyl}pyridine: *m*/*z* (ES) 482 (MH)⁺
For 10Ag: 2- {[4-(1,2-dimethyl-1-{4-[5-(pyrrolidin-3-ylmethyl)-1*H*-1,2,4-triazol-3-yl]phenyl}-propyl)phenoxy]methyl}pyridine: *m*/*z* (ES) 482 (MH)⁺

### EXAMPLE 11

### Preparation of 2-({4-[2,2-dimethyl-1-(4-{2-[(3R)-pyrrolidin-3-ylmethyl]-2H-1,2,3-triazol-4-yl}phenyl)propyl]phenoxy}methyl)pyridine (11e)

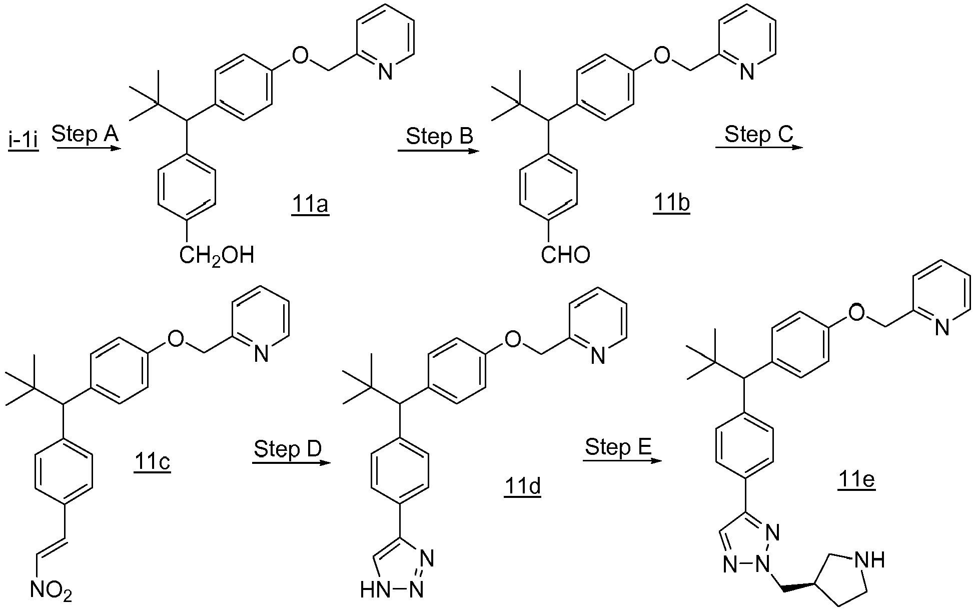

### Step A: Preparation of (4-{2,2-dimethyl-1-[4-(pyridin-2-ylmethoxy)phenyl]propyl}phenyl)-methanol (11a)

DIBAL-H (5.93 mL of a 1.5 M toluene solution) was added to a solution of i-1i (990 mg, 2.54 mmol) in toluene (19.0 mL) at -50 °C. After 20 min, the reaction mixture was warmed to -10 °C. After 1.5 h, the reaction mixture was quenched with satd. aq. sodium-potassium tartrate and extracted with EtOAc. The combined organic extracts were washed with brine, dried and concentrated *in vacuo* to afford the title compound 11a. *m*/*z* (ES) 362 (MH)⁺.

### Step B: Preparation of 4-{2,2-dimethyl-1-[4-(pyridin-2-ylmethoxy)phenyl]propyl}-benzaldeh. (11b)

DMSO (414 µL, 5.84 mmol) was added slowly dropwise to a stirred solution of oxalyl chloride (354 µL, 4.06 mmol) in DCM (30.0 mL) at -78 °C. After 20 min, a solution of 11a (2.54 mmol, theoretical) in DCM (17.0 mL) was added slowly dropwise, which after an additional 45 min, was followed by the addition of triethylamine (1.42 mL, 10.2 mmol). After an additional 15 min, the reaction mixture was allowed to warm to rt over 30 min. The reaction mixture was quenched with satd. aq. sodium bicarbonate and extracted with EtOAc. The combined organic extracts were washed with water and brine, dried and concentrated *in vacuo.* The crude residue was purified by flash chromatography on silica gel (gradient elution; 0%-70% EtOAc/hexanes as eluent) to afford the title compound 11b. *m*/*z* (ES) 360 (MH)⁺.

### Step C: Preparation of 2-{[4-(2,2-dimethyl-1-{4-[(E)-2-nitrovinyl]phenyl}propyl)-phenoxy]methyl}pyridine (11c)

Ammonium acetate (68.0 mg, 0.885 mmol) was added to a stirred solution of 11b (795 mg, 2.21 mmol) in nitromethane (4.00 mL), and the resulting mixture was heated to reflux. After 1.5 h, the reaction mixture was diluted with EtOAc, and washed with water and brine. The combined organic extracts were dried, concentrated *in vacuo* and purified by flash chromatography on silica gel (gradient elution; 0%-60% EtOAc/hexanes as eluent) to afford the title compound 11c. *m*/*z* (ES) 403 (MH)⁺.

### Step D: Preparation of 2-[(4-{2,2-dimethyl-1-[4-(1H-1,2,3-triazol-4-yl)phenyl]propyl}-phenoxy)methyl]pyridine (11d)

A stirred mixture of sodium azide (232 mg, 3.56 mmol) and 11c (477 mg, 1.19 mmol) in DMSO (5.00 mL) was heated to 50 °C. After 16 h, the reaction mixture was diluted with EtOAc, and washed with water and brine. The combined organic extracts were dried, concentrated *in vacuo* and purified by flash chromatography on silica gel (gradient elution; 0%-100% EtOAc/hexanes as eluent) to afford the title compound 11d. *m*/*z* (ES) 399 (MH)⁺.

### Step E: Preparation of 2-({4-[2,2-dimethyl-1-(4-{2-[(3R)-pyrrolidin-3-ylmethyl]-2H-1,2,3-triazol-4-yl}phenyl)propyl]phenoxy}methyl)pyridine (11e)

Compound 11e was prepared following the procedures described in example 2, step C, substituting compound 11d for intermediate i-18Bg. *m*/*z* (ES) 482 (MH)⁺.

Following procedures similar to those described above for making Compound 11e, the following compounds in Table 11 can be prepared:

**Table 11**

| | | | | | | |
|---|---|---|---|---|---|---|
| 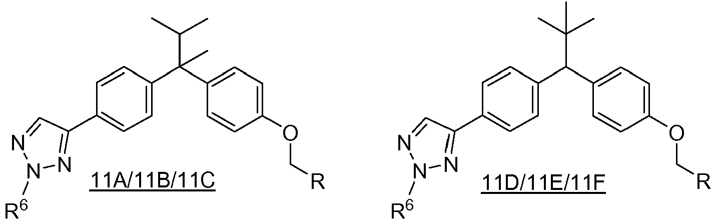 | | | | | | |

| Ex. 11A | Ex. 11B | Ex. 11C | Ex. 11D | Ex. 11E | Ex. 11F | R⁶ |
|---|---|---|---|---|---|---|
| R=2-PYR | R=2-PYM | R=2-TZE | R=2-PYR | R=2-PYM | R=2-TZE | |
| a | a | a | a | a | a | Me |
| b | b | b | b | b | b | Et |
| c | c | c | c | c | c | 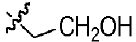 |
| d | d | d | d | d | d | 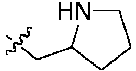 |
| e | e | e | -- | e | e | 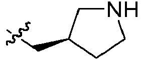 |
| f | f | f | f | f | f | 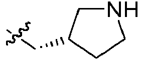 |
| g | g | g | g | g | g | 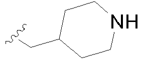 |

Table 11. Parent Ion *m*/*z* (MH)⁺ data for compounds.
For 11Da: 2-[(4-{2,2-dimethyl-1-[4-(2-methyl-2*H*-1,2,3-triazol-4-yl)phenyl]propyl}phenoxy)-methyl]pyridine: *m*/*z* (ES) 413 (MH)⁺
For 11Dc: 2-[4-(4-{2,2-dimethyl-1-[4-(pyridin-2-ylmethoxy)phenyl]propyl}phenyl)-2*H*-1,2,3-triazol-2-yl] ethanol: *m*/*z* (ES) 443 (MH)⁺
For 11Df:2-({4-[2,2-dimethyl-1-(4-{2-[(3S)-pyrrolidin-3-ylmethyl]-2*H*-1,2,3-triazol-4-yl}phenyl)propyl]phenoxy}methyl)pyridine: *m*/*z* (ES) 481 (MH)⁺

### EXAMPLE 12

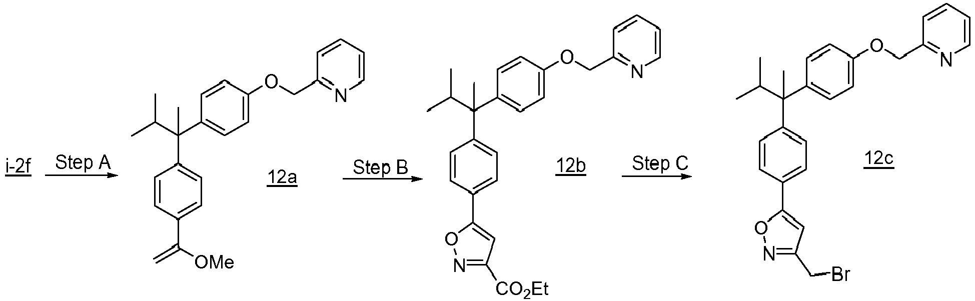

### Step A: Preparation of 2-[(4-{1-[4-(1-methoxyvinyl)phenyl]-1,2-dimethylpropyl}-phenoxy)methyl]pyridine (12a)

Tebbe reagent (7.34 mL of a 0.5 M toluene solution) was added to a solution of i-2f (1.43 g, 3.67 mmol) in THF (10.0 mL) at rt. After 16 h, the reaction mixture was filtered through alumina, and the solids were rinsed with EtOAc. The combined organic extracts were concentrated *in vacuo* to afford the title compound 12a. *m*/*z* (ES) 374 (M-13)⁺

### Step B: Preparation of ethyl 5-(4-{1,2-dimethyl-1-[4-(pyridin-2-ylmethoxy)phenyl]-propyl}phenyl)isoxazole-3-carboxylate (12b)

Ethyl 2-chloro-2-(hydroxyimino)acetate (1.67 g, 11.01 mmol) was added to a stirred solution of triethylamine (5.12 mL, 36.7 mmol) and 12a (crude product from Step A; 3.67 mmol, theoretical) in THF (100 mL). Excess TFA was added, and the resulting mixture was heated to 50 °C. After 2 h, the reaction mixture was concentrated *in vacuo,* and the crude residue was purified by flash chromatography on silica gel (gradient elution; 0%-100% EtOAc/hexanes as eluent) to afford the title compound 12b. *m*/*z* (ES) 471 (MH)⁺

### Step C: Preparation of 2-{[4-(1-{4-[3-(bromomethyl)isoxazol-5-yl]phenyl}-1,2-dimethylpropyl)phenoxy]methyl}pyridine (12c)

Lithium aluminum hydride (166 µL of a 1.0 M THF solution) was added to a stirred solution of 12b (65.0 mg, 0.138 mmol) in diethyl ether (1.00 mL) at 0 °C. After 15 min, the reaction mixture was quenched with 1.0 N sodium hydroxide and extracted with EtOAc. The combined organic extracts were dried and concentrated *in vacuo* to afford an intermediate that was converted to the title compound 12c following procedures as described in example 7, step E, substituting the aforementioned intermediate for compound 7d. *m*/*z* (ES) 493 (MH)⁺

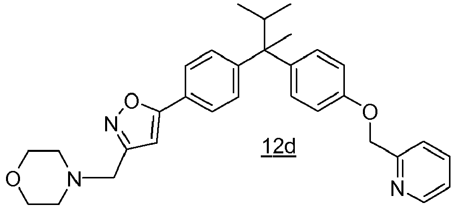

### Step D: Preparation of 4-{[5-(4-{1,2-dimethyl-1-[4-(pyridm-2-ylmethoxy)phenyl]propyl}-phenyl)isoxazol-3-yl]methyl}morpholine (12d)

Compound 12d can be prepared following the procedures described in example 7, step F, substituting compound 12c for compound 7e. *m*/*z* (ES) 457 (MH)⁺

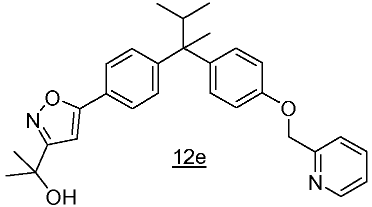

### Step E: Preparation of 2-[5-(4-{1,2-dimethyl-1-[4-(pyridin-2-ylmethoxy)phenyl]propyl}-phenyl)isoxazol-3-yl]propan-2-ol (12e)

Methyl magnesium bromide (334 mL of a 1.4 M (3:1) toluene:THF solution) was added to a stirred solution of 12b (55.0 mg, 0.117 mmol) in THF (1.00 mL) at 0 °C. After 1 h, the reaction mixture was quenched with water, and the resulting mixture was purified directly by flash chromatography on silica gel (gradient elution; 0%-100% EtOAc/hexanes as eluent) to afford the title compound 12e. *m*/*z* (ES) 457 (MH)⁺

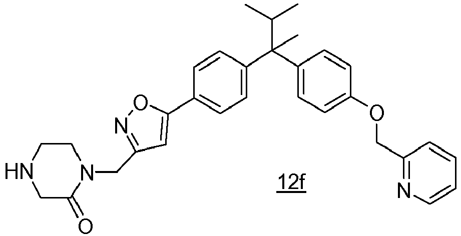

### Step F: Preparation of 1-{[5-(4-{1,2-dimethyl-1-[4-(pyridin-2-ylmethoxy)phenyl]propyl}-phenyl)isoxazol-3-yl]methyl}piperazin-2-one (12f)

A stirred solution of 12c (20.0 mg, 0.0407 mmol), *tert*-butyl 3-oxopiperazine-1-carboxylate (16.4 mg, 0.0819 mmol) and cesium carbonate (67.0 mg, 0.205 mmol) in DMF (1.00 mL) was heated to 60 °C. After 2 h, the reaction mixture was purified directly by preparative reversed phase HPLC on YMC Pack Pro C18 stationary phase (acetonitrile/water as eluent, 0.1 % TFA as modifier) to afford an intermediate compound that was deprotected to afford compound 12f following the procedures described. *m*/*z* (ES) 511 (MH)⁺

Following procedures similar to those described above, the following compounds in Table 12 can be prepared:

**Table 12**

| | | | | | | |
|---|---|---|---|---|---|---|
| 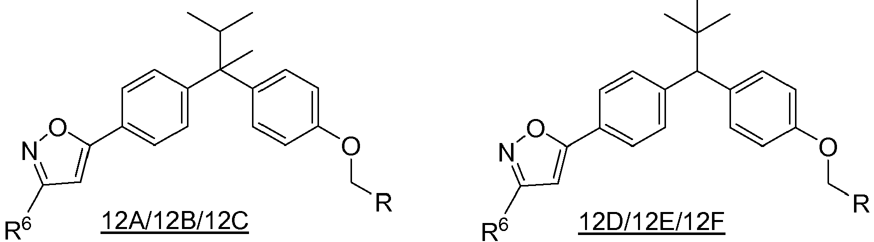 | | | | | | |

| Ex. 12A | Ex. 12B | Ex. 12C | Ex. 12D | Ex. 12E | Ex. 12F | R⁶ |
|---|---|---|---|---|---|---|
| R=2-PYR | R=2-PYM | R=2-TZE | R=2-PYR | R=2-PYM | R=2-TZE | |
| -- | a | a | a | a | a | -CH₂OH |
| -- | b | b | b | b | b | -C(CH₃)₂OH |
| c | c | c | c | c | c | 1-OH-c-Pr |
| d | d | d | d | d | d | 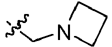 |
| e | e | e | e | e | e | 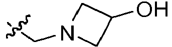 |
| f | f | f | f | f | f | 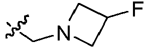 |
| g | g | g | g | g | g | 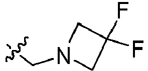 |
| h | h | h | h | h | h | 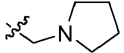 |
| i | i | i | i | i | i | 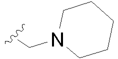 |
| -- | J | J | J | J | J | 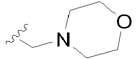 |
| -- | k | k | k | k | k | 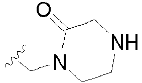 |
| l | l | l | l | l | l | 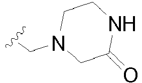 |

Table 12. Parent Ion *m*/*z* (MH)⁺ data for compounds.
For 12Ac: 1-[5-(4-{1,2-dimethyl-1-[4-(pyridin-2-ylmethoxy)phenyl]propyl}phenyl)isoxazol-3-yl]cyclopropanol: *m*/*z* (ES) 455 (MH)⁺
For 12Ae: 1- {[5-(4- {1,2-dimethyl-1-[4-(pyridin-2-ylmethoxy)phenyl]propyl}phenyl)isoxazol-3-yl]methyl}azetidin-3-ol: *m*/*z* (ES) 484 (MH)⁺
For 12Af: 2-({4-[1-(4- {3-[(3-fluoroazetidin-1-yl)methyl]isoxazol-5-yl}phenyl)-1,2-dimethylpropyl]phenoxy}methyl)pyridine: *m*/*z* (ES) 486 (MH)⁺
For 12Ag: 2-({4-[1-(4-{3-[(3,3-difluoroazetidin-1-yl)methyl]isoxazol-5-yl}phenyl)-1,2-dimethylpropyl]phenoxy}methyl)pyridine: *m*/*z* (ES) 504 (MH)⁺
For 12Ah: 2-({4-[1-(4-{3-[(pyrrolidin-1-yl)methyl]isoxazol-5-yl}phenyl)-1,2-dimethylpropyl]-phenoxy}methyl)pyridine: *m*/*z* (ES) 482 (MH)⁺
For 12A1: 4- {[5-(4-{1,2-dimethyl-1-[4-(pyridin-2-ylmethoxy)phenyl]propyl}phenyl)isoxazol-3-yl]methyl}piperazin-2-one: *m*/*z* (ES) 511 (MH)⁺

### EXAMPLE 13

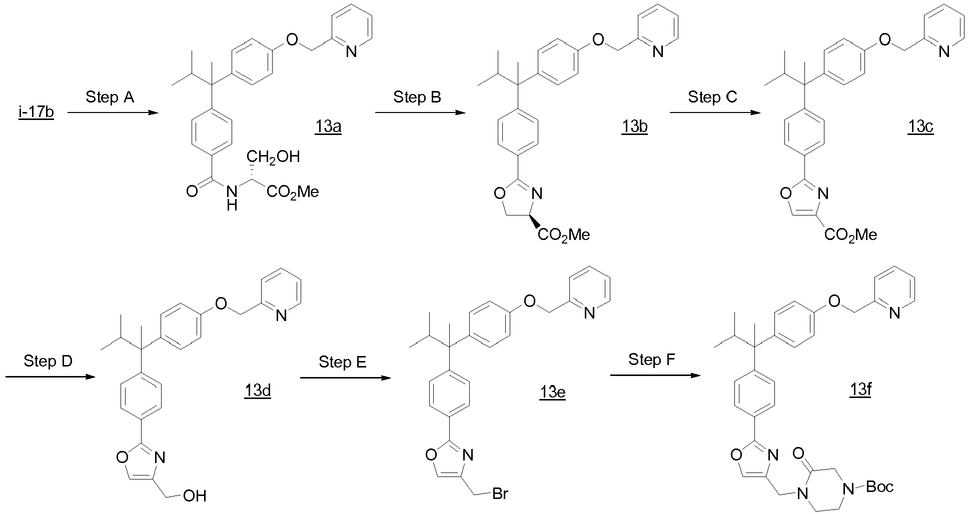

### Step A: Preparation of methyl (2R)-2-[(4-{1,2-dimethyl-1-[4-(pyridin-2-ylmethoxy)-pheny]lpropyl}benzoyl)amino]-3-hydroxypropanoate (13a)

Compound 13a can be prepared following the procedures described in example 5, step A, substituting D-serine methyl ester for *tert*-butyl 4-aminopiperidine-1-carboxylate. *m*/*z* (ES) 477 (MH)⁺

### Step B : Preparation of methyl (4R)-2-(4-{1,2-dimethyl-1-[4-(pyridin-2-ylmethoxy)phenyl]-propyl}phenyl)-4,5-dihydro-1,3-oxazole-4-carboxylate (13b)

DAST (352 µL, 2.184 mmol) was added dropwise to a stirred solution of 13a (1.04 g, 2.184 mmol) in DCM (30.0 mL) at -78 °C. After 2 h, potassium carbonate (362 mg, 2.184 mmol) was added, and the resulting mixture was allowed to warm to rt. The reaction mixture was poured idnto water and extracted with chloroform. The combined organic extracts were washed with water and brine, dried and concentrated *in vacuo.* The crude residue was purified by flash chromatography on silica gel (gradient elution; 40%-80% EtOAc/hexanes as eluent) to afford the title compound 13b. *m*/*z* (ES) 459 (MH)⁺.

### Step C: Preparation of methyl 2-(4-{1,2-dimethyl-1-[4-(pyridin-2-ylmethoxy)phenyl]-propyl}phenyl)-1,3-oxazole-4-carboxylate (13c)

Bromotrichloromethane (596 µL, 6.00 mmol) was added dropwise to a stirred solution of DBU (904 µL, 6.00 mmol) and 13b (550 mg, 1.20 mmol) in DCM (30.0 mL) at -20 °C. The resulting mixture was warmed to 0 °C and allowed to stir for 1 h. The reaction mixture was poured into satd. aq. sodium bicarbonate and extracted with EtOAc. The combined organic extracts were washed with water and brine, dried and concentrated *in vacuo.* The crude residue was purified by flash chromatography on silica gel (gradient elution; 20%-60% EtOAc/hexanes as eluent) to afford the title compound 13c. *m*/*z* (ES) 457 (MH)⁺.

### Step D : Preparation of [2-(4-{1,2-dimethyl-1-[4-(pyridin-2-ylmethoxy)phenyl]propyl}-phenyl)-1,3-oxazol-4-yl]methanol (13d)

Compound 13d can be prepared following the procedures as described and referred to in example 12, step C, substituting compound 13c for compound 12b. *m*/*z* (ES) 429 (MH)⁺

### Step E : Preparation of [2-(4-{1,2-dimethyl-1-[4-(pyridin-2-ylmethoxy)phenyl]propyl}-phenyl)-1,3-oxazol-4-yl]bromomethane (13e)

Compound 13e can be prepared following the procedures described in example 7, step E, substituting compound 13d for compound 7d. *m*/*z* (ES) 491 (MH)⁺

### Step F: Preparation of tert-butyl 4-{[2-(4-{1,2-dimethyl-1-[4-(pyridin-2-ylmethoxy)-phenyl]propyl}phenyl)-1,3-oxazol-4-yl]methyl}-3-oxopiperazine-1-carboxylate (13f)

Compound 13f can be prepared following the procedures described in example 12, step F, substituting compound 13e for compound 12c. *m*/*z* (ES) 611 (MH)⁺

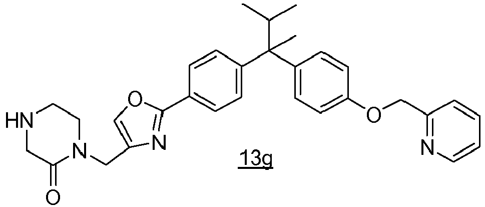

### Step G: Preparation of 1-{[2-(4-{1,2-dimethyl-1-[4-(pyridin-2-ylmethoxy)phenyl]propyl}-phenyl)-1,3-oxazol-4-yl]methyl}piperazin-2-one (13g)

Compound 13g can be prepared following the procedures described in example 2, step B, substituting compound 13f for compound 2a. *m*/*z* (ES) 511 (MH)⁺

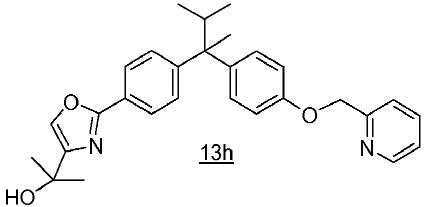

### Step H: Preparation of 2-[2-(4-{1,2-dimethyl-1-[4-(pyridin-2-ylmethoxy)phenyl]propyl}-phenyl)-1,3-oxazol-4-yl]propan-2-ol (13h)

Compound 13h can be prepared following the procedures described in example 12, step E, substituting compound 13c for compound 12b.

Following procedures similar to those described above for making Compounds 13f, 13g and 13h, the following compounds in Table 13 can be prepared:

**Table 13**

| | | | | | | |
|---|---|---|---|---|---|---|
| 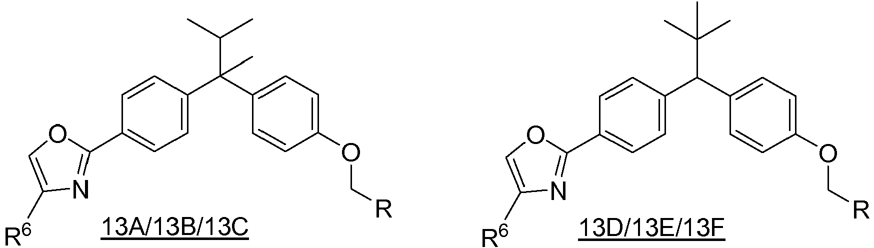 | | | | | | |

| Ex. 13A | Ex. 13B | Ex. 13C | Ex. 13D | Ex. 13E | Ex. 13F | R⁶ |
|---|---|---|---|---|---|---|
| R=2-PYR | R=2-PYM | R=2-TZE | R=2-PYR | R=2-PYM | R=2-TZE | |
| a | a | a | a | a | a | 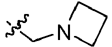 |
| b | b | b | b | b | b | 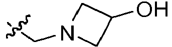 |
| c | c | c | c | c | c | 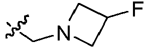 |
| d | d | d | d | d | d | 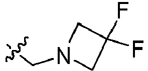 |
| e | e | e | e | e | e | 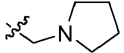 |
| -- | f | f | f | f | f | 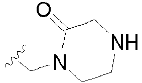 |
| g | g | g | g | g | g | 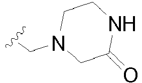 |
| -- | h | h | h | h | h | -CO₂Me |
| -- | i | i | i | i | i | -CH₂OH |
| -- | J | J | J | J | J | -C(CH₃)₂OH |
| k | k | k | k | k | k | -CH₂CN |

Table 13. Parent Ion *m*/*z* (MH)⁺ data for compounds.
For 13Ab: 1- {[2-(4-{1,2-dimethyl-1-[4-(pyridin-2-ylmethoxy)phenyl]propyl}phenyl)-1,3-oxazol-4-yl]methyl}azetidin-3-ol: *m*/*z* (ES) 484 (MH)⁺
For 13Ac: 2-({4-[1-(4- {4-[(3-fluoroazetidin-1-yl)methyl]-1,3-oxazol-2-yl}phenyl)-1,2-dimethylpropyl]phenoxy}methyl)pyridine: *m*/*z* (ES) 487 (MH)⁺
For 13Ad: 2-({4-[1-(4-{4-[(3,3-difluoroazetidin-1-yl)methyl]-1,3-oxazol-2-yl}phenyl)-1,2-dimethylpropyl]phenoxy}methyl)pyridine: *m*/*z* (ES) 504 (MH)⁺
For 13Ae: 2-({4-[1-(4-{4-[(pyrrolidin-1-yl)methyl]-1,3-oxazol-2-yl}phenyl)-1,2-dimethylpropyl]-phenoxy}methyl)pyridine: *m*/*z* (ES) 483 (MH)⁺
For 13Ag: 4-{[2-(4-{1,2-dimethyl-1-[4-(pyridin-2-ylmethoxy)phenyl]propyl}phenyl)-1,3-oxazol-4-yl]methyl}piperazin-2-one: *m*/*z* (ES) 511 (MH)⁺
For 13Ak: [2-(4-{1,2-dimethyl-1-[4-(pyridin-2-ylmethoxy)phenyl]propyl}phenyl)-1,3-oxazol-4-yl]acetonitrile: : *m*/*z* (ES) 438 (MH)⁺
For 13Ch: methyl 2-(4- {1,2-dimethyl-1-[4-(1,3-thiazol-2-ylmethoxy)phenyl]propyl}phenyl)-1,3-oxazole-4-carboxylate: *m*/*z* (ES) 463 (MH)⁺
For 13Cj: 2-[2-(4-{1,2-dimethyl-1-[4-(1,3-thiazol-2-ylmethoxy)phenyl]propyl}phenyl)-1,3-oxazol-4-yl]propan-2-ol: *m*/*z* (ES) 445 (MH)⁺

### EXAMPLE 14

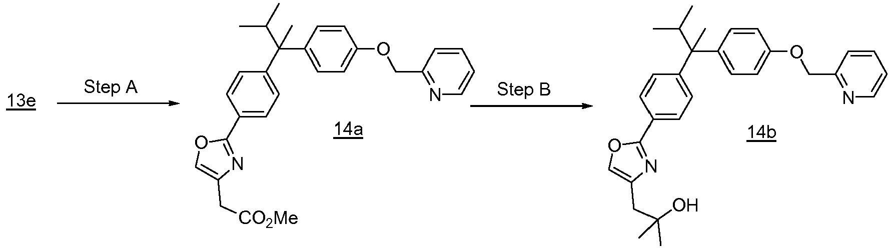

### Step A: Preparation of methyl 2-(4-{1,2-dimethyl-1-[4-(pyridin-2-ylmethoxy)phenyl]-propyl}phenyl)-1,3-oxazole-4-acetate (14a)

Bis(triphenylphosphine)palladium(II) chloride (0.5 mg, mmol) was added to a stirred solution of potassium carbonate (53.0 mg, 0.382 mmol) and 13e (170 mg, 0.347 mmol) in THF:methanol (6.00 mL of a 5:1 mixture). The reaction mixture was saturated with carbon monoxide and allowed to stir at rt under a carbon monoxide atmosphere (balloon) for 24 h. The reaction mixture was diluted with EtOAc, filtered through a short column of Celite®, and concentrated *in vacuo.* The crude residue was purified by preparative thin-layer chromatography on silica gel (50% EtOAc/hexanes as eluent) to afford the title compound 14a. *m*/*z* (ES) 471 (MH)⁺.

### Step B: Preparation of (14b)

Compound 14b was prepared following the procedures described in example 12, step E, substituting compound 14a for compound 12b. *m*/*z* (ES) 471 (MH)⁺.

Following procedures similar to those described above, the following compounds in Table 14 can be prepared:

**Table 14**

| | | | | | | |
|---|---|---|---|---|---|---|
| 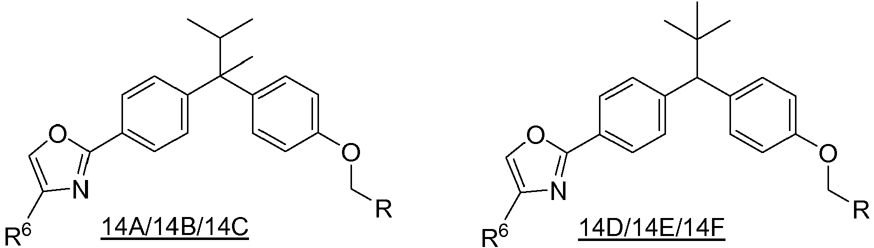 | | | | | | |

| Ex. 14A | Ex. 14B | Ex. 14C | Ex. 14D | Ex. 14E | Ex. 14F | R⁶ |
|---|---|---|---|---|---|---|
| R=2-PYR | R=2-PYM | R=2-TZE | R=2-PYR | R=2-PYM | R=2-TZE | |
| -- | a | a | a | a | a | 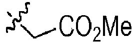 |
| -- | b | b | b | b | b | 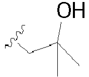 |

### EXAMPLE 15

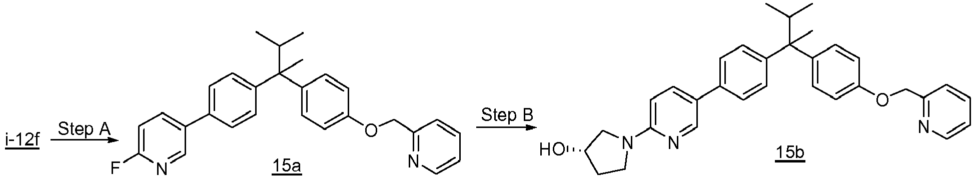

### Step A: Preparation of 5-(4-{1,2-dimethyl-1-[4-(pyridin-2-ylmethoxy)phenyl]propyl}-phenyl)-2-fluoropyridine (15a)

2-Fluoropyridine-5-boronic acid (926 mg, 6.56 mmol) was added to a stirred solution of i-12f (1.50 g, 3.28 mmol), sodium carbonate (8.20 mL of a 2.0 M aqueous solution) and [1,1'-bis(diphenylphosphino)ferrocene]dichloropalladium (53.6 mg, 0.0732 mmol) in THF (20.0 mL) and ethanol (20.0 mL). The resulting mixture was degassed and heated to 90 °C. After 2 h, the reaction mixture was cooled to rt, filtered through a short column of Celite® and concentrated *in vacuo.* The crude residue was purified by flash chromatography on silica gel (gradient elution; 0%-50% EtOAc/hexanes as eluent) to afford the title compound 15a. *m*/*z* (ES) 427 (MH)⁺.

### Step B: Preparation of (3S)-1-[5-(4-{1,2-dimethyl-1-[4-(pyridin-2-ylmethoxy)phenyl]-propyl}phenyl)pyridin-2-yl]pyrrolidin-3-ol (15b)

(*S*)-3-pyrrolidinol (110 mg, 1.15 mmol) was added to a stirred solution of 15a (25.0 mg, 0.0587 mmol) in NMP (2.00 mL), and the resulting mixture was heated to 110 °C. After 24 h, the reaction mixture was cooled to rt and diluted with EtOAc. The organic phase was washed with brine, dried and concentrated *in vacuo.* The crude residue was purified by preparative thin layer chromatography (50% EtOAc/hexanes as eluent) to afford the title compound 15b. *m*/*z* (ES) 495 (MH)⁺.

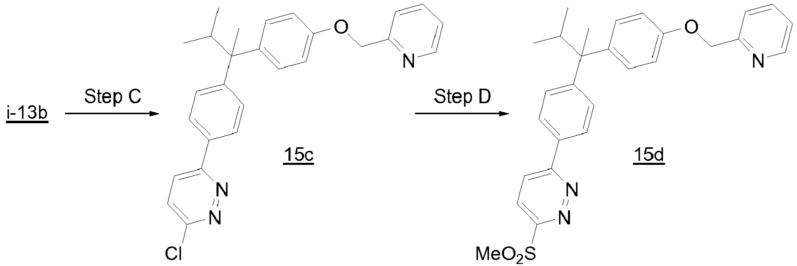

### Step C : Preparation of 3-chloro-6-(4-{1,2-dimethyl-1-[4-(pyridin-2-ylmethoxy)phenyl]-propyl}phenyl]pyridazine (15c)

3,6-Dichloropyridazine (462 mg, 3.10 mmol) was added to a stirred solution of i-13b (1.29 g, 2.82 mmol), sodium carbonate (2.82 mL of a 2.0 M aqueous solution) and and [1,1'-bis(diphenylphosphino)ferrocene] dichloropalladium (322 mg, 0.395 mmol) in toluene (7.50 mL) and ethanol (15.0 mL), and the resulting mixture was heated to reflux. After 1.5 h, the reaction mixture was filtered through Celite®, poured into satd. aq. sodium bicarbonate and extracted with EtOAc. The combined organic extracts were washed with brine, dried, and concentrated *in vacuo.* The crude residue was purified by flash chromatography on silica gel (gradient elution; 0%-40% EtOAc/hexanes as eluent) to afford the title compound 15c. *m*/*z* (ES) 444 (MH)⁺.

### Step D: Preparation of 3-(4-{1,2-dimethyl-1-[4-(pyridin-2-ylmethoxy)phenyl]propyl}-phenyl)-6-(methylsulfonyl)pyridazine (15d)

A solution of methanesulfinic acid, sodium salt (625 mg, 6.13 mmol) and 15c (1.36 g, 3.06 mmol) in DMF (20.0 mL) was heated to 120 °C. After 18 h, the reaction mixture was cooled to rt, poured into 1.0 M aqueous sodium carbonate and extracted with EtOAc. The organic extracts were dried, concentrated *in vacuo* and purified by flash chromatography on silica gel (gradient elution; 0%-50% EtOAc/hexanes as eluent) to afford the title compound 15d. *m*/*z* (ES) 488 (MH)⁺.

Following procedures similar to that described above, the following compounds in Table 15 can be prepared:

**Table 15**

| | | | | | | |
|---|---|---|---|---|---|---|
| 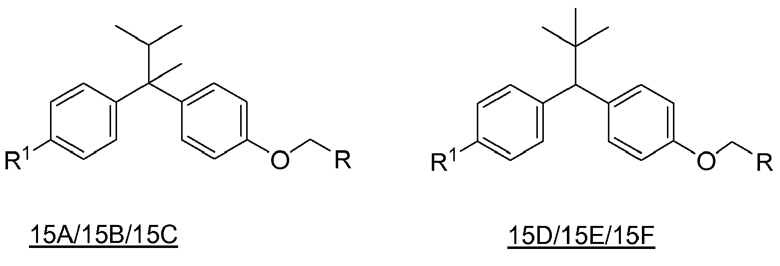 | | | | | | |

| Ex. 15A | Ex. 15B | Ex. 15C | Ex. 15D | Ex. 15E | Ex. 15F | R¹ |
|---|---|---|---|---|---|---|
| R=2-PYR | R=2-PYM | R=2-TZE | R=2-PYR | R=2-PYM | R=2-TZE | |
| a | a | a | a | a | a | 2-pyridyl |
| b | b | b | b | b | b | 3-pyridyl |
| c | c | c | c | c | c | 4-pyridyl |
| d | d | d | d | d | d | 2-pyrimidinyl |
| e | e | e | e | e | e | 4-pyrimidinyl |
| f | f | f | f | f | f | 6-F-3-pyridyl |
| g | g | g | g | g | g | 6-amino-3-pyridyl |
| h | h | h | h | h | h | 6-OMe-3-pyridyl |
| i | i | i | i | i | i | 6-nitro-3-pyridyl |
| J | J | J | J | J | j | 6-amino-2-pyridyl |
| k | k | k | k | k | k | 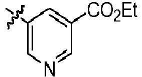 |
| l | l | l | l | l | l | 2-Me-4-pyrimidinyl |
| m | m | m | m | m | m | 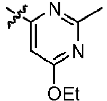 |
| n | n | n | n | n | n | 2-Cl-5-pyrimidinyl |
| o | o | o | o | o | o | 6-OH-3-pyridyl |
| p | p | p | p | p | p | 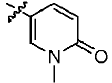 |
| q | q | q | q | q | q | 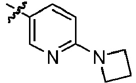 |
| r | r | r | r | r | r | 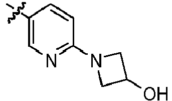 |
| s | s | s | s | s | s | 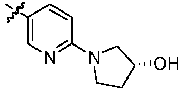 |
| t | t | t | t | t | t | 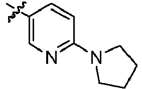 |
| u | u | u | u | u | u | 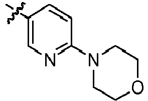 |
| v | v | v | v | v | v | 2-pyrazinyl |
| w | w | w | w | w | w | 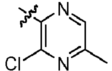 |
| x | x | x | x | x | x | 3-pyridazinyl |
| y | y | y | y | y | y | 6-Me-3-pyridazinyl |
| z | z | z | z | z | z | 6-OMe-3-pyridazinyl |
| aa | aa | aa | aa | aa | aa | 6-CF₃-3-Dvridazinyl |
| | | | | | | 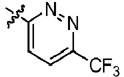 |
| ab | ab | ab | ab | ab | ab | 6-F-3-pyridazinyl |
| -- | ac | ac | ac | ac | ac | 6-Cl-3-pyridazinyl |
| ad | ad | ad | ad | ad | ad | 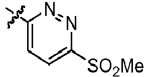 |
| ae | ae | ae | ae | ae | ae | 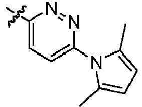 |
| af | af | af | af | af | af | 5-amino-2-pyrazinyl |
| ag | ag | ag | ag | ag | ag | 2-amino-4-pyrimidinyl |
| ah | ah | ah | ah | ah | ah | 2-amino-4-pyrimidinyl |

Table 15. Parent Ion *m*/*z* (MH)⁺ data for compounds
For 15Aa: 2-(4- {1,2-dimethyl-1-[4-(pyridin-2-ylmethoxy)phenyl]propyl}phenyl)pyridine: *m*/*z* (ES) 409 (MH)⁺
For 15Ab: 3-(4-{1,2-dimethyl-1-[4-(pyridin-2-ylmethoxy)phenyl]propyl}phenyl)pyridine: *m*/*z* (ES) 409 (MH)⁺
For 15Ac: 2-(4- {1,2-dimethyl-1-[4-(pyridin-2-ylmethoxy)phenyl]propyl}phenyl)pyridine: *m*/*z* (ES) 409 (MH)⁺
For 15Ad: 2-(4- {1,2-dimethyl-1-[4-(pyridin-2-ylmethoxy)phenyl]propyl}phenyl)pyrimidine: *m*/*z* (ES) 410 (MH)⁺
For 15Ae: 5-(4- {1,2-dimethyl-1-[4-(pyridin-2-ylmethoxy)phenyl]propyl}phenyl)pyrimidine: *m*/*z* (ES) 410 (MH)⁺
For 15Af: 5-(4-{1,2-dimethyl-1-[4-(pyridin-2-ylmethoxy)phenyl]propyl}phenyl)-2-fluoropyridine: *m*/*z* (ES) 428 (MH)⁺
For 15Ag: 5-(4-{1,2-dimethyl-1-[4-(pyridin-2-ylmethoxy)phenyl]propyl}phenyl)-2-aminopyridine: *m*/*z* (ES) 424 (MH)⁺
For 15Ah: 5-(4- {1,2-dimethyl-1-[4-(pyridin-2-ylmethoxy)phenyl]propyl}phenyl)-2-methoxy-pyridine: *m*/*z* (ES) 440 (MH)⁺
For 15Ai: 5-(4-{1,2-dimethyl-1-[4-(pyridin-2-ylmethoxy)phenyl]propyl}phenyl)-2-nitropyridine: *m*/*z* (ES) 454 (MH)⁺
For 15Aj: 6-(4-{1,2-dimethyl-1-[4-(pyridin-2-ylmethoxy)phenyl]propyl}phenyl)pyridin-2-amine: *m*/*z* (ES) 424 (MH)⁺
For 15Ak: ethyl 5-(4-{1,2-dimethyl-1-[4-(pyridin-2-ylmethoxy)phenyl]propyl}phenyl)nicotinate: m/z (ES) 481 (MH)⁺
For 15Al: 4-(4-{1,2-dimethyl-1-[4-(pyridin-2-ylmethoxy)phenyl]propyl}phenyl)-2-methylpyrimidine: *m*/*z* (ES) 424 (MH)⁺
For 15Am: 4-(4-{1,2-dimethyl-1-[4-(pyridin-2-ylmethoxy)phenyl]propyl}phenyl)-6-ethoxy-2-methylpyrimidine: *m*/*z* (ES) 468 (MH)⁺
For 15An: 2-chloro-5-(4-{1,2-dimethyl-1-[4-(pyridin-2-ylmethoxy)phenyl]propyl}phenyl)-pyrimidine: *m*/*z* (ES) 444 (MH)⁺
For 15Ao: 5-(4- {1,2-dimethyl-1-[4-(pyridin-2-ylmethoxy)phenyl]propyl}phenyl)pyridine-2-ol: *m*/*z* (ES) 426 (MH)⁺
For 15Ap: 5-(4- {1,2-dimethyl-1-[4-(pyridin-2-ylmethoxy)phenyl]propyl}phenyl)-1-methylpyridin-2(1*H*)-one: *m*/*z* (ES) 440 (MH)⁺
For 15Aq: 2-azetidin-1-yl-5-(4- {1,2-dimethyl-1-[4-(pyridin-2-ylmethoxy)phenyl]propyl}phenyl)-pyridine: *m*/*z* (ES) 478 (MH)⁺
For 15Ar: 1-[5-(4- {1,2-dimethyl-1-[4-(pyridin-2-ylmethoxy)phenyl]propyl}phenyl)pyridin-2-yl]-azetidin-3-ol: *m*/*z* (ES) 480 (MH)⁺
For 15As: (3*R*)-1-[5-(4- {1,2-dimethyl-1-[4-(pyridin-2-ylmethoxy)phenyl]propyl}phenyl)pyridin-2-yl]pyrrolidin-3-ol: *m*/*z* (ES) 495 (MH)⁺
For 15At: 5-(4- {1,2-dimethyl-1-[4-(pyridin-2-ylmethoxy)phenyl]propyl}phenyl)-2-pyrrolidin-1-yl-pyridine: *m*/*z* (ES) 479 (MH)⁺
For 15Au: 4-[5-(4- {1,2-dimethyl-1-[4-(pyridin-2-ylmethoxy)phenyl]propyl}phenyl)pyridin-2-yl]-morpholine: *m*/*z* (ES) 495 (MH)⁺
For 15Av: 2-(4- {1,2-dimethyl-1-[4-(pyridin-2-ylmethoxy)phenyl]propyl}phenyl)pyrazine: *m*/*z* (ES) 411 (MH)⁺
For 15Aw: 3-chloro-2-(4- {1,2-dimethyl-1-[4-(pyridin-2-ylmethoxy)phenyl]propyl}phenyl)-5-methylpyrazine: *m*/*z* (ES) 458 (MH)⁺
For 15Ax: 3-(4- {1,2-dimethyl-1-[4-(pyridin-2-ylmethoxy)phenyl]propyl}phenyl)pyridazine: *m*/*z* (ES) 419 (MH)⁺
For 15Ay: 3-(4- {1,2-dimethyl-1-[4-(pyridin-2-ylmethoxy)phenyl]propyl}phenyl)-6-methylpyridazine: *m*/*z* (ES) 424 (MH)⁺
For 15Az: 3-(4- {1,2-dimethyl-1-[4-(pyridin-2-ylmethoxy)phenyl]propyl}phenyl)-6-methoxy-pyridazine: *m*/*z* (ES) 440 (MH)⁺
For 15Aaa: 3-(4-{1,2-dimethyl-1-[4-(pyridin-2-ylmethoxy)phenyl]propyl}phenyl)-6-(trifluoromethyl)pyridazine: *m*/*z* (ES) 478 (MH)⁺
For 15Aab: 3-(4- {1,2-dimethyl-1-[4-(pyridin-2-ylmethoxy)phenyl]propyl}phenyl)-6-fluoro-pyridazine: *m*/*z* (ES) 429 (MH)⁺
For 15Aad: 3-(4- {1,2-dimethyl-1-[4-(pyridin-2-ylmethoxy)phenyl]propyl}phenyl)-6-chloro-pyridazine: *m*/*z* (ES) 444 (MH)⁺
For 15Aae: 3-(4- {1,2-dimethyl-1-[4-(pyridin-2-ylmethoxy)phenyl]propyl}phenyl)-6-(2,5-dimethyl-1*H*-pyrrol-1-yl)pyridazine: *m*/*z* (ES) 503 (MH)⁺
For 15Aaf: 5-(4- {1,2-dimethyl-1-[4-(pyridin-2-ylmethoxy)phenyl]propyl}phenyl)pyrazin-2-amine: *m*/*z* (ES) 425 (MH)⁺
For 15Aag: 4-(4- {1,2-dimethyl-1-[4-(pyridin-2-ylmethoxy)phenyl]propyl}phenyl)pyrimidin-2-amine: *m*/*z* (ES) 425 (MH)⁺
For 15Aah: 5-(4- {1,2-dimethyl-1-[4-(pyridin-2-ylmethoxy)phenyl]propyl}phenyl)pyrimidin-2-amine: *m*/*z* (ES) 425 (MH)⁺ .
For 15Aaa: 3-(4- {1,2-dimethyl-1-[4-(pyrimidin-2-ylmethoxy)phenyl]propyl}phenyl)-6-(trifluoromethyl)pyridazine: *m*/*z* (ES) 479 (MH)⁺
For 15Caa: 3-(4- {1,2-dimethyl-1-[4-(1,3-thiazol-2-ylmethoxy)phenyl]propyl}phenyl)-6-(trifluoromethyl)pyridazine: *m*/*z* (ES) 484 (MH)⁺
For 15Cac: 3-chloro-6-(4- {1,2-dimethyl-1-[4-(1,3-thiazol-2-ylmethoxy)phenyl]propyl}phenyl)-pyridazine: *m*/*z* (ES) 450 (MH)⁺
For 15Dy: 3-(4- {2,2-dimethyl-1-[4-(pyridin-2-ylmethoxy)phenyl]propyl}phenyl)-6-methylpyridazine: *m*/*z* (ES) 424 (MH)⁺

### EXAMPLE 16

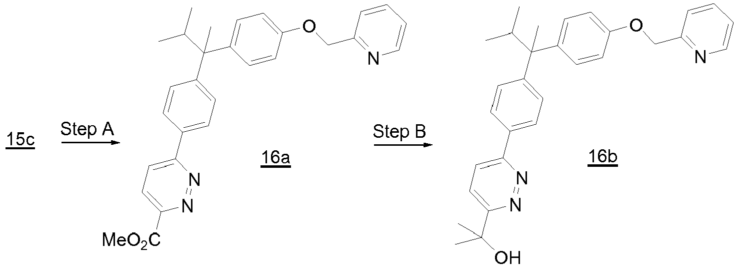

Step A: Preparation of methyl 6-(4-{1,2-dimethyl-1-[4-(pyridin-2-ylmethoxy)phenyl]-propyl}phenyl)pyridazine-3-carboxylate (16a) 1,1'-[bis(diphenylphosphino)ferrocene]dichloropalladium (18 mg, 0.023 mmol) was added to a solution of anhydrous triethylamine (314 µL, 2.25 mmol) and 15c (100 mg, 0.23 mmol) in DMF:methanol (2.00 mL of a 1:1 mixture). The reaction mixture was saturated with carbon monoxide and then heated to 65 °C under a carbon monoxide atmosphere (balloon). After 24 h, the reaction mixture was cooled to rt and filtered through a short column of Celite^{®}, and the filter cake was rinsed with EtOAc. The filtrate was partially concentrated *in vacuo* to approximately ¼ of its original volume, and then purified directly by flash chromatography on silica gel (gradient elution; 0%-70% EtOAc/hexanes as eluent) to afford the title compound 16a. *m*/*z* (ES) 468 (MH)⁺.

### Step B: Preparation of 2-[6-(4-{1,2-dimethyl-1-[4-(pyridin-2-ylmethoxy)phenyl]propyl}-phenyl)py ridazin-3-yl]propan-2-ol (16b)

Compound 16b was prepared following the procedures described in example 12, step E, substituting compound 16a for compound 12b. *m*/*z* (ES) 468 (MH)⁺

Following procedures similar to that described above, the following compounds in Table 16 can be prepared:

**Table 16**

| | | | | | | |
|---|---|---|---|---|---|---|
| 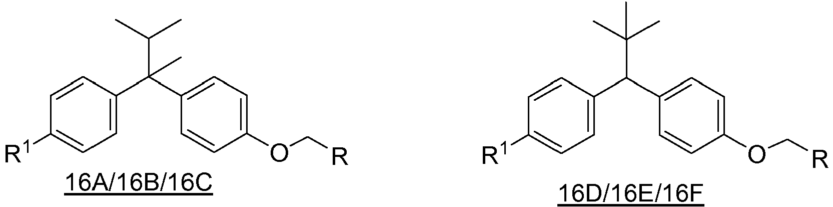 | | | | | | |

| Ex. 16A | Ex. 16B | Ex. 16C | Ex. 16D | Ex. 16E | Ex. 16F | R¹ |
|---|---|---|---|---|---|---|
| R=2-PYR | R=2-PYM | R=2-TZE | R=2-PYR | R=2-PYM | R=2-TZE | |
| -- | a | a | a | a | a | 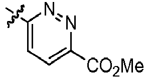 |
| -- | b | b | b | b | b | 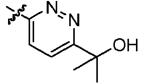 |

Table 16. Parent Ion *m*/*z* (MH)⁺ data for compounds
For 16Ca: methyl 6-(4- {1,2-dimethyl-1-[4-(thiazol-2-ylmethoxy)phenyl]propyl}phenyl)-pyridazine-3-carboxylate: *m*/*z* (ES) 474 (MH)⁺
For 16Cb: 2-[6-(4-{1,2-dimethyl-1-[4-(thiazol-2-ylmethoxy)phenyl]propyl}phenyl)py ridazin-3-yl]propan-2-ol: *m*/*z* (ES) 474 (MH)⁺

### EXAMPLE 17

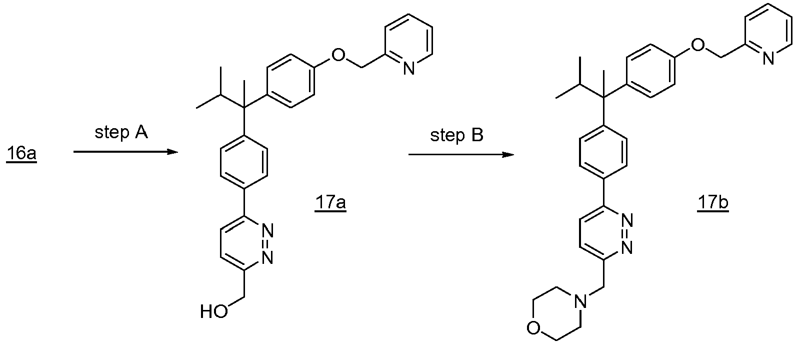

### Step A: Preparation of [6-(4-{1,2-dimethyl-1-[4-(pyridin-2-ylmethoxy)phenyl]propyl}-phenyl)pyridazin-3-yl]methanol (17a)

Compound 17a was prepared following procedures as described in example 13, step D, substituting compound 16a for compound 13c. *m*/*z* (ES) 440 (MH)⁺

### Step B: Preparation of 4-{[6-(4-{1,2-dimethyl-1-[4-(pyridin-2-ylmethoxy)phenyl]propyl}-phenyl)pyridazin-3-yl]methyl}morpholine (17b)

Compound 17b can be prepared following the procedures described in example 7, steps E-F, initially substituting compound 17a for compound 7d.

Following procedures similar to those described above, the following compounds in Table 17 can be prepared:

**Table 17**

| | | | | | | |
|---|---|---|---|---|---|---|
| 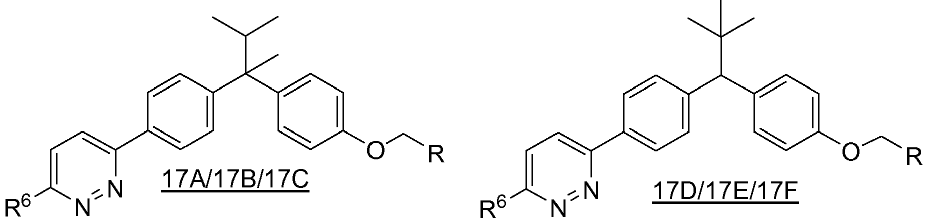 | | | | | | |

| Ex. 17A | Ex. 17B | Ex. 17C | Ex. 17D | Ex. 17E | Ex. 17F | R⁶ |
|---|---|---|---|---|---|---|
| R=2-PYR | R=2-PYM | R=2-TZE | R=2-PYR | R=2-PYM | R=2-TZE | |
| -- | a | a | a | a | a | -CH₂OH |
| b | b | b | b | b | b | 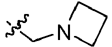 |
| c | c | c | c | c | c | 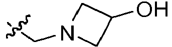 |
| d | d | d | d | d | d | 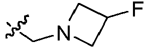 |
| e | e | e | e | e | e | 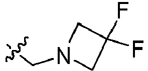 |
| f | f | f | f | f | f | 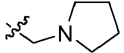 |
| g | g | g | g | g | g | 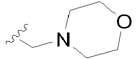 |

### EXAMPLE 18

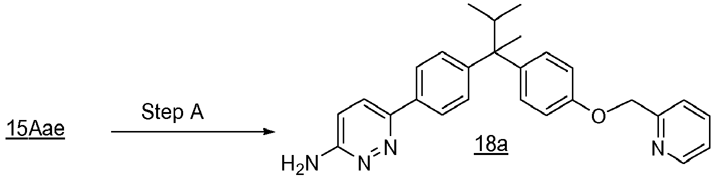

### Preparation of 3-(4-{1,2-dimethyl-1-[4-(pyridin-2-ylmethoxy)phenyl]propyl}phenyl)-6-aminopyridazine (18a)

Hydroxylamine hydrochloride (43.0 mg, 0.619 mmol) was added to a stirred solution of triethylamine (4.00 mL, 0.0287 mmol) and 15Aae (31.0 mg, 0.617 mmol) in ethanol:water (210 mL of a 2:1 mixture, respectively), and the resulting mixture was heated to reflux for 21 h. The reaction mixture was cooled to rt, poured into satd. aq. sodium bicarbonate and extracted with EtOAc. The combined organic extracts were washed with brine, dried and concentrated *in vacuo.* The crude residue was purified by flash chromatography on silica gel (gradient elution; 0%-10% methanol/chlorofom as eluent) to afford the title compound 18a. *m*/*z* (ES) 425 (MH)⁺

Following procedures similar to those described above, the following compounds in Table 18 can be prepared:

**Table 18**

| | | | | |
|---|---|---|---|---|
| 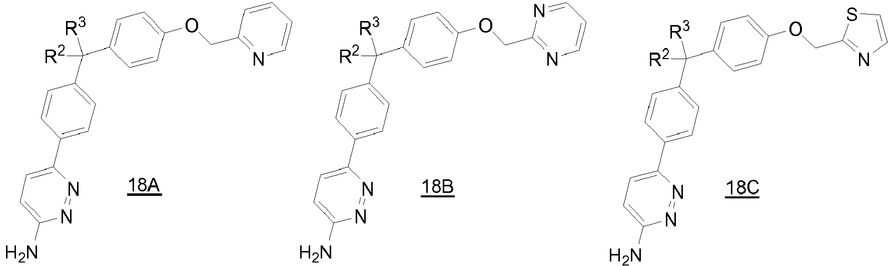 | | | | |

| Ex. 18A | Ex. 18B | Ex. 18C | R2 | R3 |
|---|---|---|---|---|
| a | a | a | *ⁱ*Pr | H |
| b | b | b | *^{t}*Bu | H |
| c | c | c | c-Pr | H |
| d | d | d | c-Bu | H |
| e | e | e | 1-Me-c-Pr | H |
| f | f | f | 1-Me-c-Bu | H |
| -- | g | g | ⁱPr | Me |

For 18Ab: 6-(4- (2,2-dimethyl-1-[4-(pyridin-2-ylmethoxy)phenyl]propyl)phenyl)pyridazin-3-amine: *m*/*z* (ES) 425 (MH)⁺

### EXAMPLE 19

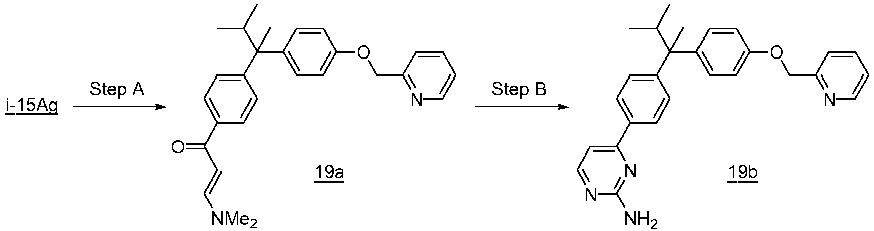

### Step A: Preparation of (2E)-3-(dimethylamino)-1-(4-{1,2-dimethyl-1-[4-(pyridine-2-ylmethoxy)phenyl]propyl}phenyl)prop-2-en-1-one (19a)

Compound 19a was prepared following the procedures described in example 4, step A, substituting intermediate i-15Ag, for intermediate i-15b. *m*/*z* (ES) 429 (MH)⁺

### Step B: Preparation of 2-amino-5-(4-{1,2-dimethyl-1-[4₋(pyridin-2-ylmethoxy)phenyl]-propyl}phenyl)pyrimidine (19b)

A stirred solution of guanidine hydrochloride (13.4 mg, 0.140 mmol), sodium methoxide (280 mL of a 0.5 M methanol solution) and 19a (30.0 mg, 0.0700 mmol) in ethanol (500 mL) was heated in a sealed tube to 120 °C under nitrogen. After 20 h, the reaction mixture was cooled to rt, poured into satd. aq. sodium bicarbonate and extracted with EtOAc. The combined organic extracts were washed with water, dried and concentrated *in vacuo.* The crude residue was purified by preparative reversed phase high performance liquid chromatography on YMC Pack Pro C18 phase (gradient elution; 10%-90% acetonitrile/water as eluent, 0.05% TFA modifier) to afford the title compound 19b. m/z (ES) 425 (MH)⁺

Following procedures similar to that described above, the following compounds in Table 19 can be prepared:

**Table 19**

| | | | | |
|---|---|---|---|---|
| 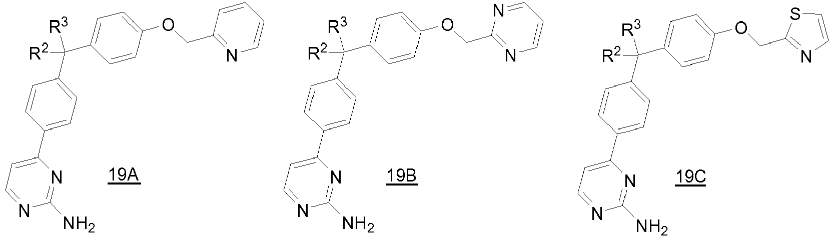 | | | | |

| Ex. 19A | Ex. 19B | Ex. 19C | R2 | R3 |
|---|---|---|---|---|
| a | a | a | *ⁱ*Pr | H |
| b | b | b | *^{t}*Bu | H |
| c | c | c | c-Pr | H |
| d | d | d | c-Bu | H |
| e | e | e | 1-Me-c-Pr | H |
| f | f | f | 1-Me-c-Bu | H |
| -- | g | g | *ⁱ*Pr | Me |

### EXAMPLE 20

### Preparation of 6-(4-{2,2-dimethyl-1-[4-(pyridin-2-ylmethoxy)phenyl]propyl}phenyl)imidazo[2,1-b][1,3,4]thiadiazole (20a]

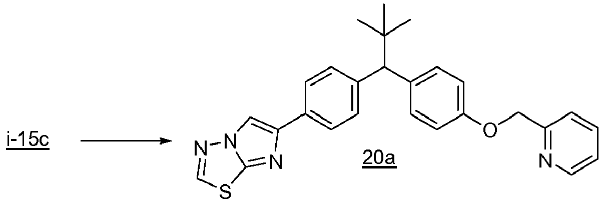

A stirred solution of 1,3,4-thiadiazol-2-amine (7.80 mg, 0.0700 mmol) and i-15c (35.0 mg, 0.0770 mmol) in EtOH (2.00 mL) was heated in a sealed tube to 120 °C. After 16 h, the reaction was quenched with satd. aq. sodium bicarbonate and extracted with EtOAc. The combined organic extracts were washed with brine, dried and concentrated *in vacuo.* The crude residue was purified by preparative reversed phase high performance liquid chromatography on YMC Pack Pro C18 phase (gradient elution; 0%-60% acetonitrile/water as eluent, 0.05% TFA modifier) to afford the title compound 20a. *m*/*z* (ES) 456 (MH)⁺.

Following procedures similar to that described above, the following compounds in Table 20 can be prepared:

**Table 20**

| | | | | | | |
|---|---|---|---|---|---|---|
| 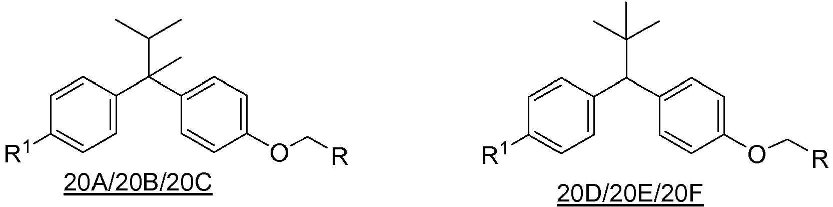 | | | | | | |

| Ex. 20A | Ex. 20B | Ex. 20C | Ex. 20D | Ex. 20E | Ex. 20F | R¹ |
|---|---|---|---|---|---|---|
| R=2-PYR | R=2-PYM | R=2-TZE | R=2-PYR | R=2-PYM | R=2-TZE | |
| a | a | a | -- | a | a | 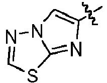 |
| b | b | b | b | b | b | 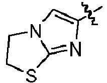 |
| c | c | c | c | c | c | 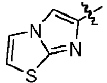 |
| d | d | d | d | d | d | 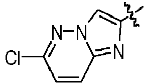 |
| e | e | e | e | e | e | 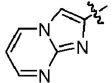 |
| f | f | f | f | f | f | 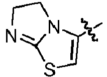 |
| g | g | g | g | g | g | 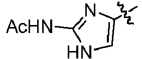 |

Table 20. Parent Ion *m*/*z* (MH)⁺ data for compounds
For 20Db: 6-(4-{2,2-dimethyl-1-[4-(pyridin-2-ylmethoxy)phenyl]propyl}phenyl)-2,3-dihydroimidazo[2,1-*b*][1,3]thiazole: *m*/*z* (ES) 457 (MH)⁺
For 20Dc: 6-(4-{2,2-dimethyl-1-[4-(pyridin-2-yfmethoxy)phenyl]propyl}phenyl)imidazo[2,1-*b*][1,3]thiazole: *m*/*z* (ES) 455 (MH)⁺
For 20Dd: 6-chloro-2-(4- {2,2-dimethyl-1-[4-(pyridin-2-ylmethoxy)phenyl]propyl}phenyl)-imidazo[1,2-*b*]pyridazine: *m*/*z* (ES) 484 (MH)⁺
For 20De: 2-(4- {2,2-dimethyl-1-[4-(pyridin-2-ylmethoxy)phenyl]propyl}phenyl)imidazo[1,2-*a*]-pyrimidine: *m*/*z* (ES) 450 (MH)⁺
For 20Df: 3-(4-{2,2-dimethyl-1-[4-(pyridin-2-ylmethoxy)phenyl]propyl}phenyl)-5,6-dihydroimidazo[2,1-*b*][1,3]thiazole: *m*/*z* (ES) 457 (MH)⁺
For 20Dg: *N*-[4-(4-{2,2-dimethyl-1-[4-(pyridin-2-ylmethoxy)phenyl]propyl}phenyl)-1*H*-imidazol-2-yl]acetamide: *m*/*z* (ES) 456 (MH)⁺

### EXAMPLE 21

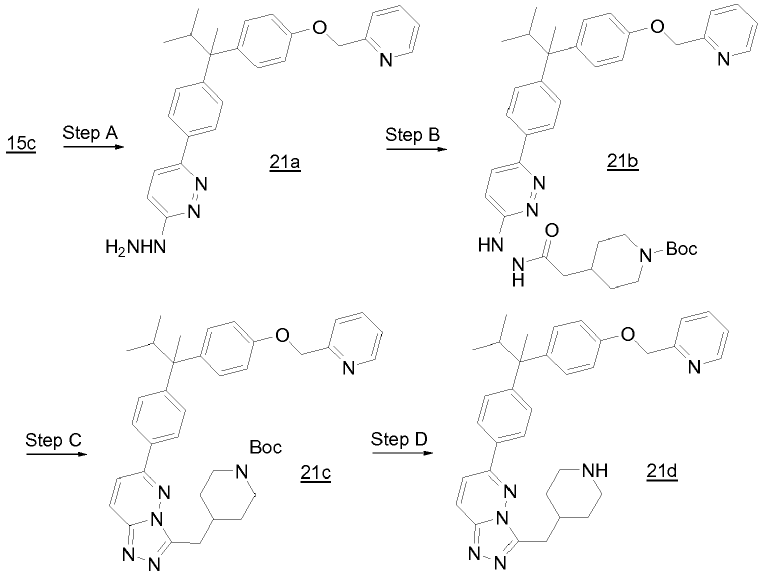

### Step A: Preparation of 3-(4-{1,2-dimethyl-1-[4-(pyridin-2-ylmethoxy)phenyl]-propyl}phenyl)-6-hydrazinopyridazine (21a)

Compound 21a was prepared following the procedures described in example 3, step A, substituting compound 15c for intermediate i-1h.

### Step B: Preparation of tert-butyl 4-(2-{2-[6-(4-{1,2-dimethyl-1-[4-(pyridin-2-ylmethoxy)-phoyl]propyl}phenyl)pyridazin-3-yl]hydrazino}-2-oxoethyl)piperidine-1-carboxylate (21b)

Compound 21b was prepared following the procedures described in example 7, step B, substituting compound 21a for compound 7a. *m*/*z* (ES) 665 (MH)⁺

### Step C: Preparation of tert-butyl4-{[6-(4-{1,2-dimethyl-1-[4-(pyridin-2-ylmethoxy)-phenyl]propyl}phenyl)[1,2,4]triazolo[4,3-b]pyridazin-3-yl]methyl}piperidine-1-carboxylate (21c)

A stirred solution of triethylamine (105 µL, 0.753 mmol) and 21b (100 mg, 0.151 mmol) in xylene (800 µL) was heated in a sealed tube at 140 °C. After 18 h, the reaction mixture was cooled to rt, poured into satd. aq. sodium bicarbonate and extracted with EtOAc. The combined organic extracts were concentrated *in vacuo,* and the resulting crude residue was purified by flash chromatography on silica gel {gradient elution; 0%-50% [(85:15:1) DCM:methanol:ammonium hydroxide]/DCM as eluent} to afford the title compound 21c. *m*/*z* (ES) 647 (MH)⁺

### Step D: Preparation of 6-(4-{1,2-dimethyl-1-[4-(pyridin-2-ylmethoxy)phenyl]propyl}-phenyl)-3-(piperidin-4-ylmethyl)[1,2,4]triazolo[4,3-b]pyridazine

Compound 21d was prepared following the procedures described in example 2, step B, substituting compound 21c for compound 2a. *m*/*z* (ES) 547 (MH)⁺

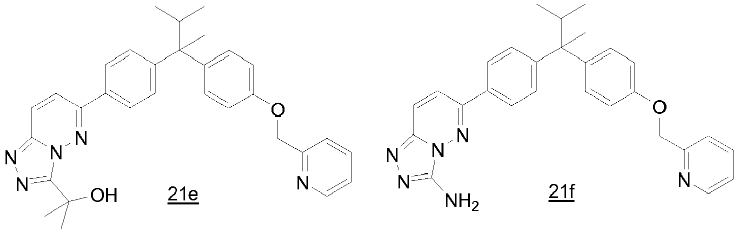

### Step E: Preparation of 2-[6-(4-{1,2-dimethyl-1-[4-(pyridin-2-ylmethoxy)phenyl]propyl}-bhenyl)[1,2,4]triazolo[4,3-b]pyridazin-3-yl]propan-2-ol (21e)

A stirred solution of 2-hydroxy-2-methylpropiohydrazide (40.0 mg, 0.338 mmol), triethylamine (47.0 µL, 0.338 mmol) and 15c (30.0 mg, 0.068 mmol) in xylene (800 µL) was heated in a sealed tube to 140 °C. After 18 h, the reaction mixture was cooled to rt, poured into satd. aq. sodium bicarbonate and extracted with EtOAc. The combined organic extracts were concentrated *in vacuo,* and the resulting crude residue was purified by flash chromatography on silica gel {gradient elution; 0%-100% [(85:15:1) DCM:methanol:ammonium hydroxide]/DCM as eluent} to afford the title compound 21e. *m*/*z* (ES) 508 (MH)+

### Step F: Preparation of 6-(4-{1,2-dimethyl-1-[4-(pyridin-2-ylmethoxy)phenyl]propyl}-phenyl)[1,2,4]triazolo[4,3-b]pyridazin-3-amine (21f)

Cyanogen bromide (11.0 mg, 0.103 mmol) was added to a stirred solution of 21a (40.0 mg, 0.091 mmol) in ethanol (300 mL) at rt. After 5 h, reaction mixure was poured into satd. aq. sodium bicarbonate, extracted with EtOAc. The combined organic extracts were dried, filtered and concentrated *in vacuo.* The crude residue was purified by preparative reversed phase high performance liquid chromatography on YMC Pack Pro C18 phase (gradient elution; 0%-70% acetonitrile/water as eluent, 0.05% TFA modifier) to give the title compound 21f. *m*/*z* (ES) 465 (MH)⁺.

Following procedures similar to that described above, the following compounds in Table 21 can be prepared:

**Table 21**

| | | | | | | |
|---|---|---|---|---|---|---|
| 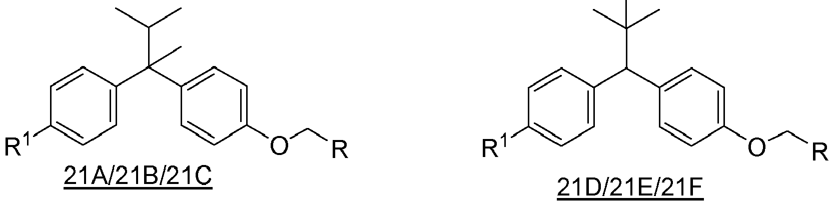 | | | | | | |

| Ex. 21A | Ex. 21B | Ex. 21C | Ex. 21D | Ex. 21E | Ex. 21F | R¹ |
|---|---|---|---|---|---|---|
| R=2-PYR | R=2-PYM | R=2-TZE | R=2-PYR | R=2-PYM | R=2-TZE | |
| a | a | a | a | a | a | 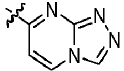 |
| b | b | b | b | b | b | 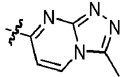 |
| c | c | c | c | c | c | 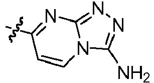 |
| d | d | d | d | d | d | 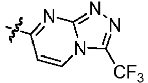 |
| e | e | e | e | e | e | 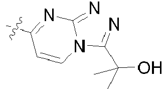 |
| f | f | f | f | f | f | 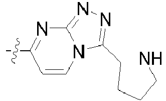 |
| g | g | g | g | g | g | 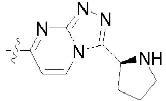 |
| h | h | h | h | h | h | 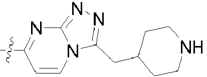 |
| i | i | i | i | i | i | 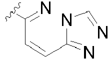 |
| J | J | J | J | J | j | 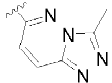 |
| -- | k | k | k | k | k | 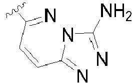 |
| l | l | l | l | l | l | 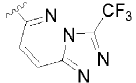 |
| m | m | m | m | m | m | 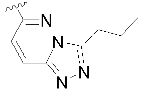 |
| n | n | n | n | n | n | 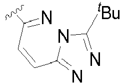 |
| o | o | o | o | o | o | 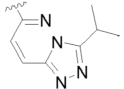 |
| -- | p | p | p | p | p | 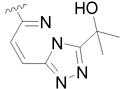 |
| q | q | q | q | q | q | 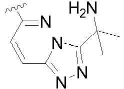 |
| r | r | r | r | r | r | 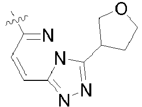 |
| s | s | s | s | s | s | 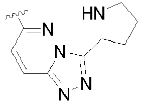 |
| t | t | t | t | t | t | 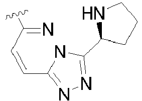 |
| -- | u | u | u | u | u | 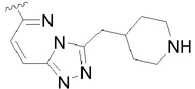 |

Table 21. Parent Ion *m*/*z* (MH)⁺ data for compounds
For 21Aa: 7-(4- {1,2-dimethyl-1-[4-(pyridin-2-ylmethoxy)phenyl]propyl}phenyl)-[1,2,4]triazolo[4,3-*a*]pyrimidine: *m*/*z* (ES) 450 (MH)⁺
For 21Ae: 2-[7-(4-{1,2-dimethyl-1-[4-(pyridin-2-ylmethoxy)phenyl]propyl}phenyl)-[1,2,4]triazolo[4,3-*a*]pyrimidin-3-yl]propan-2-ol: *m*/*z* (ES) 508 (MH)⁺
For 21Ai: 6-(4-{1,2-dimethyl-1-[4-(pyridin-2-ylmethoxy)phenyl]propyl}phenyl)[1,2, 4]-triazolo[4,3-*b*]pyridazine: *m*/*z* (ES) 450 (MH)⁺
For 21Aj: 6-(4-{1,2-dimethyl-1-[4-(pyridin-2-ylmethoxy)phenyl]propyl}phenyl)-3-methyl-[1,2,4]triazolo[4,3-*b*]pyridazine: *m*/*z* (ES) 464 (MH)⁺
For 21Am: 6-(4-{1,2-dimethyl-1-[4-(pyridin-2-ylmethoxy)phenyl]propyl}phenyl)-3-propyl-[1,2,4]triazolo[4,3-*b*]pyridazine: *m*/*z* (ES) 492 (MH)⁺
For 21An: 3-*ter*t-butyl-6-(4-{1,2-dimethyl-1-[4-(pyridin-2-ylmethoxy)phenyl]propyl}phenyl)-[1,2,4]triazolo[4,3-*b*]pyridazine: *m*/*z* (ES) 506 (MH)⁺
For 21Ao: 6-(4-{1,2-dimethyl-1-[4-(pyridin-2-ylmethoxy)phenyl]propyl}phenyl)-3-isopropyl-[1,2,4]triazolo[4,3-*b*]pyridazine: *m*/*z* (ES) 492 (MH)⁺
For 21Aq: 2-[6-(4-{1,2-dimethyl-1-[4-(pyridin-2-ylmethoxy)phenyl]propyl}phenyl)-[1,2,4]triazolo[4,3-*b*]pyridazin-3-yl]propan-2-amine: *m*/*z* (ES) 471 (MH)⁺
For 21Ar: 6-(4-{1,2-dimethyl-1-[4-(pyridin-2-ylmethoxy)phenyl]propyl}phenyl)-3-(tetrahydrofuran-3-yl)[1,2,4]triazolo[4,3-*b*]pyridazine: *m*/*z* (ES) 520 (MH)⁺
For 21As: 6-(4-{1,2-dimethyl-1-[4-(pyridin-2-ylmethoxy)phenyl]propyl}phenyl)-3-[(2R)-pyrrolidin-2-yl][1,2,4]triazolo[4,3-*b*]pyridazine: *m*/*z* (ES) 519 (MH)⁺
For 21At: 6-(4-{1,2-dimethyl-1-[4-(pyridin-2-ylmethoxy)phenyl]propyl}phenyl)-3-[(2*S*)-pyrrolidin-2-yl][1,2,4]triazolo[4,3-*b*]pyridazine: *m*/*z* (ES) 519 (MH)⁺

### EXAMPLE 22

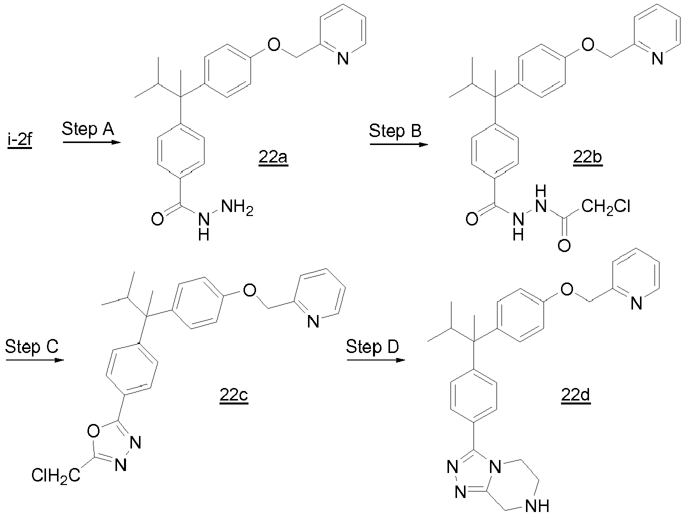

### Step A: Preparation of 4-{1,2-dimethyl-1-[4-(pyridine-2-ylmethoxy)phenyl]propyl}-benzohydrazide (22a)

Compound 22a was prepared following the procedures described in example 3, step A, substituting intermediate i-2f for intermediate i-1h. *m*/*z* (ES) 453 (MH)⁺

### Step B: Preparation of N'-(chloroacetyl)-4-{1,2-dimethyl-1-[4-(pyridin-2-ylmethoxy)-phenyl]propyl}benzohydrazide (22b)

A stirred solution of chloroacetyl chloride (22.0 µL, 0.281 mmol) in acetonitrile (200 µL) was added to 22a (84.0 mg, 0.216 mmol) in acetonitrile (500 µL) at 0 °C. After 5 min, a solution of sodium hydroxide (11.0 mg, 0.281 mmol) in water (200 µL) was added, and the resulting mixture was allowed to warm to rt over 10 min. The reaction mixture was poured into water and extracted with DCM. The combined organic extracts were washed with water and brine, dried and concentrated *in vacuo* to afford the title compound 22b. *m*/*z* (ES) 466 (MH)⁺

### Step C: Preparation of 2-{[4-(1-{4-[5-(chloromethyl)-1,3,4-oxadiazol-2-yl]phenyl}-1,2-dimethyl propyl)phenoxy]methyl}pyridine (22c)

Phosphoryloxy trichloride (181 µL, 1.950 mmol) was added to a stirred solution of 22b (91.0 mg, 0.195 mmol) in acetonitrile (200 µL), and the resulting mixture was heated to reflux. After 18 h, the reaction mixture was cooled to rt and carefully added to pre-cooled satd. aq. sodium bicarbonate and extracted with EtOAc. The combined organic extracts were washed with water and brine, dried and concentrated *in vacuo.* The crude residue was purified by flash chromatography on silica gel (gradient elution; 0% 75% EtOAc/hexanes as eluent) to afford the title compound 22c. *m*/*z* (ES) 448 (MH)⁺

### Step D: Preparation of 3-(4-{1,2-dimethyl-1-[4-(pyridin-2-ylmethoxy)phenyl]propyl} - phenyl)-5,6,7,8-tetrahydro[1,2,4]triazolo[4,3-a]pyrazine (22d)

A stirred solution of ethylenediamine (52.0 µL, 0.774 mmol) and 22c (58.0 mg, 0.129 mmol) in methanol was heated at reflux. After 18 h, the reaction mixture was poured into water and extracted with EtOAc. The combined organic extracts were washed with water and brine, dried and concentrated *in vacuo.* The crude residue was purified by preparative reversed phase high performance liquid chromatography on YMC Pack Pro C18 phase (gradient elution; 10%-35% acetonitrile/water as eluent, 0.05% TFA modifier) to give the title compound 22d. *m*/*z* (ES) 454 (MH)⁺.

Following procedures similar to that described above, the following compounds in Table 22 can be prepared:

**Table 22**

| | | | | |
|---|---|---|---|---|
| 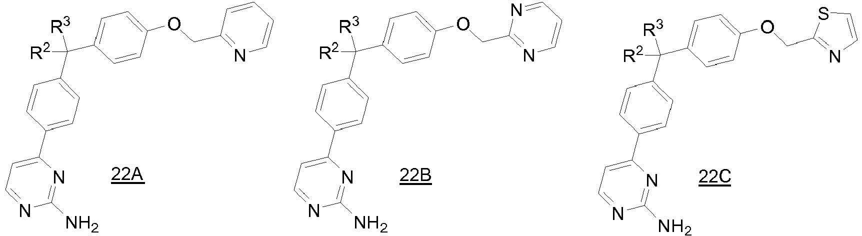 | | | | |

| Ex. 22A | Ex. 22B | Ex. 22C | R² | R³ |
|---|---|---|---|---|
| a | a | a | *ⁱ*Pr | H |
| b | b | b | *^{t}*Bu | H |
| c | c | c | c-Pr | H |
| d | d | d | c-Bu | H |
| e | e | e | 1-Me-c-Pr | H |
| f | f | f | 1-Me-c-Bu | H |
| -- | g | g | *ⁱ*Pr | Me |

### EXAMPLE 23

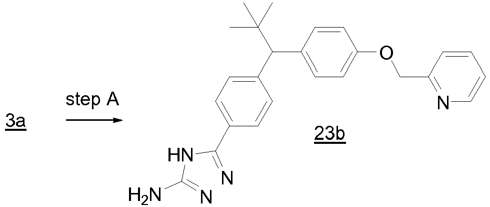

### Step A: Preparation of 2-(4-{2-[4-(pyridin-2-ylmethoxy)phenyl]-2,2-dimethyl-prop-1 - yl}benzoyl)hydrazinecarboximidamide (23a)

S-methylpseudothiourea sulfate (2.00 equiv.) is added to a stirred solution of 3a (1.00 equiv.) in dioxane at ambient temperature. A solution of NaOH (1.10 equiv.) in water (0.25 M) is added to the resulting mixture. After the reaction is deemed complete, heating to 100 °C, if necessary, the reaction mixture is diluted with water and extracted with EtOAc. The combined organic extracts are washed with brine, dried and concentrated *in vacuo.* The crude residue is purified by preparative reversed phase HPLC on YMC Pack Pro C18 stationary phase (CH₃CN/H₂O as eluent, 0.05% TFA as modifier) to afford the title compound 23a.

Following procedures similar to those described above, the following compounds in Table 23 can be prepared:

**Table 23**

| | | | | |
|---|---|---|---|---|
| 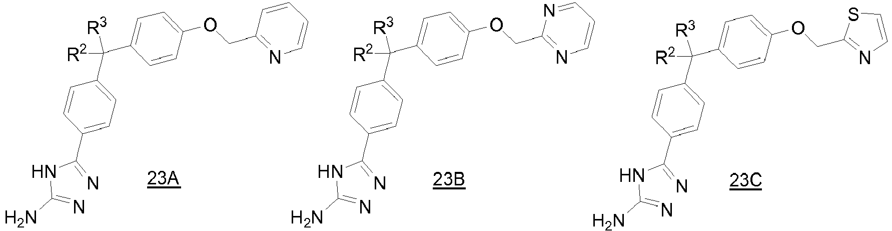 | | | | |

| Ex. 23A | Ex.23B | Ex. 23C | R² | R³ |
|---|---|---|---|---|
| a | a | a | *ⁱ*Pr | H |
| -- | b | b | *^{t}*Bu | H |
| c | c | c | c-Pr | H |
| d | d | d | c-Bu | H |
| e | e | e | 1-Me-c-Pr | H |
| f | f | f | 1-Me-c-Bu | H |
| g | g | g | *ⁱ*Pr | Me |

### EXAMPLE 24

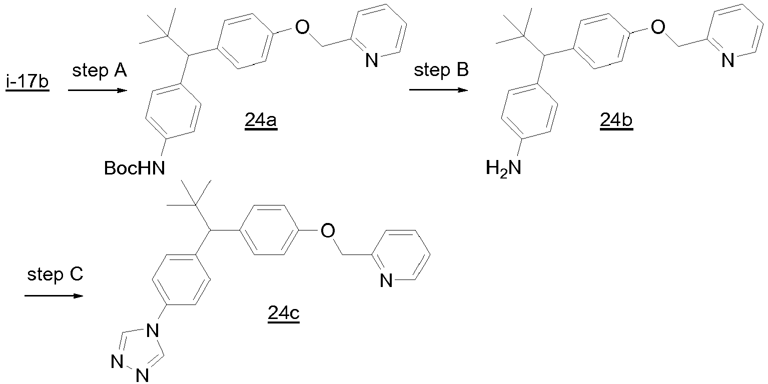

### Step A: Preparation of tert-butyl(4-{2-[4-(pyridine-2-ylmethoxy)phenyl]-2,2-dimethyl-prop-1-yl}phenyl)carbamate (24a)

Isobutyl chloroformate (1.50 equiv.) is added to a stirred suspension of triethylamine (3.50 equiv.) and i-17b (1.00 equiv.) in acetone (0.1 M) at rt. After 1 h, the reaction mixture is cooled to 0 °C, and a solution of sodium azide (6.5 equiv.) in water/acetone (1.0 M) is added. The resulting mixture is stirred at 0 °C until the reaction is deemed complete. The reaction mixture is poured into satd. aq. sodium bicarbonate and extracted with toluene. The combined organic extracts are washed with satd. aq. sodium bicarbonate, dried and partially concentrated *in vacuo* to approximately ¼ of its original volume. (Care should be taken not to concentrate the potentially unstable intermediate acyl azide to dryness.) tert-Butanol (to a final solute concentration of approximately 0.1 M) is added to the acyl azide solution, and the resulting mixture is heated to reflux until the reaction is deemed complete. After cooling to rt, the reaction mixture is concentrated *in vacuo* and can be purified by flash chromatography on silica gel to afford the title compound 24a.

### Step B: Preparation of 4-{2-[4-(pyridin-2-ylmethoxy)phenyl]-2,2-dimethyl-prop-1-yl}aniline (24b)

A solution of 4.0 M HCl in dioxane/water (10:1 volume ratio) is added to a stirred solution of 24a (1.00 equiv.) in dioxane/water (20:1 volume ratio, 0.1 M) at 0 °C. After 30 min, the reaction is warmed to rt and allowed to stir until deemed complete. The reaction mixture is quenched with satd. aq. sodium bicarbonate and extracted with EtOAc. The combined organic extracts are washed with satd. aq. sodium bicarbonate solution, dried and concentrated *in vacuo* to afford the title compound 24b.

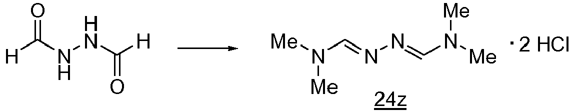

### Preparation of N'-[(1E)-(dimethylamino)methylene]-N,N-dimethylhydrazono formamide dihydrochloride (24z)

Thionyl chloride (2.00 mL, 28.4 mmmol) was added to a stirred solution of diformylhydrazine (1.02 g, 11.4 mmol) in DMF (22 mL) at 0 °C. After 10 min, the yellow mixture was warmed to rt and allowed to stir overnight. The resulting precipitate was collected and rinsed with DMF, followed by diethyl ether to afford the title compound 24z. *m*/*z* (ES) 143 (MH)⁺.

### Step C: Preparation of 2-[(4-{2-[4-(4H-1,2,4-triazol-4-yl)phenyl]-2,2-dimethyl-prop-1-yl}phenoxy)methyl]pyridine (24c)

24z (1.20 equiv.), triethylamine (2.20 equiv.), andp-TSA (0.200 equiv.) are added to a stirred solution of 24b (1.00 equiv.) in toluene (0.1 M), and the resulting mixture is heated to reflux until the reaction is deemed complete. After cooling to rt, the reaction mixture is diluted with EtOAc and the phase is washed with satd. aq. sodium bicarbonate, water and brine. The organic phase is dried, concentrated *in vacuo* and can be purified by flash chromatography on silica gel to afford the title compound 24c.

Following procedures similar to those described above, the following compounds in Table 24 can be prepared:

**Table 24**

| | | | | |
|---|---|---|---|---|
| 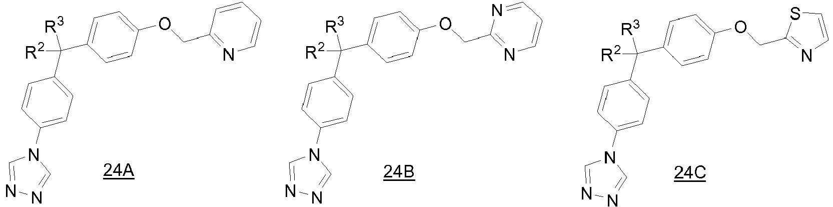 | | | | |

| Ex. 24A | Ex. 24B | Ex. 24C | R² | R³ |
|---|---|---|---|---|
| a | a | a | *ⁱ*Pr | H |
| -- | b | b | *^{t}*Bu | H |
| c | c | c | c-Pr | H |
| d | d | d | c-Bu | H |
| e | e | e | 1-Me-c-Pr | H |
| f | f | f | 1-Me-c-Bu | H |
| g | g | g | *ⁱ*Pr | Me |

### EXAMPLE 25

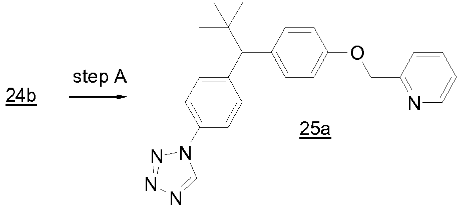

### Step A: Preparation of 2-[(4-{2-[4-(1H-tetrazol-1-yl)phenyl]-2,2-dimethyl-prop-1-yl}phenoxy)methyl]pyridine (25a)

Triethylorthoformate (4.00 equiv.), and sodium azide (2.75 equiv.) are added to a stirred solution of 24b (1.00 equiv.) in glacial acetic acid (0.5 M). The resultant mixture is heated to 95 °C until the reaction is deemed complete. After cooling to rt, the reaction mixture is diluted with EtOAc. The organic phase is washed with satd. aq. sodium bicarbonate, dried and concentrated *in vacuo.* The crude residue can be purified by flash chromatography on silica gel to afford the title compound 25a.

Following procedures similar to those described above, the following compounds in Table 25 can be prepared:

**Table 25**

| | | | | |
|---|---|---|---|---|
| 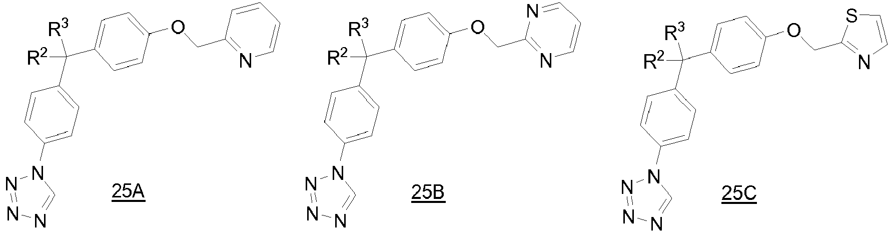 | | | | |

| Ex. 25A | Ex. 25B | Ex. 25C | R² | R³ |
|---|---|---|---|---|
| a | a | a | *ⁱ*Pr | H |
| -- | b | b | *^{t}*Bu | H |
| c | c | c | c-Pr | H |
| d | d | d | c-Bu | H |
| e | e | e | 1-Me-c-Pr | H |
| f | f | f | 1-Me-c-Bu | H |
| g | g | g | *ⁱ*Pr | Me |

### EXAMPLE 26

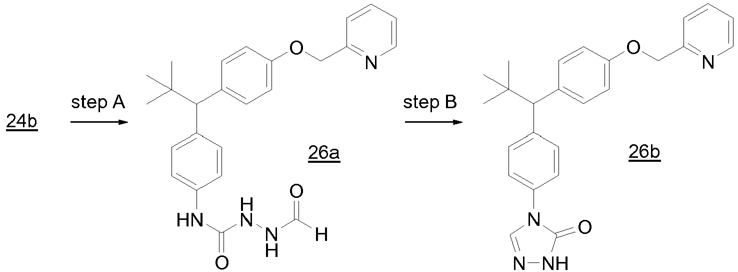

### Step A: Preparation of N-(4-{2,2-dimethyl-1-[4-(pyridin-2-ylmethoxy)phenyl]propyl}-phenyl)-2-formylhydrazinecarboxamide (26a)

Triphosgene (0.500 equiv.) and pyridine (3.25 equiv.) are added to a stirred solution of 24b (1.00 equiv.) in DCM (0.05 M) at 0 °C. After 30 min, formic hydrazide (5.00 equiv.) and triethylamine (2.50 equiv.) are added, and the resulting mixture is allowed to stir at 0 °C until the reaction is deemed complete. The reaction mixture is quenched with satd. aq. sodium bicarbonate solution, extracted with EtOAc, dried and concentrated *in vacuo.* The crude residue can be purified by flash chromatography on silica gel to afford the title compound 26a.

### Step B: Preparation of 4-(4-{2,2-dimethyl-1-[4-(pyridin-2-ylmethoxy)phenyl]propyl}-phenyl)-2,4-dihydro-3H-1,2,4-triazol-3-one (26b)

Sodium hydroxide (1.60 equiv. of a 1.25 N aqueous solution) is added to a stirred suspension of 26a (1.00 equiv.) in n-butanol (0.1 M), and the resulting mixture is heated to reflux until the reaction is deemed complete. After cooling to rt, the reaction mixture is poured into satd. aq. ammonium chloride and extracted with EtOAc. The combined organic extracts are washed with satd. aq. sodium bicarbonate, dried and concentrated *in vacuo.* The crude residue can be purified by flash chromatography on silica gel to afford the title compound 26b.

Following the procedures similar to those described above, the following compounds in Table 26 can be prepared:

**Table 26**

| | | | | |
|---|---|---|---|---|
| 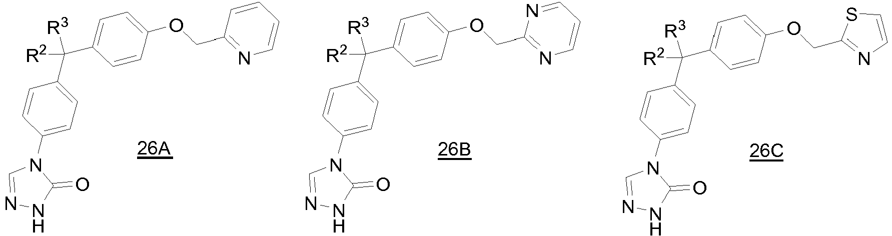 | | | | |

| Ex. 26A | Ex. 26B | Ex. 26C | R² | R³ |
|---|---|---|---|---|
| a | a | a | *ⁱ*Pr | H |
| -- | b | B | *^{t}*Bu | H |
| c | c | C | c-Pr | H |
| d | d | D | c-Bu | H |
| e | e | E | 1-Me-c-Pr | H |
| f | f | F | 1-Me-c-Bu | H |
| g | g | G | *ⁱ*Pr | Me |

### EXAMPLE 27

### Step A: Preparation of methyl 4-{2,2-dimethyl-1-[4-(1-pyrimidin-2-ylpropoxy)phenyl]-propyl}benzoate (27a)

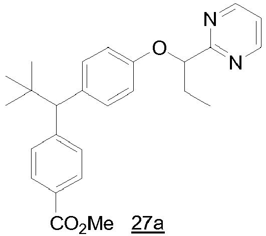

Cesium carbonate (4.0 equiv.), and 2-(1-bromopropyl)pyrimidine (1.5 equiv.) are added to a stirred solution of i-1g (1.0 equiv.) in DMF at rt, heating to 60 °C if necessary, until the reaction is deemed complete. After cooling to rt, the reaction mixture is partitioned between EtOAc and water. The organic layer is washed with water and brine, dried and concentrated *in vacuo.* The crude product can be purified by flash chromatography on silica gel to afford the title compound 27a.

### Step B: Preparation of 5-(4-{2,2-dimethyl-1-[4-(1-pyrimidin-2-ylpropoxy)phenyl]propyl} - phenyl)-1,3,4-oxadiazol-2-amine (27b)

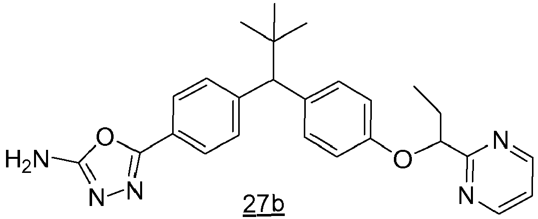

Compound 27b can be prepared, following the procedures described in example 1, substituting compound 27a for intermediate i-11Ab.

Following procedures similar to those described above, the following compounds in Table 27 can be prepared:

**Table 27**

| | | | | | | |
|---|---|---|---|---|---|---|
| 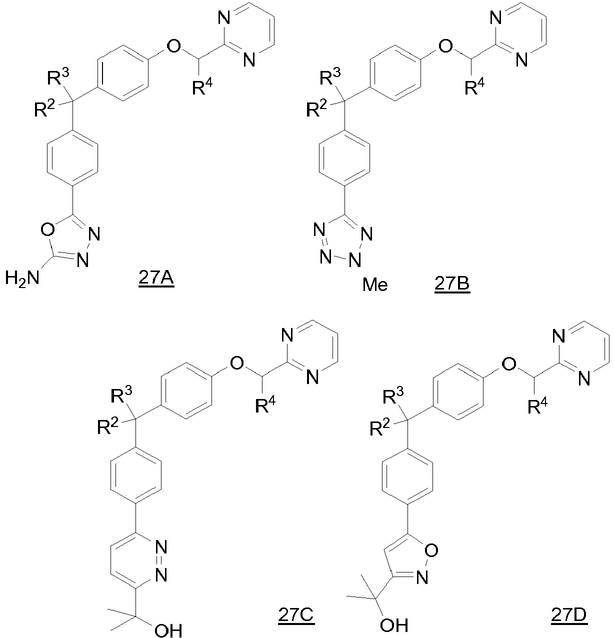 | | | | | | |

| Ex. 27A | Ex. 27B | Ex. 27C | Ex. 27D | R² | R³ | R⁴ |
|---|---|---|---|---|---|---|
| a | a | a | a | *i*Pr | H | Me |
| b | b | b | b | *i*Pr | Me | |
| c | c | c | c | *t*Bu | H | |
| d | d | d | d | *i*Pr | H | Et |
| e | e | e | e | *i*Pr | Me | |
| | f | f | f | *t*Bu | H | |

### EXAMPLE 28

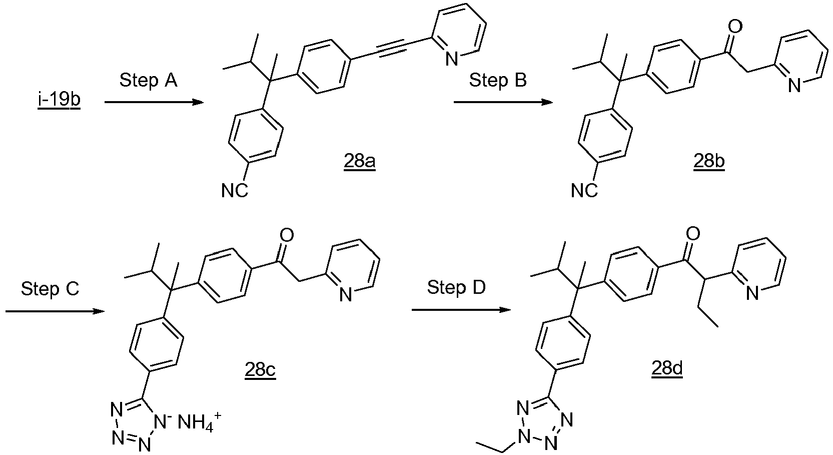

### Step A: Preparation of 4-{1,2-dimethyl-1-[4-(pyridin-2-ylethynyl)phenyl]propyl}-benzonitrile (28a)

Compound 28a can be prepared following the procedures described in scheme i-14, substituting intermediate i-19b for intermediate i-12f. *m*/*z* (ES) 351 (MH)⁺.

### Step B: Preparation of 4-{1,2-dimethyl-1-[4-(pyridin-2-ylacetyl)phenyl]propyl}benzonitrile (28b)

Sulfuric acid (50.0 mL of a 18 M solution) was added dropwise over 1 h added to a stirred suspension of 28a (3.10 g, 8.83 mmol) in water (15.0 mL) at 0 °C, and the resulting mixture was warmed to rt and allowed to stir for 1 h. The reaction mixture was carefully poured over ice, and the resulting mixture was adjusted to pH ∼ 10 by the addition of 2 N aqueous sodium hydroxide. After extracting with EtOAc, and the combined organic extracts were washed with brine and concentrated *in vacuo.* The crude residue was purified by flash chromatography on silica gel (gradient elution; 20%-35% EtOAc/hexanes as eluent) to afford the title compound 28b. *m*/*z* (ES) 369 (MH)⁺.

### Step C: Preparation of 5-(4-{1,2-dimethyl-1-[4-(pyridin-2-ylacetyl)phenyl]propyl}phenyl)-tetrazole-1-ide (28c)

Compound 28c was prepared following the procedures described in scheme i-18, substituting compound 28b for intermediate i-14Ab. *m*/*z* (ES) 412 (MH)⁺.

### Step D: Preparation of 1-(4-{1-[4-(2-ethyl-2H-tetrazol-5-yl)phenyl]-1,2-dimethylpropyl}-phenyl)-2-pyridin-2-ylbutan-1-one (28d)

Ethyl iodide (662 µL, 8.26 mmol) was added to a stirred solution of potassium carbonate (2.85 g, 20.1 mmol) and 28c (1.70 g, 4.13 mmol) in DMF (50.0 mL) at rt. After 18 h, the reaction mixture was diluted with EtOAc, and the organic phase was washed with satd. aq. sodium bicarbonate and brine, dried and concentrated *in vacuo.* The crude residue was purified by flash chromatography on silica gel (gradient elution; 0%-100% EtOAc/hexanes as eluent) to afford the title compound 28d. *m*/*z* (ES) 468 (MH)⁺.

### EXAMPLE 29

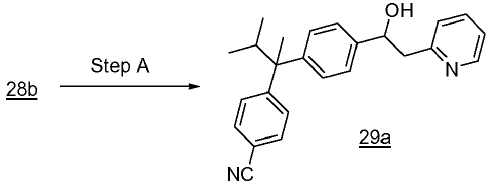

### Preparation of 4-{1-[4-(1-hydroxy-2-pyridin-2-ylethyl)phenyl]-1,2-dimethylpropyl}benzonitrile (29a)

Sodium borohydride (87.0 mg, 2.30 mmol) was added to a stirred solution of 28b (850 mg, 2.30 mmol) in ethanol (20.0 mL) at rt. After 1 h, the reaction mixture was quenched with 0.1 N HCl, then adjusted to pH ∼10 by the addition of satd. aq. sodium bicarbonate. The mixture was extracted with EtOAc, the combined organic extracts were concentrated *in vacuo,* and the crude residue was purified by flash chromatography on silica gel (gradient elution; 0%-100% EtOAc/hexanes as eluent) to afford the title compound 29a. *m*/*z* (ES) 371 (MH)⁺.

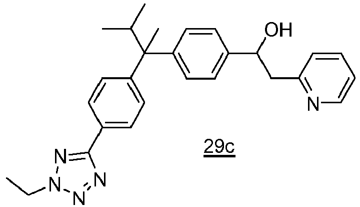

### Preparation of 1-(4-{1-[4-(2-ethyl-2H-tetrazol-5-yl)phenyl]-1,2-dimethylpropyl}phenyl)-2-pyridin-2-ylethanol (29c)

Compound 29c was prepared following the procedures described in scheme i-18, substituting compound 29b for intermediate i-14Ab, to yield an intermediate compound that was substituted for compound 28b in example 28, step D, to afford the title compound 29c. *m*/*z* (ES) 442 (MH)⁺.

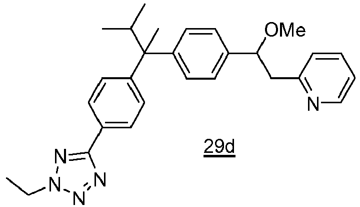

### Preparation of 2-[2-(4-{1-[4-(2-ethyl-2H-tetrazol-5-yl)phenyl]-1,2-dimethylpropyl}phenyl)-2-methoxyethyl]pyridine (29d)

Sodium bis(trimethylsilyl)amide (68.0 µL of a 2.0 M THF solution) was added to a stirred solution of 29c (30.0 mg, 0.0679 mmol) in THF (2.00 mL). After 10 min, iodomethane (8.50 µL, 0.140 mmol) was added, and the resulting mixture was allowed to stir at rt. After 1 h, the reaction mixture was diluted with EtOAc, and the organic phase was washed with satd. aq. sodium bicarbonate and brine, dried and concentrated *in vacuo.* The crude residue was purified by flash chromatography on silica gel (gradient elution; 0%-100% EtOAc/hexanes as eluent) to afford the title compound 29d. *m*/*z* (ES) 456 (MH)⁺.

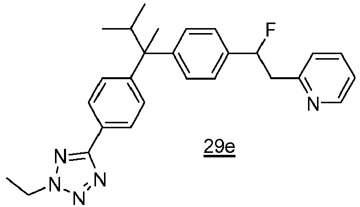

### Preparation of 2-[2-(4-{1-[4-(2-ethyl-2H-tetrazol-5-yl)phenyl]-1,2-dimethylpropyl}phenyl)-2-fluoroethyl]pyridine (29e)

[Bis(2-methoxyethyl)amino]sulfur trifluoride (1.00 mL of a 50% toluene solution) was added to a stirred solution of 29c (40.0 mg, 0.091 mmol) in DCM (1.00 mL), and the resulting mixture was allowed to stir at rt. After 18 h, the reaction mixture was diluted with EtOAc, and the organic phase was washed with satd. aq. sodium bicarbonate and brine, dried and concentrated *in vacuo.* The crude residue was purified by flash chromatography on silica gel (gradient elution; 30%-70% EtOAc/hexanes as eluent) to afford the title compound 29e. *m*/*z* (ES) 444 (MH)⁺.

Following procedures similar to those described above, the following compounds in Table 29 can be prepared:

**Table 29**

| | |
|---|---|
| 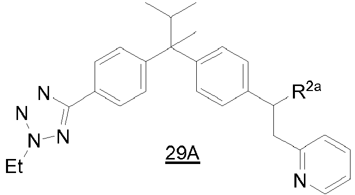 | |

| Ex. 29A | R^{2a} |
|---|---|
| -- | -OH |
| -- | -OMe |
| c | -OEt |
| d | -OCF₃ |
| e | -OBn |
| f | F |

Table 29. Parent Ion *m*/*z* (MH)⁺ data for compounds.
For 29Ac: 2- [2-ethoxy-2-(4-{1-[4-(2-ethyl-2H-tetrazol-5-yl)phenyl]-1,2-dimethylpropyl}phenyl) ethyl]pyridine: *m*/*z* (ES) 470 (MH)⁺.
For 29Ad: 2-[2-(4-{1-[4-(2-ethyl-2H-tetrazol-5-yl)phenyl]-1,2-dimethylpropyl}phenyl)-2-(trifluoromethoxy)ethyl]pyridine: *m*/*z* (ES) 510 (MH)⁺.

### EXAMPLE 30

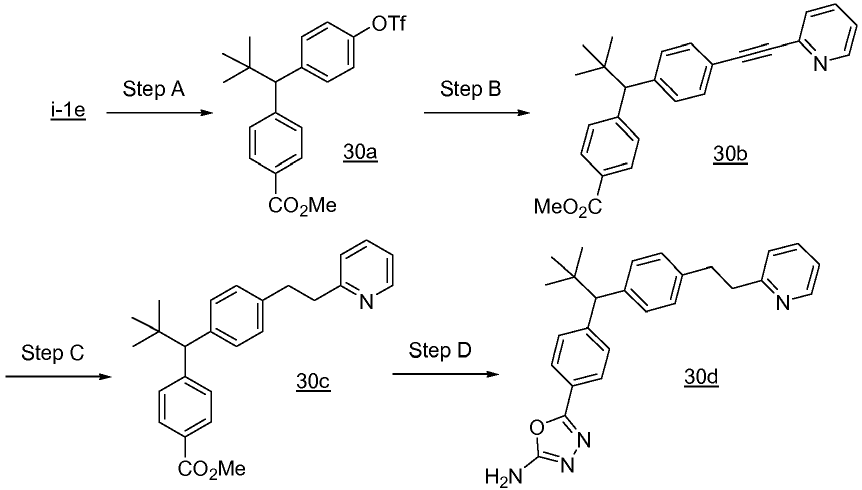

### Step A: Preparation of methyl 4-[2,2-dimethyl-1-(4-{[(trifluoromethyl)sulfonyl]oxy}-phenyl)propyl]benzoate (30a)

Compound 30a was prepared following the procedures described in scheme i-6, step C, substituting intermediate i-1e for intermediate i-6b. *m*/*z* (ES) 431 (MH)⁺.

### Step B: Preparation of methyl 4-{2,2-dimethyl-1-[4-(pyridin-2-ylethynyl)phenyl]propyl}-benzoate (30b)

Compound 30b was prepared following the procedures described in scheme i-19, step A, substituting compound 30a for intermediate i-12c. *m*/*z* (ES) 384 (MH)⁺.

### Step C: Preparation of methyl 4-{2,2-dimethyl-1-[4-(2-pyridin-2-ylethyl)phenyl]propyl}-benzoate (30c)

A mixture of palladium (20.0 mg of a 10% wt. powder on activated carbon) and 30b (20.0 mg, 0.053 mmol) in ethanol (2.00 mL) was hydrogenated at atmospheric pressure for 96 h. The mixture was filtered through a short column of Celite^{®} and concentrated *in vacuo* to afford the title compound 30c. *m*/*z* (ES) 388 (MH)⁺.

### Step D: Preparation of 5-(4-{2,2-dimethyl-1-[4-(2-pyridin-2-ylethyl)phenyl]propyl}-phenyl)-1,3, 4-oxadiazol-2-amine (30d)

Compound 30d was prepared following the procedures described in example 1, substituting compound 30c for intermediate i-11Ab. *m*/*z* (ES) 413 (MH)⁺.

### EXAMPLE 31

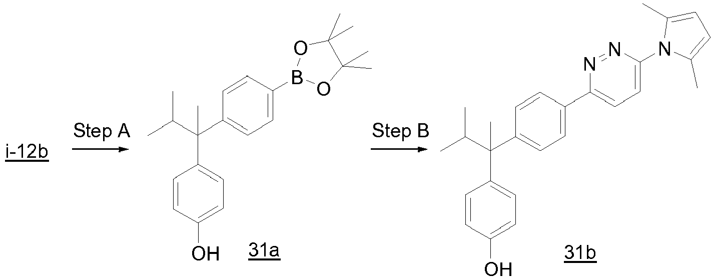

### Step A: Preparation of 4-{1-[4-(4,4,5,5-tetramethyl-1,3,2-dioxaborolan-2-yl)phenyl]-1,2-dimethylpropyl}phenol (31a)

Compound 31a was prepared following the procedures described in scheme i-13, substituting intermediate i-12b for intermediate i-12f. *m*/*z* (ES) 367 (MH)⁺.

### Step B: Preparation of 4-(1-{4-[6-(2,5-dimethyl-1H-pyrrol-1-yl)pyridazin-3-yl]phenyl}-1,2-dimethylpropyl)phenol (31b)

Compound 31b was prepared from 31a and i-20a following the procedures described in scheme 15, step C, substituting compound 31a for intermediate i-13b. *m*/*z* (ES) 412 (MH)⁺.

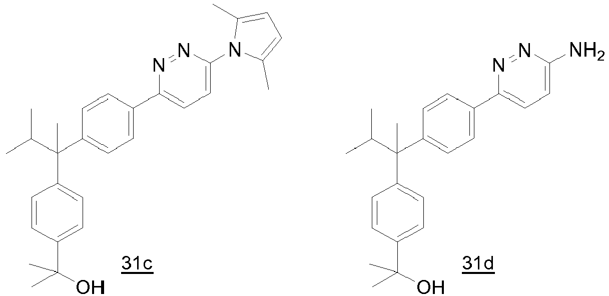

### Preparation of 2-[4-(1-{4-[6-(2,5-dimethyl-1H-pyrrol-1-yl)pyridazin-3-yl]phenyl}-1,2-dimethylpropyl)phenyl]propan-2-ol (31c)

Compound 31c was prepared following the procedures described in scheme i-6, step C, substituting compound 31b for intermediate i-6b. The product of this reaction was carried forward following procedures as described in example 16, step A, substituting it for 15c. The product of this reaction was subsequently carried forward following procedures as described in example 12, step E, substituting it for compound 12b, to afford the title compound 31c. *m*/*z* (ES) 454 (MH)⁺.

### Preparation of 2-(4-{1-[4-(6-aminopyridazin-3-yl)phenyl]-1,2-dimethylpropyl}phenyl)propan-2-ol (31d)

A stirred solution of ammonium hydroxide (100 µL of a 28 % ammonia solution in water) and 31c (43.0 mg, 0.095 mmol) in water (1.00 mL) and EtOH (500 µL) was irradiated at 120 °C in a microwave reactor for 10 min. After cooling to rt, the reaction mixture was poured into satd. aq. sodium bicarbonate and extracted with DCM. The combined organic extracts were dried, filtered and concentrated *in vacuo.* The crude residue was purified by preparative thin layer chromatography on silica gel (5% methanol/DCM as eluent) to afford the title compound 31d. *m*/*z* (ES) 376 (MH)⁺.

Following procedures similar to those described in Example 31 and the preceding schemes, the following additional compounds represented in Table 31 can be prepared:

**Table 31**

| | | | | |
|---|---|---|---|---|
| 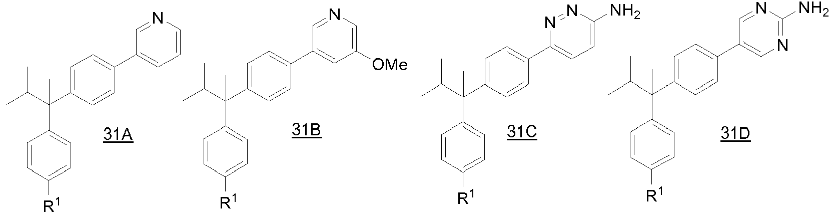 | | | | |

| Ex. #31A | Ex. #31B | Ex. #31C | Ex. #31D | R¹ |
|---|---|---|---|---|
| a | a | a | a | CO₂Me |
| b | b | -- | b | 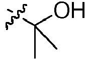 |
| c | c | c | c | CN |
| d | d | d | d | 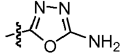 |
| e | e | e | e | 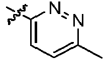 |
| f | f | f | f | 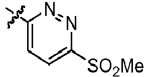 |
| g | g | g | g | 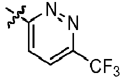 |
| h | h | h | h | 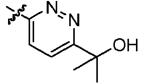 |

Table 31. Parent Ion *m*/*z* (MH)⁺ data for compounds.
For 31Aa: methyl 4-[1,2-dimethyl-1-(4-pyridin-3-ylphenyl)propyl]benzoate: *m*/*z* (ES) 360 (MH)⁺.
For 31Ad: 5-{4-[1,2-dimethyl-1-(4-pyridin-3-ylphenyl)propyl]phenyl}-1,3,4-oxadiazol-2-amine: *m*/*z* (ES) 385 (MH)⁺.
For 31Ae: 3-{4-[1,2-dimethyl-1-(4-pyridin-3-ylphenyl)propyl]phenyl}-6-methylpyridazine: m/z (ES) 394 (MH)⁺.
For 31Ag: 3-{4-[1,2-dimethyl-1-(4-pyridin-3-ylphenyl)propyl]phenyl}-6-(trifluoromethyl)-pyridazine: *m*/*z* (ES) 448 (MH)⁺.
For 31Ba: methyl 4-{1-[4-(5-methoxypyridin-3-yl)phenyl]-1,2-dimethylpropyl}benzoate: *m*/*z* (ES) 390 (MH)⁺.
For 31Be: 3-(4-{1-[4-(5-methoxypyridin-3-yl)phenyl]-1,2-dimethylpropyl}phenyl)-6-methylpyridazine: *m*/*z* (ES) 424 (MH)⁺.
For 31Bf: 3-(4-{1-[4-(5-methoxypyridin-3-yl)phenyl]-1,2-dimethylpropyl}phenyl)-6-(methylsulfonyl)pyridazine: *m*/*z* (ES) 488 (MH)⁺.
For 31Bh: 2-[6-(4-{1-[4-(5-methoxypyridin-3-yl)phenyl]-1,2-dimethylpropyl}phenyl)pyridazin-3-yl]propan-2-ol: *m*/*z* (ES) 468 (MH)⁺.
For 31Ch: 2-[6-(4-{1-[4-(6-aminopyridazin-3-yl)phenyl]-1,2-dimethylpropyl}phenyl)pyridazin-3-yl]propan-2-ol: *m*/*z* (ES) 454 (MH)⁺.
For 31Db: 2-(4-{1-[4-(2-aminopyrimidin-5-yl)phenyl]-1,2-dimethylpropyl}phenyl)propan-2-ol: *m*/*z* (ES) 376 (MH)⁺.
For 31Dc: 4-{1-[4-(2-aminopyrimidin-5-yl)phenyl]-1,2-dimethylpropyl}benzonitrile: *m*/*z* (ES) 343 (MH)⁺.
For 31Dh: 2-[6-(4-{1-[4-(2-aminopyrimidin-5-yl)phenyl]-1,2-dimethylpropyl}phenyl)pyridazin-3-yl]propan-2-ol: *m*/*z* (ES) 454 (MH)⁺.

### FLAP Binding Assay

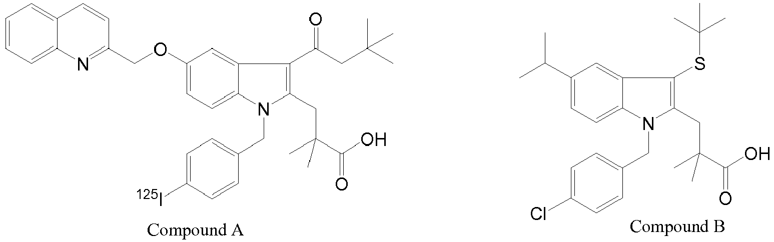

A 100,000 x g pellet from human leukocyte 10,000 x g supernatants (1) is the source of FLAP. The 100,000 x g pellet membranes were resuspended in Tris-Tween assay buffer (100 mM Tris HCl pH 7.4, 140 mM NaCl, 2 mM EDTA, 0.5 mM dithiothreitol, 5% glycerol, 0.05% Tween 20) to yield a final protein concentration of 50 µg to 150 µg/ml. Aliquots (100 µl) of membrane suspension were added to 12 mm x 75 mm polypropylene tubes containing 100 µl Tris-Tween assay buffer, 30,000 cpm of Compound A in 5 µl MeOH:assay buffer (1:1), and 2 µl dimethyl sulfoxide or competitor (i.e., the compound to be tested) in dimethyl sulfoxide. Compound B (10 µM final concentration) was used to determine non-specific binding. After a 20 minute incubation at room temperature, tube contents were diluted to 4 ml with cold 0.1 M Tris HCl pH 7.4, 0.05% Tween 20 wash buffer and the membranes were collected by filtration of GFB filters presoaked in the wash buffer. Tubes and filters were rinsed with 2 x 4 ml aliquots of cold wash buffer. Filters were transferred to 12 mm x 3.5 mm polystyrene tubes for determination of radioactivity by gamma-scintillation counting.

Specific binding is defined as total binding minus non-specific binding. Total binding was Compound A bound to membranes in the absence of competitor; non-specific binding was Compound A bound in the presence of 10 uM Compound B. Preparation of Compound A is described in reference 1, below. The IC₅₀ values were obtained by computer analysis (see reference 2, below) of the experimental data. Representative tested compounds of the invention were determined to have an IC₅₀ < 200 nM, and most of the tested compounds had an IC₅₀ < 100 nM.

### REFERENCES:

1. Charleson, S., Prasti, P., Leger, S., Gillard, J.W, Vickers, P.J., Mancini, J.A., Charleson, P., Guay, J., Ford-Hutchinson, A.W., and Evans, J.F. (1992) Characterization of a 5-lipoxygenase-activating protein binding assay: correlation of affinity for 5-lipoxygenase-activating protein with leukotriene synthesis inhibition. Mol Pharmacol 41:873-879.
2. Kinetic, EBDA, Ligand, Lowry: A collection of Radioligand Binding Analysis Programs by G.A. McPherson. Elsevier-BIOSOFT.
   WHILE THE INVENTION HAS BEEN DESCRIBED WITH REFERENCE TO CERTAIN PARTICULAR EMBODIMENTS THEREOF, NUMEROUS ALTERNATIVE EMBODIMENTS WILL BE APPARENT TO THOSE SKILLED IN THE ART FROM THE TEACHINGS DESCRIBED HEREIN. ALL PATENTS, PATENT APPLICATIONS AND PUBLICATIONS CITED HEREIN ARE INCORPORATED BY REFERENCE IN THEIR ENTIRETY.

## Claims

1. A compound represented by structural formula I 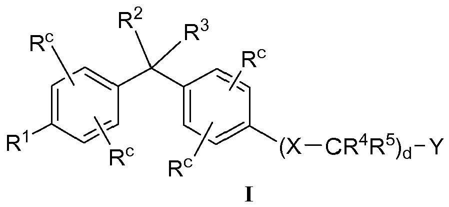 and pharmaceutically acceptable salts thereof wherein:
**R¹** is selected from the group consisting of:
(a) a 5-membered aromatic or partially unsaturated heterocyclic ring containing 2 to 4 heteroatoms selected from N, S and O, wherein the heterocyclic ring is optionally substituted with (i) R⁶, or (ii) oxo and R⁶ provided R⁶ is not oxo;
(b) a 6-membered aromatic or partially unsaturated heterocyclic ring containing 1 to 2 heteroatoms selected from N and O, wherein the heterocyclic ring is optionally substituted with (i) R⁶, (ii) oxo and R⁶ provided R⁶ is not oxo, or (iii) methyl and R⁶;
(c) an 8-membered aromatic or partially unsaturated ortho-fused bicyclic ring system containing 3-5 heteroatoms selected from one sulfur and 2-4 of nitrogen wherein one carbon in the ring is optionally substituted with a group selected from =O, =S, -SMe, -NH₂, -CF₃, -Cl, -C₁₋₄alkyl optionally substituted with 1-3 of fluoro or a group selected from -NH₂, - OH, -OC₁₋₄alkyl, and -CN;
(d) a 9-membered aromatic or partially unsaturated ortho-fused bicyclic ring system containing 3-4 nitrogen atoms, wherein one carbon in the ring is optionally substituted with a group selected from =O, =S, -SMe, -NH₂, -CF₃, -Cl, pyrrolidinyl, tetrahydrofuranyl, -C₁-₄alkyl optionally substituted with 1-3 of fluoro or a group selected from -NH₂, - OH, -OC₁₋₄alkyl, -CN, and R¹⁰;
(e) -C₁₋₆alkyl, -C₂₋₆alkenyl, and -C₂₋₆alkynyl, said alkyl, alkenyl and alkynyl groups being optionally substituted with R¹² and optionally substituted with R¹³;
(f) -C₃₋₆cycloalkyl optionally substituted with 1-3 substituents selected from the group consisting of fluoro, -NH₂, -OH and -C₁₋₃alkyl optionally substituted with 1-3 of fluoro;
(g) -O-R^{6a}; and
(h) -H, -OH, -CN, -CO₂R⁹, -C(O)NR⁷R⁸, -NR⁷R⁸, -NR^{b}SOₚR^{a}, -NR^{b}C(O)R^{a}, -NR^{b}C(O)NR^{a}R^{b}, -S(O)ₚR^{a}, and -S(O)ₚNR^{a}R^{b};
**p** is an integer selected from 0, 1 and 2;
**R²** is selected from the group consisting of (a) -C₁₋₆alkyl optionally substituted with 1-5 of fluoro or hydroxy, (b) -C₃₋₆cycloalkyl, and (c) 1-methyl-C₃₋₆cycloalkyl;
**R³** is selected from the group consisting of -H, -F, -OH, and -C₁₋₃alkyl optionally substituted with 1-5 fluoro;
**X** is selected from the group consisting of -O-, -S- and -CR^{2a} R^{3a}-;
**R**^{**2**a} is selected from the group consisting of -H, -OH, -OC₁₋₆alkyl optionally substituted with 1-3 of fluoro, -O-benzyl and -fluoro;
**R^{3a}** is selected from the group consisting of -H, fluoro and -C₁₋₆alkyl;
or **CR^{2a}R^{3a}** represents carbonyl;
**R⁴** is selected from the group consisting of -H, -C₁₋₆alkyl optionally substituted with 1-3 of fluoro, and -C₃₋₆cycloalkyl optionally substituted with 1-3 of fluoro;
**R⁵** is selected from the group consisting of -H, fluoro and methyl;
or **R⁴** and **R⁵** taken together with the carbon to which they are attached represents a -C₃₋₆cycloalkyl ring, for example cyclopropyl-1,1-diyl;
**d** is an integer selected from 1 (one) and 0 (zero);
**R⁶** is selected from the group consisting of (a) -C₁₋₆alkyl optionally substituted with one or more substituents selected from the group consisting of -OH, -NH₂, -N(CH₃)₂, -NH(C=O)O^{t}Bu, -CN, -O-C₁₋₄alkyl and fluoro, (b) -C₁₋₆alkyl-R¹⁰, (c) -OC₁₋₆alkyl optionally substituted with one or more substituents selected from the group consisting of -OH, -NH₂ and fluoro, (d) -C₃₋₆ cycloalkyl optionally substituted with one or more substituents selected from the group consisting of methyl, -OH, -NH₂, -NH(C=O)O^{t}Bu, -CF₃ and fluoro, (e) -NR⁷R⁸, (f) -SO₂C₁₋₃alkyl, (g) -(CH₂)₀₋₃CO₂-R⁸, (h) -OH, (i) =O (oxo), (j) -SH, (k) =S, (l) -SMe, (m) -Cl, (n) 1-5 of fluoro, (o) -CF₃, (p) -CN and (q) R¹⁰;
**R^{6a}** is selected from the group consisting of (1) -C₁₋₆alkyl optionally substituted with R¹² and optionally substituted with R¹³, (2) -C₃₋₆cycloalkyl optionally substituted with R¹² and optionally substituted with R¹³ and (3) -C₂₋₆alkyl-R¹⁰;
**R⁷** is selected from the group consisting of (a) -H, (b) -C₁₋₆alkyl optionally substituted with one or more substituents selected from the group consisting of -F, -NH₂ and -OH, (c) -C₃₋₆cycloalkyl optionally substituted with one or more substituents selected from the group consisting of methyl, -CF₃, -F, -NH₂ and -OH, (d) -COC₁₋₆alkyl optionally substituted with one or more substituents selected from the group consisting of -F and -OH, (e) -C(O)OC₁₋₆alkyl optionally substituted with one or more substituents selected from the group consisting of methyl, phenyl, -CF₃, -F and -OH, (f) a 4-6 membered saturated heterocyclic ring containing one N and/or one O, wherein the ring is bonded to the nitrogen in -NR⁷R⁸ through a carbon atom in the ring, and wherein the ring is optionally substituted with one or more substituents selected from the group consisting of methyl, -CF₃, -F, -CH₂CH₂F, -CH2CHF2, -CH₂CF₃, -NH₂ and -OH, and wherein the ring is optionally bridged by a -CH₂CH₂- group, (g) a 5-membered unsaturated heterocyclic ring comprising one, two or three heteroatoms selected from N, O and S, and (h) benzyl;
**R⁸** is selected from the group consisting of (a) -H, (b) -C₁₋₆alkyl optionally substituted with one or more substituents selected from the group consisting of -F, -NH₂ and -OH, and (c) -C₃₋₆cycloalkyl optionally substituted with one or more substituents selected from the group consisting of methyl, -CF₃, -F, -NH₂ and -OH;
or **R⁷** and **R⁸** together represent - (CH₂)3-5- which is bonded with the nitrogen to which **R⁷** and **R⁸** are attached to form a 4-6 membered ring, wherein the ring is optionally substituted with a substituent selected from the group consisting of -CH₃, -CF₃, -F and -OH;
**R⁹** is selected from the group consisting of-H, -C₁₋₆alkyl and -C₃₋₆cycloalkyl;
**R¹⁰** is a heterocyclic ring selected from the group consisting of (a) azetidinyl optionally substituted with one or more of methyl, -F and -OH, (b) pyrrolidinyl optionally substituted with one or more of methyl, -F and -OH, (c) piperidinyl optionally substituted with one or more of methyl, -F and -OH, (d) piperazinyl optionally substituted with =O, and (e) morpholinyl optionally substituted with one or more of methyl and -F;
**Y** is a 6-membered aromatic or partially unsaturated heterocyclic ring containing 1 to 2 N heteroatoms, wherein the heterocyclic ring is optionally substituted with R¹¹;
**R¹¹** is selected from the group consisting of -F, -NH₂, -OH, -OC₃₋₄cycloalkyl, -C₁₋₃alkyl optionally substituted with 1-3 fluoro, and -OC₁₋₃alkyl optionally substituted with phenyl or 1-3 fluoro;
**R¹²** is selected from the group consisting of -CO₂R⁹, -C(O)NR⁷R⁸, -N(R^{a})₂, - NR^{b}SOₚR^{a}, -NR^{b}C(O)R^{a}, -NR^{b}C(O)NR^{a}R^{b}, -S(O)ₚNR^{a}R^{b}, -S(O)ₚR^{a}, -F, -CF₃, phenyl, Het and Z¹;
**R¹³** is selected from the group consisting of -OH, -NH₂ and 1-5 of -F;
each **R^{a}** is independently selected from the group consisting of (a) -H, (b) -C₁₋₆alkyl, -C₂₋₆alkenyl and -C₂₋₆alkynyl, wherein each is optionally substituted with 1-3 of fluoro, and optionally substituted with 1-2 substituents selected from the group consisting of: -OH, - OC₁₋₄alkyl, cyano, -NH₂, -NHC₁₋₄alkyl, and -N(C₁₋₄alkyl)₂, (c) -C₃₋₆cycloalkyl, optionally substituted with 1-3 of fluoro, and optionally substituted with 1-2 substituents selected from the group consisting of: -C₁₋₄alkyl, -OH, -OC₁₋₄alkyl, -CN, -NH₂, -NHC₁₋₄alkyl, -N(C₁₋₄alkyl)₂, and -CF₃, (d) Het and Het-C₁₋₄alkylene-, the Het moieties being optionally substituted on carbon with 1-2 substituents selected from the group consisting of -F, -OH, -CO₂H, -C₁₋₄alkyl, - CO₂C₁₋₄alkyl, -OC₁₋₄alkyl, -NH2, -NHC₁₋₄alkyl, -N(C₁₋₄alkyl)₂, -NHC(O)C₁₋₄alkyl, oxo, - C(O)NHC₁₋₄alkyl and -C(O)N(C₁₋₄alkyl)₂; and optionally substituted on nitrogen when present with a group selected from -C₁₋₄alkyl and -C₁₋₄acyl; and the alkylene portion of Het-C₁₋₄alkylene- being optionally substituted with 1-3 of fluoro and optionally substituted with a member selected from the group consisting of -OH, -CN, -OC₁₋₄alkyl, -NH₂, -NHC₁₋₄alkyl, and -N(C₁₋₄alkyl)₂, (e) Z² and Z²-C₁₋₄alkylene-, the alkylene portion of Z²-C₁₋₄alkylene- being optionally substituted with 1-3 of fluoro and optionally substituted with a member selected from the group consisting of -OH, -CN, -OC₁₋₄alkyl, -NH₂, -NHC₁₋₄alkyl, and -N(C₁₋₄alkyl)₂;
each **R^{b}** is independently selected from the group consisting of -H and -C₁₋₃alkyl optionally substituted with 1-2 members selected from the group consisting of -NH₂, -OH, -F, -CN and -CF3;
each **R^{c}** is independently selected from the group consisting of -H, -F, -Cl, -OH, -CN, -C₁₋₄alkyl optionally substituted with 1-3 of fluoro, and -OC₁₋₄alkyl optionally substituted with 1-3 of fluoro;
**Het** is selected from the group consisting of azetidinyl, pyrrolidinyl, piperidinyl, piperazinyl, morpholinyl, tetraydrofuranyl and β-lactamyl, δ- lactamyl, γ-lactamyl and tetrahydropyranyl;
**Z¹** is selected from the group consisting of: (a) Z², (b) an 8-membered aromatic or partially unsaturated ortho-fused bicyclic ring system containing 3-5 heteroatoms selected from one sulfur and 2-4 of nitrogen wherein one carbon in the ring is optionally substituted with a group selected from =O, =S, -SMe, -NH₂, -CF₃, -Cl, -C₁₋₄alkyl optionally substituted with 1-3 of fluoro and optionally substituted with a group selected from -NH₂, -OH, -OC₁₋₄alkyl, and - CN, and (c) a 9-membered aromatic or partially unsaturated *ortho*-fused bicyclic ring system containing 3-4 nitrogen atoms, wherein one carbon in the ring is optionally substituted with a group selected from =O, =S, -SMe, -NH₂, -CF₃, -Cl, -C₁₋₄alkyl optionally substituted with 1-3 of fluor and optionally substituted with a group selected from -NH₂, -OH, -OC₁₋₄alkyl, and -CN;
and **Z²** is selected from the group consisting of: (a) a 5-membered aromatic or partially unsaturated heterocyclic ring containing 2-4 nitrogen atoms, wherein one nitrogen in the ring is optionally substituted with a group selected from -C₁₋₄alkyl optionally substituted with 1-3 of fluoro and optionally substituted with a group selected from -NH₂, -OH, and -CN, and one carbon in the ring is optionally substituted with a group selected from =O, =S, -SMe, -NH₂, - CF₃, -Cl, -C₁₋₄alkyl optionally substituted with 1-3 of fluoro and optionally substituted with a group selected from -NH₂, -OH, -OC₁₋₄alkyl, and -CN, and -OC₁₋₄alkyl optionally substituted with -OH or 1-3 of fluoro; (b) a 5-membered aromatic or partially unsaturated heterocyclic ring containing 2-3 heteroatoms selected from one oxygen or one sulfur and 1-2 of nitrogen, wherein one nitrogen in the ring is optionally substituted with a group selected from C₁₋₄alkyl optionally substituted with 1-3 of fluoro and optionally substituted with a group selected from -NH₂, -OH, and -CN, and one carbon in the ring is optionally substituted with a group selected from =O, =S, - SMe, -NH2, -CF3, -Cl, C₁₋₄alkyl optionally substituted with 1-3 of fluoro and optionally substituted with a group selected from -NH₂, -OH, -OC₁₋₄alkyl, and -CN, and -OC₁₋₄alkyl optionally substituted with -OH or 1-3 of fluoro; and (c) a 6-membered aromatic or partially unsaturated heterocyclic ring containing 1-2 nitrogen atoms, wherein one nitrogen in the ring is optionally substituted with a group selected from -C₁₋₄alkyl optionally substituted with 1-3 of fluoro and optionally substituted with a group selected from -NH₂, -OH, and -CN, and one carbon in the ring is optionally substituted with a group selected from =O, =S, -SMe, -NH₂, - CF₃, -Cl, -C₁₋₄alkyl optionally substituted with 1-3 of fluoro and optionally substituted with a group selected from -NH₂, -OH, -OC₁₋₄alkyl, and -CN, and -OC₁₋₄alkyl optionally substituted with-OH or 1-3 offluoro; provided that when -(X-CR⁴R⁵)_{d}-Y is 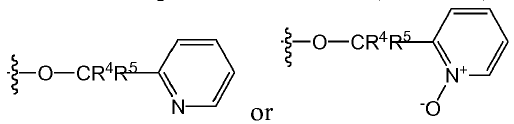 and R⁴ is -H or -C₁₋₄alkyl and R⁵ is -H or methyl, then R¹ is not -CO₂R⁹, OH, CN, 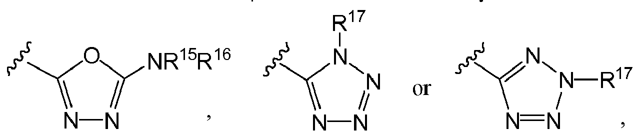 wherein
**R¹⁵** is selected from the group consisting of -H, -C₁₋₆alkyl optionally substituted with 1 to 3 fluoro, -C₃₋₆cycloalkyl optionally substituted with 1-3 fluoro, -COC₁₋₆alkyl and -COC₃₋₆cycloalkyl;
**R¹⁶** is selected from the group consisting of -H, -C₁₋₆alkyl optionally substituted with 1 to 3 fluoro, and -C₃₋₆cycloalkyl optionally substituted with 1 to 3 fluoro;
**R¹⁷** is selected from the group consisting of -H, -C₁₋₆alkyl optionally substituted with 1 to 3 fluoro, -C₃₋₆cycloalkyl optionally substituted with 1 to 3 fluoro and -CH₂-R¹⁸;
**R¹⁸** is selected from the group consisting of pyrrolidinyl optionally substituted on nitrogen with methyl, piperidinyl optionally substituted on nitrogen with methyl, and morpholinyl optionally substituted on nitrogen with methyl.

2. The compound of claim 1 wherein Y is a 6-membered heterocyclic ring, containing 1 to 2 N heteroatoms, wherein the heterocyclic ring is optionally substituted with R¹¹.

3. The compound of claim 2 having Formula Ia 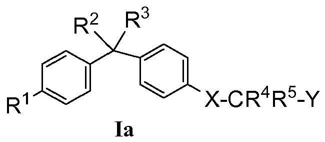 wherein Y is selected from the group consisting of pyridinyl, pyridazinyl and pyrimidinyl, and Y is optionally substituted with -F, -OCH₃ and -NH₂.

4. The compound of claim 3 wherein X is selected from the group consisting of -O- and -CR^{2a}R^{3a}.

5. The compound of claim 4 wherein -CR^{2a}R^{3a} is selected from the goup consisting of -CH₂-, -CHF-, -CH(OH)-, -C(O)-, -CH(OC₁₋₃alkyl)-, -CH(O-benzyl)- and - CH(OCF₃)-, and -CR⁴R⁵- is selected from the group consisting of -CH₂-, -CH(CH₃)- and - CH(CH₂CH₃)-.

6. The compound of claim 4 wherein X is O, and -CR4R5- is selected from the group consisiting of -CH₂-, -CH(CH₃)- and -CH(CH₂CH₃)-.

7. The compound of claim 1 wherein R² is selected from the group consisting of (a) -C₁₋₄alkyl optionally substituted with one to three fluorine atoms, (b) -C₃₋₆cycloalkyl and (c) 1-methyl-C₃₋₄cycloalkyl, and R³ is selected from the group consisting of -H, -C₁₋₃alkyl and - OH.

8. The compound of claim 6 wherein (a) R³ is methyl and R² is ⁱpropyl or (b) R3 is -H and R² is tbutyl.

9. The compound of claim 2 having Formula Ic: 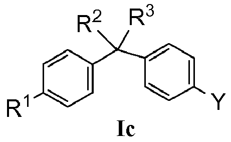 wherein Y is a 6-membered heterocyclic ring containing 1 to 2 N heteroatoms, wherein the heterocyclic ring is substituted with R¹¹.

10. The compound of claim 9 wherein R² is selected from the group consisting of (a) -C₁₋₄alkyl optionally substituted with one to three fluorine atoms, (b) -C₃₋₆cycloalkyl and (c) 1-methyl-C₃₋₄cycloalkyl, and R³ is selected from the group consisting of -H, -C₁₋₃alkyl and-OH.

11. The compound of claim 10 wherein (a) R³ is methyl and R² is ⁱpropyl or (b) R³ is -H and R² is tbutyl.

12. The compound of claim 1 selected from the group consisting of:
4- {[5-(4-{1,2-dimethyl-1-[4-(pyridin-2-ylmethoxy)phenyl]propyl}phenyl)isoxazol-3-yl]-methyl}morpholine;
1- {[5-(4-{1,2-dimethyl-1-[4-(pyridin-2-ylmethoxy)phenyl]propyl}phenyl)isoxazol-3-yl]-methyl}azetidin-3-ol;
3-(4-{1,2-dimethyl-1-[4-(pyridin-2-ylmethoxy)phenyl]propyl}phenyl)-6-(methylsulfonyl)-pyridazine;
3-(4-{1,2-dimethyl-1-[4-(pyridin-2-ylmethoxy)phenyl]propyl}phenyl)-6-methylpyridazine;
3-(4-{1,2-dimethyl-1-[4-(pyridin-2-ylmethoxy)phenyl]propyl}phenyl)-6-(trifluoromethyl)-pyridazine;
2-[6-(4-{1,2-dimethyl-1-[4-(pyridin-2-ylmethoxy)phenyl]propyl}phenyl)pyridazin-3-yl]propan-2-ol;
2-(4-{1-[4-(6-aminopyridazin-3-yl)phenyl]-1,2-dimethylpropyl}phenyl)propan-2-ol;
2-(4-{1-[4-(2-aminopyrimidin-5-yl)phenyl]-1,2-dimethylpropyl}phenyl)propan-2-ol;
2-[6-(4-{1-[4-(5-methoxypyridin-3-yl)phenyl]-1,2-dimethylpropyl}phenyl)pyridazin-3-yl]propan-2-ol;
2-[6-(4-{11-[4-(6-aminopyridazin-3-yl)phenyl]-1,2-dimethylpropyl}phenyl)pyridazin-3-yl]propan-2-ol;
2-[6-(4-{1-[4-(2-aminopyrimidin-5-yl)phenyl]-1,2-dimethylpropyl}phenyl)pyridazin-3-yl]propan-2-ol;
and the pharmaceutically acceptable salts thereof.

13. A pharmaceutical composition comprised of a therapeutically effective amount of a compound of any preceding claim or a pharmaceutically acceptable salt thereof and a pharmaceutically acceptable carrier.

14. Use of a compound of Formula I, as defined in any one of Claims 1 to 12, or a pharmaceutically acceptable salt thereof, in the manufacture of a medicament for treating atherosclerosis; preventing or reducing the risk of an atherosclerotic disease event in a patient at risk for having an atherosclerotic disease event; the prophylaxis or treatment of asthma; treating allergic rhinitis; or treating COPD in a patient in need of such treatment.

15. A compound of Formula I, as defined in any one of Claims 1 to 12, or a pharmaceutically acceptable salt thereof, for use in a method of treatment of the human body by therapy.

16. A compound for use according to Claim 15 wherein the use is in the manufacture of a medicament for treating atherosclerosis; preventing or reducing the risk of an atherosclerotic disease event in a patient at risk for having an atherosclerotic disease event; the prophylaxis or treatment of asthma; treating allergic rhinitis; or treating COPD in a patient in need of such treatment.

17. A combination of a compound of Formula I, as defined in any one of Claims 1 to 12, or a pharmaceutically acceptable salt thereof, and at least one additional therapeucitally active agent.
